(19) Europäisches Patentamt
European Patent Office
Office européen des brevets

(11) **EP 4 015 520 B1**

(12) # EUROPEAN PATENT SPECIFICATION

(45) Date of publication and mention of the grant of the patent:
**19.02.2025 Bulletin 2025/08**

(21) Application number: **20895574.0**

(22) Date of filing: **21.10.2020**

(51) International Patent Classification (IPC):
***C07D 491/052*** *(2006.01)* ***A61K 31/519*** *(2006.01)*
***A61P 35/00*** *(2006.01)* ***C07D 519/00*** *(2006.01)*

(52) Cooperative Patent Classification (CPC):
**A61P 35/00; A61K 31/519; C07D 491/052; C07D 519/00**

(86) International application number:
**PCT/CN2020/122335**

(87) International publication number:
**WO 2021/109737 (10.06.2021 Gazette 2021/23)**

(54) **OXYGEN-CONTAINING HETEROCYCLIC COMPOUND, PREPARATION METHOD AND APPLICATION THEREOF**

SAUERSTOFFHALTIGE HETEROCYCLISCHE VERBINDUNG, VERFAHREN ZU IHRER HERSTELLUNG UND IHRE VERWENDUNG

COMPOSÉ HÉTÉROCYCLIQUE CONTENANT DE L'OXYGÈNE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2019 CN 201911212840**
**29.04.2020 CN 202010368798**
**10.10.2020 CN 202011077052**

(43) Date of publication of application:
**22.06.2022 Bulletin 2022/25**

(73) Proprietor: **280 Bio, Inc.**
**San Francisco, CA 94080 (US)**

(72) Inventors:
- **XU, Zusheng**
  **Shanghai 201203 (CN)**
- **LOU, Yangtong**
  **Shanghai 201203 (CN)**
- **XIE, Tiegang**
  **Shanghai 201203 (CN)**

(74) Representative: **Barker Brettell LLP**
**100 Hagley Road**
**Edgbaston**
**Birmingham B16 8QQ (GB)**

(56) References cited:
**EP-A1- 4 105 211**
**WO-A1-2010/064705**
**WO-A1-2019/099524**
**CN-A- 102 480 961**
**CN-A- 108 473 504**
**US-A1- 2019 248 767**
**WO-A1-2010/064705**
**WO-A1-2010/064705**
**WO-A1-2021/107160**
**CN-A- 108 473 504**
**CN-A- 109 843 856**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present disclosure relates to an oxygen-containing heterocyclic compound, a preparation method therefor and use thereof.

## Background

Ras (Rat sarcoma viral oncogene) was first identified in rat sarcoma. In mammals, the ras gene family is composed of three members: H-ras, K-ras and N-ras, and the fourth exon of K-ras has two variants: A and B. Ras genes are widely present in various eukaryotes, such as mammals, Drosophila, fungi, nematodes and yeast, and are expressed at varying degrees in different tissues. H-Ras is mainly expressed in skin and skeletal muscle; K-Ras is mainly expressed in colon and thymus; and N-Ras is highly expressed in testis. Ras protein acts as a molecular switch in signal transduction of cells and regulates signal transduction by binding GTP/GDP and exchanging between them, thereby regulating life processes such as cell proliferation, differentiation, aging and apoptosis.

Ras mutation is closely related to the occurrence and development of tumors. Ras gene is mutated in at least 30% of human tumors and is considered to be one of the most powerful drivers of cancer. Mutations in the Ras proto-oncogenes are mainly point mutations. More than 150 different Ras point mutations have been found, most of which are mutations at positions 12 and 13 of glycine and position 61 of glutamine.

For decades, efforts have been made to research and develop a small molecule inhibitor targeting Ras, but so far, no related drugs have been marketed. Scientists have always wanted to develop a competitive inhibitor of GTP that directly acts on Ras proteins, but without success, due to strong affinity between GTP and Ras (pmol/L level), high concentration of GTP in cells (0.5 mM), lack of pockets that are conducive to the binding of small molecules in the RAS protein structure, etc. In recent years, people have made some progress in drug research and development using allosteric sites of the K-Ras G12C mutant. In 2013, a research team reported the discovery of K-Ras G12C small molecule inhibitors (Nature, 2013, 503, 548-551) and they identified a new binding pocket, i.e., an allosteric pocket, located below molecular switch II region from the K-Ras G12C mutant, wherein these inhibitors bind to the allosteric pocket and form a covalent bond with nearby Cys12, thereby selectively inhibiting the activation of K-Ras G12C. Other researchers have reported KRAS inhibitors with cellular activity (Science, 2016, 351, 604-608). Currently, there are several drugs in clinical development. In 2018, Amgen began clinical trials on compound AMG510, which is the first small molecule inhibitor that directly targets KRAS to enter the clinic.

In summary, after decades of unremitting efforts, people have gradually deepened their understanding of Ras, but no medicinal compounds have been successfully developed so far. Finding compounds with better inhibitory effects on Ras is still a research hotspot and difficulty in the field of new drug development.

WO2019/099524A1 and CN109843856A respectively relates to compounds that inhibit KRas G12C, which relates to compounds that irreversibly inhibit the activity of KRas G12C, pharmaceutical compositions comprising the compounds and methods of use therefor. US2019/0248767A1 relates to tetrahydroquinazoline derivatives useful as anticancer agents. WO2019/099524A1, CN109843856A and US2019/0248767A1 all disclose pyrimidines fused to a partially saturated ring as KRAS inhibitors for the treatment of cancer.

## Content of the present invention

The technical problem to be solved in the present disclosure is that there is no effective drug in the prior art as an Ras inhibitor for clinical treatment, and therefore, the present disclosure provides an oxygen-containing heterocyclic compound, a preparation method therefore and use thereof, wherein the oxygen-containing heterocyclic compound is expected to treat and/or prevent various Ras-mediated diseases.

The present disclosure solves the above-mentioned technical problem through the following technical solutions.

The present disclosure provides an oxygen-containing heterocyclic compound represented by formula I, a pharmaceutically acceptable salt thereof, a stereoisomer thereof, a tautomer thereof or an isotopic compound thereof,

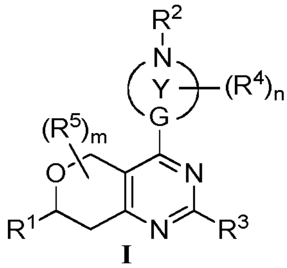

I

wherein $R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, -C(=O)$R^{65}$, -N$R^{63}R^{64}$, -C(-O)O$R^{66}$, -C(=O)N$R^{69}R^{610}$, $C_{1-6}$ alkyl, $C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;

$R^{1-6-1}$, $R^{1-6-2}$, $R^{1-6-3}$, $R^{1-6-4}$, $R^{1-6-5}$ and $R^{1-6-6}$ are independently cyano, halogen, hydroxyl, $C_{1-6}$ alkoxy, $C_{1-6}$ alkyl, -C(=O)$R^{65-2}$, -N$R^{63-2}R^{64-2}$, -C(=O)O$R^{66-2}$, or -C(=O)N$R^{69-2}R^{610-2}$;

$R^{65}$, $R^{65-2}$, $R^{63}$, $R^{63-2}$, $R^{64}$, $R^{64-2}$ , $R^{66}$, $R^{66-2}$, $R^{69}$, $R^{69-2}$, $R^{610}$ and $R^{610-2}$ are independently hydrogen or $C_{1-6}$ alkyl;

m is 0, 1 or 2;

$R^5$ is independently $C_{1-6}$ alkyl;

$R^3$ is -O$R^{31}$, -S$R^{32}$ or -N$R^{33}R^{34}$;

$R^{31}$, $R^{32}$ and $R^{34}$ are independently $C_{1-6}$ alkyl substituted with one or more $R^{31-1}$, $R^{33}$ is independently H, $C_{1-6}$ alkyl, or $C_{1-6}$ alkyl substituted with one or more $R^{31-1}$;

$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, -O$R^d$, -S$R^{d1}$, -N$R^{e1}R^{e2}$, or -C(=O)N$R^{e3}R^{e4}$;

$R^{d15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, -N$R^{e5}R^{e6}$ or -C(=O)N$R^{e7}R^{e8}$;

$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;

$R^{1-8-1}$ and $R^{1-8-2}$ are independently cyano, halogen, hydroxyl, $C_{1-6}$ alkoxy, -C(=O)$R^{e9}$, -N$R^{e10}R^{e11}$, -C(=O)O$R^{e12}$, or -C(=O)N$R^{e13}R^{e14}$;

$R^{e5}$, $R^{e6}$, $R^{e7}$, $R^{e8}$, $R^{e9}$, $R^{e10}$, $R^{e11}$, $R^{e12}$, $R^{e13}$ and $R^{e14}$ are independently hydrogen or $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;

G is N, C or CH;

n is 0, 1, 2 or 3;

$R^4$ is independently $C_{1-6}$ alkyl, $C_{1-6}$ alkyl substituted with one or more $R^{4-1}$, oxo, $-C(=O)OR^{4a}$ or $-C(=O)NR^{4b}R^{4c}$;

$R^{4-1}$ is independently halogen, cyano, hydroxyl, $C_{1-6}$ alkoxy, $-NR^{4i}R^{4j}$, $-C(=O)OR^{4d}$ or $-C(=O)NR^{4e}R^{4f}$; $R^{4a}$, $R^{4e}$, $R^{4f}$, $R^{4i}$ and $R^{4j}$ are independently hydrogen or $C_{1-6}$ alkyl;

$R^{4a}$, $R^{4b}$ and $R^{4c}$ are independently hydrogen or $C_{1-6}$ alkyl;

$R^2$ is CN, $-C(=O)-C(R^a)=C(R^b)(R^f)$, $-C(=O)-C{\equiv}CR^f$, $-S(=O)_2-C(R^a)=C(R^b)(R^f)$ or $-S(=O)_2-C{\equiv}CR^f$;

$R^a$ is independently hydrogen, deuterium, halogen or $C_{1-6}$ alkyl;

$R^b$ and $R^f$ are independently hydrogen, deuterium, $C_{1-6}$ alkyl, $C_{1-6}$ alkyl-C(=O)-, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment, with regard to an oxygen-containing heterocyclic compound represented by formula I, a pharmaceutically acceptable salt thereof, a stereoisomer thereof, a tautomer thereof or an isotopic compound thereof, some groups are as defined as follows, and the unmentioned group definitions are as described in any one of the embodiments of the present disclosure (this content is hereinafter referred to simply as "in a certain embodiment"). With regard to an oxygen-containing heterocyclic compound represented by formula I,

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $-C(=O)R^{65}$, $-NR^{63}R^{64}$, $-C(=O)OR^{66}$, $-C(=O)NR^{69}R^{610}$, $C_{1-6}$ alkyl, $C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;

$R^{1-6-1}$, $R^{1-6-2}$, $R^{1-6-3}$, $R^{1-6-4}$, $R^{1-6-5}$ and $R^{1-6-6}$ are independently cyano, halogen, hydroxyl, $C_{1-6}$ alkoxy, $C_{1-6}$ alkyl, $-C(=O)R^{65-2}$, $-NR^{63-2}R^{64-2}$, $-C(=O)OR^{66-2}$, or $-C(=O)NR^{69-2}R^{610-2}$;

$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ and $R^{610-2}$ are independently hydrogen or $C_{1-6}$ alkyl;

m is 0, 1 or 2;

$R^5$ is independently $C_{1-6}$ alkyl;

$R^3$ is $-OR^{31}$, $-SR^{32}$ or $-NR^{33}R^{34}$;

$R^{31}$, $R^{32}$, $R^{33}$ and $R^{34}$ are independently $C_{1-6}$ alkyl substituted with one or more $R^{31-1}$;

$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, $-OR^d$, $-SR^{d1}$, $-NR^{e1}R^{e2}$, or $-C(=O)NR^{e3}R^{e4}$;

$R^{d15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;

$R^{1-8-1}$ and $R^{1-8-2}$ are independently cyano, halogen, hydroxyl, $C_{1-6}$ alkoxy, $-C(=O)R^{e9}$, $-NR^{e10}R^{e11}$, $-C(=O)OR^{e12}$, or $-C(=O)NR^{e13}R^{e14}$;

$R^{e5}$, $R^{e6}$, $R^{e7}$, $R^{e8}$, $R^{e9}$, $R^{e10}$, $R^{e11}$, $Re^{12}$, $R^{e13}$ and $R^{e14}$ are independently hydrogen or $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;

G is N, C or CH;

n is 0, 1, 2 or 3;

$R^4$ is independently $C_{1-6}$ alkyl, $C_{1-6}$ alkyl substituted with one or more $R^{4-1}$, oxo, $-C(=O)OR^{4a}$ or $-C(=O)NR^{4b}R^{4c}$;

$R^{4-1}$ is independently halogen, cyano, hydroxyl, $C_{1-6}$ alkoxy, $-NR^{4i}R^{4j}$, $-C(=O)OR^{4d}$ or $-C(=O)NR^{4e}R^{4f}$; $R^{4a}$, $R^{4e}$, $R^{4f}$, $R^{4i}$ and $R^{4j}$ are independently hydrogen or $C_{1-6}$ alkyl;

$R^{4a}$, $R^{4b}$ and $R^{4c}$ are independently hydrogen or $C_{1-6}$ alkyl;

$R^2$ is $-C(=O)-C(R^a)=C(R^b)(R^f)$, $-C(=O)-C{\equiv}CR^f$, $-S(=O)_2-C(R^a)=C(R^b)(R^f)$ or $-S(=O)_2-C{\equiv}CR^f$:

$R^a$ is independently hydrogen, deuterium, halogen or $C_{1-6}$ alkyl;

$R^b$ and $R^f$ are independently hydrogen, deuterium, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen.

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; for example, $C_{6-20}$ aryl, "5-12 membered heteroaryl containing 1-4 heteroatoms selected from one or more of O, S and N", or $C_{6-20}$ aryl substituted with one or more $R^{1-6}$;

$R^{1-6}$ and $R^{1-7}$ are independently halogen, $C_{1-6}$ alkyl, or $C_{1-6}$ alkyl substituted with one or more $R^{1-6-1}$;

$R^{1-6-1}$ is independently halogen.

In a certain embodiment:

$R^1$ is $C_{6-20}$ aryl, or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$,

$R^{1-6-1}$ is independently halogen.

In a certain embodiment:
m is 0.
In a certain embodiment:

$R^3$ is $-OR^{31}$, $-SR^{32}$ or $-NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl.

In a certain embodiment:

$R^3$ is $-SR^{32}$;

$R^{32}$ is $C_{1-6}$ alkyl substituted with one or more $R^{31-1}$;

$R^{31-1}$ is independently $-NR^{e1}R^{e2}$;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl.

In a certain embodiment:

$R^3$ is $-NR^{33}R^{34}$;

$R^{34}$ is independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{e1}$, $R^{e2}$ and $R^{d15}$ are independently $C_{1-6}$ alkyl.

In a certain embodiment:

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring; for example, a monocyclic ring;

G is N, C or CH.

In a certain embodiment:

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl, cyano, or -C(=O)$NR^{4e}R^{4f}$; $R^{4e}$ and $R^{4f}$ are independently hydrogen or $C_{1-6}$ alkyl; for example, hydrogen.

In a certain embodiment:

n is 0 or 1;

$R^4$ is independently $C_{1-6}$ alkyl substituted with one or more $R^{4-1}$;

$R^{4-1}$ is independently -C(=O)$NR^{4e}R^{4f}$; $R^{4e}$ and $R^{4f}$ are independently hydrogen or $C_{1-6}$ alkyl; for example, hydrogen.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl or cyano.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently cyano.

In a certain embodiment:

$R^2$ is CN, -C(=O)-C($R^a$)=C($R^b$)($R^f$), -C(=O)-C=C-Me or, -S$(=O)_2$-C($R^a$)=C($R^b$)($R^f$);

$R^a$ is independently hydrogen or halogen;

$R^b$ and $R^f$ are independently hydrogen, $C_{1-6}$ alkyl, $C_{1-6}$ alkyl-C(=O)-, or $C_{1-6}$ alkyl substituted with one or more $R^{b-1}$;

$R^{b-1}$ is independently -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^2$ is CN or -C(=O)-C($R^a$)=C($R^b$)($R^f$);

$R^a$ is independently hydrogen;

$R^b$ and $R^f$ are independently hydrogen, $C_{1-6}$ alkyl-C(=O)-.

In a certain embodiment:

$R^2$ is -C(=O)-C($R^a$)=C($R^b$)($R^f$), -C(=O)-C=C-Me or -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, or $C_{1-6}$ alkyl substituted with one or more $R^{b-1}$,

$R^{b-1}$ is independently halogen, hydroxyl, $C_{1-6}$ alkoxy, or -N$R^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^2$ is -C(=O)-C($R^a$)=C($R^b$)($R^f$), -C(=O)-C=C-Me or -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, or $C_{1-6}$ alkyl substituted with one or more $R^{b-1}$,

$R^{b-1}$ is independently -N$R^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, $C_{1-6}$ alkyl, $C_{1-6}$ alkyl substituted with one or more $R^{1-6-1}$; $R^{1-6-1}$ is independently halogen;

m is 0;

$R^3$ is -O$R^{31}$, -S$R^{32}$ or -N$R^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or -N$R^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano, hydroxyl or $-C(=O)NR^{4e}R^{4f}$; $R^{4e}$ and $R^{4f}$ are independently hydrogen or $C_{1-6}$ alkyl;

$R^2$ is CN, $-C(=O)-C(R^a)=C(R^b)(R^f)$, -C(=O)-C=C-Me or, $-S(=O)_2-C(R^a)=C(R^b)(R^f)$;

$R^a$ is independently hydrogen or halogen;

$R^b$ and $R^f$ are independently hydrogen, $C_{1-6}$ alkyl, $C_{1-6}$ alkyl-C(=O)-, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano or hydroxyl;

$R^2$ is $-C(=O)-C(R^a)=C(R^b)(R^f)$, -C(=O)-C=C-Me or $-S(=O)_2-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:
$R^2$ is $-C(=O)-C(R^a)=C(R^b)(R^f)$; $R^a$ is independently hydrogen or halogen; $R^b$ and $R^f$ are independently hydrogen or $C_{1-6}$ alkyl.

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano;

$R^2$ is $-C(=O)-C(R^a)=C(R^b)(R^f)$, $-C(=O)-C≡C-Me$ or $-S(=O)_2-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl substituted with one or more $R^{b-1}$,

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; for example, $C_{6-20}$ aryl, "5-12 membered heteroaryl containing 1-4 heteroatoms selected from one or more of O, S and N", or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$;

$R^{1-6-1}$ is independently halogen;

m is 0;

$R^3$ is $-OR^{31}$, $-SR^{32}$ or $-NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N;

n is 0 or 1;

$R^4$ is independently $C_{1-6}$ alkyl substituted with one or more $R^{4-1}$;

$R^{4-1}$ is independently cyano or $-C(=O)NR^{4e}R^{4f}$; $R^{4e}$ and $R^{4f}$ are independently hydrogen or $C_{1-6}$ alkyl;

$R^2$ is CN, $-C(=O)-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl-C(=O)-.

In a certain embodiment:

$R^1$ is $C_{6-20}$ aryl, or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$;

$R^{1-6-1}$ is independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N;

n is 0 or 1;

$R^4$ is independently $C_{1-6}$ alkyl, or $C_{1-6}$ alkyl substituted with one or more $R^{4-1}$;

$R^{4-1}$ is independently cyano;

$R^2$ is -C(=O)-C($R^a$)=C($R^b$)($R^f$);

$R^a$ is independently hydrogen or halogen;

$R^b$ and $R^f$ are independently hydrogen or $C_{1-6}$ alkyl, or $C_{1-6}$ alkyl substituted with one or more $R^{b-1}$;

$R^{b-1}$ is independently -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment:

$R^1$ is $C_{6-20}$ aryl, or $C_{6-20}$ aryl substituted with one or more $R^{1-6}$;

$R^{1-6}$ is independently halogen, $C_{1-6}$ alkyl, or $C_{1-6}$ alkyl substituted with one or more R1’6’1;

$R^{1-6-1}$ is independently halogen;

m is 0;

$R^3$ is -$OR^{31}$ or -$NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or -$NR^{e1}R^{e2}$;

$R^{e1}$, $R^{e2}$ and $R^{d15}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N;

n is 0 or 1;

$R^4$ is independently $C_{1-6}$ alkyl, or $C_{1-6}$ alkyl substituted with one or more $R^{4-1}$;

$R^{4-1}$ is independently cyano;

$R^2$ is -C(=O)-C($R^a$)=C($R^b$)($R^f$);

$R^a$ is independently hydrogen or halogen;

$R^b$ and $R^f$ are independently hydrogen or $C_{1-6}$ alkyl.

In a certain embodiment:
the oxygen-containing heterocyclic compound represented by formula I has a structure as follows:

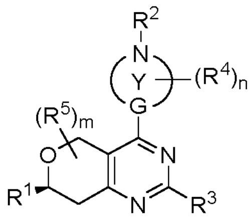
I-2

,

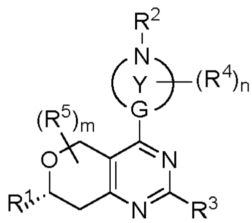
I-3

or "a mixture of

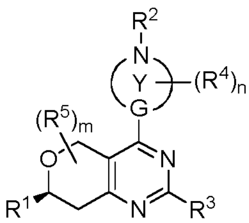
I-2

and

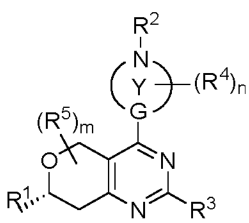
I-3

, with a molar ratio of, for example, 1:1".

In a certain embodiment: the oxygen-containing heterocyclic compound represented by formula I has a structure as follows:

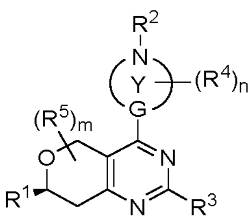
I-2

.

In a certain embodiment:
when $R^1$ is $C_{6-20}$ aryl, then the $C_{6-20}$ aryl may be phenyl or naphthyl, or may be phenyl or 1-naphthyl.

In a certain embodiment:
when $R^1$ is "5-12 membered heteroaryl containing 1-4 heteroatoms selected from one or more of O, S and N", then the "5-12 membered heteroaryl containing 1-4 heteroatoms selected from one or more of O, S and N" may be "9-10 membered heteroaryl containing 1-4 heteroatoms selected from one or more of O, S and N", or may be "9-10 membered heteroaryl containing 1 heteroatom selected from one of O, S and N", or may be isoquinolyl, or may be

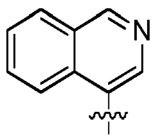

.

In a certain embodiment:
when $R^1$ is $C_{6-20}$ aryl substituted with one or more $R^{1-6}$, then the $C_{6-20}$ aryl may be phenyl or naphthyl, or may be phenyl or 1-naphthyl.

In a certain embodiment:

when $R^1$ is $C_{6-20}$ aryl substituted with one or more $R^{1-6}$, then the more $R^{1-6}$ may be two or three $R^{1-6}$.

In a certain embodiment:
when $R^{1-6}$ is independently halogen, then the halogen may be fluorine, chlorine, bromine or iodine, or may be fluorine or chlorine.

In a certain embodiment:
when $R^{1-6}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{1-6}$ is independently $C_{1-6}$ alkyl substituted with one or more $R^{1-6-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{1-6}$ is independently $C_{1-6}$ alkyl substituted with one or more $R^{1-6-1}$, then the more $R^{1-6-1}$ may be two or three $R^{1-6-1}$.

In a certain embodiment:
when $R^{1-6-1}$ is independently halogen, then the halogen may be fluorine, chlorine, bromine or iodine, or may be fluorine.

In a certain embodiment:
when $R^{1-6}$ is independently $C_{1-6}$ alkyl substituted with one or more $R^{1-6-1}$, then the $C_{1-6}$ alkyl substituted with one or more $R^{1-6-1}$ may be trifluoromethyl.

In a certain embodiment:
when $R^1$ is $C_{6-20}$ aryl substituted with one or more $R^{1-6}$, then the $C_{6-20}$ aryl substituted with one or more $R^{1-6}$ is

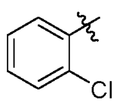, 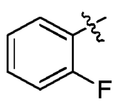, 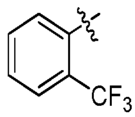, 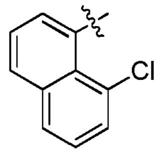 or 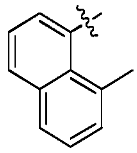.

In a certain embodiment:
when $R^1$ is $C_{6-20}$ aryl substituted with one or more $R^{1-6}$, then the $C_{6-20}$ aryl substituted with one or more $R^{1-6}$ is

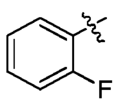, 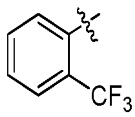, 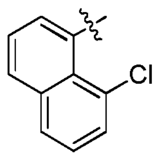 or 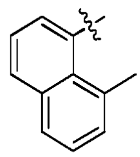.

In a certain embodiment:
when $R^{33}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl, ethyl, n-propyl or isopropyl.

In a certain embodiment:
when $R^{31}$, $R^{33}$ and $R^{34}$ are independently $C_{1-6}$ alkyl substituted with one or more $R^{31-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl, ethyl, n-propyl or isopropyl.

In a certain embodiment:
when $R^{31}$, $R^{33}$ and $R^{34}$ are independently $C_{1-6}$ alkyl substituted with one or more $R^{31-1}$, then the more $R^{31-1}$ may be two or three $R^{31-1}$.

In a certain embodiment:
when $R^{31-1}$ is independently "4-10 membered heterocycloalkyl containing 1-3 heteroatoms selected from one or more of O and N" substituted with one or more $R^{d15}$, then the "4-10 membered heterocycloalkyl containing 1-3 heteroatoms selected from one or more of O and N" may be "5-7 membered heterocycloalkyl containing 1-3 heteroatoms selected from one or more of O and N", or may be "5-7 membered heterocycloalkyl containing 1 heteroatom selected from one of O and N", or may be tetrahydropyrrolyl, or more particularly, tetrahydropyrrole-2-yl.

In a certain embodiment:
when $R^{31-1}$ is independently "4-10 membered heterocycloalkyl containing 1-3 heteroatoms selected from one or more of O and N" substituted with one or more $R^{d15}$, then the more $R^{d15}$ may be two or three $R^{d15}$.

In a certain embodiment:
when $R^{d15}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{d15}$ is independently halogen, then the halogen may be fluorine, chlorine, bromine or iodine, or may be fluorine.
In a certain embodiment:
when $R^{e1}$ and $R^{e2}$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl or ethyl.
In a certain embodiment:
when $R^{31}$, $R^{33}$ and $R^{34}$ are independently $C_{1-6}$ alkyl substituted with one or more $R^{31-1}$, then the $C_{1-6}$ alkyl substituted with one or more $R^{31-1}$ may be

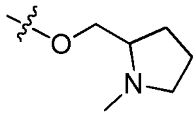,

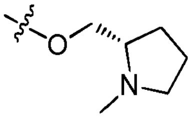, 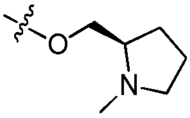, 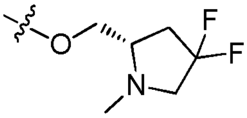, 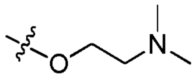, 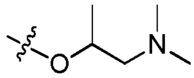,

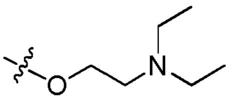, 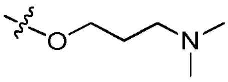 or 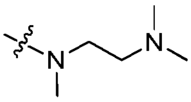.

In a certain embodiment:
when ring Y is a 4-12 membered heterocyclic ring containing 1-4 N atoms, then the 4-12 membered heterocyclic ring containing 1-4 N atoms may be a 6-9 membered heterocyclic ring containing 1-2 N atoms or may be

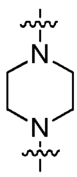, 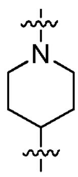, 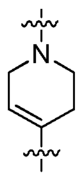 or 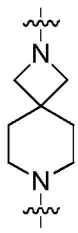,

which, at its upper end, is connected to $R^2$.
In a certain embodiment:
the oxygen-containing heterocyclic compound represented by formula I has a structure as follows:

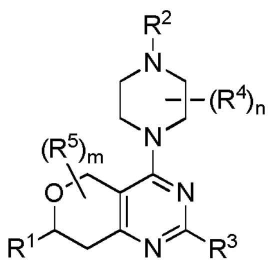

**II**

.

In a certain embodiment:
when $R^4$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.
In a certain embodiment:
when $R^4$ is independently $C_{1-6}$ alkyl substituted with one or more $R^{4-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^4$ is independently $C_{1-6}$ alkyl substituted with one or more $R^{4-1}$, then the more $R^{4-1}$ may be two or three $R^{4-1}$.

In a certain embodiment:
when $R^4$ is independently $C_{1-6}$ alkyl substituted with one or more $R^{4-1}$, then the $C_{1-6}$ alkyl substituted with one or more $R^{4-1}$ may be hydroxymethyl, cyanomethyl or

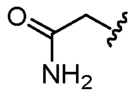

;

for example, cyanomethyl or

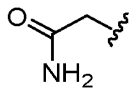

.

In a certain embodiment:
when $R^4$ is independently $C_{1-6}$ alkyl substituted with one or more $R^{4-1}$, then the $C_{1-6}$ alkyl substituted with one or more $R^{4-1}$ may be

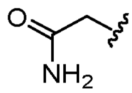

.

In a certain embodiment:
when $R^4$ is independently $C_{1-6}$ alkyl substituted with one or more $R^{4-1}$, then the $C_{1-6}$ alkyl substituted with one or more $R^{4-1}$ may be hydroxymethyl or cyanomethyl.

In a certain embodiment:
when $R^4$ is independently $C_{1-6}$ alkyl substituted with one or more $R^{4-1}$, then the $C_{1-6}$ alkyl substituted with one or more $R^{4-1}$ may be hydroxymethyl.

In a certain embodiment:
when $R^4$ is independently $C_{1-6}$ alkyl substituted with one or more $R^{4-1}$, then the $C_{1-6}$ alkyl substituted with one or more $R^{4-1}$ may be cyanomethyl.

In a certain embodiment:
when $R^a$ is independently halogen, then the halogen may be fluorine, chlorine, bromine or iodine, or may be fluorine.

In a certain embodiment:
when $R^b$ and $R^f$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl-C(=O)-, then the $C_{1-6}$ alkyl in the $C_{1-6}$ alkyl-C(=O)- may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl substituted with one or more $R^{b-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl substituted with one or more $R^{b-1}$, then the more $R^{b-1}$ may be two or three $R^{b-1}$.

In a certain embodiment:
when $R^{10j}$ and $R^{10k}$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{10j}$ and $R^{10k}$ taken together with the N atom to which they are attached form "4-10 membered heterocycloalkyl containing 1-3 heteroatoms selected from one or more of O and N", then the "4-10 membered heterocycloalkyl containing 1-3 heteroatoms selected from one or more of O and N" may be "5-6 membered heterocycloalkyl containing 2 heteroatoms selected from O and N", or may be

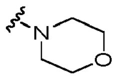.

In a certain embodiment:
the $R^2$ may be CN,

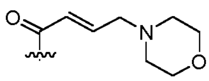, 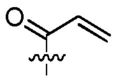, 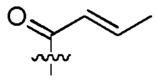, 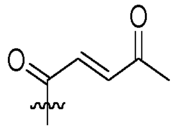,

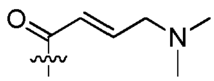, 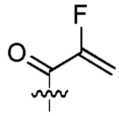, 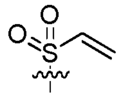 or 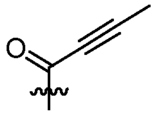.

In a certain embodiment:
the $R^2$ may be CN,

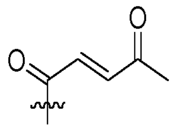.

In a certain embodiment:
the $R^2$ may be

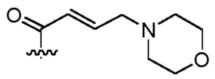, 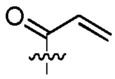, 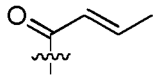, 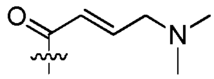,

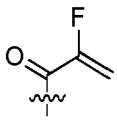, 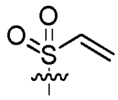 or 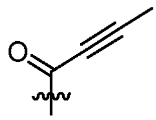.

In a certain embodiment:
the $R^2$ may be

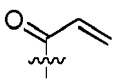, 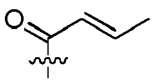, 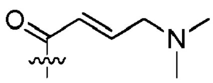, 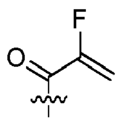, 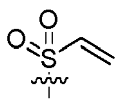

or

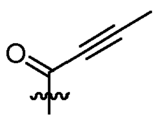.

In a certain embodiment, the oxygen-containing heterocyclic compound represented by formula I has any one of the following structures:

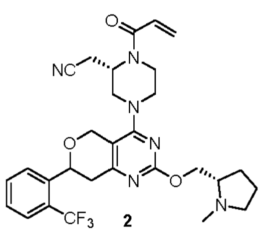
2
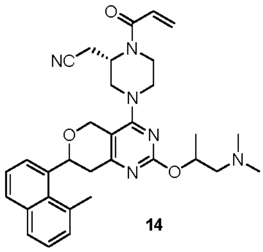
14
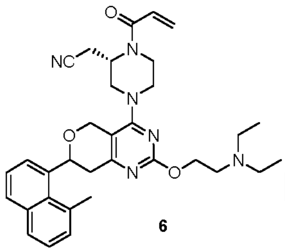
6
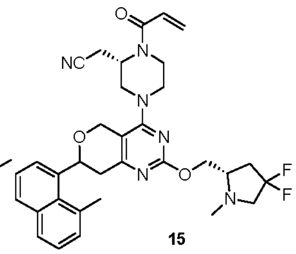
15

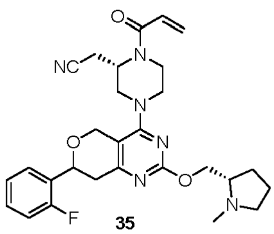
35
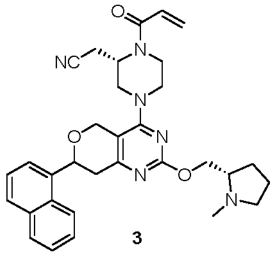
3
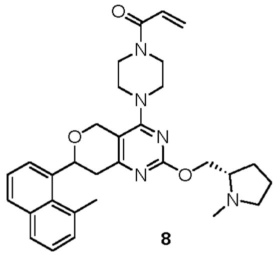
8
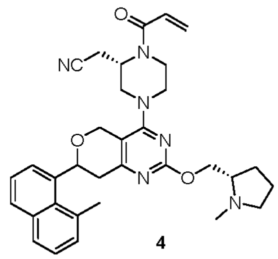
4

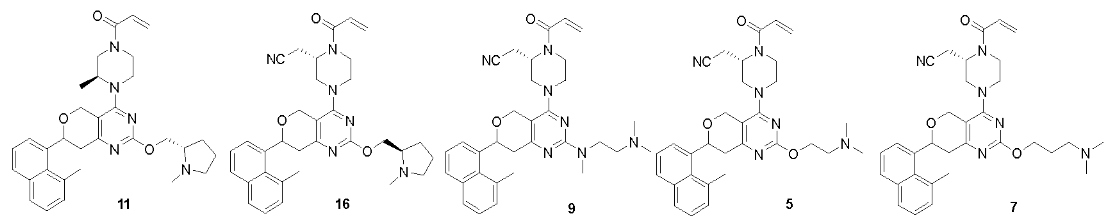
11 16 9 5 7

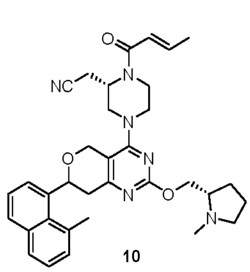
10
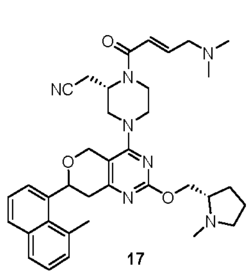
17
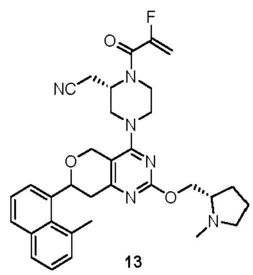
13
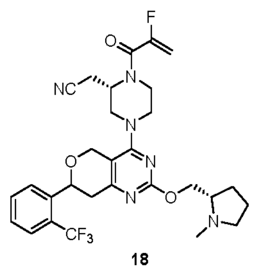
18

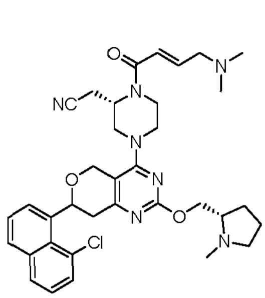
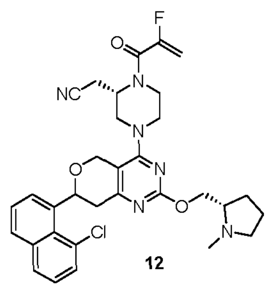
12
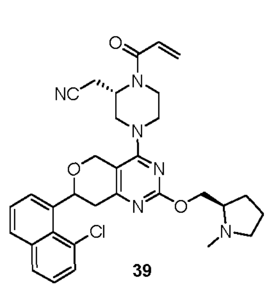
39
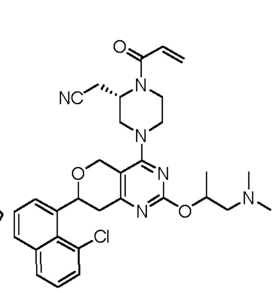

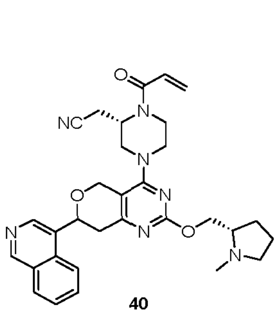
40
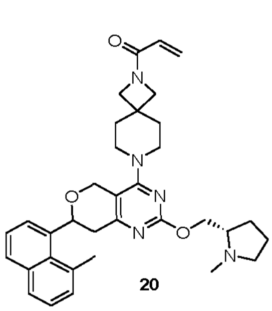
20
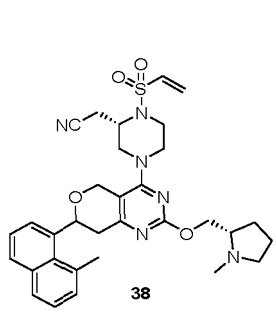
38
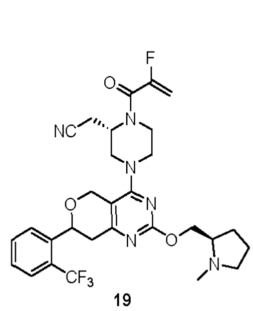
19

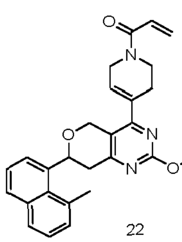
22
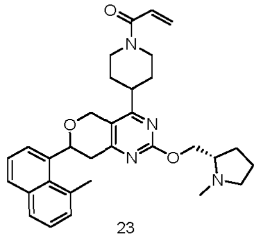
23
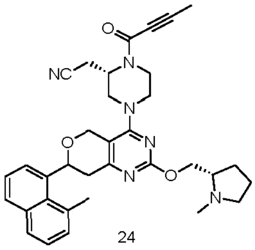
24
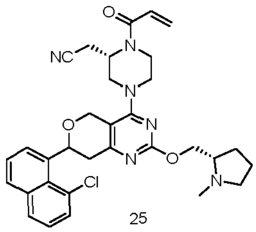
25

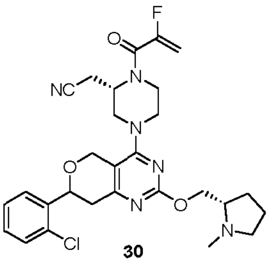
**30**
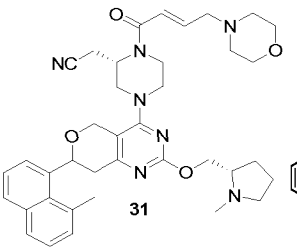
**31**
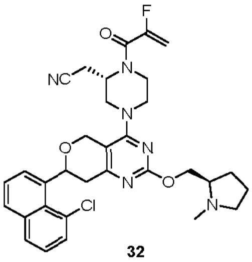
**32**
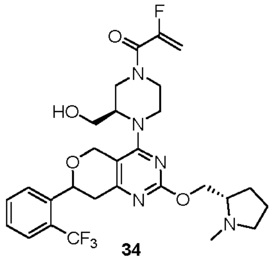
**34**

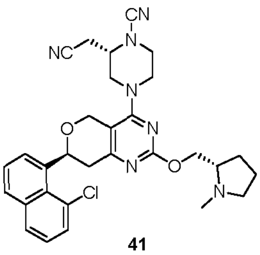
**41**
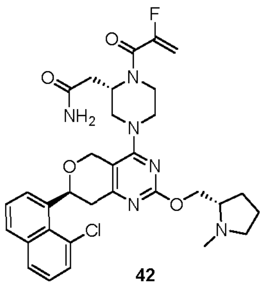
**42**
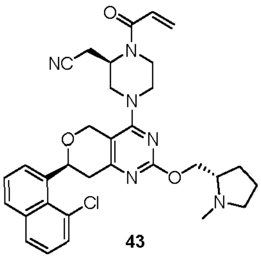
**43**
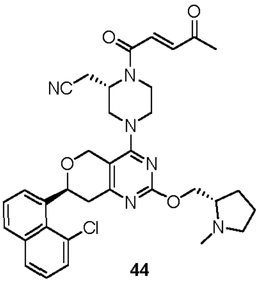
**44**

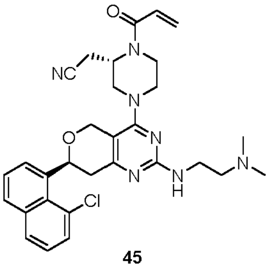
**45**
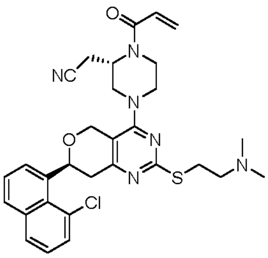
**46**
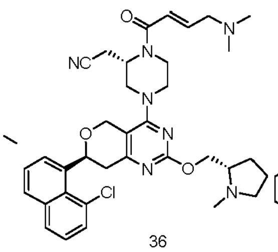
36
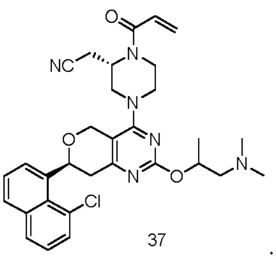
37
.

In a certain embodiment, the oxygen-containing heterocyclic compound represented by formula I is any one of the following compounds:

compound

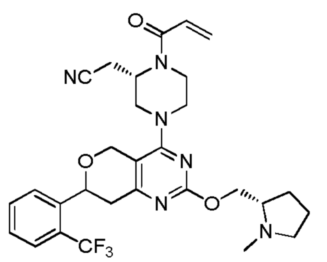

, which has a retention time of 0.92 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

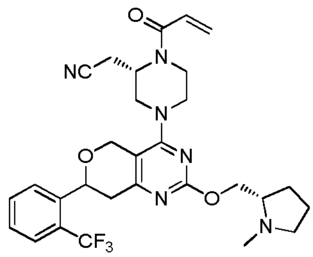

, which has a retention time of 2.74 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

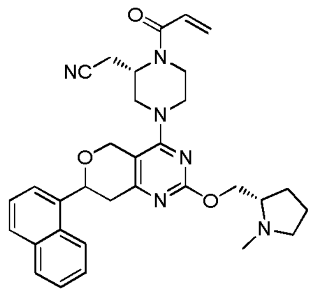

, which has a retention time of 0.97 min under the following conditions: equipment: SFC Method Station (Thar, Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar;

compound

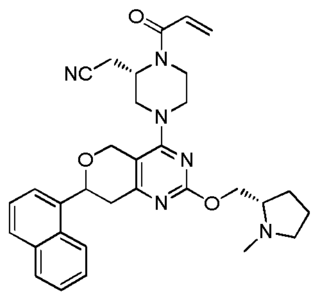

, which has a retention time of 2.40 min under the following conditions: equipment: SFC Method Station (Thar, Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar;

compound

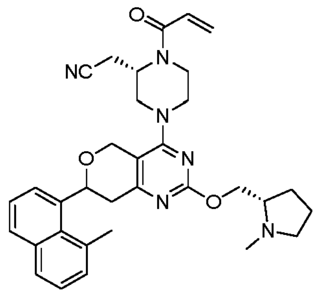

, which has a retention time of 0.97 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar;

compound

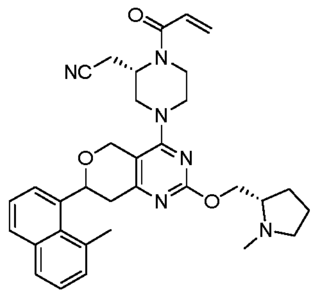

, which has a retention time of 1.94 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar;

compound

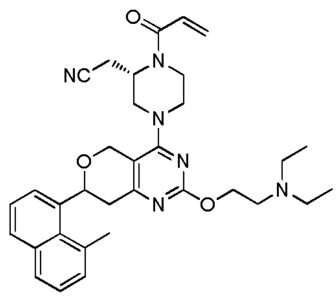

, which has a retention time of 1.22 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;

compound

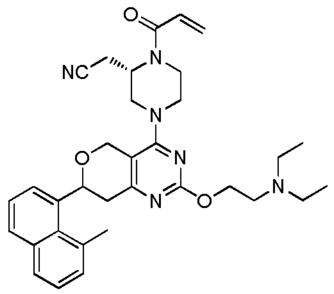

, which has a retention time of 2.67 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;

compound

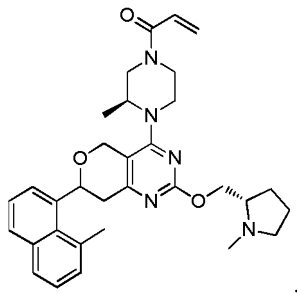

, which has a retention time of 3.26 min under the following conditions: instrument: SFC Method Station (Thar, Waters); chromatographic column: R,R-WHELK-O1 4.6 * 100 mm, 5 μm (REGIS); column temperature: 40 °C; mobile phase: $CO_2$/(MeOH/CAN = 3 : 2 (0.1% TEA)) = 55/45; flow rate: 4.0 ml/min; wavelength: 254 nm; back pressure: 120 bar;

compound

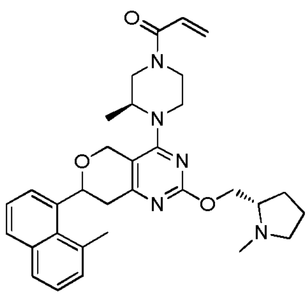

, which has a retention time of 4.16 min under the following conditions: instrument: SFC Method Station (Thar, Waters); chromatographic column: R,R-WHELK-O1 4.6 * 100 mm, 5 μm (REGIS); column temperature: 40 °C; mobile phase: $CO_2$/(MeOH/CAN = 3 : 2 (0.1% TEA)) = 55/45; flow rate: 4.0 ml/min; wavelength: 254 nm; back pressure: 120 bar.

compound

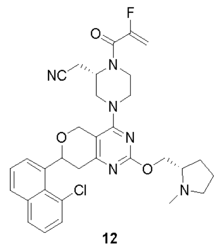

12

, which has a retention time of 1.36 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

compound

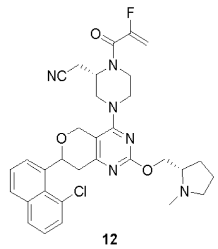

12

, which has a retention time of 2.77 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

compound

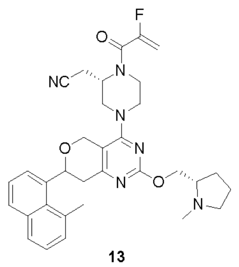

13

, which has a retention time of 1.17 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

compound

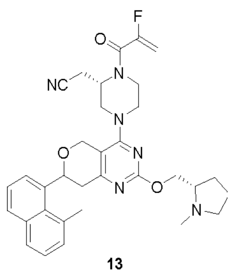

13

, which has a retention time of 2.76 min under the following conditions: instrument: SFC Method Station (Thar, Waters); chromatographic column: OJ-H 4.6 * 100 mm, 5 μm (Daicel); column temperature: 40 °C; mobile phase:

$CO_2$/MeOH (0.1% TEA) = 60/40; flow rate: 4.0 ml/min; wavelength: 254 nm; back pressure: 120 bar.

compound

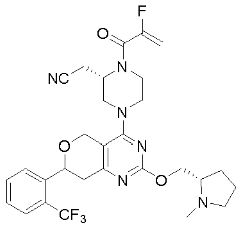

18

, which has a retention time of 0.78 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar.

compound

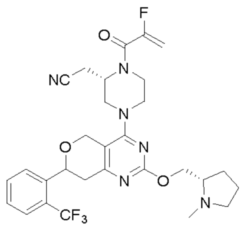

18

, which has a retention time of 2.42 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar.

compound

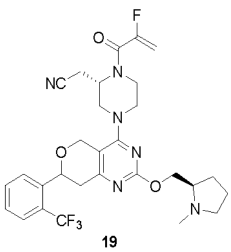

19

, which has a retention time of 0.79 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar.

compound

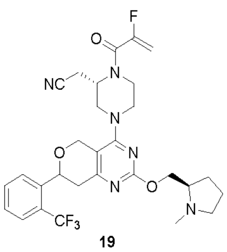

19

, which has a retention time of 2.29 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar.

compound

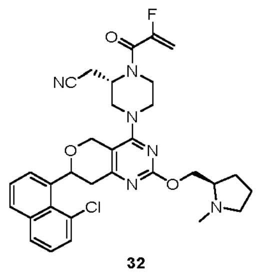

32

, which has a retention time of 1.45 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

compound

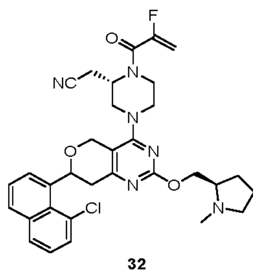

32

, which has a retention time of 2.81 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

In a certain embodiment, the oxygen-containing heterocyclic compound represented by formula I is any one of the following compounds:

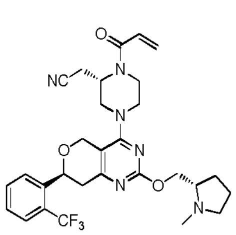 , 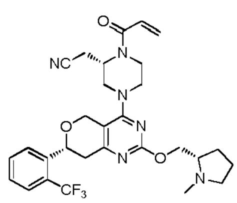 , 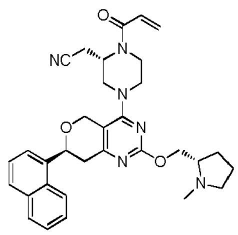 , 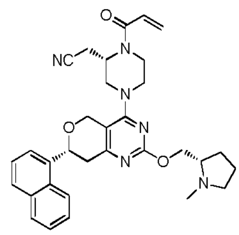 ,

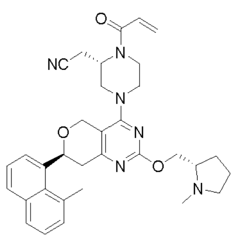 , 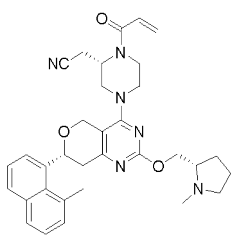 , 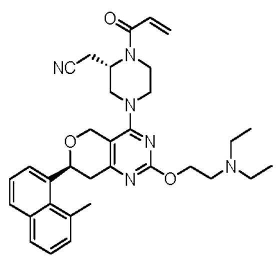 , 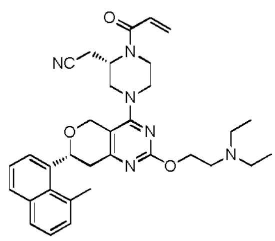 ,

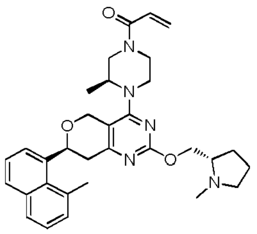 , 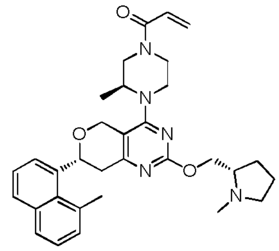 , 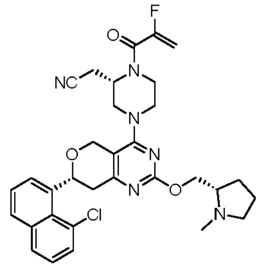 , 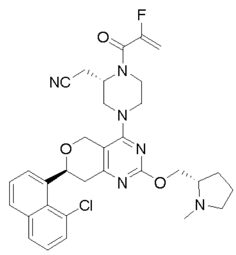 ,

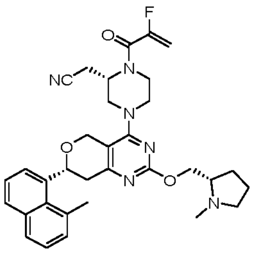 , 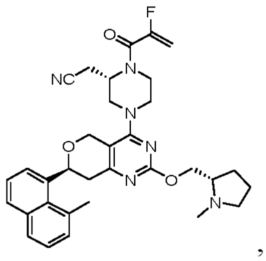 , 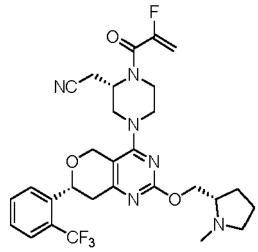 , 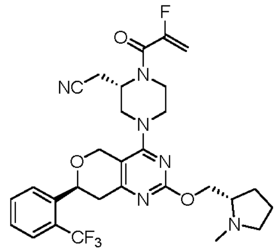 ,

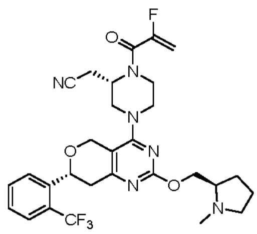 , 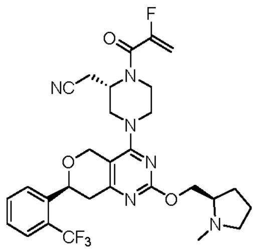 , 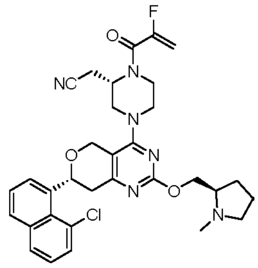 , 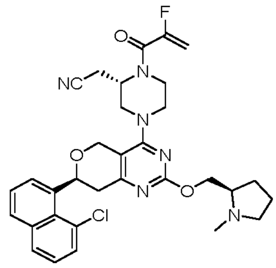 .

The present disclosure also provides a method for preparing the above-mentioned oxygen-containing heterocyclic compound represented by formula I, the method comprising route one or route two: route one:

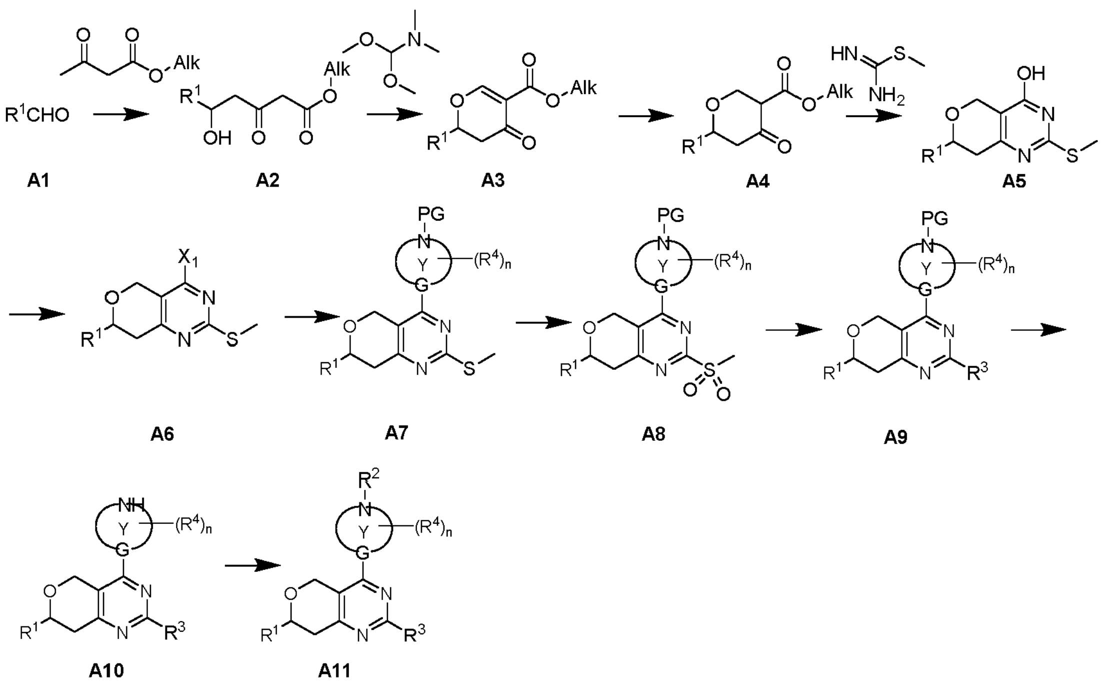


$R^1$, $R^2$, $R^3$, $R^4$, G, n and Y are all as defined above; $X_1$ is a leaving group selected from OTf and Cl Alk is alkyl (for example, $C_{1-6}$ alkyl); PG is an amino protecting group selected from Boc and Cbz; route two:

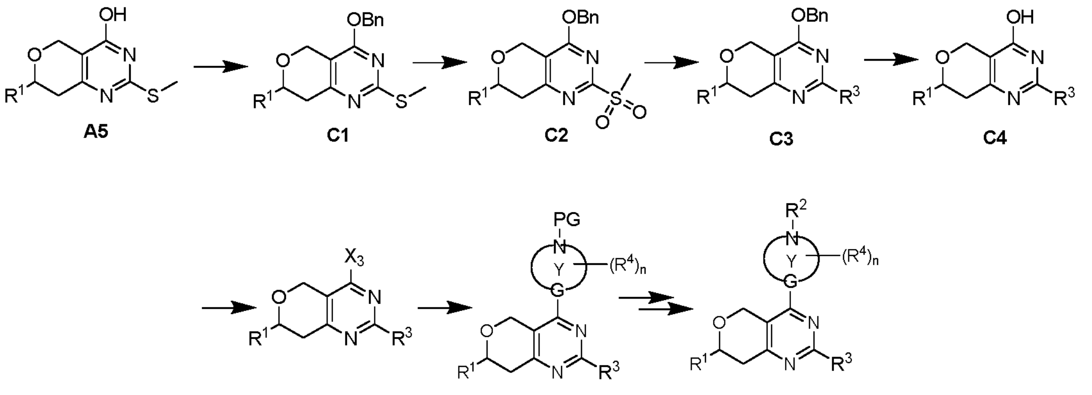

A5 C1 C2 C3 C4

C5 A9 A11

$R^1$, $R^2$, $R^3$, $R^4$, G, n and Y are all as defined above; $X_3$ is a leaving group (OTf or Cl); PG is an amino protecting group (Boc or Cbz).

The route one is described in detail as follows: aldehyde compound A1 is condensed with acetyl acetate to obtain compound A2; A2 is condensed with DMF-DMA to obtain compound A3; A3 is reduced to A4; A5 is obtained from A4 after ring formation; the hydroxyl group in A5 is converted into a leaving group and A6 is obtained; A6 is converted into A7 by means of nucleophilic substitution, coupling, etc.; A7 is oxidized to obtain A8; A8 is further converted into A9; and A9 is deprotected and further converted into A11.

The route two is described in detail as follows: compound A5 is protected by Bn; C1 is oxidized to obtain C2; C2 is converted into C3 by nucleophilic substitution; C3 is subjected to Bn deprotection and converted into C4; the hydroxyl group in C4 is converted into a leaving group and C5 is obtained; C5 is converted into A9 by means of nucleophilic substitution, coupling, etc.; A9 is deprotected and further converted into A11.

The conditions and steps adopted for the chemical reactions involved in the various reaction routes described in the present disclosure all can be carried out with reference to conventional conditions and steps for such reactions in the art, and specific reference may be made to the literatures: R. Larock, Comprehensive Organic Transformations, VCH Publishers (1989); T. W. Greene and P. G. M. Wuts, Protective Groups in Organic Synthesis, 3rd ED., John Wiley and Sons (1999); L. Fieser and M. Fieser, Fieser and Fieser's Reagents for Organic Synthesis, John Wiley and Sons (1994); L. Paquette, ed., Encyclopedia of Reagents for Organic Synthesis, John Wiley and Sons (1995) subsequent versions.

The present application cites the entire contents of the above literatures. In addition, other target compounds of the present disclosure can also be obtained from the compounds obtained by the above-mentioned methods through modifying peripheral positions with reference to related methods of the above literatures.

The present disclosure also provides a compound represented by formula A5, A6, A7, A8, A9, A10, C1, C2, C3, C4 or C5:

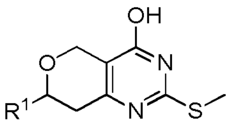

A5,

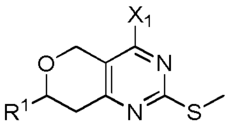

A6,

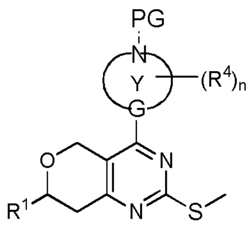

A7,

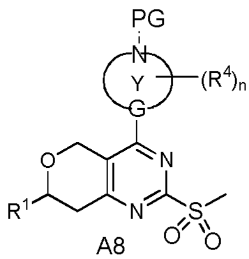

A8,

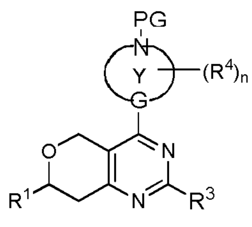

A9

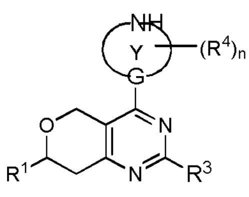

A10

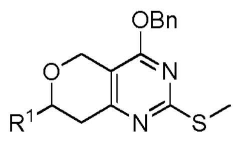

**C1**

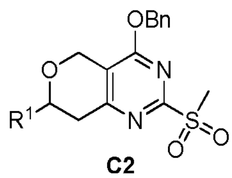

**C2**

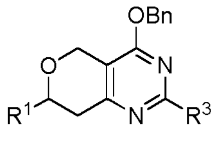
C3,
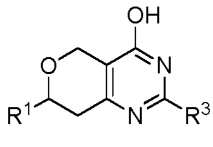
C4,
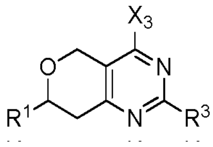
C5;

wherein $R^1$, $R^3$, $R^4$, G, Y and n are as defined above;

$X^1$ and $X^3$ are independently leaving groups selected from OTf and Cl PG is an amino protecting group selected from Boc and Cbz.

In a certain embodiment, the compound represented by formula A5, A6, A7, A8, A9, A10, C1, C2, C3, C4 or C5 may be any one of the following compounds:

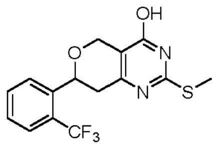
2-f
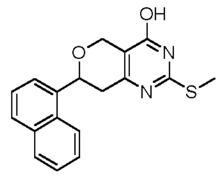
3-f
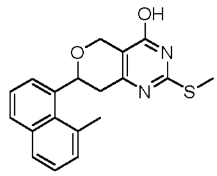
4-f
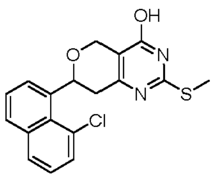
12-f

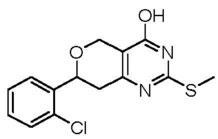
30-f

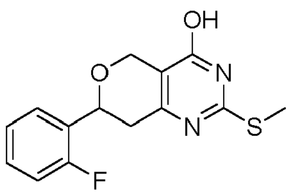
35-f
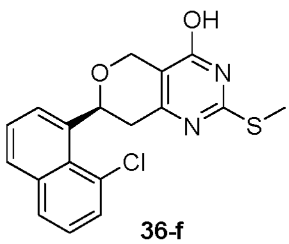
36-f
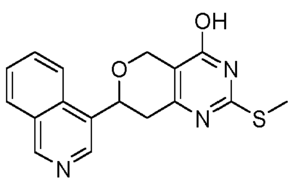
40-f

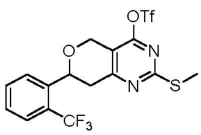
2-e
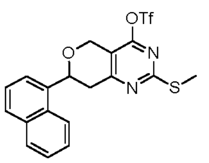
3-e
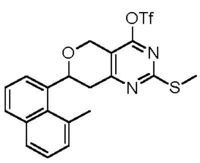
4-e
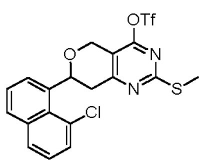
12-e
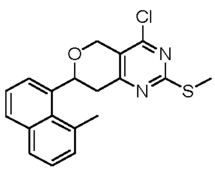
20-e

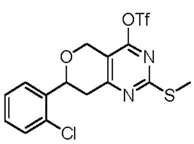
30-e
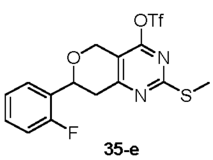
35-e
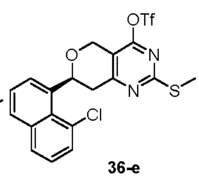
36-e
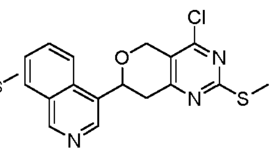
40-e
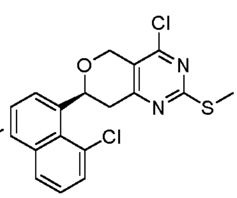
43-e

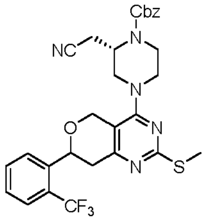
2-d

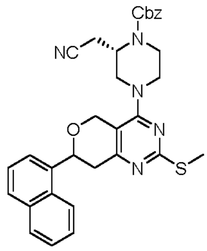
3-d

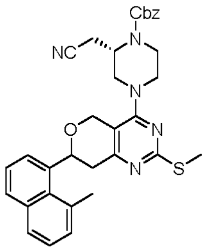
4-d

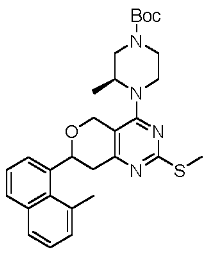
11-d

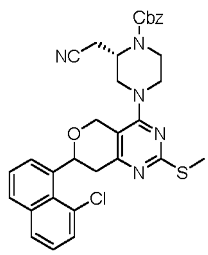
12-d

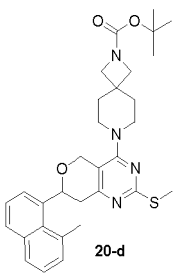
20-d

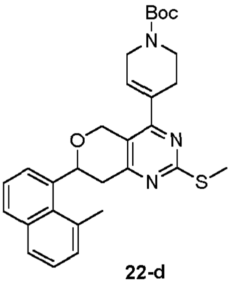
22-d

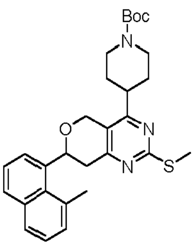
23-d

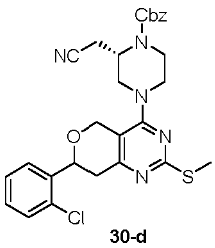
30-d

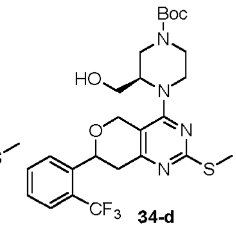
34-d

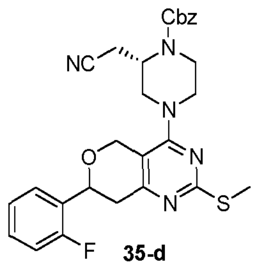
35-d

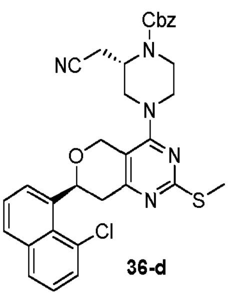
36-d

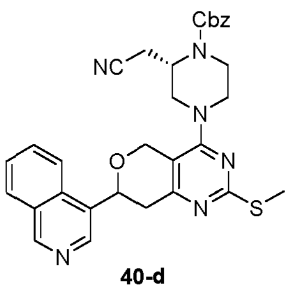
40-d

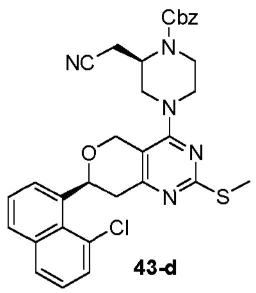
43-d

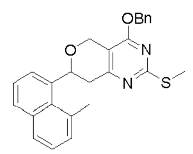
8-g

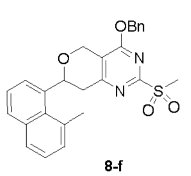
8-f

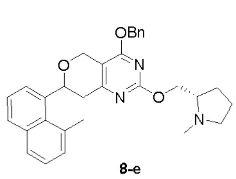
8-e

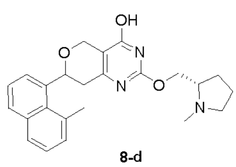
8-d

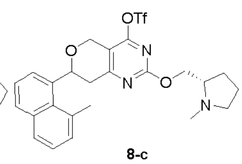
8-c

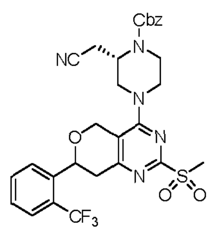
2-c

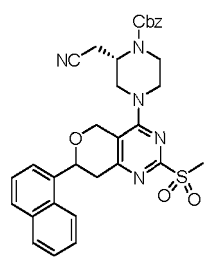
3-c

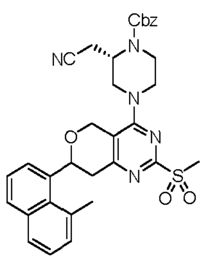
4-c

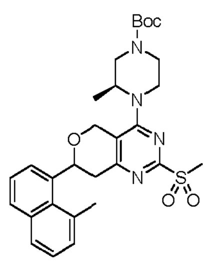
11-c

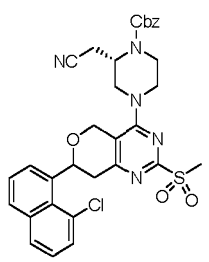
12-c

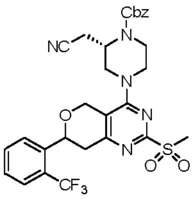
19-c

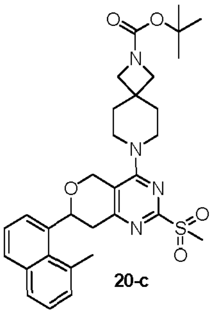
20-c

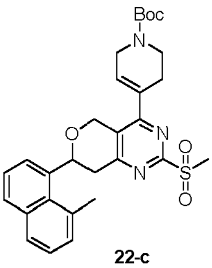
22-c

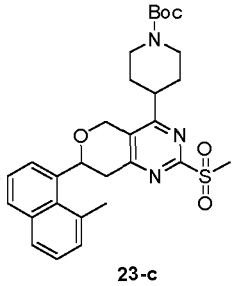
23-c

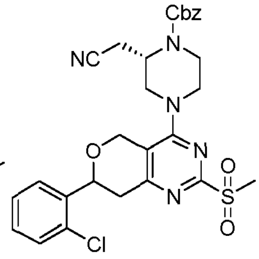
30-c

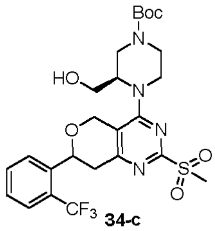
34-c

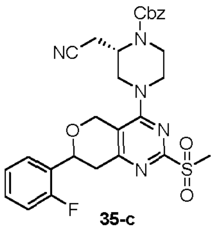
35-c

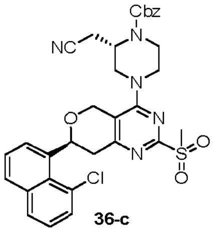
36-c

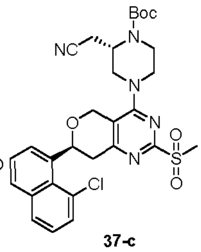
37-c

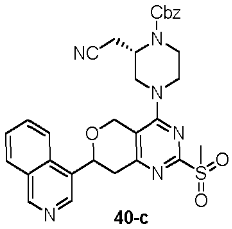
40-c

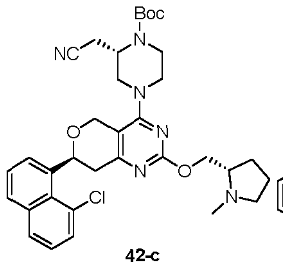
42-c

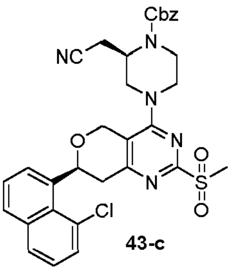
43-c

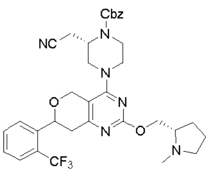
2-b

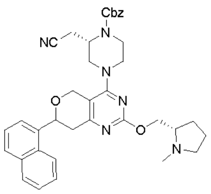
3-b

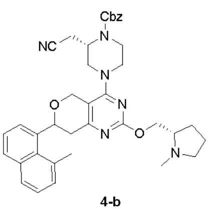
4-b

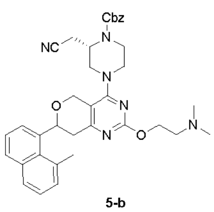
5-b

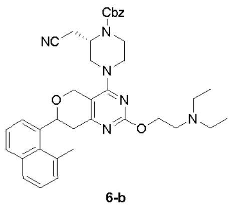
6-b

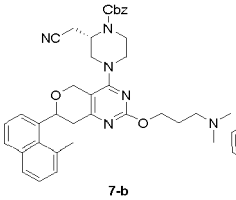
7-b

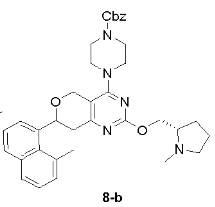
8-b

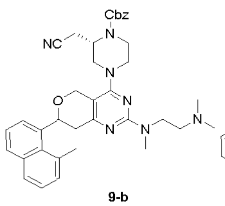
9-b

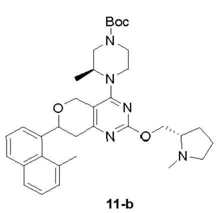
11-b

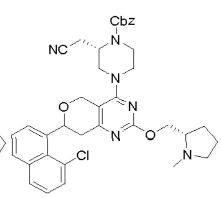
12-b

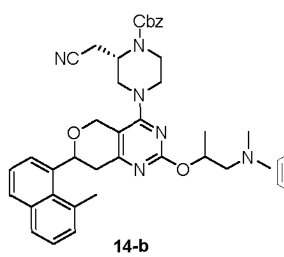
14-b

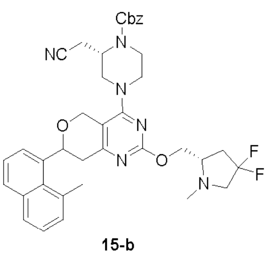
15-b

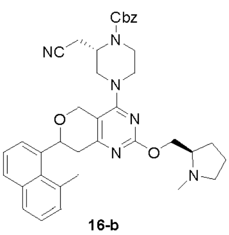
16-b

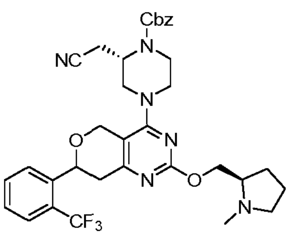
19-b

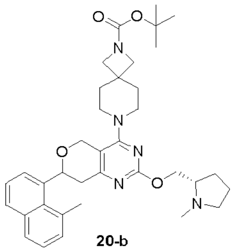
20-b

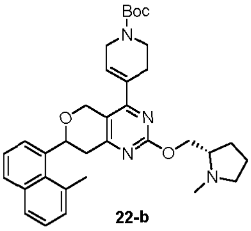
22-b

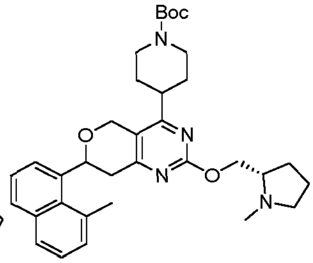
23-b

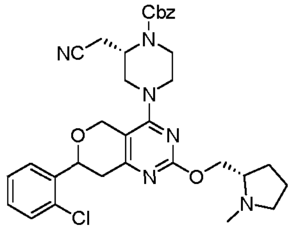
30-b

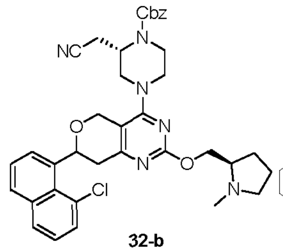
32-b

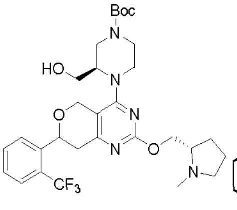
34-b

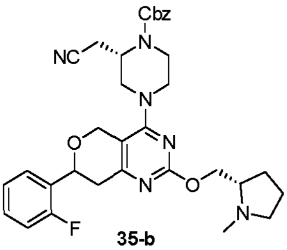
35-b

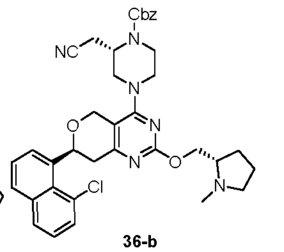
36-b

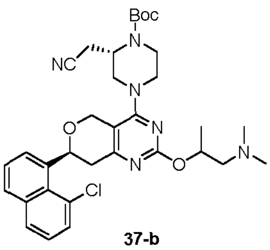
37-b

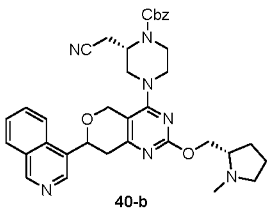
40-b

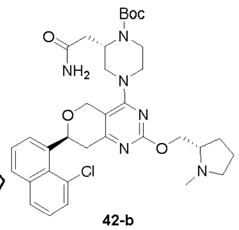
42-b

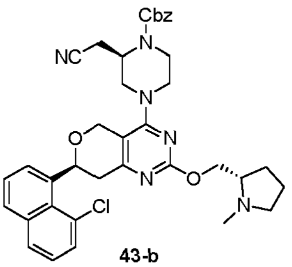
43-b

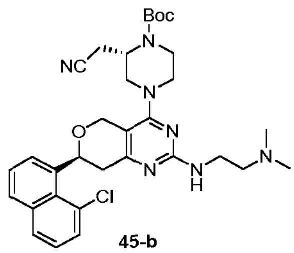
45-b

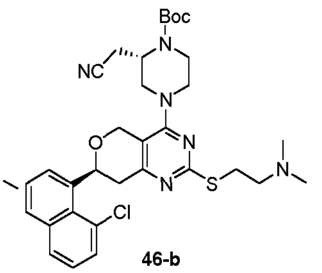
46-b

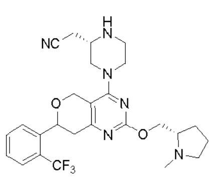
2-a

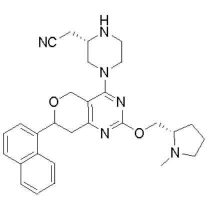
3-a

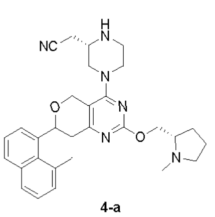
4-a

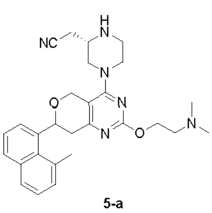
5-a

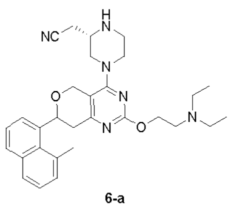
6-a

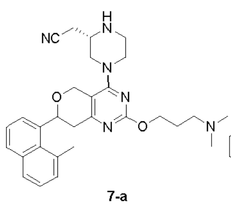
7-a

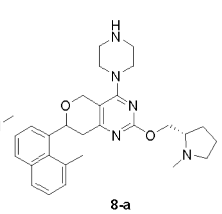
8-a

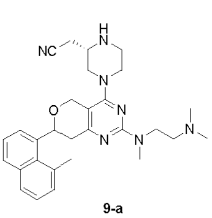
9-a

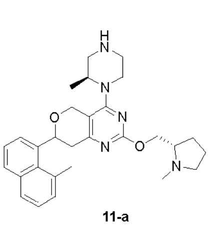
11-a

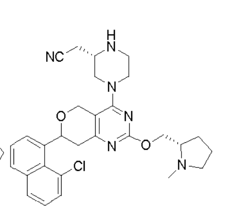
12-a

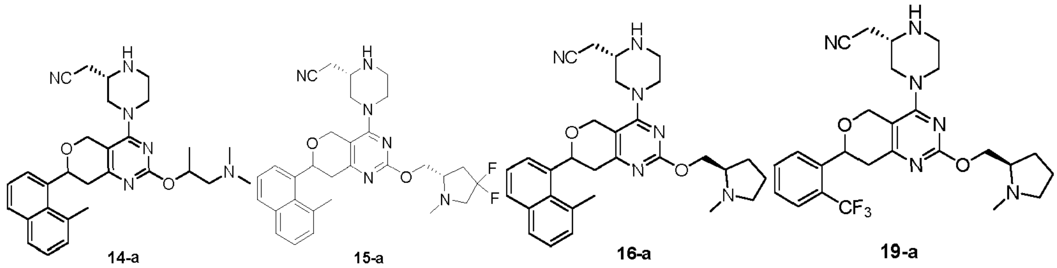

14-a 15-a 16-a 19-a

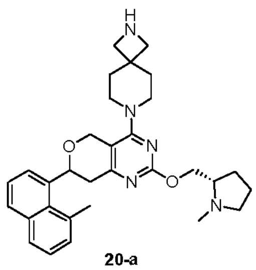

20-a

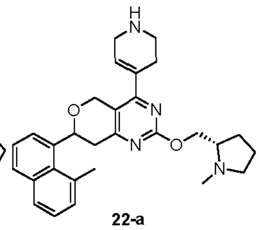

22-a

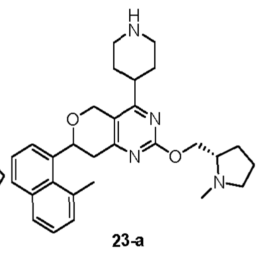

23-a

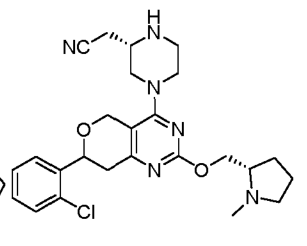

30-a

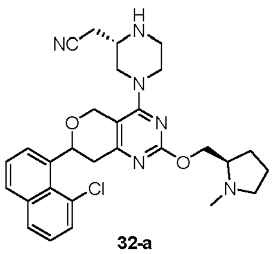

32-a

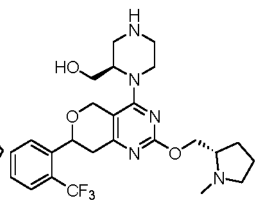

34-a

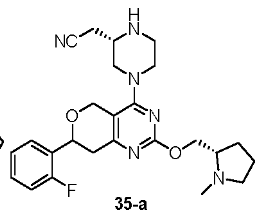

35-a

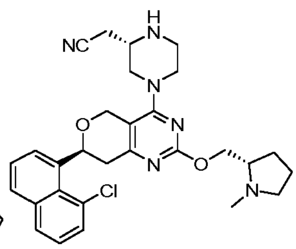

36-a

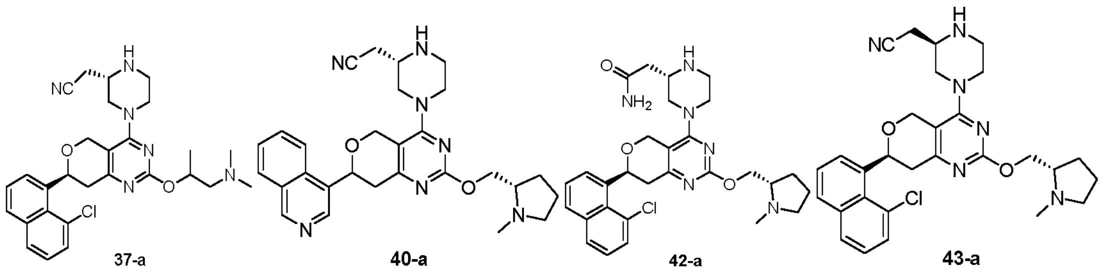

37-a 40-a 42-a 43-a

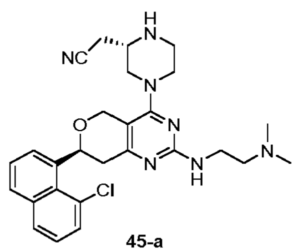

45-a

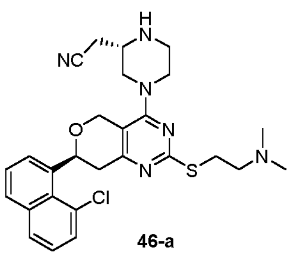

46-a

The present disclosure also provides a pharmaceutical composition, comprising substance **A** and a pharmaceutical adjuvant; the substance **A** is the above-mentioned oxygen-containing heterocyclic compound represented by formula **I,** a pharmaceutically acceptable salt thereof, a stereoisomer thereof, a tautomer thereof or an isotopic compound thereof.

The present disclosure also provides use of substance **A** in the preparation of an RAS inhibitor, wherein the substance **A** is the above-mentioned oxygen-containing heterocyclic compound represented by formula **I,** a pharmaceutically acceptable salt thereof, a stereoisomer thereof, a tautomer thereof or an isotopic compound thereof.

The RAS comprises, for example, KRAS G12C, HRAS G12C or NRAS G12C mutation; for example, KRAS G12C.

The present disclosure also provides use of substance **A** in the preparation of a medicament, wherein the medicament is used to treat or prevent an RAS-mediated disease;
the substance **A** is the above-mentioned oxygen-containing heterocyclic compound represented by formula **I,** a pharmaceutically acceptable salt thereof, a stereoisomer thereof, a tautomer thereof or an isotopic compound thereof.

The RAS comprises, for example, KRAS G12C, HRAS G12C or NRAS G12C mutation; for example, KRAS G12C.

The RAS-mediated disease is, for example, cancer. The cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The present disclosure also provides use of substance **A** in the manufature of a medicament, wherein the medicament is used to treat or prevent cancer;
the substance **A** is the above-mentioned oxygen-containing heterocyclic compound represented by formula **I,** a pharmaceutically acceptable salt thereof, a stereoisomer thereof, a tautomer thereof or an isotopic compound thereof.

The cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The present disclosure also provides an oxygen-containing heterocyclic compound represented by formula **I,** a pharmaceutically acceptable salt thereof, a solvate thereof, a solvate of the pharmaceutically acceptable salt thereof, a crystal form thereof, a stereoisomer thereof, a tautomer thereof or an isotopic compound thereof:

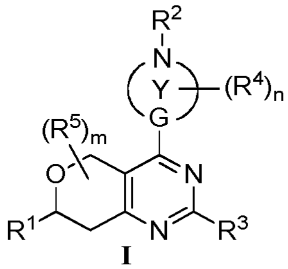

**I**

wherein $R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $-C(=O)R^{65}$, $-NR^{63}R^{64}$, $-C(=O)OR^{66}$, $-C(=O)NR^{69}R^{610}$, $C_{1-6}$ alkyl, $C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;

$R^{1-6-1}$, $R^{1-6-2}$, $R^{1-6-3}$ , $R^{1-6-4}$, $R^{1-6-5}$ and $R^{1-6-6}$ are independently cyano, halogen, hydroxyl, $C_{1-6}$ alkoxy, $C_{1-6}$ alkyl, $-C(=O)R^{65-2}$, $-NR^{63-2}R^{64-2}$, $-C(=O)OR^{66-2}$, or $-C(=O)NR^{69-2}R^{610-2}$;

$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ and $R^{610-2}$ are independently hydrogen or $C_{1-6}$ alkyl;

m is 0, 1 or 2;

$R^5$ is independently $C_{1-6}$ alkyl;

$R^3$ is $-OR^{31}$, $-SR^{32}$ or $-NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or

$C_{1-6}$ alkyl substituted with one or more $R^{31-1}$;

$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, $-OR^d$, $-SR^{d1}$, $-NR^{e1}R^{e2}$, or $-C(=O)NR^{e3}R^{e4}$;

$R^{d15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;

$R^{1-8-1}$ and $R^{1-8-2}$ are independently cyano, halogen, hydroxyl, $C_{1-6}$ alkoxy, $-C(=O)R^{e9}$, $-NR^{e10}R^{e11}$, $-C(=O)OR^{e12}$, or $-C(=O)NR^{e13}R^{e14}$;

$R^{e5}$, $R^{e6}$, $R^{e7}$, $R^{e8}$, $R^{e9}$, $R^{e10}$, $R^{e11}$, $R^{e12}$, $R^{e13}$ and $R^{e14}$ are independently hydrogen or $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;

G is N, C or CH;

n is 0, 1, 2 or 3;

$R^4$ is independently $C_{1-6}$ alkyl, $C_{1-6}$ alkyl substituted with one or more $R^{4-1}$, oxo, $-C(=O)OR^{4a}$ or $-C(=O)NR^{4b}R^{4c}$;

$R^{4-1}$ is independently halogen, cyano, hydroxyl, $C_{1-6}$ alkoxy, $-NR^{4i}R^{4j}$, $-C(=O)OR^{4d}$ or $-C(=O)NR^{4e}R^{4f}$; $R^{4d}$, $R^{4e}$, $R^{4f}$, $R^{4i}$ and $R^{4j}$ are independently hydrogen or $C_{1-6}$ alkyl;

$R^{4a}$, $R^{4b}$ and $R^{4c}$ are independently hydrogen or $C_{1-6}$ alkyl;

$R^2$ is CN, $-C(=O)-C(R^a)=C(R^b)(R^f)$, $-C(=O)-C\equiv CR^f$, $-S(=O)_2-C(R^a)=C(R^b)(R^f)$ or $-S(=O)_2-C\equiv CR^f$;

$R^a$ is independently hydrogen, deuterium, halogen or $C_{1-6}$ alkyl;

$R^b$ and $R^f$ are independently hydrogen, deuterium, $C_{1-6}$ alkyl, $C_{1-6}$ alkyl-C(=O)-, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment, with regard to an oxygen-containing heterocyclic compound represented by formula I, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, some groups are as defined as follows, and the unmentioned group definitions are as described in any one of the embodiments of the present disclosure (this content is hereinafter referred to simply as "in a certain embodiment"). With regard to an oxygen-containing heterocyclic compound represented by formula I,

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $-C(=O)R^{65}$, $-NR^{63}R^{64}$, $-C(=O)OR^{66}$, $-C(=O)NR^{69}R^{610}$, $C_{1-6}$ alkyl,

$C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;

$R^{1-6-1}$, $R^{1-6-2}$, $R^{1-6-3}$, $R^{1-6-4}$, $R^{1-6-5}$ and $R^{1-6-6}$ are independently cyano, halogen, hydroxyl, $C_{1-6}$ alkoxy, $C_{1-6}$ alkyl, $-C(=O)R^{65-2}$, $-NR^{63-2}R^{64-2}$, $-C(=O)OR^{66-2}$, or $-C(=O)NR^{69-2}R^{610-2}$;

$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ and $R^{610-2}$ are independently hydrogen or $C_{1-6}$ alkyl;

m is 0, 1 or 2;

$R^5$ is independently $C_{1-6}$ alkyl;

$R^3$ is $-OR^{31}$, $-SR^{32}$ or $-NR^{33}R^{34}$;

$R^{31}$, $R^{32}$, $R^{33}$ and $R^{34}$ are independently $C_{1-6}$ alkyl substituted with one or more $R^{31-1}$;

$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, $-OR^d$, $-SR^{d1}$, $-NR^{e1}R^{e2}$, or $-C(=O)NR^{e3}R^{e4}$;

$R^{d15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;

$R^{1-8-1}$ and $R^{1-8-2}$ are independently cyano, halogen, hydroxyl, $C_{1-6}$ alkoxy, $-C(=O)R^{e9}$, $-NR^{e10}R^{e11}$, $-C(=O)OR^{e12}$, or $-C(=O)NR^{e13}R^{e14}$;

$R^{e5}$, $R^{e6}$, $R^{e7}$, $R^{e8}$, $R^{e9}$, $R^{e10}$, $R^{e11}$, $R^{e12}$, $R^{e13}$ and $R^{e14}$ are independently hydrogen or $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;

G is N, C or CH;

n is 0, 1, 2 or 3;

$R^4$ is independently $C_{1-6}$ alkyl, $C_{1-6}$ alkyl substituted with one or more $R^{4-1}$, oxo, $-C(=O)OR^{4a}$ or $-C(=O)NR^{4b}R^{4c}$;

$R^{4-1}$ is independently halogen, cyano, hydroxyl, $C_{1-6}$ alkoxy, $-NR^{4i}R^{4j}$, $-C(=O)OR^{4d}$ or $-C(=O)NR^{4e}R^{4f}$; $R^{4d}$, $R^{4e}$, $R^{4f}$, $R^{4i}$ and $R^{4j}$ are independently hydrogen or $C_{1-6}$ alkyl;

$R^{4a}$, $R^{4b}$ and $R^{4c}$ are independently hydrogen or $C_{1-6}$ alkyl;

$R^2$ is $-C(=O)-C(R^a)=C(R^b)(R^f)$, $-C(=O)-C{\equiv}CR^f$, $-S(=O)_2-C(R^a)=C(R^b)(R^f)$ or $-S(=O)_2-C{\equiv}CR^f$;

$R^a$ is independently hydrogen, deuterium, halogen or $C_{1-6}$ alkyl;

$R^b$ and $R^f$ are independently hydrogen, deuterium, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen.

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; for example, $C_{6-20}$ aryl, "5-12 membered heteroaryl containing 1-4 heteroatoms selected from one or more of O, S and N", or $C_{6-20}$ aryl substituted with one or more $R^{1-6}$;

$R^{1-6}$ and $R^{1-7}$ are independently halogen, $C_{1-6}$ alkyl, or $C_{1-6}$ alkyl substituted with one or more $R^{1-6-1}$;

$R^{1-6-1}$ is independently halogen.

In a certain embodiment:

$R^1$ is $C_{6-20}$ aryl, or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$;

$R^{1-6-1}$ is independently halogen.

In a certain embodiment:
m is 0.
In a certain embodiment:

$R^3$ is $-OR^{31}$, $-SR^{32}$ or $-NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl.

In a certain embodiment:

$R^3$ is $-SR^{32}$;

$R^{32}$ is $C_{1-6}$ alkyl substituted with one or more $R^{31-1}$;

$R^{31-1}$ is independently $-NR^{e1}R^{e2}$;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl.

In a certain embodiment:

$R^3$ is $-NR^{33}R^{34}$;

$R^{34}$ is independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl.

In a certain embodiment:

$R^3$ is $-NR^{33}R^{34}$;

$R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$;

$R^{d15}$ is independently $C_{1-6}$ alkyl.

In a certain embodiment:

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{e1}$, $R^{e2}$ and $R^{d15}$ are independently $C_{1-6}$ alkyl.

In a certain embodiment:

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring; for example, a monocyclic ring;

G is N, C or CH.

In a certain embodiment:

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl, cyano, or $-C(=O)NR^{4e}R^{4f}$; $R^{4e}$ and $R^{4f}$ are independently hydrogen or $C_{1-6}$ alkyl; for example, hydrogen.

In a certain embodiment:

n is 0 or 1;

$R^4$ is independently $C_{1-6}$ alkyl substituted with one or more $R^{4-1}$;

$R^{4-1}$ is independently $-C(=O)NR^{4e}R^{4f}$; $R^{4e}$ and $R^{4f}$ are independently hydrogen or $C_{1-6}$ alkyl; for example, hydrogen.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl or cyano.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently hydroxyl.

In a certain embodiment:

n is 0 or 1;

$R^4$ is independently $C_{1-6}$ alkyl, or $C_{1-6}$ alkyl substituted with one or more $R^{4-1}$;

$R^{4-1}$ is independently cyano.

In a certain embodiment:

$R^2$ is CN, -C(=O)-C($R^a$)=C($R^b$)($R^f$), -C(=O)-C=C-Me or, -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, $C_{1-6}$ alkyl-C(=O)-, or $C_{1-6}$ alkyl substituted with one or more $R^{b-1}$;

$R^{b-1}$ is independently -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^2$ is CN or -C(=O)-C($R^a$)=C($R^b$)($R^f$);

$R^a$ is independently hydrogen;

$R^b$ and $R^f$ are independently hydrogen, $C_{1-6}$ alkyl-C(=O)-.

In a certain embodiment:

$R^2$ is -C(=O)-C($R^a$)=C($R^b$)($R^f$), -C(=O)-C=C-Me or -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^2$ is -C(=O)-C($R^a$)=C($R^b$)($R^f$), -C(=O)-C=C-Me or -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, or $C_{1-6}$ alkyl substituted with one or more $R^{b-1}$;

$R^{b-1}$ is independently -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, $C_{1-6}$ alkyl, $C_{1-6}$ alkyl substituted with one or more $R^{1-6-1}$; $R^{1-6-1}$ is independently halogen;

m is 0;

$R^3$ is -$OR^{31}$, -$SR^{32}$ or -$NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or $C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or -$NR^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano, hydroxyl or -C(=O)$NR^{4e}R^{4f}$; $R^{4e}$ and $R^{4f}$ are independently hydrogen or $C_{1-6}$ alkyl;

$R^2$ is CN, -C(=O)-C($R^a$)=C($R^b$)($R^f$), -C(=O)-C=C-Me or, -S(=O)$_2$-C($R^a$)=C($R^b$)($R^f$);

$R^a$ is independently hydrogen or halogen;

$R^b$ and $R^f$ are independently hydrogen, $C_{1-6}$ alkyl, $C_{1-6}$ alkyl-C(=O)-, or $C_{1-6}$ alkyl substituted with one or more $R^{b-1}$;

$R^{b-1}$ is independently halogen, hydroxyl, $C_{1-6}$ alkoxy, or -$NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and

N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano or hydroxyl;

$R^2$ is $-C(=O)-C(R^a)=C(R^b)(R^f)$, $-C(=O)-C{\equiv}C-Me$ or $-S(=O)_2-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:
$R^2$ is $-C(=O)-C(R^a)=C(R^b)(R^f)$; $R^a$ is independently hydrogen or halogen; $R^b$ and $R^f$ are independently hydrogen or $C_{1-6}$ alkyl.

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;

$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;

$R^{1-8-1}$ is independently halogen; $R^{e5}$, $R^{e6}$, $R^{e7}$ and $R^{e8}$ are independently hydrogen or $C_{1-6}$ alkyl;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;

G is N, C or CH;

n is 0 or 1;

$R^4$ is independently $C_{1-6}$ alkyl, or $C_{1-6}$ alkyl substituted with one or more $R^{4-1}$;

$R^{4-1}$ is independently cyano;

$R^2$ is $-C(=O)-C(R^a)=C(R^b)(R^f)$, $-C(=O)-C{=}C-Me$ or $-S(=O)_2-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl substituted with one or more $R^{b-1}$;

$R^{b-1}$ is independently halogen, hydroxyl, $C_{1-6}$ alkoxy, or $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

In a certain embodiment:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; for example, $C_{6-20}$ aryl, "5-12 membered heteroaryl containing 1-4 heteroatoms selected from one or more of O, S and N", or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$;

$R^{1-6-1}$ is independently halogen;

m is 0;

$R^3$ is $-OR^{31}$, $-SR^{32}$ or $-NR^{33}R^{34}$;

$R^{31}$, $R^{32}$ and $R^{34}$ are independently $C_{1-6}$ alkyl substituted with one or more $R^{31-1}$; $R^{33}$ is independently H, $C_{1-6}$ alkyl, or

$C_{1-6}$ alkyl substituted with one or more $R^{31-1}$; for example, H or $C_{1-6}$ alkyl;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N;

n is 0 or 1;

$R^4$ is independently $C_{1-6}$ alkyl substituted with one or more $R^{4-1}$;

$R^{4-1}$ is independently cyano or $-C(=O)NR^{4e}R^{4f}$; $R^{4e}$ and $R^{4f}$ are independently hydrogen or $C_{1-6}$ alkyl;

$R^2$ is CN, $-C(=O)-C(R^a)=C(R^b)(R^f)$;

$R^a$ is independently hydrogen or halogen;

$R^b$ and $R^f$ are independently hydrogen, $C_{1-6}$ alkyl, or $C_{1-6}$ alkyl-C(=O)-.

In a certain embodiment:

$R^1$ is $C_{6-20}$ aryl, or $C_{6-20}$ aryl substituted with one or more $R^{1-6}$;

$R^{1-6}$ is independently halogen, $C_{1-6}$ alkyl, or $C_{1-6}$ alkyl substituted with one or more $R^{1-6-1}$;

$R^{1-6-1}$ is independently halogen;

m is 0;

$R^3$ is $-OR^{31}$ or $-NR^{33}R^{34}$;

$R^{31}$, $R^{33}$ and $R^{34}$ are independently $C_{1-6}$ alkyl substituted with one or more $R^{31-1}$;

$R^{31-1}$ is independently "5-7 membered heterocycloalkyl containing 1-3 heteroatoms selected from one or more of O and N" substituted with one or more $R^{d15}$, or $-NR^{e1}R^{e2}$;

$R^{d15}$ is independently $C_{1-6}$ alkyl or halogen;

$R^{e1}$ and $R^{e2}$ are independently $C_{1-6}$ alkyl;

ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;

G is N;

n is 0 or 1;

$R^4$ is independently $C_{1-6}$ alkyl, or $C_{1-6}$ alkyl substituted with one or more $R^{4-1}$;

$R^{4-1}$ is independently cyano;

$R^2$ is $-C(=O)-C(R^a)=C(R^b)(R^f)$;

$R^a$ is independently hydrogen or halogen;

$R^b$ and $R^f$ are independently hydrogen or $C_{1-6}$ alkyl, or $C_{1-6}$ alkyl substituted with one or more $R^{b-1}$;

$R^{b-1}$ is independently $-NR^{10j}R^{10k}$;

$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

In a certain embodiment:

$R^1$ is $C_{6-20}$ aryl, or $C_{6-20}$ aryl substituted with one or more $R^{1-6}$;
$R^{1-6}$ is independently halogen, $C_{1-6}$ alkyl, or $C_{1-6}$ alkyl substituted with one or more $R^{1-6-1}$;
$R^{1-6-1}$ is independently halogen;
m is 0;
$R^3$ is $-OR^{31}$ or $-NR^{33}R^{34}$;
$R^{31}$, $R^{33}$ and $R^{34}$ are independently $C_{1-6}$ alkyl substituted with one or more $R^{31-1}$;
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{e1}$, $R^{e2}$ and $R^{d15}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;
G is N;
n is 0 or 1;
$R^4$ is independently $C_{1-6}$ alkyl, or $C_{1-6}$ alkyl substituted with one or more $R^{4-1}$;
$R^{4-1}$ is independently cyano;
$R^2$ is $-C(=O)-C(R^a)=C(R^b)(R^f)$;
$R^a$ is independently hydrogen or halogen;
$R^b$ and $R^f$ are independently hydrogen or $C_{1-6}$ alkyl.

In a certain embodiment:
the oxygen-containing heterocyclic compound represented by formula I has a structure as follows:

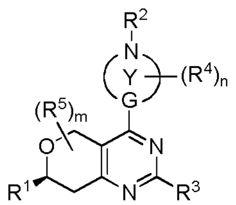
I-2
,
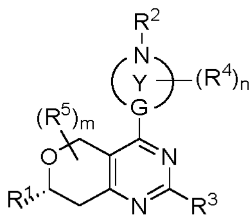
I-3

or "a mixture of

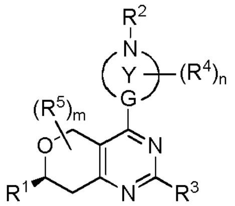
I-2
and
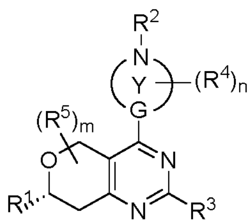
I-3

, with a molar ratio of, for example, 1 : 1".

In a certain embodiment: the oxygen-containing heterocyclic compound represented by formula I has a structure

as follows:

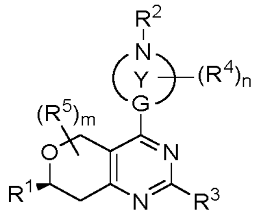

I-2

.

In a certain embodiment:
when $R^1$ is $C_{6-20}$ aryl, then the $C_{6-20}$ aryl may be phenyl or naphthyl, or may be phenyl or 1-naphthyl.

In a certain embodiment:
when $R^1$ is "5-12 membered heteroaryl containing 1-4 heteroatoms selected from one or more of O, S and N", then the "5-12 membered heteroaryl containing 1-4 heteroatoms selected from one or more of O, S and N" may be "9-10 membered heteroaryl containing 1-4 heteroatoms selected from one or more of O, S and N", or may be "9-10 membered heteroaryl containing 1 heteroatom selected from one of O, S and N", or may be isoquinolyl, or may be

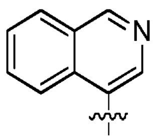

.

In a certain embodiment:
when $R^1$ is $C_{6-20}$ aryl substituted with one or more $R^{1-6}$, then the $C_{6-20}$ aryl may be phenyl or naphthyl, or may be phenyl or 1-naphthyl.

In a certain embodiment:
when $R^1$ is $C_{6-20}$ aryl substituted with one or more $R^{1-6}$, then the more $R^{1-6}$ may be two or three $R^{1-6}$.

In a certain embodiment:
when $R^{1-6}$ is independently halogen, then the halogen may be fluorine, chlorine, bromine or iodine, or may be fluorine or chlorine.

In a certain embodiment:
when $R^{1-6}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{1-6}$ is independently $C_{1-6}$ alkyl substituted with one or more $R^{1-6-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{1-6}$ is independently $C_{1-6}$ alkyl substituted with one or more $R^{1-6-1}$, then the more $R^{1-6-1}$ may be two or three $R^{1-6-1}$.

In a certain embodiment:
when $R^{1-6-1}$ is independently halogen, then the halogen may be fluorine, chlorine, bromine or iodine, or may be fluorine.

In a certain embodiment:
when $R^{1-6}$ is independently $C_{1-6}$ alkyl substituted with one or more $R^{1-6-1}$, then the $C_{1-6}$ alkyl substituted with one or more $R^{1-6-1}$ may be trifluoromethyl.

In a certain embodiment:
when $R^1$ is $C_{6-20}$ aryl substituted with one or more $R^{1-6}$, then the $C_{6-20}$ aryl substituted with one or more $R^{1-6}$ is

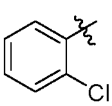, 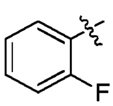, 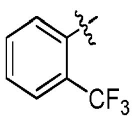, 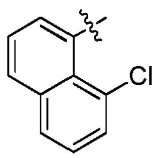 or 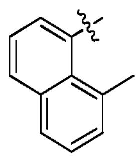.

In a certain embodiment:
when $R^1$ is $C_{6-20}$ aryl substituted with one or more $R^{1-6}$, then the $C_{6-20}$ aryl substituted with one or more $R^{1-6}$ is

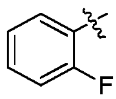, 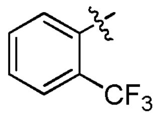, 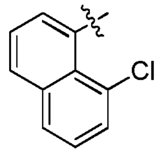 or 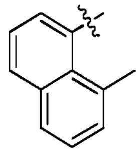.

In a certain embodiment:
when $R^{33}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl, ethyl, n-propyl or isopropyl.

In a certain embodiment:
when $R^{31}$, $R^{33}$ and $R^{34}$ are independently $C_{1-6}$ alkyl substituted with one or more $R^{31-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl, ethyl, n-propyl or isopropyl.

In a certain embodiment:
when $R^{31}$, $R^{33}$ and $R^{34}$ are independently $C_{1-6}$ alkyl substituted with one or more $R^{31-1}$, then the more $R^{31-1}$ may be two or three $R^{31-1}$.

In a certain embodiment:
when $R^{31-1}$ is independently "4-10 membered heterocycloalkyl containing 1-3 heteroatoms selected from one or more of O and N" substituted with one or more $R^{d15}$, then the "4-10 membered heterocycloalkyl containing 1-3 heteroatoms selected from one or more of O and N" may be "5-7 membered heterocycloalkyl containing 1-3 heteroatoms selected from one or more of O and N", or may be "5-7 membered heterocycloalkyl containing 1 heteroatom selected from one of O and N", or may be tetrahydropyrrolyl, or more particularly, tetrahydropyrrole-2-yl.

In a certain embodiment:
when $R^{31-1}$ is independently "4-10 membered heterocycloalkyl containing 1-3 heteroatoms selected from one or more of O and N" substituted with one or more $R^{d15}$, then the more $R^{d15}$ may be two or three $R^{d15}$.

In a certain embodiment:
when $R^{d15}$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{d15}$ is independently halogen, then the halogen may be fluorine, chlorine, bromine or iodine, or may be fluorine.

In a certain embodiment:
when $R^{e1}$ and $R^{e2}$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl or ethyl.

In a certain embodiment:
when $R^{31}$, $R^{33}$ and $R^{34}$ are independently $C_{1-6}$ alkyl substituted with one or more $R^{31-1}$, then the $C_{1-6}$ alkyl substituted with one or more $R^{31-1}$ may be

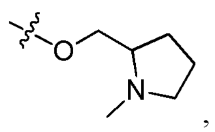,

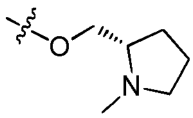, 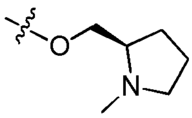, 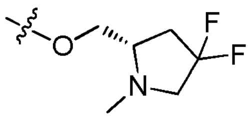, 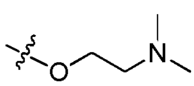, 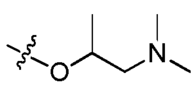,

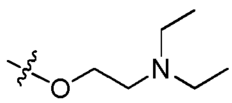, 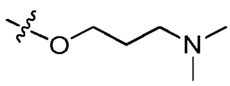 or 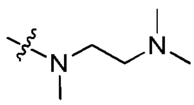.

In a certain embodiment:
when ring Y is a 4-12 membered heterocyclic ring containing 1-4 N atoms, then the 4-12 membered heterocyclic ring containing 1-4 N atoms may be a 6-9 membered heterocyclic ring containing 1-2 N atoms or may be

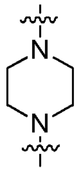, 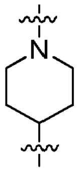, 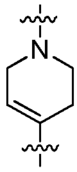 or 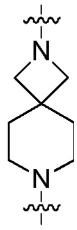

, the upper end of which is connected to $R^2$.

In a certain embodiment:
the oxygen-containing heterocyclic compound represented by formula I has a structure as follows:

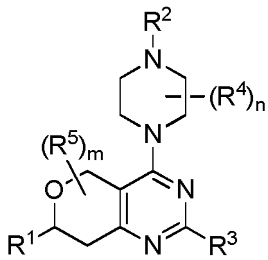

**II**

.

In a certain embodiment:
when $R^4$ is independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^4$ is independently $C_{1-6}$ alkyl substituted with one or more $R^{4-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^4$ is independently $C_{1-6}$ alkyl substituted with one or more $R^{4-1}$, then the more $R^{4-1}$ may be two or three $R^{4-1}$.

In a certain embodiment:
when $R^4$ is independently $C_{1-6}$ alkyl substituted with one or more $R^{4-1}$, then the $C_{1-6}$ alkyl substituted with one or more $R^{4-1}$ may be hydroxymethyl, cyanomethyl or

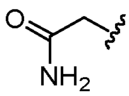;

for example, cyanomethyl or

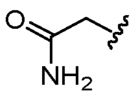.

In a certain embodiment:
when $R^4$ is independently $C_{1-6}$ alkyl substituted with one or more $R^{4-1}$, then the $C_{1-6}$ alkyl substituted with one or more $R^{4-1}$ may be

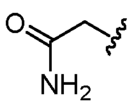.

In a certain embodiment:
when $R^4$ is independently $C_{1-6}$ alkyl substituted with one or more $R^{4-1}$, then the $C_{1-6}$ alkyl substituted with one or more $R^{4-1}$ may be hydroxymethyl or cyanomethyl.

In a certain embodiment:

when $R^4$ is independently $C_{1-6}$ alkyl substituted with one or more $R^{4-1}$, then the $C_{1-6}$ alkyl substituted with one or more $R^{4-1}$ may be hydroxymethyl.

In a certain embodiment:
when $R^4$ is independently $C_{1-6}$ alkyl substituted with one or more $R^{4-1}$, then the $C_{1-6}$ alkyl substituted with one or more $R^{4-1}$ may be cyanomethyl.

In a certain embodiment:
when $R^a$ is independently halogen, then the halogen may be fluorine, chlorine, bromine or iodine, or may be fluorine.

In a certain embodiment:
when $R^b$ and $R^f$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl-C(=O)-, then the $C_{1-6}$ alkyl in the $C_{1-6}$ alkyl-C(=O)- may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl substituted with one or more $R^{b-1}$, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^b$ and $R^f$ are independently $C_{1-6}$ alkyl substituted with one or more $R^{b-1}$, then the more $R^{b-1}$ may be two or three $R^{b-1}$.

In a certain embodiment:
when $R^{10j}$ and $R^{10k}$ are independently $C_{1-6}$ alkyl, then the $C_{1-6}$ alkyl may be $C_{1-4}$ alkyl, or may be methyl, ethyl, n-propyl, isopropyl, n-butyl, isobutyl, sec-butyl or tert-butyl, or may be methyl.

In a certain embodiment:
when $R^{10j}$ and $R^{10k}$ taken together with the N atom to which they are attached form "4-10 membered heterocycloalkyl containing 1-3 heteroatoms selected from one or more of O and N", then the "4-10 membered heterocycloalkyl containing 1-3 heteroatoms selected from one or more of O and N" may be "5-6 membered heterocycloalkyl containing 2 heteroatoms selected from O and N", or may be

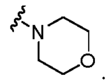.

In a certain embodiment:
the $R^2$ may be CN,

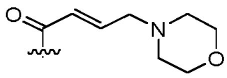, 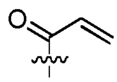, 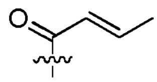, 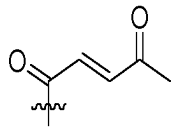,

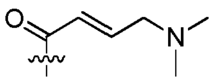, 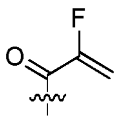, 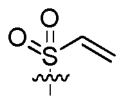 or 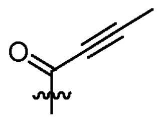.

In a certain embodiment:
the $R^2$ may be CN,

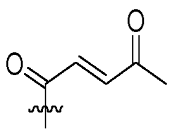.

In a certain embodiment:
the $R^2$ may be

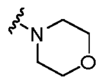, 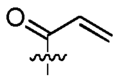, 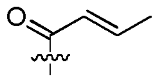, 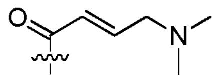, 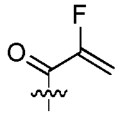,

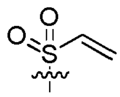 or 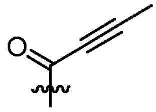.

In a certain embodiment:
the $R^2$ may be

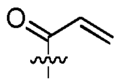, 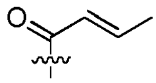, 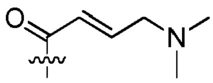, 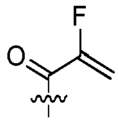, 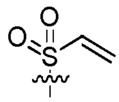

or

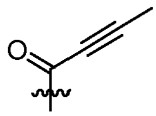.

In a certain embodiment, the oxygen-containing heterocyclic compound represented by formula I has any one of the following structures:

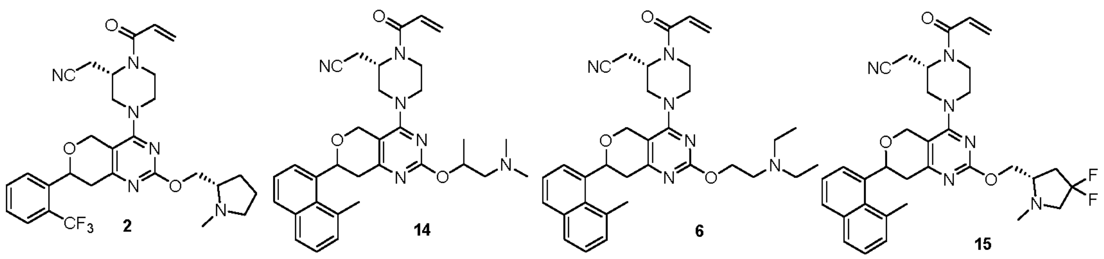

2 14 6 15

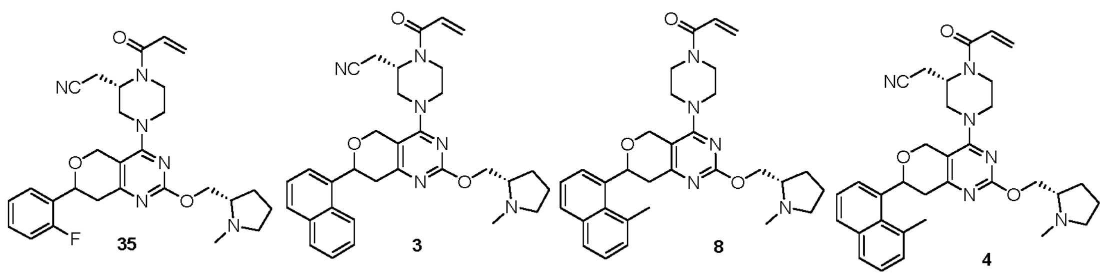

35 3 8 4

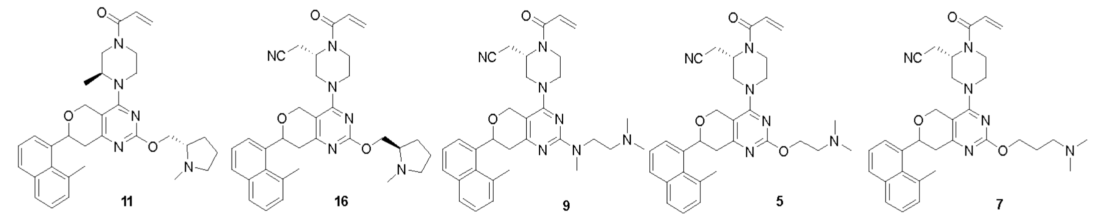

11 16 9 5 7

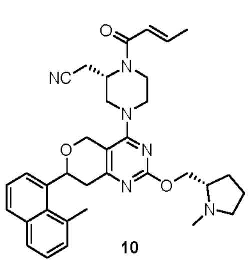
10
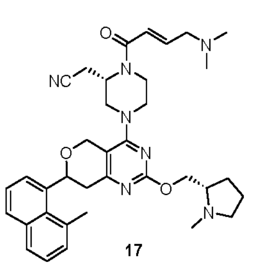
17
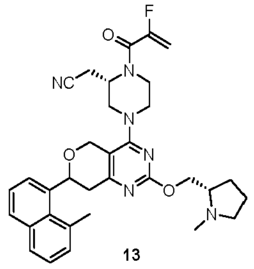
13
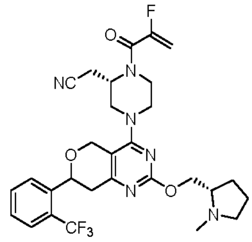
18

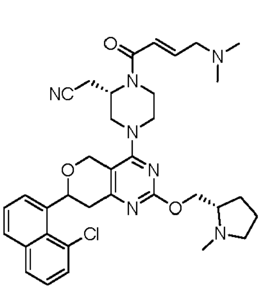
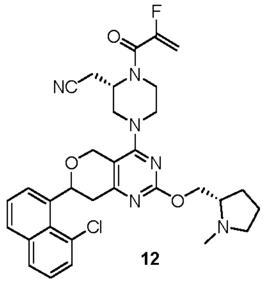
12
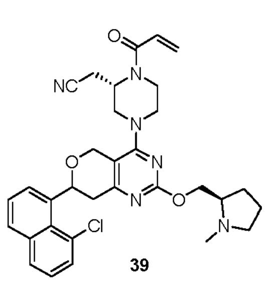
39
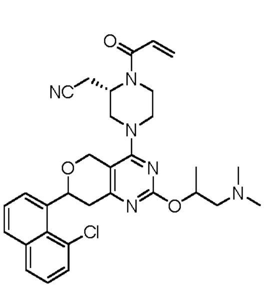

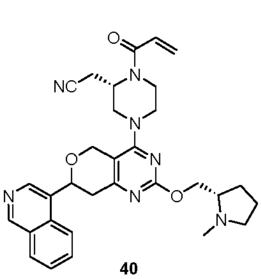
40
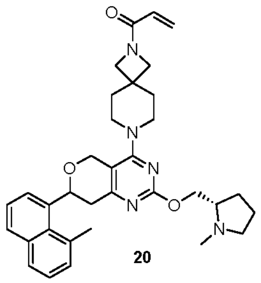
20
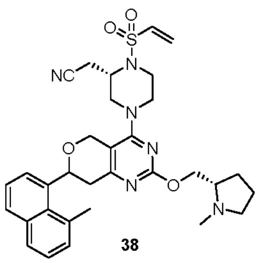
38
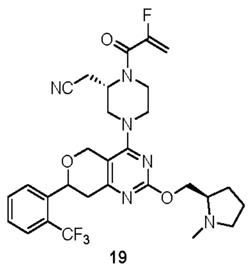
19

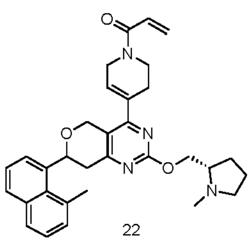
22
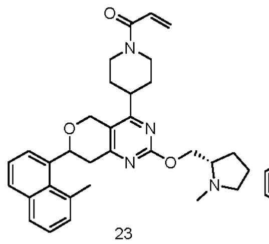
23
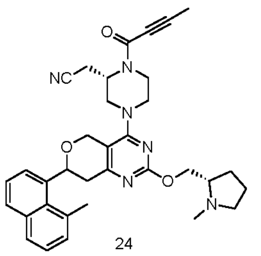
24
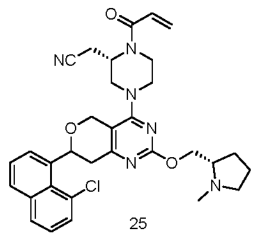
25

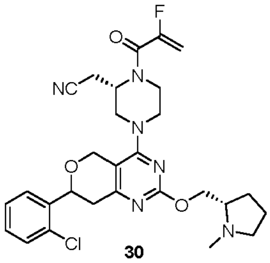
30
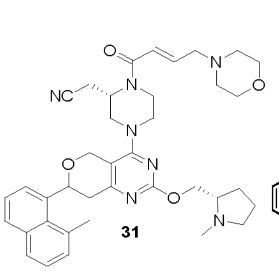
31
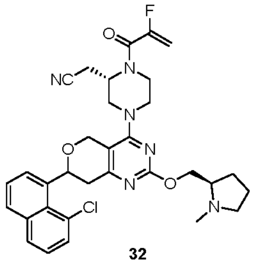
32
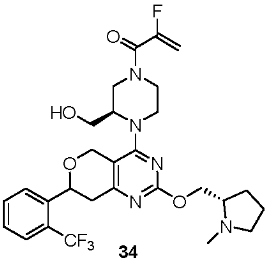
34

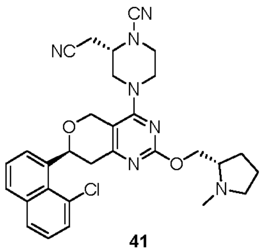
41
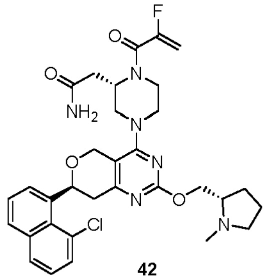
42
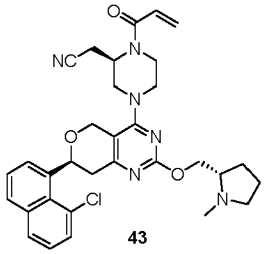
43
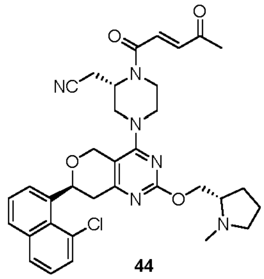
44

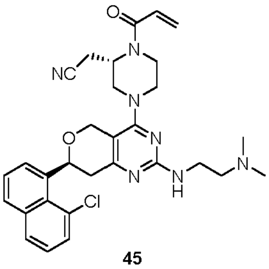
45
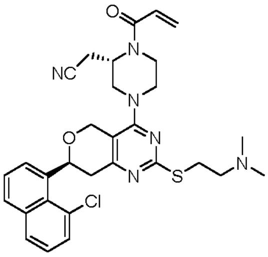
46
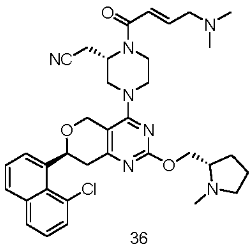
36
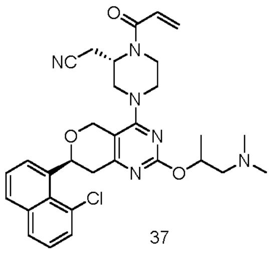
37
.

In a certain embodiment, the oxygen-containing heterocyclic compound represented by formula I is any one of the following compounds:

compound

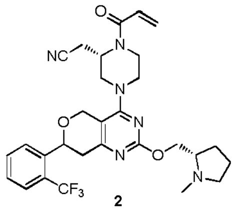
2

, which has a retention time of 0.92 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

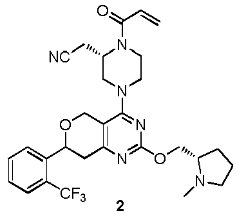
2

, which has a retention time of 2.74 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

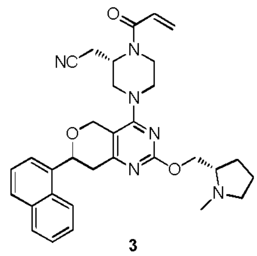

3

, which has a retention time of 0.97 min under the following conditions: equipment: SFC Method Station (Thar, Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar;

compound

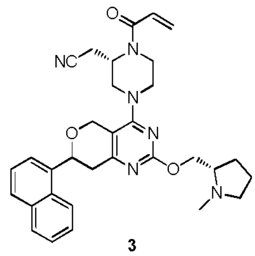

3

, which has a retention time of 2.40 min under the following conditions: equipment: SFC Method Station (Thar, Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar;

compound

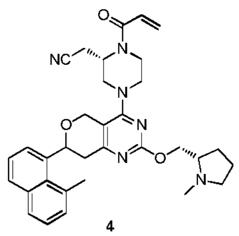

4

, which has a retention time of 0.97 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar;

compound

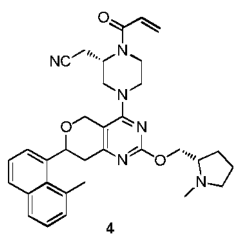

4

, which has a retention time of 1.94 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar;

compound

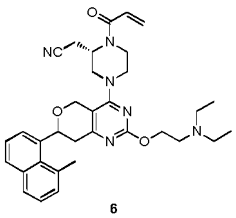

6

, which has a retention time of 1.22 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;

compound

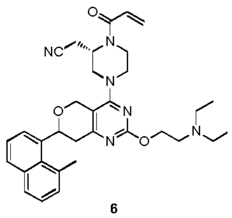

6

, which has a retention time of 2.67 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;

compound

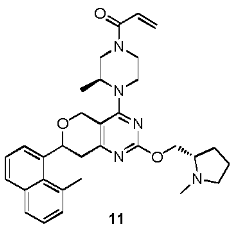

11

, which has a retention time of 3.26 min under the following conditions: instrument: SFC Method Station (Thar, Waters); chromatographic column: R,R-WHELK-O1 4.6 * 100 mm, 5 μm (REGIS); column temperature: 40 °C; mobile phase: COz/(MeOH/CAN = 3 : 2 (0.1% TEA)) = 55/45; flow rate: 4.0 ml/min; wavelength: 254 nm; back pressure: 120 bar;

compound

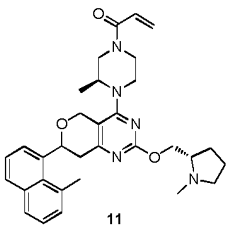

11

,

which has a retention time of 4.16 min under the following conditions: instrument: SFC Method Station (Thar, Waters); chromatographic column: R,R-WHELK-01 4.6 * 100 mm, 5 μm (REGIS); column temperature: 40 °C; mobile phase: COz/(MeOH/CAN = 3 : 2 (0.1% TEA)) = 55/45; flow rate: 4.0 ml/min; wavelength: 254 nm; back pressure: 120 bar;

compound

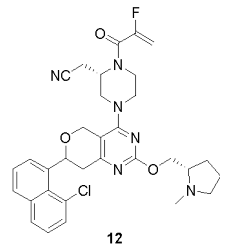

12

, which has a retention time of 1.36 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

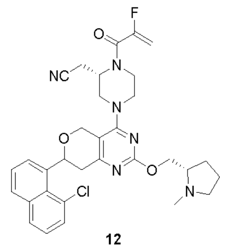

12

, which has a retention time of 2.77 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

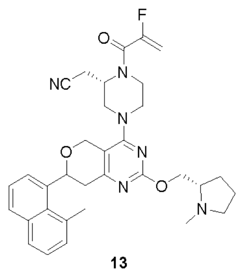

13

, which has a retention time of 1.17 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

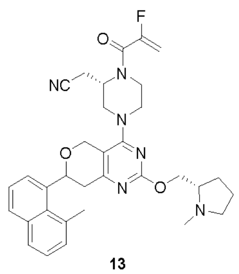

13

, which has a retention time of 2.76 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

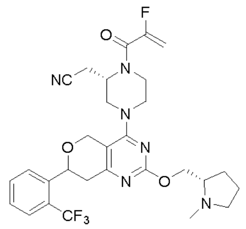

18

, which has a retention time of 0.78 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

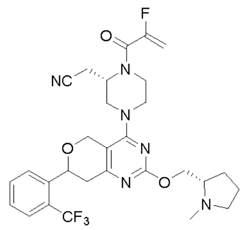

18

, which has a retention time of 2.42 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

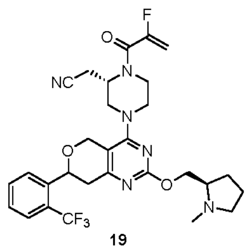

19

, which has a retention time of 0.79 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

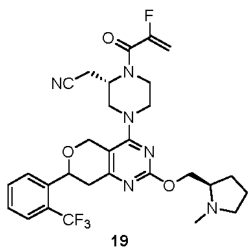

19

, which has a retention time of 2.29 min under the following conditions: instrument: SFC Method Station (Thar,

Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

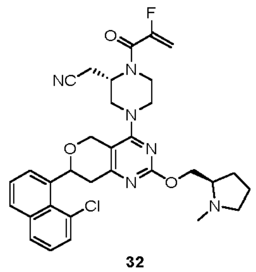

32

, which has a retention time of 1.45 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

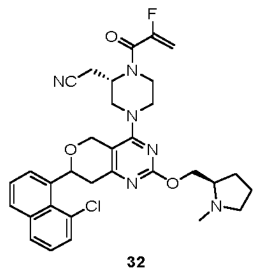

32

, which has a retention time of 2.81 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

In a certain embodiment, the oxygen-containing heterocyclic compound represented by formula **I** is any one of the following compounds:

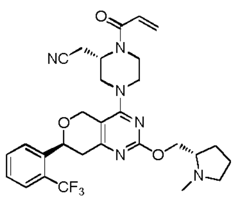 , 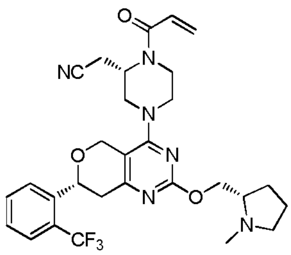 , 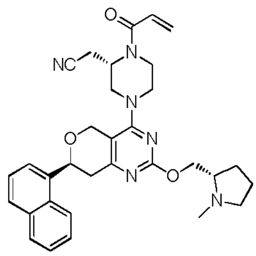 , 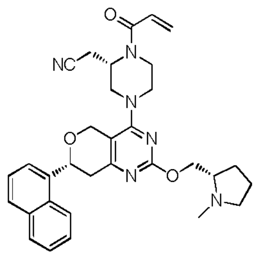 ,

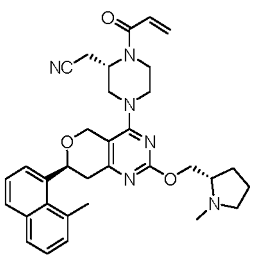 , 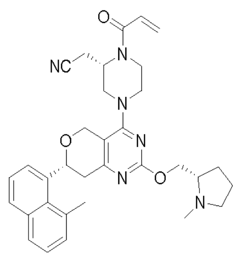 , 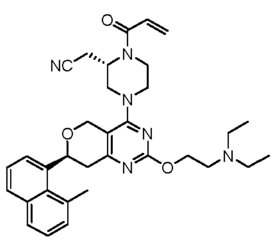 , 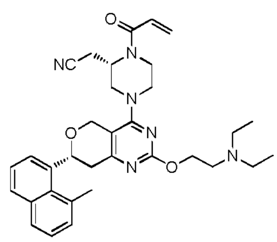 ,

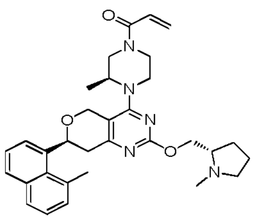,
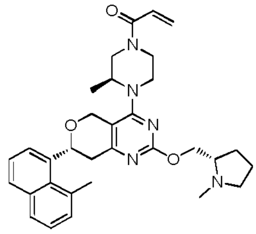,
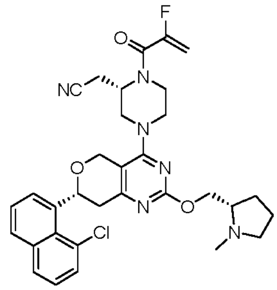,
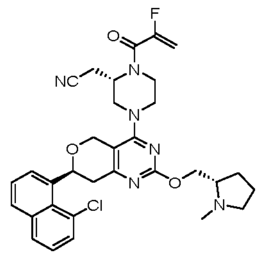,

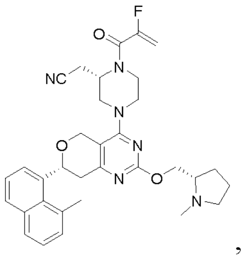,
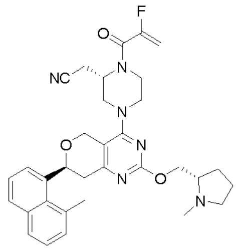,
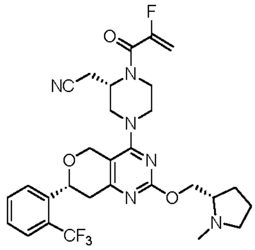,
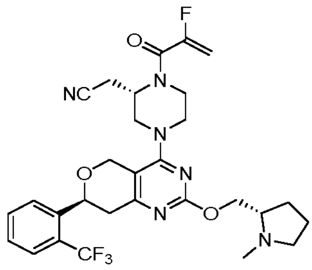,

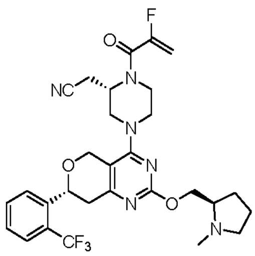,
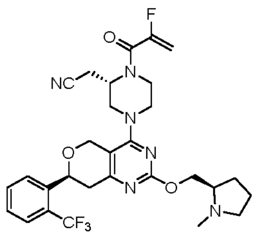,
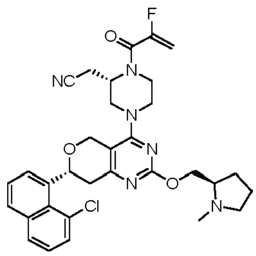,
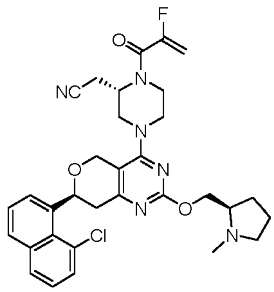.

The present disclosure also provides a method for preparing the above-mentioned oxygen-containing heterocyclic compound represented by formula **I,** the method comprising route one or route two: route one:

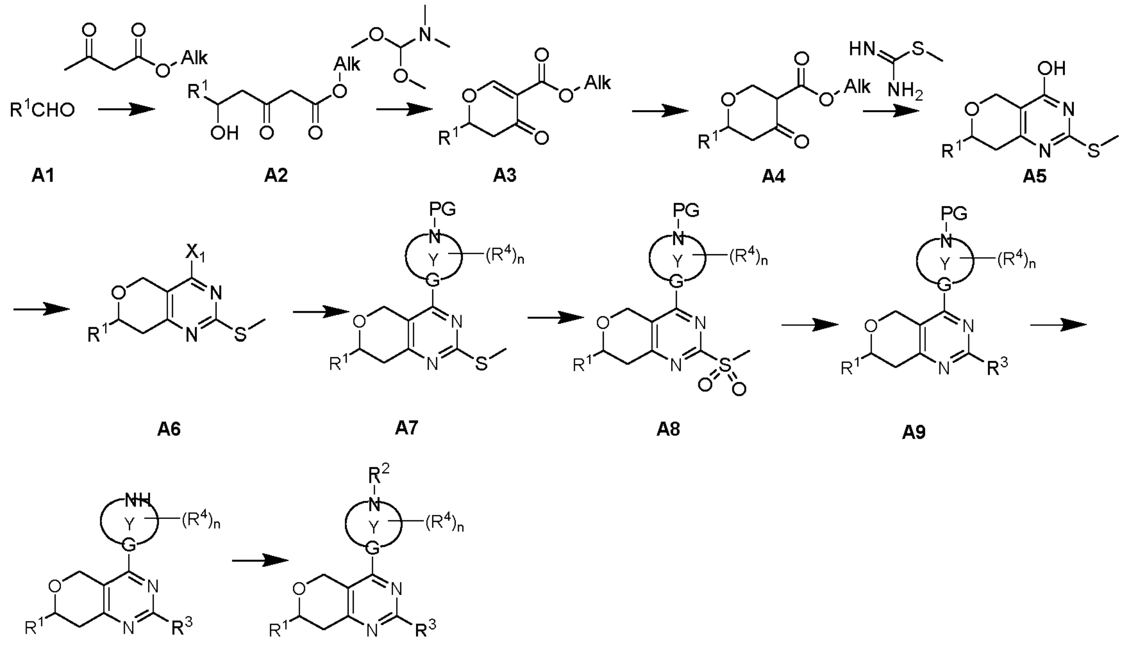

$R^1$, $R^2$, $R^3$, $R^4$, G, n and Y are all as defined above; $X_1$ is a leaving group (for example, OTf or Cl), etc.; Alk is alkyl

(for example, $C_{1-6}$ alkyl); PG is an amino protecting group (for example, Boc or Cbz); route two:

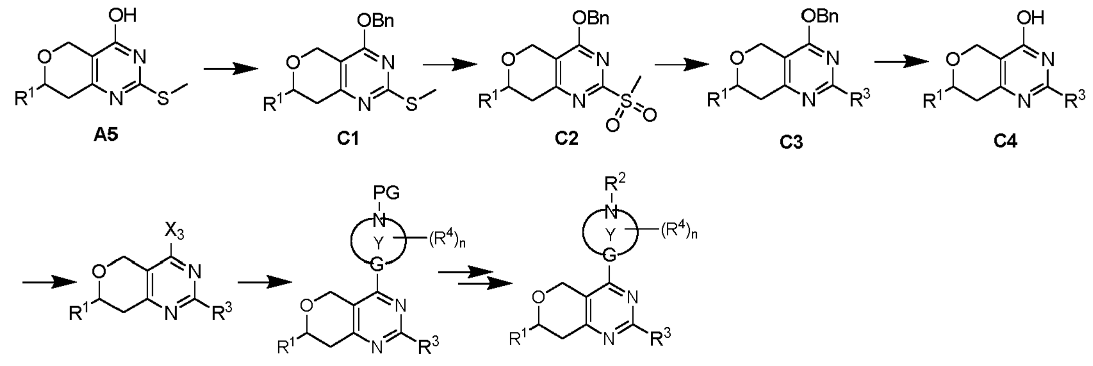

A5 C1 C2 C3 C4

C5 A9 A11

$R^1$, $R^2$, $R^3$, $R^4$, G, n and Y are all as defined above; $X_3$ is a leaving group selected from OTf and Cl PG is an amino protecting group selected from Boc and Cbz.

The route one is described in detail as follows: aldehyde compound A1 is condensed with acetyl acetate to obtain compound A2; A2 is condensed with DMF-DMA to obtain compound A3; A3 is reduced to A4; A5 is obtained from A4 after ring formation; the hydroxyl group in A5 is converted into a leaving group and A6 is obtained; A6 is converted into A7 by means of nucleophilic substitution, coupling, etc.; A7 is oxidized to obtain A8; A8 is further converted into A9; and A9 is deprotected and further converted into A11.

The route two is described in detail as follows: compound A5 is protected by Bn; C1 is oxidized to obtain C2; C2 is converted into C3 by nucleophilic substitution; C3 is subjected to Bn deprotection and converted into C4; the hydroxyl group in C4 is converted into a leaving group and C5 is obtained; C5 is converted into A9 by means of nucleophilic substitution, coupling, etc.; A9 is deprotected and further converted into A11.

The conditions and steps adopted for the chemical reactions involved in the various reaction routes described in the present disclosure all can be carried out with reference to conventional conditions and steps for such reactions in the art, and specific reference may be made to the literatures: R. Larock, Comprehensive Organic Transformations, VCH Publishers (1989); T. W. Greene and P. G. M. Wuts, Protective Groups in Organic Synthesis, 3rd ED., John Wiley and Sons (1999); L. Fieser and M. Fieser, Fieser and Fieser's Reagents for Organic Synthesis, John Wiley and Sons (1994); L. Paquette, ed., Encyclopedia of Reagents for Organic Synthesis, John Wiley and Sons (1995) subsequent versions.

The present application cites the entire content of the above literatures. In addition, other target compounds of the present disclosure can also be obtained from the compounds obtained by the above-mentioned methods through modifying peripheral positions with reference to related methods of the above literatures.

The present disclosure also provides a compound represented by formula A5, A6, A7, A8, A9, A10, C1, C2, C3, C4 or C5:

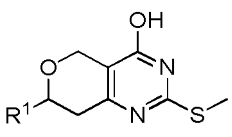
A5

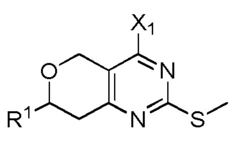
A6

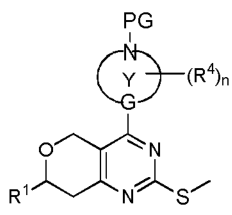
A7

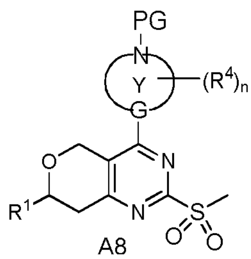
A8

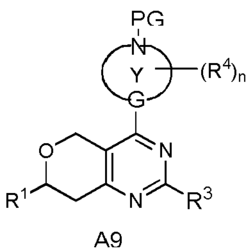
A9

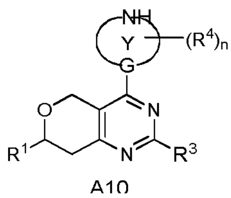
A10

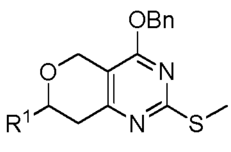
**C1**

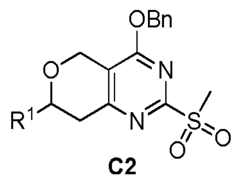
**C2**

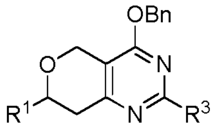
C3,
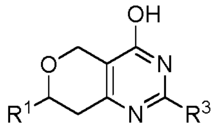
C4,
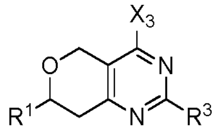
C5;

wherein $R^1$, $R^3$, $R^4$, G, Y and n are as defined above;

$X^1$ and $X^3$ are independently leaving groups selected from OTf and Cl; PG is an amino protecting group selected from Boc and Cbz.

In a certain embodiment, the compound represented by formula A5, A6, A7, A8, A9, A10, C1, C2, C3, C4 or C5 may be any one of the following compounds:

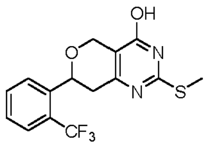
2-f
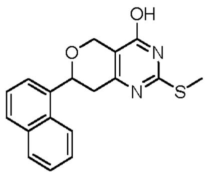
3-f
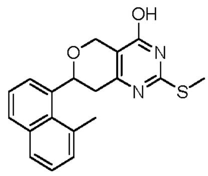
4-f
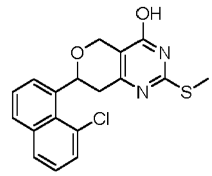
12-f
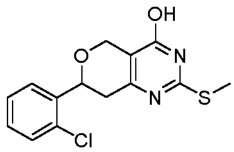
30-f

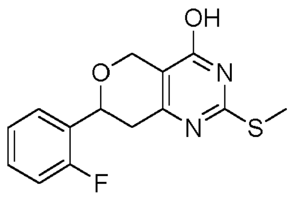
35-f
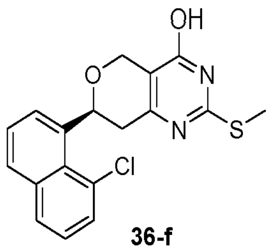
36-f
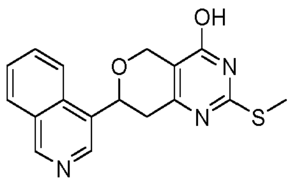
40-f

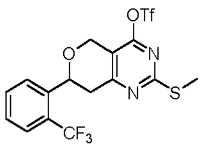
2-e
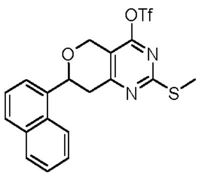
3-e
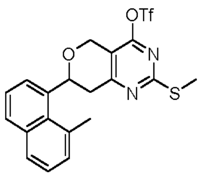
4-e
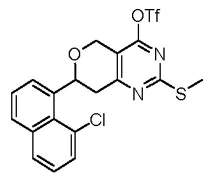
12-e
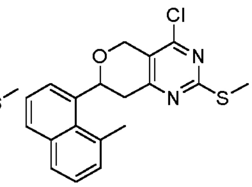
20-e

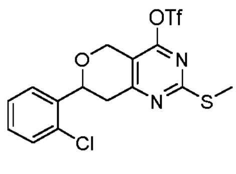
30-e
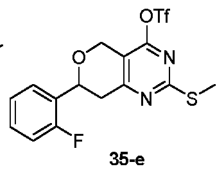
35-e
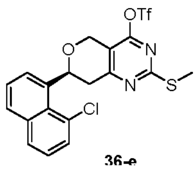
36-e
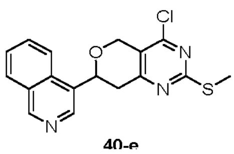
40-e
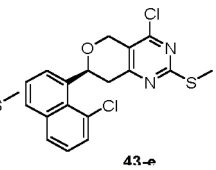
43-e

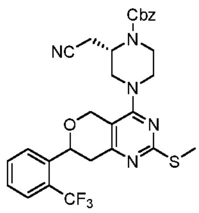
2-d
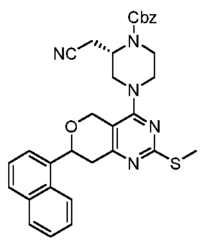
3-d
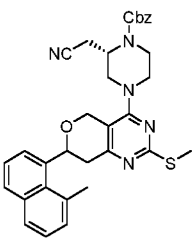
4-d
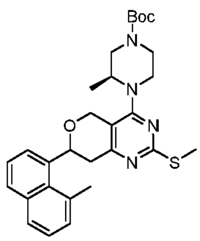
11-d
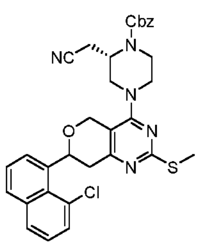
12-d

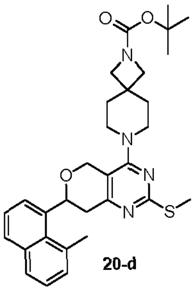
20-d

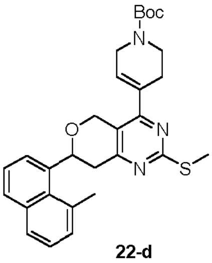
22-d

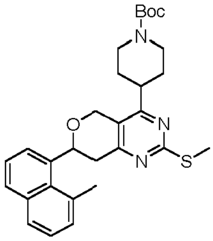
23-d

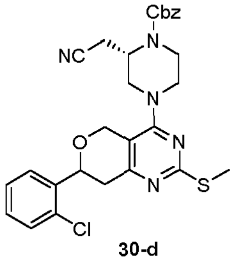
30-d

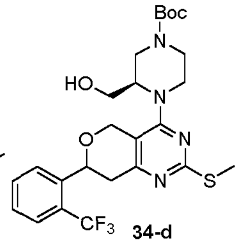
34-d

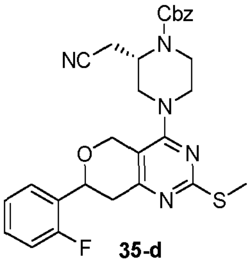
35-d

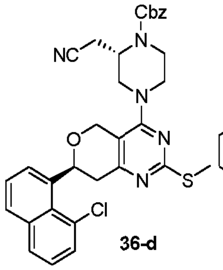
36-d

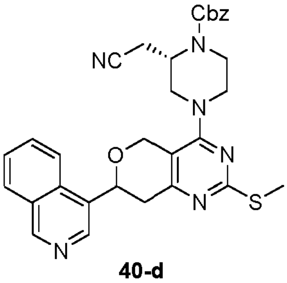
40-d

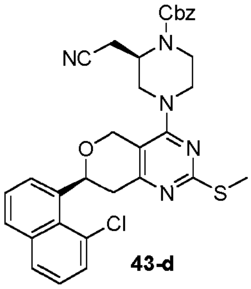
43-d

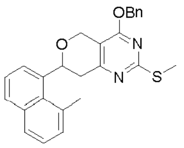
8-g

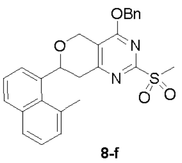
8-f

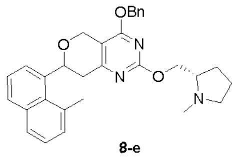
8-e

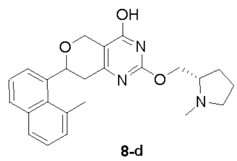
8-d

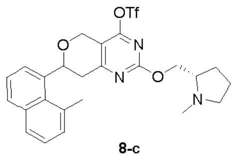
8-c

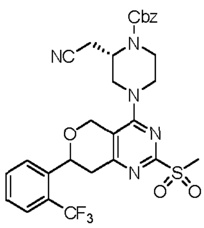
2-c

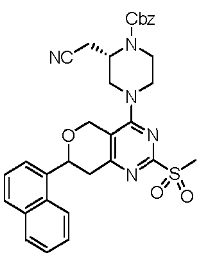
3-c

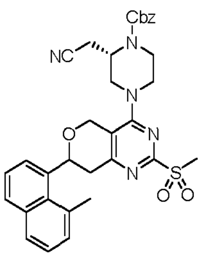
4-c

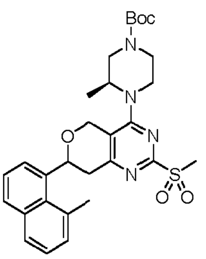
11-c

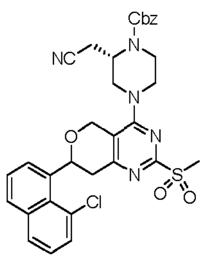
12-c

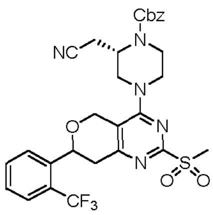
19-c

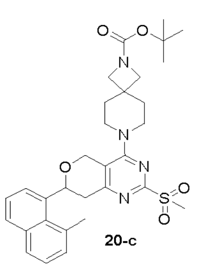
20-c

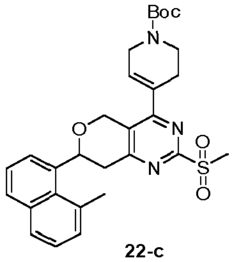
22-c

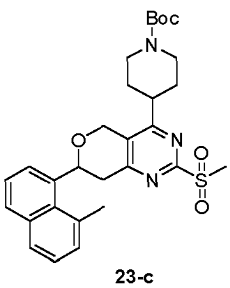
23-c

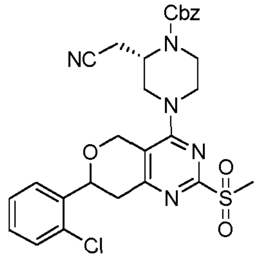

30-c

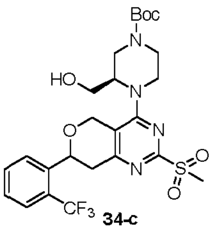

34-c

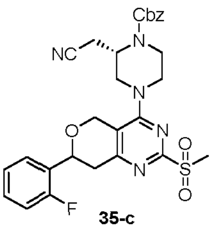

35-c

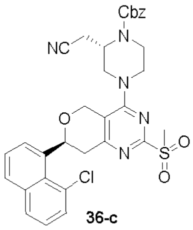

36-c

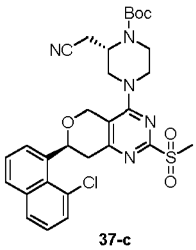

37-c

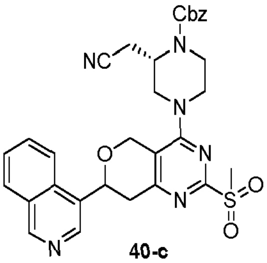

40-c

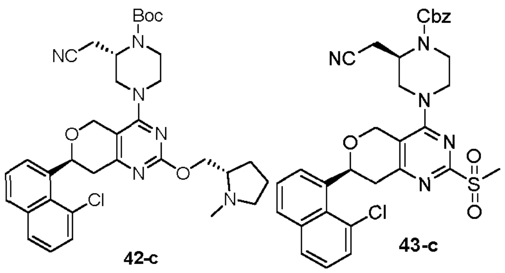

42-c 43-c

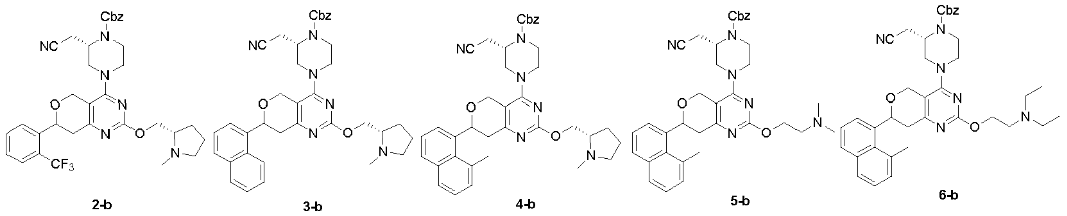

2-b 3-b 4-b 5-b 6-b

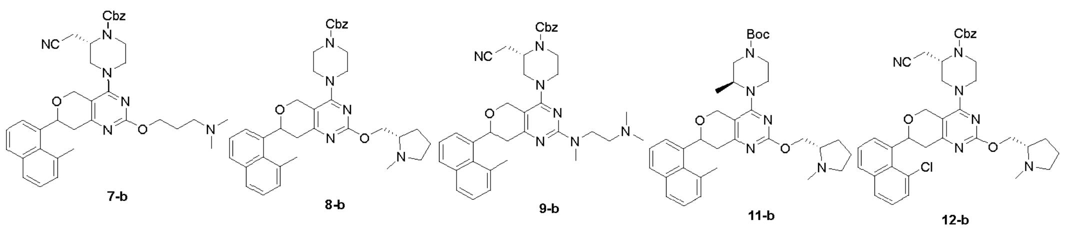

7-b 8-b 9-b 11-b 12-b

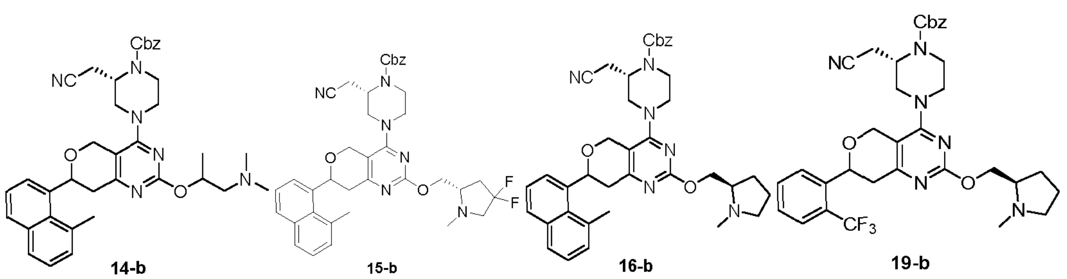

14-b 15-b 16-b 19-b

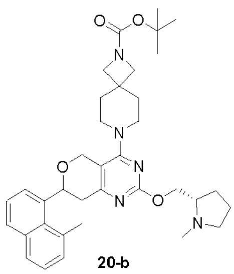
20-b

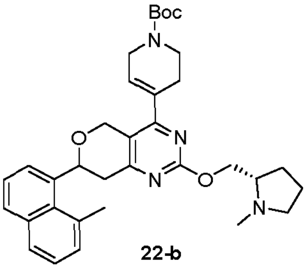
22-b

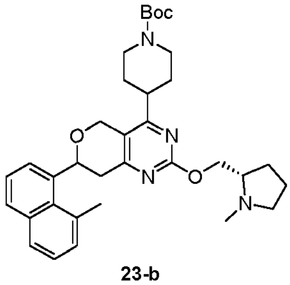
23-b

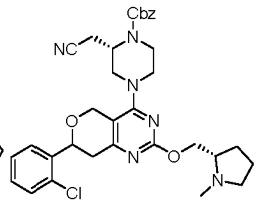
30-b

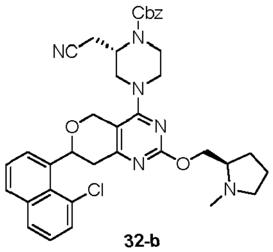
32-b

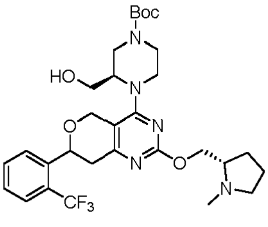
34-b

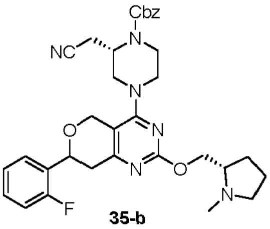
35-b

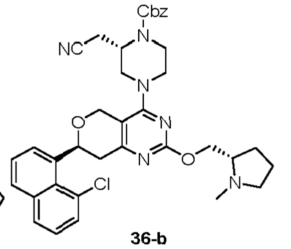
36-b

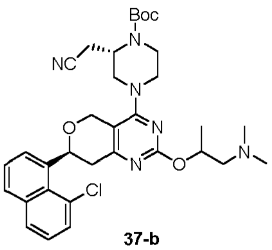
37-b

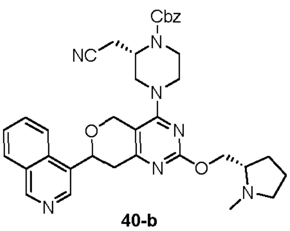
40-b

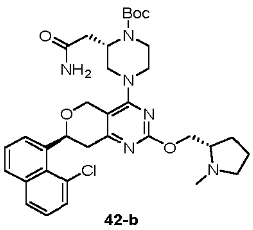
42-b

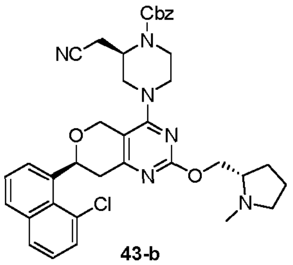
43-b

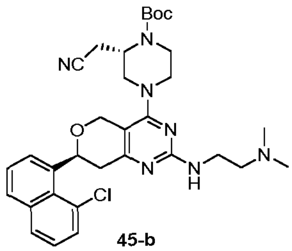
45-b

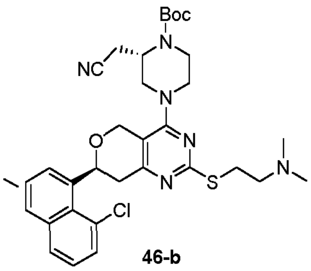
46-b

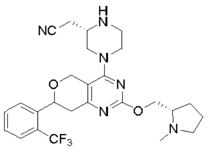
2-a

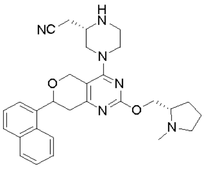
3-a

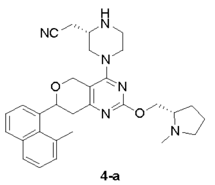
4-a

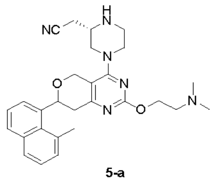
5-a

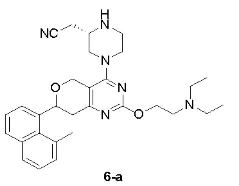
6-a

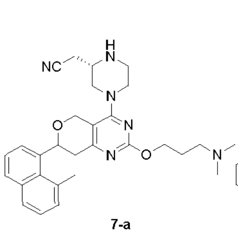
7-a

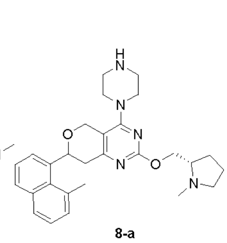
8-a

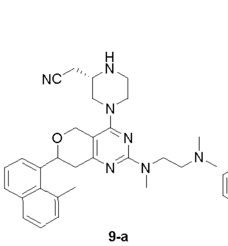
9-a

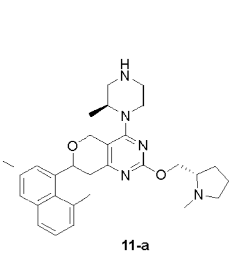
11-a

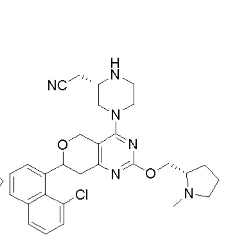
12-a

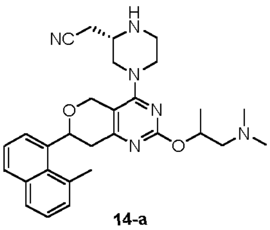
14-a
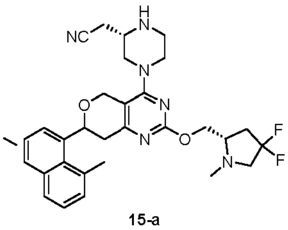
15-a
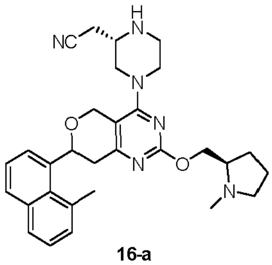
16-a
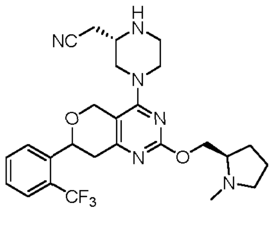
19-a

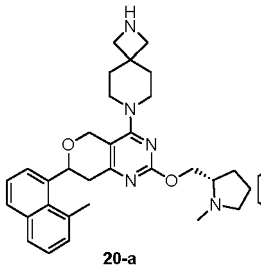
20-a
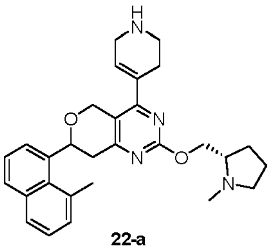
22-a
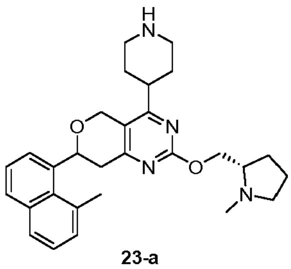
23-a
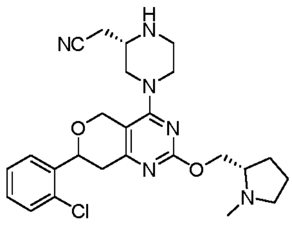
30-a

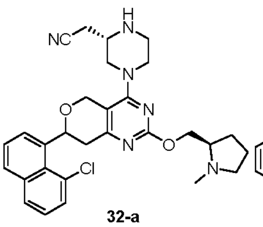
32-a
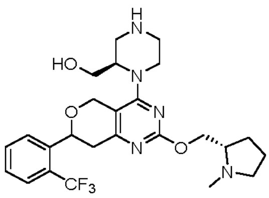
34-a
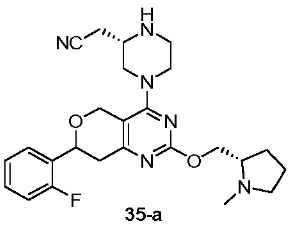
35-a
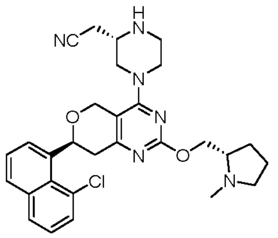
36-a

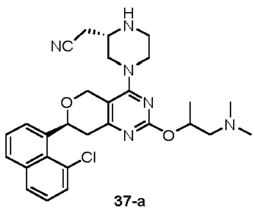
37-a
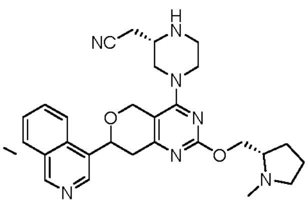
40-a
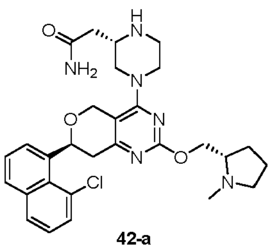
42-a
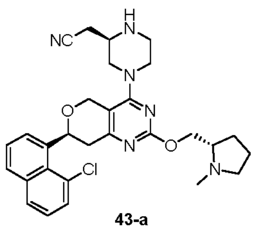
43-a

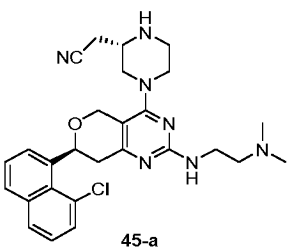
45-a
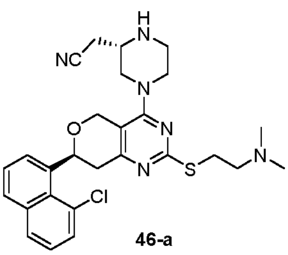
46-a
.

The present disclosure also provides a pharmaceutical composition, comprising substance **A** and a pharmaceutical adjuvant; the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The present disclosure also provides use of substance **A** in the preparation of an RAS inhibitor, wherein the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The RAS comprises, for example, KRAS G12C, HRAS G12C or NRAS G12C mutation; for example, KRAS G12C.

The present disclosure also provides use of substance **A** in the preparation of a medicament, wherein the medicament is used to treat or prevent an RAS-mediated disease;
The substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The RAS comprises, for example, KRAS G12C, HRAS G12C or NRAS G12C mutation; for example, KRAS G12C.

The RAS-mediated disease is, for example, cancer; the cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The present disclosure also provides use of substance **A** in the preparation of a medicament, wherein the medicament is used to treat or prevent cancer;
the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The present disclosure also provides a method for inhibiting RAS, the method comprising administrating to a patient an therapeutically effective amount of substance **A;**
the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The RAS comprises, for example, KRAS G12C, HRAS G12C or NRAS G12C mutation; for example, KRAS G12C.

The present disclosure also provides a method for treating or preventing an RAS-mediated disease, the method comprising administrating to a patient an therapeutically effective amount of substance **A;**
the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof.

The RAS comprises, for example, KRAS G12C, HRAS G12C or NRAS G12C mutation; for example, KRAS G12C.

The RAS-mediated disease is, for example, cancer; the cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The present disclosure also provides a method for treating or preventing cancer, the method comprising administrating to a patient an therapeutically effective amount of substance **A;**
the substance **A** is the above-mentioned oxygen-containing heterocyclic compound represented by formula **I,** a pharmaceutically acceptable salt thereof, a solvate thereof, a solvate of the pharmaceutically acceptable salt thereof, a crystal form thereof or an isotopic compound thereof. The methods for treatment of the human or animal body by surgery or therapy and diagnostic methods practised on the human or animal body are not a part of the present disclosure.

The cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The term "more" refers to 2, 3, 4 or 5.

The term "pharmaceutically acceptable salt" refers to a salt prepared from compounds of the present disclosure with relatively non-toxic, pharmaceutically acceptable acids or bases. When compounds of the present disclosure contain relatively acidic functional groups, base addition salts can be obtained by contacting the neutral form of such compounds with a sufficient amount of pharmaceutically acceptable bases, either in pure solution or a suitable inert solvent. The pharmaceutically acceptable base addition salts include but are not limited to: lithium salt, sodium salt, potassium salt, calcium salt, aluminum salt, magnesium salt, zinc salt, bismuth salt, ammonium salt and diethanolamine salt. When compounds of the present disclosure contain relatively basic functional groups, acid addition salts can be obtained by

contacting the neutral form of such compounds with a sufficient amount of pharmaceutically acceptable acids, either in pure solution or a suitable inert solvent. The pharmaceutically acceptable acids include inorganic acids, and the inorganic acids include but are not limited to: hydrochloric acid, hydrobromic acid, hydroiodic acid, nitric acid, carbonic acid, phosphoric acid, phosphorous acid and sulfuric acid. The pharmaceutically acceptable acids include organic acids, and the organic acids include but are not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, hydrogen tartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)) and amino acid (such as glutamic acid and arginine). When compounds of the present disclosure contain relatively acidic functional groups and relatively basic functional groups, such compounds can be converted into base addition salts or acid addition salts. For details, reference can be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to substance formed by combining compounds of the present disclosure with stoichiometric or non-stoichiometric solvents. The solvent molecules in the solvate may be present in a regular arrangement or a non-ordered arrangement. The solvents include but are not limited to: water, methanol and ethanol.

With regard to term "solvate of a pharmaceutically acceptable salt", the "pharmaceutically acceptable salt" and "solvate" as described above refer to: 1. substance prepared by compounds of the present disclosure and relatively non-toxic, pharmaceutically acceptable acids or bases; and 2. substance formed by combining compounds of the present disclosure with stoichiometric or non-stoichiometric solvents. The "solvate of a pharmaceutically acceptable salt" includes, but is not limited to, the hydrochloric acid monohydrate of compounds of the present disclosure.

The term "stereoisomer" refers to an isomer in which the atoms or atomic groups in a molecule have the same interconnection order but different spatial arrangements, such as cis-trans isomers, optical isomers or atropisomers. These stereoisomers can be separated, purified and enriched by means of asymmetric synthesis methods or chiral separation methods (including but not limited to thin layer chromatography, rotation chromatography, column chromatography, gas chromatography and high-pressure liquid chromatography) or can also be obtained by means of chiral resolution via forming bonds (chemical bonding, etc.) or forming salts (physical bonding) with other chiral compounds, etc.

The term "tautomer" refers to a functional group isomer resulting from the rapid movement of an atom in two positions in a molecule. For example, acetone and 1-propene-2-ol can be converted into each other by the rapid movement of hydrogen atoms on oxygen and $\alpha$-carbon.

The term "crystal form" refers to substance in which ions or molecules are arranged strictly and periodically in a three-dimensional space according to a certain way and are repeated regularly and periodically at a certain interval; and since the periodic arrangements are different, there can be multiple crystal forms, which is also known as polymorphism.

The term "isotopic compound" refers to a compound in which one or more atoms are substituted with one or more atoms having a specific atomic mass or mass number. Examples of isotopes that can be incorporated into compounds of the present disclosure include, but are not limited to, isotopes of hydrogen, carbon, nitrogen, oxygen, fluorine, sulfur and chlorine (e.g., 2H, 3H, 13C, 14C, 15N, 18O, 17O, 18F, 35S and 36Cl). The isotopic compounds of the present disclosure can generally be prepared by substituting non-isotopically-labeled reagents with isotopically-labeled reagents according to the methods described herein.

When any variable (such as $R^{1-6}$) appears multiple times in the definition of a compound, the definition of the variable in each position is irrespective of that in the other positions, and these definitions of the variable are independent from and do not interfere with each other. Therefore, if a group is substituted with one, two or three $R^{1-6}$ groups, the group may be substituted with up to three $R^{1-6}$ groups, and the definition of $R^{1-6}$ in this position is independent from that in the other positions. In addition, a combination of substituents and/or variables are only allowed if the combination produces a stable compound.

The term "halogen" refers to fluorine, chlorine, bromine or iodine.

The term "alkyl" refers to straight or branched alkyl having specified number of carbon atoms. Examples of alkyl include methyl, ethyl, n-propyl, isopropyl, n-butyl, tert-butyl, isobutyl, sec-butyl, n-pentyl, n-hexyl, n-heptyl, n-octyl and similar alkyl groups.

The term "alkoxy" refers to group -O-$R^X$, wherein $R^X$ is alkyl as defined above.

The term "cycloalkyl" refers to saturated monocyclic alkyl, preferably saturated monocyclic alkyl having 3-7 ring carbon atoms, more preferably 3-6 carbon atoms, for example, cyclopropyl, cyclobutyl, cyclopentyl or cyclohexyl.

The term "heterocycloalkyl" refers to a saturated monocyclic group having heteroatoms, preferably a 3-7 membered saturated monocyclic group containing one, two or three ring heteroatoms independently selected from N, O or S. Examples of heterocycloalkyl include: pyrrolidinyl, tetrahydrofuryl, tetrahydropyranyl, tetrahydrothienyl, tetrahydropyridyl, tetrahydropyrrolyl, azetidinyl, thiazolidinyl, oxazolidinyl, piperidinyl, morpholinyl, thiomorpholinyl, piperazinyl, azepanyl, diazepanyl, oxazepanyl, etc. Preferred heterocyclyl is morpholin-4-yl, piperidin-1-yl, pyrrolidin-1-yl,

thiomorpholin-4-yl and 1,1-dioxo-thiomorpholin-4-yl.

The term "saturated heterocyclic ring" refers to a saturated cyclic group having heteroatoms and can be a monocyclic ring, a bridged ring or a spiro ring. The monocyclic "saturated heterocyclic ring" is the "heterocycloalkyl" as described above.

The term "partly saturated heterocyclic ring" refers to a partially saturated cyclic group having heteroatoms, which is neither fully saturated nor aromatic and can be a monocyclic ring, a bridged ring or a spiro ring. Examples of "partly saturated heterocyclic ring" include: pyranoid ring and 1,2,5,6-tetrahydropyridine.

The term "aryl" refers to an aromatic group composed of carbon atoms, in which each ring is aromatic, and examples include phenyl or naphthyl.

The term "heteroaryl" refers to an aromatic group containing heteroatoms, in which each ring is aromatic; preferred heteroaryl is an aromatic 5-6 membered monocyclic ring or 9-10 membered bicyclic ring containing 1, 2 or 3 heteroatoms independently selected from nitrogen, oxygen or sulfur, for example, furyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzoimidazolyl, indolyl, indazolyl, benzothiazolyl, benzoisothiazolyl, benzoxazolyl, benzoisoxazolyl, quinolyl, isoquinolyl, etc.

The term "pharmaceutical adjuvant" refers to excipients and additives used in the production of drugs and formulation of prescriptions, and is all substances contained in a pharmaceutical preparation except for active ingredients. Reference can be made to Pharmacopoeia of the People's Republic of China (2015 edition, four volumes), or Handbook of Pharmaceutical Excipients (Raymond C Rowe, 2009 Sixth Edition).

The term "treat/treating/treatment" refers to therapeutic therapy. With regard to specific conditions, "treat/treating/treatment" refers to: (1) alleviating one or more biological manifestations of a disease or condition; (2) interfering with (a) one or more points in the biological cascade resulting from or caused by a condition or (b) one or more of biological manifestations of a condition; (3) improving one or more symptoms, impacts or side effects related to a condition, or one or more symptoms, impacts or side effects related to a condition or the treatment thereof; or (4) alleviating a condition or one or more biological manifestations of the condition.

The term "prevent/preventing/prevention" refers to a reduction in the risk of acquiring or developing a disease or disorder.

The term "therapeutically effective amount" refers to an amount of a compound that is sufficient to effectively treat the diseases or conditions described herein when administered to a patient. The "therapeutically effective amount" will vary according to compounds, conditions and severity thereof, and age of patients to be treated, but can be adjusted by those of skill in the art as needed.

The term "patient" refers to any animal, preferably mammal, most preferably human, which is about to receive or has received the compound or composition according to examples of the present disclosure. The term "mammal" includes any mammal. Examples of mammals include, but are not limited to, cattle, horses, sheep, pigs, cats, dogs, mice, rats, rabbits, guinea pigs, monkeys, humans, etc., most preferably humans.

On the basis of not departing from common knowledge in the art, the above-mentioned various preferred conditions can be combined in any manner, such that various preferred examples of the present disclosure are obtained.

Reagents and starting materials used in the present disclosure are all commercially available.

In the present disclosure, the room temperature refers to the ambient temperature, which is 10 °C to 35 °C.

The positive effect of the present disclosure lies in: the oxygen-containing heterocyclic compound is expected to treat and/or prevent various Ras-mediated diseases.

## Detailed description of the preferred embodiment

The present disclosure is further illustrated by the following examples, but the present disclosure is not limited thereto. Experimental methods with specific conditions are not indicated in the following examples, but can be chosen according to conventional methods and conditions or commodity instructions.

In the present disclosure, the room temperature refers to the ambient temperature, which is 10 °C to 35 °C. Overnight refers to 8 to 15 hours. Reflux refers to the reflux temperature of the solvent under normal pressure.

The following is a list of abbreviations used in the examples:

DMF N,N-dimethylformamide

HATU 2-(7-azobenzotriazole)-tetramethylurea hexafluorophosphate

EDCI 1-(3-dimethylaminopropyl)-3-ethylcarbodiimide hydrochloride

DIPEA diisopropylethylamine

$Pd(PPh_3)_4$ palladium tetraphenylphosphine

$Pd(dppf)Cl_2$ [1,1'-bis(diphenylphosphino)ferrocene]palladium dichloride dichloromethane complex

LiHMDSbis-(trimethylsilyl) lithium amide

MCPBA m-chloroperoxybenzoic acid

**Example 1** Synthetic route of compound **1**

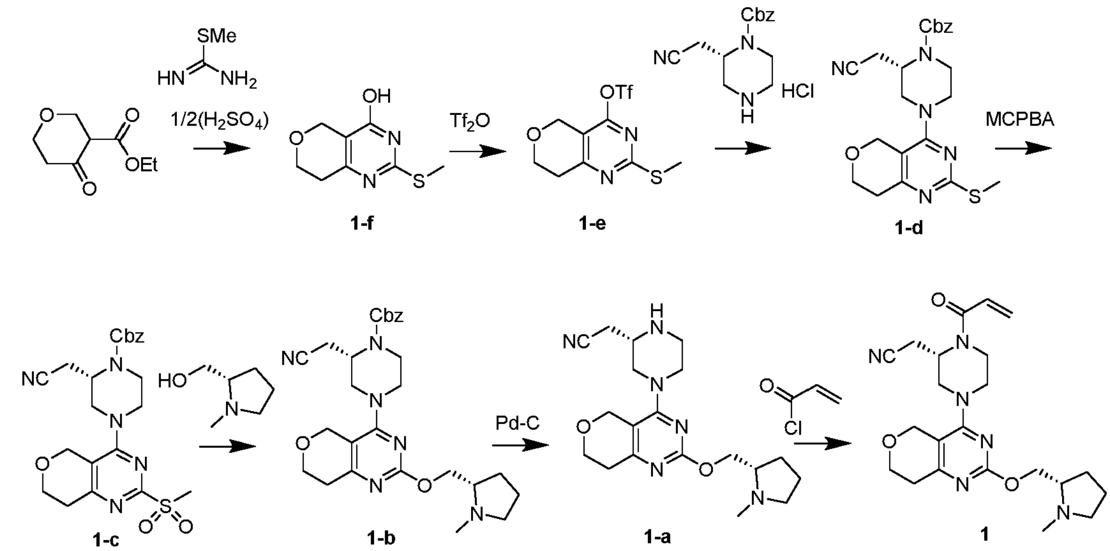


1-f 1-e 1-d

1-c 1-b 1-a 1

Synthesis of compound **1-f**

In an ice-water bath, to a solution of compound ethyl 4-carbonyl-tetrahydropyran-3-carboxylate (1 g, 5.81 mmol) in methanol (20 mL) were added 2-methyl-2-thiourea sulfate (1.45 g, 10.43 mmol) and sodium methylate (1.57 g, 29.07 mmol) respectively. After completion of the addition, the reaction mixture was stirred at room temperature for 3 hours. LCMS monitoring indicated that the reaction was incomplete, and by-products were produced; the pH was adjusted to 5 with 1 M dilute hydrochloric acid; 30 mL of water and 30 mL of ethyl acetate were respectively added, and the mixture was stirred for 10 minutes. The mixture was filtered to give compound **1-f** (684 mg, 59%) as a white solid. LC-MS (ESI): m/z = 199.1 $[M+H]^+$.

Synthesis of compound **1-e**

At room temperature, **1-f** (684 mg, 3.45 mmol) was dissolved in dichloromethane (20 mL), and DIPEA (1.14 mL, 6.91 mmol) was added. Under ice-water bath cooling, trifluoromethanesulfonic anhydride (0.871 mL, 5.18 mmol) was added dropwise with stirring. After completion of the dropwise addition, the mixture was continuously stirred in an ice-water bath for 1 hour. LCMS monitoring indicated that the reaction was complete; the reaction was then quenched with a saturated sodium bicarbonate solution, extracted with dichloromethane (30 mL * 4), dried, concentrated and subjected to a silica gel column to give compound **1-e** (996 mg, 87%) as a light brown oil. LC-MS (ESI): m/z = 331.2 $[M+H]^+$.

Synthesis of compound **1-d**

At room temperature, **1-e** (400 mg, 1.21 mmol) was dissolved in DMF (15 mL), and compound benzyl (S)-2-cyanomethylpiperazine-1-carboxylate hydrochloride (429 mg, 1.45 mmol) and DIPEA (0.6 mL, 3.64 mmol) were respectively added. The reaction mixture was replaced three times with nitrogen and heated to 100 °C and stirred for 1 hour under nitrogen protection. LCMS monitoring indicated that the reaction was complete, and the reaction mixture was cooled to room temperature, quenched with a saturated sodium bicarbonate solution and extracted with ethyl acetate (50 mL*3). The organic layer was washed three times with saturated brine, dried, concentrated and subjected to a silica gel

column to give compound **1-d** (522 mg, 98%) as a white solid. LC-MS (ESI): m/z = 440.4 $[M+H]^+$.

Synthesis of compound **1-c**

At room temperature, **1-d** (522 mg, 1.19 mmol) was dissolved in ethyl acetate (30 mL), and MCPBA (601.5 mg, 2.97 mmol) was added. After completion of the addition, the reaction mixture was stirred at room temperature for 1 hour. TLC monitoring indicated that the reaction was complete; the reaction was then quenched with a saturated sodium bicarbonate solution, extracted with ethyl acetate (50 mL * 2), dried, concentrated and subjected to a silica gel column to give compound **1-c** (552 mg, 99%) as a white solid. LC-MS (ESI): m/z = 472.4 $[M+H]^+$.

Synthesis of compound **1-b**

**1-c** (552 mg, 1.17 mmol) was dissolved in toluene (15 mL); under ice-water bath cooling, N-methyl-L-prolinol (243.7 μL, 2.05 mmol) and sodium tert-butoxide (225 mg, 2.34 mmol) were respectively added. After completion of the addition, under nitrogen protection, the mixture was continuously stirred in an ice-water bath for 30 minutes. TLC monitoring indicated that the reaction was complete; the reaction was then quenched with water, extracted twice with ethyl acetate, dried, concentrated and subjected to a silica gel column to give compound **1-b** (443 mg, 75%) as a white solid. LC-MS (ESI): m/z = 507.5 $[M+H]^+$.

Synthesis of compound **1-a**

**1-b** (150 mg, 0.296 mmol) was dissolved in ethyl acetate (30 mL), and 10% palladium-carbon (450 mg) was added; after replaced three times with hydrogen, the reaction mixture was stirred at room temperature under hydrogen atmosphere for 3 hours. TLC monitoring indicated that the reaction was complete; the product was filtered through diatomite, rinsed with methanol and subjected to rotary evaporation to give **1-a** (74 mg, 67%) as a white solid, which was used directly in the next step without further purification. LC0000000000000000000000000000b-MS (ESI): m/z = 373.4 $[M+H]^+$.

Synthesis of compound **1**

At room temperature, to a solution of **1-a** (74 mg, 0.199 mmol) in dichloromethane (30 mL) were added DIPEA (164 μL, 0.995 mmol) and acryloyl chloride (24 μL, 0.298 mmol) respectively. Under nitrogen atmosphere, at room temperature, the reaction mixture was reacted overnight; LCMS monitoring indicated that the reaction was complete; the reaction was then quenched with a saturated sodium bicarbonate solution and extracted with dichloromethane (30 mL x 3). The organic phase was dried, subjected to rotary evaporation and purified by preparative TLC (DCM : MeOH = 10 : 1) to give compound **1** (20 mg, 24%) as a white solid. LC-MS (ESI): m/z = 427.2 $[M+H]^+$; $^1H$ NMR (400 MHz, $CDCl_3$): $\delta$ 6.65-6.50 (m, 1H), 6.41-6.24 (m, 1H), 6.16-5.69 (m, 1H), 4.78-4.67 (m, 1H), 4.67-4.52 (m, 2H), 4.38 (dd, 1H, *J*= 11.6 Hz, *J*= 4.8 Hz), 4.12-3.93 (m, 3H), 3.59-3.48 (m, 2H), 3.36 (dd, 2H, *J*= 14 Hz, J = 3.6 Hz), 3.26-3.00 (m, 4H), 2.95-2.81 (m, 3H), 2.77 (s, 3H), 2.73-2.58 (m, 2H), 2.26-1.88 (m, 4H).

**Example 2** Synthetic route of compound **2**

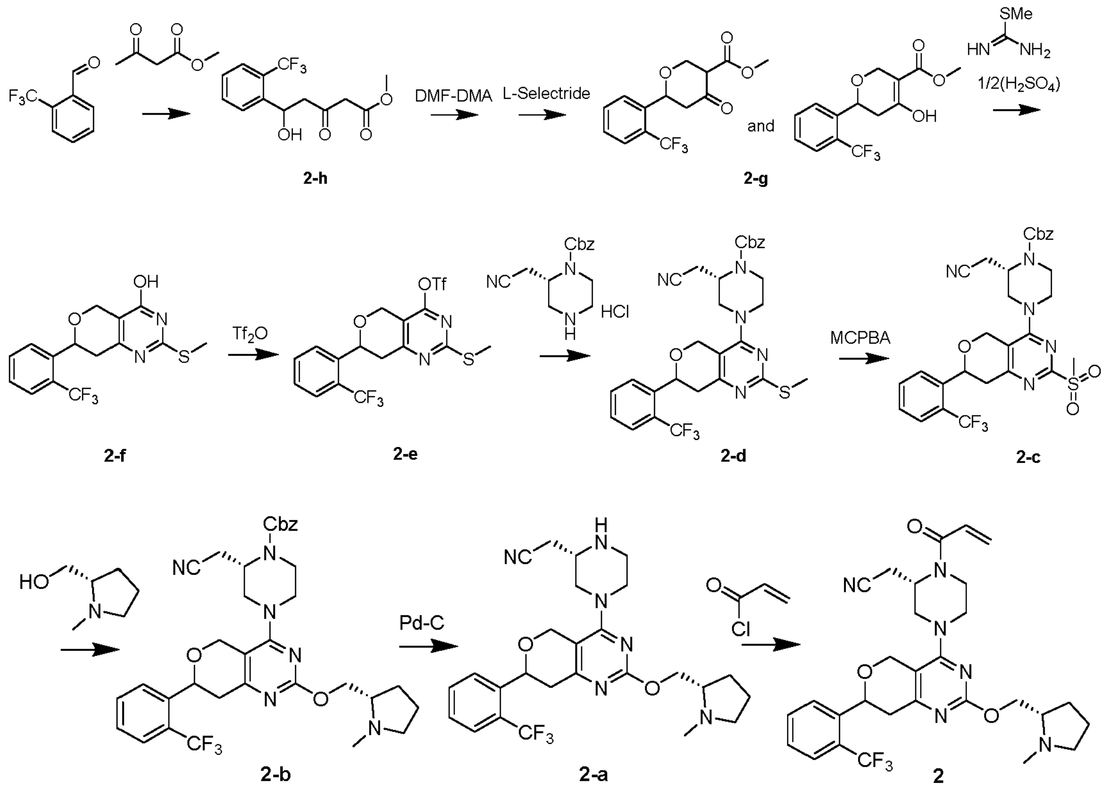


2-h, 2-g (and), 2-f, 2-e, 2-d, 2-c, 2-b, 2-a, 2

Synthesis of compound **2-h**

At room temperature, NaH (60%, 3.6 g, 90.0 mmol) was added to THF (100 mL); under nitrogen atmosphere, methyl acetoacetate (10.4 g, 90.0 mmol) was added at room temperature. Under nitrogen atmosphere, the mixture was stirred for 30 minutes at room temperature, and then n-BuLi (2.5 M, 36 mL, 90.0 mmol) was added dropwise at -15 °C to -10 °C; after completion of the addition, the reaction mixture was kept at this temperature and stirred for 30 minutes, and a solution of 2-trifluoromethylbenzaldehyde (5.2 g, 29.9 mmol) in THF (10 mL) was added dropwise; after completion of the addition, the mixture was stirred at low temperature (-10 °C to 0 °C) for 2 hours. The reaction was quenched by adding a saturated ammonium chloride solution (100 mL) and extracted with ethyl acetate (100 mL*3); the organic phase was washed with saturated brine (100 mL), dried over anhydrous sodium sulfate, filtered and concentrated to give a crude product; the crude product was separated and purified through a flash column chromatography (EA/PE = 1/3) to give compound **2-h** (5.8 g, 67%) as a pale yellow liquid, which was used directly in the next reaction.

Synthesis of compound **2-g**

Under nitrogen atmosphere, at room temperature, to a solution of compound **2-h** (5.8 g, 20.0 mmol) in DCM (120 mL) was added DMF-DMA (3.2 mL, 24.1 mmol). After completion of the addition, the mixture was stirred at room temperature for 45 minutes. $BF_3 \cdot Et_2O$ (3.2 mL, 25.4 mmol) was added and continuously stirred at room temperature for 1 hour. The reaction mixture was diluted by adding dichloromethane (200 mL), washed successively with a saturated $NaHCO_3$ solution (400 mL) and saturated brine (200 mL), dried over anhydrous sodium sulfate, filtered and concentrated to give a crude product. The crude product was dissolved in THF (60 mL); at -78 °C, under nitrogen atmosphere, a solution of lithium tri-sec-butyl borohydride in tetrahydrofuran (30.0 mL, 30.0 mmol) was added dropwise, and the mixture was stirred at this temperature for 1 hour. The reaction was quenched by adding saturated ammonium chloride (200 mL) and extracted with ethyl acetate (100 mL * 3); the organic phase was washed with saturated brine (100 mL * 2) and concentrated to give compound **2-g** (3.8 g, 63%) as a yellow oil. LC-MS (ESI): m/z = 303.1 $[M+1]^+$.

Synthesis of compound **2-f**

Under nitrogen atmosphere, in an ice-water bath, to **2-g** (3.0 g, 10.0 mmol) in methanol (100 mL), sodium methylate (2.7 g, 50.0 mmol) and 2-methyl-2-thiourea sulfate (2.5 g, 8.4 mmol) were successively added. After completion of the addition, the mixture was warmed to room temperature and stirred overnight. The pH was adjusted to 5 with 1 M dilute hydrochloric acid, and the solid was precipitated out and filtered to give compound **2-f** (1.7 g, 50%) as a pale yellow solid.

LC-MS (ESI): m/z = 343.0 $[M+1]^+$.

Synthesis of compound **2-e**

Under nitrogen atmosphere, in an ice-water bath, to **2-f** (1.7 g, 5.0 mmol) in dichloromethane (40 mL), DIPEA (2.1 mL, 12.3 mmol) and trifluoromethanesulfonic anhydride (1.0 mL, 6.3 mmol) were successively added. After completion of the addition, the mixture was stirred at 0 °C for 2 hours. The reaction was quenched by adding saturated sodium bicarbonate solution (50 mL), extracted with DCM (50 mL * 2) and concentrated to give compound **2-e** (1.5 g) which was used directly in the next reaction. LC-MS (ESI): m/z = 474.9 $[M+1]^+$.

Synthesis of compound **2-d**

At room temperature, compound **2-e** (1.5 g, 3.2 mmol) was dissolved in DMF (10 mL), and DIPEA (0.9 mL, 5.6 mmol) and benzyl (S)-2-cyanomethylpiperazine-1-carboxylate hydrochloride (0.8 g, 2.8 mmol) were successively added. The mixture was stirred at 100 °C for 1 hour and then quenched by adding water (100 mL), extracted with ethyl acetate (80 mL * 2) and concentrated; the crude product was separated and purified through a flash column chromatography (EA/PE = 1/1) to give compound **2-d** (0.93 g, 50%) as a white solid. LC-MS (ESI): m/z = 584.0 $[M+1]^+$.

Synthesis of compound **2-c**

Compound **2-d** (0.4 g, 0.69 mmol) was dissolved in ethyl acetate (20 mL), and MCPBA (0.23 g, 1.4 mmol) was added at room temperature. The mixture was stirred at room temperature for 1 hour and then quenched by adding saturated sodium bicarbonate solution (50 mL) and extracted with ethyl acetate (50 mL * 2); the organic phase was concentrated, and the crude product was separated and purified through a flash column chromatography (DCM/MeOH = 9/1) to give compound **2-c** (0.33 g, 78%) as a white solid. LC-MS (ESI): m/z = 616.0 $[M+1]^+$.

Synthesis of compound **2-b**

Under ice-water bath cooling, to compound **2-c** (0.33 g, 0.54 mmol) in toluene (15 mL), N-methyl-L-prolinol (0.1 mL, 0.9 mmol) and t-BuONa (0.1 g, 0.9 mmol) were successively added. Under nitrogen atmosphere, the mixture was stirred in an ice-water bath for 0.5 hours and then quenched by adding water (10 mL) and extracted with ethyl acetate (30 mL * 2); the organic phase was concentrated and the crude product was separated and purified through a flash column chromatography (MeOH/DCM = 1/9) to give compound **2-b** (0.26 g, 74%) as a white solid. LC-MS (ESI): m/z = 651.3 $[M+1]^+$.

Synthesis of compound **2-a**

Compound **2-b** (0.26 g, 0.4 mmol) in methanolic ammonia (7 M, 50 mL) was dissolved and cooled to -78 °C, replaced twice with nitrogen and then added Pd/C (70 mg) and replaced three times with hydrogen. The reaction mixture was warmed to room temperature and stirred under hydrogen for 2 hours. The reaction mixture was filtered and concentrated to give compound **2-a** (0.16 g, 77%). LC-MS (ESI): m/z = 517.2 $[M+1]^+$.

Synthesis of compound **2**

At room temperature, compound **2-a** (0.12 g, 0.23 mmol) was dissolved in DCM (10 mL); DIPEA (75 μL, 0.45 mmol) and acryloyl chloride (25 μL, 0.23 mmol) were successively added. Under nitrogen atmosphere, the mixture was stirred at room temperature for 20 hours and then quenched by adding water (10 mL) and extracted with DCM (50 mL * 3). The organic phase was concentrated; the crude product was separated and purified through a flash column chromatography (MeOH/DCM = 1/9) to give compound **2** (58 mg, 44%) as a white solid. LC-MS (ESI): m/z = 571.3 $[M+1]^+$; $^1H$ NMR (500 MHz, $CD_3OD$): $\delta$ 7.84 (d, 1H, *J*= 7.5 Hz), 7.68-7.76 (m, 2H), 7.53 (t, 1H, *J*= 7.5 Hz), 6.73-6.97 (m, 1H), 6.30 (d, 1H, *J* = 16.5 Hz), 5.85 (d, 1H, *J*= 9.5 Hz), 5.21 (t, 1H, *J*= 11.0 Hz), 4.99-5.02 (m, 2H), 4.25-4.43 (m, 2H), 4.06-4.16 (m, 1H), 3.80-3.96 (m, 1H), 3.43-3.80 (m, 2H), 3.20-3.33 (m, 1H), 2.97-3.19 (m, 4H), 2.81-2.95 (m, 2H), 2.70-2.83 (m, 1H), 2.53(d, 3H, *J*= 4.5 Hz), 2.32-2.45 (m, 1H), 2.06-2.15 (m, 1H), 1.79-1.90 (m, 2H), 1.68-1.77 (m, 1H), 1.31-1.39 (m, 1H).

Synthesis of compounds **2-1** and **2-2**

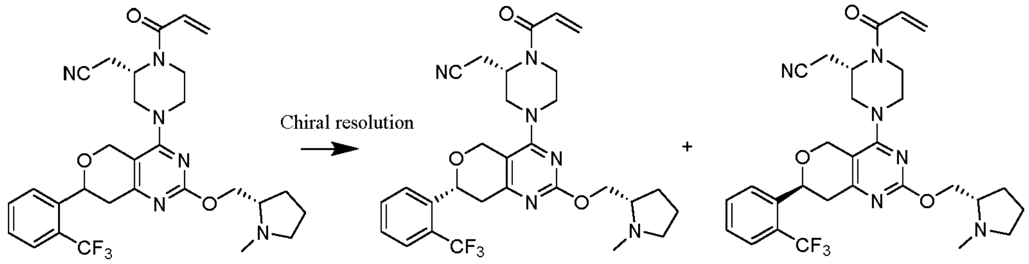


2 one of 2-1 and 2-2 + the other one of 2-1 and 2-2

Compound **2** (29 mg, 0.05 mmol) was purified by chiral resolution to give compound **2-1** (10 mg, 34%) as a white solid and compound **2-2** (10 mg, 34%) as a white solid.

| Chiral analysis conditions | Chiral preparation conditions |
|---|---|
| equipment: SFC Method Station (Thar, Waters)<br>chromatographic column: OD-H 4.6 * 100 mm, 5 μm (Daicel)<br>column temperature: 40 °C<br>mobile phase: $CO_2$/MeOH (0.1% TEA) = 65/35<br>flow rate: 4.0 ml/min<br>wavelength: 254 nm<br>back pressure: 120 bar | instrument: SFC-80 (Thar, Waters)<br>chromatographic column: OD 20 * 250 mm, 10 μm (Daicel)<br>column temperature: 35 °C<br>mobile phase: $CO_2$/MeOH (0.1% TEA) = 45/55<br>flow rate: 80 g/min<br>back pressure: 100 bar<br>detection wavelength: 214 nm<br>cycling time: 6.0 min<br>sample solution: 29 mg dissolved in 8 ml of methanol |
| 2-1: retention time: 0.92 min; d.e.% = 100.0%;<br>2-2: retention time: 2.74 min; d.e.% = 98.0%. | |

**2-1:** LC-MS (ESI): m/z = 571.2 $[M+1]^+$; $^1H$ NMR (400 MHz, $CDCl_3$): $\delta$ 7.75 (d, 1H, *J* = 7.6 Hz), 7.67 (d, 1H, *J* = 7.6 Hz), 7.63 (t, 1H, *J* = 8.0 Hz),7.44 (t, 1H, *J* = 8.0 Hz), 6.54-6.65 (m, 1H), 6.38 (dd, 1H, *J* = 16.4, 1.6 Hz), 5.83 (d, 1H, *J*= 10.0 Hz), 5.14 (dd, 1H, *J* = 10.8, 3.2 Hz), 4.88 (d, 1H, *J*= 14.0 Hz), 4.80 (d, 1H, *J*= 13.6 Hz), 4.51-5.12 (m, 2H), 4.37 (dd, 1H, *J* = 10.8, 5.2 Hz), 4.18 (dd, 1H, *J* = 10.8, 6.4 Hz), 3.84-4.10 (m, 1H), 3.70-3.83 (m, 1H), 3.32 -3.64 (m, 1H), 2.75-3.27 (m, 6H), 2.63-2.72 (m, 1H), 2.48(s, 3H), 2.25-2.32 (m, 1H), 1.98-2.10 (m, 1H), 1.69-1.90 (m, 3H), 1.28-1.39 (m, 1H).

**2-2:** LC-MS (ESI): m/z = 571.2 $[M+1]^+$; $^1H$ NMR (400 MHz, $CDCl_3$): $\delta$ 7.76 (d, 1H, *J* = 7.6 Hz), 7.67 (d, 1H, *J* = 7.6 Hz), 7.63 (t, 1H, *J* = 7.6 Hz),7.44 (t, 1H, *J* = 7.6 Hz), 6.50-6.65 (m, 1H), 6.39 (dd, 1H, *J* = 16.8, 1.6 Hz), 5.83 (d, 1H, *J*= 11.2 Hz), 5.17 (dd, 1H, *J* = 12.0, 4.0 Hz), 4.89 (d, 1H, *J*= 14.0 Hz), 4.78 (d, 1H, *J*= 13.2 Hz), 4.46-5.11 (m, 2H), 4.39 (dd, 1H, *J* = 10.4, 4.8 Hz), 4.16 (dd, 1H, *J* = 10.4, 6.4 Hz), 3.94-4.01 (m, 1H), 3.62-3.84 (m, 1H), 3.38 -3.56 (m, 1H), 2.59-3.16 (m, 7H), 2.47(s, 3H), 2.24-2.33 (m, 1H), 1.98-2.10 (m, 1H), 1.69-1.89 (m, 3H), 1.28-1.38 (m, 1H).

**Example 3** Synthetic route of compound **3**

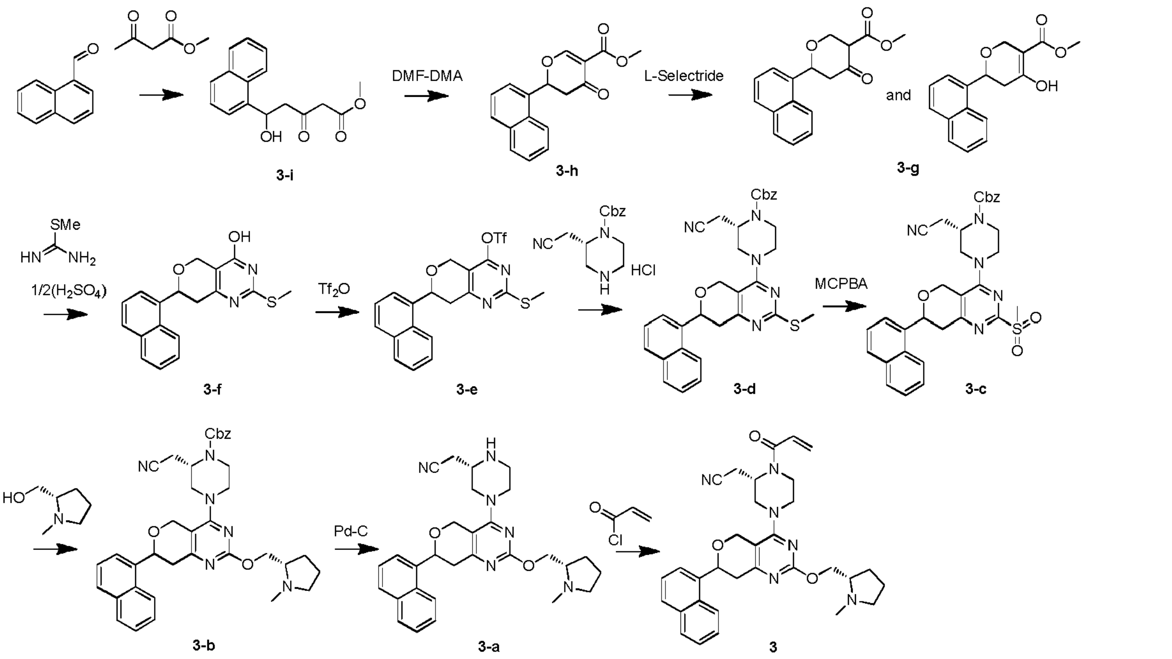


Synthesis of compound **3-i**

At room temperature, NaH (60%, 3.0 g, 75.0 mmol) was added to THF (100 mL); under nitrogen atmosphere, methyl acetoacetate (8 mL, 77.0 mmol) was added at room temperature. Under nitrogen atmosphere, the mixture was stirred at room temperature for 30 minutes, and then n-BuLi (2.5 M, 30.8 mL, 77.0 mmol) was added dropwise at -15 °C to -10 °C. After completion of the addition, the reaction mixture was kept at this temperature and stirred for 30 minutes, and then a solution of compound 1-naphthaldehyde (4.0 g, 25.6 mmol) in THF (10 mL) was added dropwise. After completion of the addition, the mixture was stirred at low temperature (-10 °C to 0 °C) for 2 hours. The reaction was quenched by adding a saturated ammonium chloride solution (100 mL) and extracted with ethyl acetate (100 mL * 3); the organic phase was washed with saturated brine (100 mL), dried over anhydrous sodium sulfate, filtered and concentrated to give a crude product; the crude product was separated and purified through a flash column chromatography (EA/PE = 1/3) to give compound **3-i** (4.5 g, 64%) as a pale yellow liquid.

Synthesis of compound **3-h**

Compound **3-i** (3.3 g, 12.1 mmol) was dissolved in DCM (120 mL), and under nitrogen atmosphere, DMF-DMA (1.6 mL, 12.0 mmol) was added at room temperature. The reaction mixture was stirred at room temperature for 45 minutes, and then $BF_3 \cdot Et_2O$ (1.6 mL, 12.7 mmol) was added. The mixture was stirred at room temperature for 1 hour and then diluted with 200 mL of dichloromethane; the organic phase was washed successively with a saturated $NaHCO_3$ solution (400 mL) and saturated brine (200 mL), dried over anhydrous sodium sulfate, filtered and concentrated to give a crude product, which was separated and purified through a flash column chromatography (EA/PE = 1/3) to give compound **3-h** (3.0 g, 88%) as a pale yellow liquid. LC-MS (ESI): m/z = 283.1 $[M+1]^+$.

Synthesis of compound **3-g**

At -78 °C, under nitrogen atmosphere, to a solution of compound **3-h** (2.3 g, 8.1 mmol) in THF (60 mL) was added dropwise a solution of lithium tri-sec-butyl borohydride in tetrahydrofuran (1 M, 8.3 mL, 8.3 mmol). After completion of the addition, the mixture was stirred at this temperature for 1 hour; the reaction was quenched by adding saturated ammonium chloride (20 mL) and extracted with ethyl acetate (100 mL * 3); the organic phase was washed with saturated sodium chloride and concentrated to give a crude product, which was separated and purified through a flash column chromatography (EA/PE = 1/4) to give compound **3-g** (2.8 g) as a yellow oil. LC-MS (ESI): m/z = 285.1 $[M+1]^+$.

Synthesis of compound **3-f**

In an ice-water bath, to a solution of compound **3-g** (2.8 g, 10.0 mmol) in methanol (100 mL) were successively added sodium methylate (2.7 g, 50.0 mmol) and 2-methyl-2-thiourea sulfate (2.6 g, 8.8 mmol). After completion of the

addition, the reaction mixture was warmed to room temperature and stirred overnight. The pH was adjusted to 5 with a 1 M hydrochloric acid solution; the solid was precipitated out, filtered, washed with water (50 mL * 3) and dried to give crude product **3-f** (1.3 g, 49% for two steps) as a pale yellow solid. LC-MS (ESI): m/z = 325.0 $[M+1]^+$.

Synthesis of compound **3-e**

In an ice-water bath, to a solution of compound **3-f** (0.65 g, 2.0 mmol) in DCM (40 mL) were successively added DIPEA (0.67 mL, 4.1 mmol) and trifluoromethanesulfonic anhydride (0.34 mL, 2.1 mmol). After completion of the addition, the mixture was stirred in an ice-water bath for 2 hours, quenched by adding a saturated sodium bicarbonate solution (50 mL) and extracted with DCM (50 mL * 2); the organic phase was dried over anhydrous sodium sulfate, filtered and concentrated to give compound **3-e** (0.62 g) which was used directly in the next reaction.

Synthesis of compound **3-d**

At room temperature, compound **3-e** (0.62 g, 1.4 mmol) was dissolved in DMF (10 mL), and DIPEA (0.45 mL, 2.8 mmol) and benzyl (S)-2-cyanomethylpiperazine-1-carboxylate hydrochloride (0.41 g, 1.4 mmol) were successively added. After completion of the addition, under nitrogen protection, the mixture was stirred at 100 °C for 1 hour, cooled to room temperature, quenched by adding water (100 mL) and extracted with ethyl acetate (80 mL * 2); the organic phase was washed with saturated brine (100 mL * 3) and concentrated; the crude product was separated and purified through a flash column chromatography (EA/PE = 1/1) to give compound **3-d** (0.5 g, 44% yield for two steps) as a white solid. LC-MS (ESI): m/z = 566.3 $[M+1]^+$.

Synthesis of compound **3-c**

Compound **3-d** (0.5 g, 0.9 mmol) was dissolved in ethyl acetate (20 mL) and MCPBA (0.46 g, 2.7 mmol) was added at room temperature. The mixture was stirred at room temperature for 1 hour and then quenched by adding saturated sodium bicarbonate solution (50 mL), extracted with ethyl acetate (50 mL * 2), filtered and concentrated; the crude product was separated and purified through a flash column chromatography (DCM/MeOH = 9/1) to give solid compound **3-c** (0.38 g, 72%).

Synthesis of compound **3-b**

In an ice-water bath, to a solution of compound **3-c** (0.38 g, 0.63 mmol) in toluene (15 mL) were successively added N-methyl-L-prolinol (0.1 mL, 0.9 mmol) and t-BuONa (0.1 g, 0.9 mmol). After completion of the addition, under nitrogen atmosphere, the mixture was stirred in an ice-water bath for 0.5 hours and then quenched by adding water (10 mL) and extracted with ethyl acetate (30 mL * 2); the organic phase was concentrated; the crude product was separated and purified through a flash column chromatography (MeOH/DCM = 1/9) to give compound **3-b** (0.3 g, 75%) as a white solid. LC-MS (ESI): m/z = 633.3 $[M+1]^+$.

Synthesis of compound **3-a**

A solution of compound **3-b** (0.13 g, 0.2 mmol) in methanolic ammonia (7 M, 50 mL) was cooled to -78 °C, replaced twice with nitrogen, and then was added 10% Pd-C (55 mg) and replaced three times with hydrogen. The reaction mixture was warmed to room temperature and stirred under hydrogen for 2 hours. The reaction mixture was filtered and concentrated to give compound **3-a** (0.1 g, 100%). LC-MS (ESI): m/z = 499.3 $[M+1]^+$.

Synthesis of compound **3**

At room temperature, compound **3-a** (0.1 g, 0.2 mmol) was dissolved in DCM (10 mL), and DIPEA (75 $\mu$L, 0.45 mmol) and acryloyl chloride (25 $\mu$L, 0.23 mmol) were successively added. Under nitrogen atmosphere, the mixture was stirred at room temperature overnight, quenched by adding water (10 mL) and extracted with DCM (50 mL * 3); the organic phase was concentrated; the crude product was separated and purified through a flash column chromatography (MeOH/DCM = 1/9) to give compound **3** (8 mg, 7%) as a white solid. LC-MS (ESI): m/z = 553.3 $[M+1]^+$; $^1$H NMR (500 MHz, $CD_3OD$): $\delta$ 8.07 (d, 1H, *J* = 7.5 Hz), 7.82 (d, 1H, *J* = 9.5 Hz), 7.78 (d, 1H, *J* = 8.5 Hz), 7.50-7.59(m, 1H), 7.36-7.48(m, 3H), 6.62-6.86(m, 1H), 6.19 (d, 1H, *J* = 17.0 Hz), 5.96-6.08 (m, 2 H), 5.73 (d, 1H, *J*= 9.5 Hz), 5.53 (dd, 1H, *J*= 4.5 Hz, *J*= 11.0 Hz), 5.46 (dd, 1H, *J* = 2.5 Hz, *J* = 9.0 Hz), 4.93-5.07 (m, 2H), 4.67-4.76 (m, 1H), 4.43-4.52(m, 2H), 4.35-4.42 (m, 1H), 3.91-4.08 (m, 1H), 3.28-3.64 (m, 1H), 3.26-3.36 (m, 2H), 3.09-3.18 (m, 2H), 2.90-3.02 (m, 2H), 2.78-2.86 (m, 1H), 2.69 (d, 3H, *J*= 12.5 Hz), 2.06-2.18 (m, 1H), 1.86-1.93 (m, 2H), 1.73-1.77 (m, 1H).

Synthesis of compounds **3-1** and **3-2**

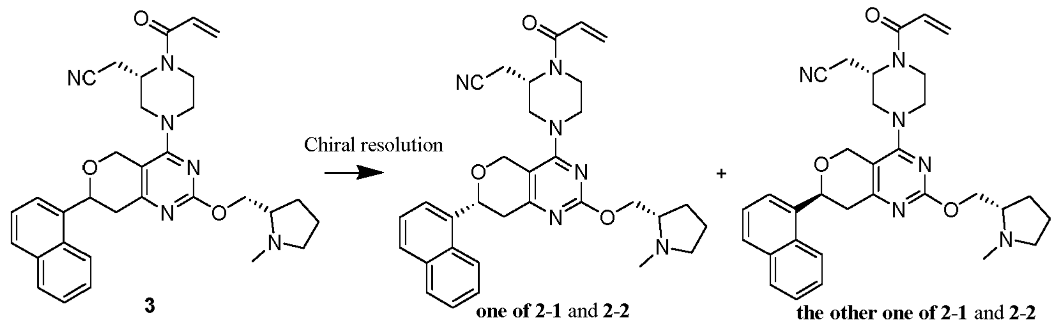


3 one of 2-1 and 2-2 + the other one of 2-1 and 2-2

According to the method for synthesizing compound **3,** compound **3** (170 mg) was synthesized and purified by chiral resolution to give compound **3-1** (40 mg, 24%) as a white solid and compound **3-2** (20 mg, 12%) as a white solid.

| Chiral analysis conditions | Chiral preparation conditions |
|---|---|
| equipment: SFC Method Station (Thar, Waters)<br>chromatographic column: AD-H 4.6 * 100 mm, 5 μm (Daicel)<br>column temperature: 40 °C<br>mobile phase: $CO_2$/ETOH (0.5% TEA) = 55/45<br>flow rate: 4.0 ml/min<br>wavelength: 254 nm<br>back pressure: 120 bar | instrument: SFC-150 (Thar, Waters)<br>chromatographic column: AD 20 * 250 mm, 10 μm (Daicel)<br>column temperature: 35 °C<br>mobile phase: $CO_2$/ETOH (0.5% TEA) = 40/60<br>flow rate: 120 g/min<br>back pressure: 100 bar<br>detection wavelength: 214 nm<br>cycling time: 5.0 min<br>sample solution: 170 mg dissolved in 20 ml of methanol |
| 3-1: retention time: 0.97 min; d.e.% = 93.5%;<br>3-2: retention time: 2.40 min; d.e.% = 99.4%. | |

**3-1:** LC-MS (ESI): m/z = 553.0 $[M+1]^+$; $^1$H NMR (400 MHz, MeOD): $\delta$ 8.06 (d, 1H, *J* = 7.6 Hz), 7.82 (d, 1H, *J* = 7.6 Hz), 7.76 (d, 1H, *J* = 8.4 Hz), 7.57 (d, 1H, *J* = 6.8 Hz), 7.38-7.44 (m, 3H), 6.62-6.86 (m, 1H), 6.19 (d, 1H, *J*= 16.0 Hz), 5.73 (d, 1H, *J* = 10.4 Hz), 5.51 (dd, 1H, *J*= 10.4, 4.0 Hz), 5.02 (d, 1H, *J* = 13.6 Hz), 4.64-4.98 (m, 1H), 4.37-4.59 (m, 1H), 4.19-4.31 (m, 2H), 3.93-3.06 (m, 1H), 3.81-3.91 (m, 1H), 3.57 -3.74 (m, 1H), 3.28-3.50 (m, 1H), 2.89-3.16 (m, 5H), 2.76-2.86 (m, 1H), 2.66-2.75 (m, 1H), 2.42(s, 3H), 2.24-2.33 (m, 1H), 1.95-2.05 (m, 1H), 1.68-1.78 (m, 2H), 1.55-1.67 (m, 1H), 1.21-1.28 (m, 1H).

**3-2:** LC-MS (ESI): m/z = 553.0 $[M+1]^+$; $^1$H NMR (400 MHz, MeOD): $\delta$ 8.07 (d, 1H, *J* = 8.0 Hz), 7.82 (d, 1H, *J* = 7.2 Hz), 7.76 (d, 1H, *J* = 8.0 Hz), 7.54 (d, 1H, *J* = 7.2 Hz), 7.37-7.44 (m, 3H), 6.33-6.85 (m, 1H), 6.19 (d, 1H, *J*= 16.4 Hz), 5.74 (d, 1H, *J*= 10.4 Hz), 5.50 (dd, 1H, *J*= 10.4, 4.0 Hz), 4.97 (d, 1H, *J*= 14.0 Hz), 4.61-4.75 (m, 1H), 4.37-4.57 (m, 1H), 4.21-4.34 (m, 2H), 4.14 (d, 1H, *J*= 13.6 Hz), 3.92-4.06 (m, 1H), 3.70-3.81 (m, 1H), 3.27 -3.42 (m, 1H), 2.89-3.17 (m, 6H), 2.69-2.80 (m, 1H), 2.46(s, 3H), 2.30-2.39 (m, 1H), 1.96-2.12 (m, 1H), 1.70-1.79 (m, 2H), 1.60-1.69 (m, 1H), 1.20-1.27 (m, 1H).

**Example 4** Synthetic route of compound **4**

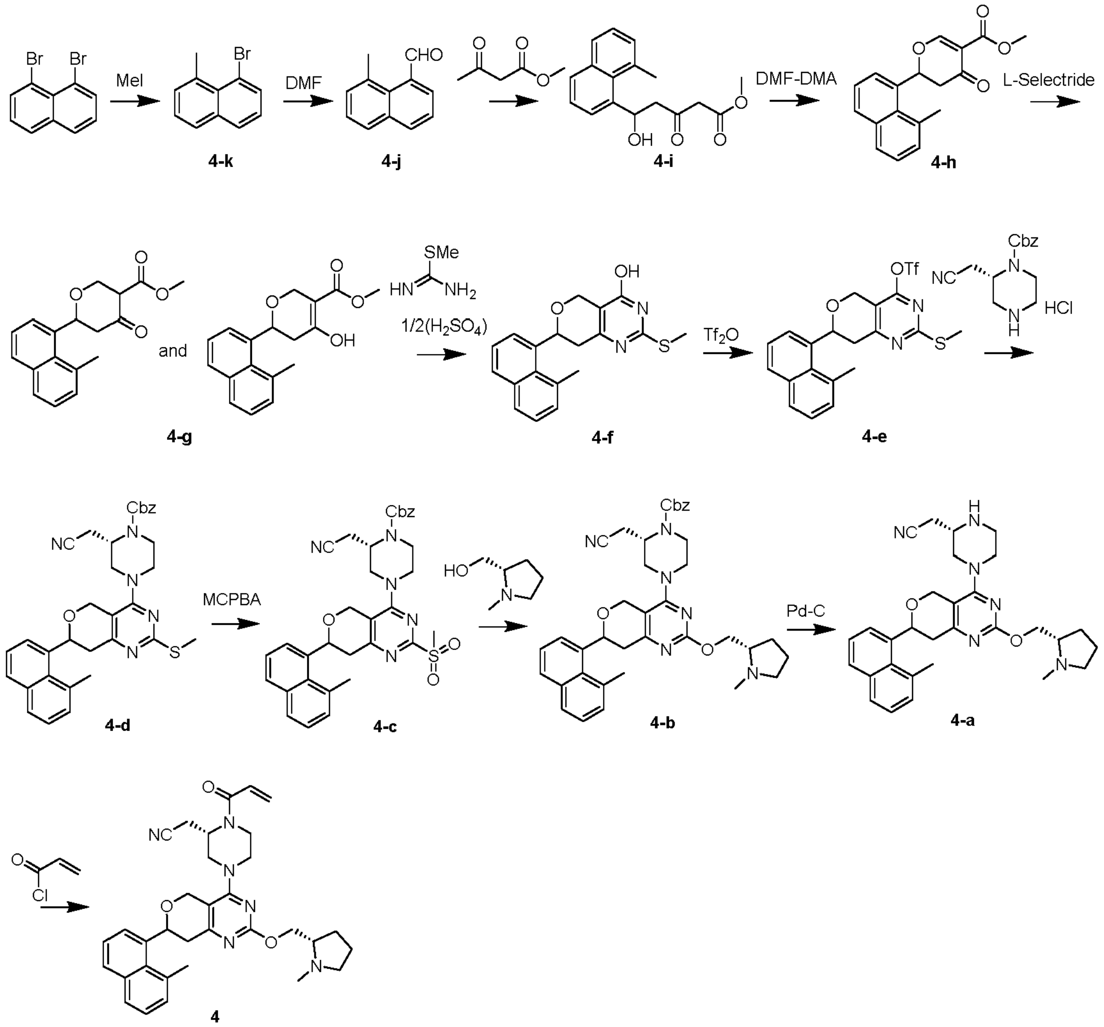


Synthesis of compound **4-k**

A solution of compound 1,8-dibromonaphthalene (5 g, 17.48 mmol) in THF (40 mL) was cooled to -78 °C, and under nitrogen protection, n-BuLi (2.5 M, 7.5 mL, 18.75 mmol) was added dropwise. After completion of the addition, the mixture was stirred at -78 °C for 20 minutes, and then iodomethane (2.2 mL, 35.2 mmol) was added dropwise at -78 °C. After completion of the addition, the reaction mixture was warmed to room temperature and stirred for 1 hour. The reaction mixture was then poured into 50 mL of saturated brine and extracted with ethyl acetate (100 mL * 2); the organic phase was dried over anhydrous sodium sulfate, filtered and concentrated to give a crude product. The crude product was separated and purified through a flash column chromatography (PE) to give compound **4-k** (3.28 g, 85% yield) as a white solid. $^1H$ NMR (500 MHz, $CDCl_3$): $\delta$ 7.85 (d, 1H, $J$ = 7.0 Hz), 7.80 (d, 1H, $J$ = 7.5 Hz), 7.75-7.71 (m, 1H), 7.40-7.33 (m, 2H), 7.23 (t, 1H, $J$= 8.0 Hz), 3.15 (s, 3H).

Synthesis of compound **4-j**

A solution of compound **4-k** (3.28 g, 14.84 mmol) in THF (110 mL) was cooled to -78 °C; under nitrogen protection, n-BuLi (2.5 M, 12 mL, 30 mmol) was added dropwise. After completion of the dropwise addition, the mixture was stirred at -78 °C for 10 minutes, and then DMF (5.8 mL, 74.55 mmol) was added dropwise at -78 °C. After completion of the addition, the reaction mixture was stirred at -78 °C for 30 minutes and then warmed to room temperature and stirred for 2 hours; the reaction was quenched with 20 mL of saturated ammonium chloride solutions, and then added to 100 mL of saturated sodium bicarbonate solution and extracted with ethyl acetate (100 mL); the organic phase was washed with saturated brine (100 mL * 2), dried over anhydrous sodium sulfate, filtered and concentrated to give a crude product. The crude product was separated and purified through a flash column chromatography (EA/PE = 1/10) to give compound **4-j** (1.5 g, 60% yield) as a white solid. LC-MS (ESI): m/z = 171.2 $[M+H]^+$; $^1H$ NMR (400 MHz, $CDCl_3$): $\delta$ 10.85 (s, 1H), 7.97 (dd, 1H, $J_1$ = 1.2 Hz, $J_2$ = 8 Hz), 7.89 (dd, 1H, $J_1$ = 1.6 Hz, $J_2$ = 7.2 Hz), 7.74-7.69 (m, 1H), 7.46 (t, 1H, $J$= 8 Hz), 7.42-7.36 (m, 2H), 2.75

(s, 3H).

Synthesis of compound **4-i**

At room temperature, NaH (60%, 423 mg, 10.58 mmol) was added to 10 mL of THF. Under nitrogen atmosphere, methyl acetoacetate (950 μL, 8.82 mmol) was added at room temperature. Under nitrogen atmosphere, the mixture was stirred at room temperature for 30 minutes, and then n-BuLi (2.5 M, 4.2 mL, 10.5 mmol) was added dropwise at -15 °C to -10 °C. After completion of the addition, the mixture was kept at this temperature for 30 minutes, and then a solution of compound **4-j** (500 mg, 2.94 mmol) in THF (10 mL) was added dropwise. After completion of the addition, the mixture was stirred at low temperature (-10 °C to 0 °C) for 2 hours, and then the reaction was quenched with a saturated ammonium chloride solution (100 mL) and extracted with ethyl acetate (80 mL * 2). The organic phase was washed with saturated brine (100 mL), dried over anhydrous sodium sulfate, filtered and concentrated to give a crude product; the crude product was separated and purified through a flash column chromatography (EA/DCM = 1/10) to give compound **4-i** (806 mg, 96% yield) as a white solid. LC-MS (ESI): m/z = 309.1 $[M+Na]^+$; $^1H$ NMR (400 MHz, $CDCl_3$): δ 7.92 (d, 1H, *J* = 7.2 Hz), 7.81 (dd, 1H, $J_1$ = 1.2 Hz, $J_2$ = 8.4 Hz), 7.77-7.72 (m, 1H), 7.49 (t, 1H, *J*= 7.2 Hz), 7.39-7.33 (m, 2H), 6.47 (d, 1H, *J* = 9.6 Hz), 3.76 (s, 3H), 3.55 (s, 2H), 3.10-2.92 (m, 3H), 2.89 (s, 3H).

Synthesis of compound **4-h**

Compound **4-i** (800 mg, 2.79 mmol) was dissolved in DCM (30 mL) at room temperature; under nitrogen atmosphere, DMF-DMA (412 μL, 3.08 mmol) was added at room temperature. At room temperature, the reaction mixture was stirred for 45 minutes, and then $BF_3 \cdot Et_2O$ (390 μL, 3.08 mmol) was added. After completion of the addition, the mixture was stirred at room temperature for 1 hour and then diluted with 200 mL of ethyl acetate. The organic phase was washed successively with a saturated $NaHCO_3$ solution (200 mL) and saturated brine (200 mL), dried over anhydrous sodium sulfate, filtered and concentrated to give crude product compound **4-h** (870 mg). The crude product was used directly in the next reaction without purification. LC-MS (ESI): m/z = 297.1 $[M+1]^+$.

Synthesis of compound **4-g**

At room temperature, compound **4-h** (770 mg, 2.59 mmol) was dissolved in THF (60 mL); at -78 °C, under nitrogen atmosphere, a solution of tri-sec-butyl lithium borohydride in tetrahydrofuran (1 M, 2.6 mL, 2.6 mmol) was added dropwise. After completion of the addition, the mixture was stirred at -78 °C for 1 hour; the reaction was then quenched by adding a saturated ammonium chloride solution (50 mL) and extracted with ethyl acetate (100 mL * 2); the organic phase was washed with saturated brine (100 mL * 2), dried over anhydrous sodium sulfate, filtered and concentrated to give a crude product; the crude product was separated and purified through a flash column chromatography (PE/EA = 4/1) to give compound **4-g** (670 mg, 86% yield) as a yellow oil. LC-MS (ESI): m/z = 299.2 $[M+1]^+$.

Synthesis of compound **4-f**

At room temperature, compound **4-g** (670 mg, 2.25 mmol) was dissolved in methanol (50 mL); at 0 °C, under nitrogen atmosphere, sodium methylate (608 mg, 11.25 mmol) and compound 2-methyl-2-thiourea sulfate (563 mg, 2.02 mmol) were successively added. After completion of the addition, the mixture was warmed to room temperature and stirred for 20 hours. The pH of the reaction mixture was adjusted to 5 with 1 M dilute hydrochloric acid; the solid was precipitated out and filtered; the filter cake was washed with water (5 mL * 2) to collect a solid, which was dried in vacuum to give crude product **4-f** (459 mg, 60% yield) as a white solid. LC-MS (ESI): m/z = 339.1 $[M+1]^+$.

Synthesis of compound **4-e**

At room temperature, compound **4-f** (459 mg, 1.36 mmol) was dissolved in DCM (18 mL); in an ice-water bath, under nitrogen atmosphere, DIPEA (673 μL, 4.08 mmol) and trifluoromethanesulfonic anhydride (343 μL, 2.04 mmol) were successively added. After completion of the addition, the reaction mixture was stirred in an ice-water bath for 2 hours and then quenched with a saturated sodium bicarbonate solution (50 mL) and extracted with DCM (50 mL * 2); the organic phase was dried over anhydrous sodium sulfate, filtered and concentrated to give a crude product; the crude product was separated and purified through a flash column chromatography (EA/PE = 1/10) to give compound **4-e** (432 mg, 68% yield) as a white solid. LC-MS (ESI): m/z = 471.1 $[M+1]^+$.

Synthesis of compound **4-d**

At room temperature, compound **4-e** (430 mg, 0.91 mmol) was dissolved in DMF (10 mL), and then DIPEA (453 μL, 2.75 mmol) and benzyl (S)-2-cyanomethylpiperazine-1-carboxylate hydrochloride (324 mg, 1.1 mmol) were successively added. After completion of the addition, under nitrogen protection, the mixture was stirred at 100 °C for 1 hour and then cooled to room temperature; the reaction was quenched with saturated brine (100 mL) and extracted with ethyl acetate (80 mL * 2). The organic phase was washed with saturated brine (100 mL * 3) and then dried over anhydrous sodium sulfate, filtered and concentrated to give a crude product; the crude product was separated and purified through a flash column chromatography (EA/PE = 1/1) to give compound **4-d** (470 mg, 68% yield) as a white solid. LC-MS (ESI): m/z = 580.3 $[M+1]^+$.

Synthesis of compound **4-c**

At room temperature, compound **4-d** (200 mg, 0.34 mmol) was dissolved in ethyl acetate (20 mL), and MCPBA (175 mg, 0.86 mmol) was added. After completion of the addition, the mixture was stirred at room temperature for 1 hour and then quenched with a saturated sodium bicarbonate solution (50 mL) and extracted with ethyl acetate (50 mL * 2); the organic phase was dried over anhydrous sodium sulfate, filtered and concentrated to give a crude product; the crude product was separated and purified through a flash column chromatography (EA/PE = 1/4) to give compound **4-c** (210 mg, 99% yield) as a white solid. LC-MS (ESI): m/z = 612.3 $[M+1]^+$.

Synthesis of compound **4-b**

At room temperature, compound **4-c** (100 mg, 0.16 mmol) was dissolved in toluene (5 mL), and then the reaction mixture was cooled to 0 °C; N-methyl-L-prolinol (34 μL, 0.29 mmol) and t-BuONa (32 mg, 0.33 mmol) were successively added. After completion of the addition, under nitrogen atmosphere, the reaction mixture was stirred in an ice-water bath for 0.5 hours and then quenched with water (10 mL) and extracted with ethyl acetate (30 mL * 2). The organic phase was dried over anhydrous sodium sulfate, filtered and concentrated to give a crude product; the crude product was separated and purified through a flash column chromatography (MeOH/DCM = 1/20) to give compound **4-b** (97 mg, 92% yield) as a white solid. LC-MS (ESI): m/z = 647.4 $[M+1]^+$.

Synthesis of compound **4-a**

At room temperature, compound **4-b** (90 mg, 0.14 mmol) was dissolved in methanolic ammonia (7 M, 50 mL); the reaction mixture was cooled to -78 °C, replaced twice with nitrogen, and then was added 10% Pd-C (75 mg) and replaced three times with hydrogen; the reaction mixture was warmed to room temperature and stirred under hydrogen for 2 hours. The reaction mixture was filtered and concentrated to give compound **4-a** (77 mg, 99% yield) as a white solid. LC-MS (ESI): m/z = 513.3 $[M+1]^+$.

Synthesis of compound **4**

At room temperature, compound **4-a** (77 mg, 0.15 mmol) was dissolved in DCM (10 mL), and DIPEA (75 μL, 0.45 mmol) and acryloyl chloride (25 μL, 0.23 mmol) were successively added. After completion of the addition, under nitrogen atmosphere, the reaction mixture was stirred at room temperature for 20 hours and then quenched with water (10 mL) and extracted with DCM (50 mL*2). The organic phase was dried over anhydrous sodium sulfate, filtered and concentrated to give a crude product; the crude product was separated and purified through a flash column chromatography (MeOH/DCM = 1/10) to give compound **4** (56 mg, 66% yield) as a white solid. LC-MS (ESI): m/z = 567.3 $[M+1]^+$; $^1H$ NMR (400 MHz, $CDCl_3$): δ 7.78-7.65 (m, 3H), 7.45-7.36 (m, 1H), 7.31-7.26 (m, 2H), 6.55-6.44 (m, 1H), 6.35-6.27 (m, 1H), 5.95-5.88 (m, 1H), 5.75 (d, 1H, *J*= 10.4 Hz), 4.94-4.84 (m, 1H), 4.81-4.59 (m, 2H), 4.52-4.37 (m, 1H), 4.22-4.11(m, 1H), 3.96-3.76 (m, 2H), 3.68-3.58 (m, 1H), 3.48-3.37 (m, 1H), 3.24-2.88 (m, 5H), 2.85 (d, 3H, *J*= 11.6 Hz), 2.78-2.58 (m, 2H), 2.50 (s, 3H), 2.40-2.27 (m, 1H), 2.08-1.68 (m, 5H).

Synthesis of compounds **4-1** and **4-2**

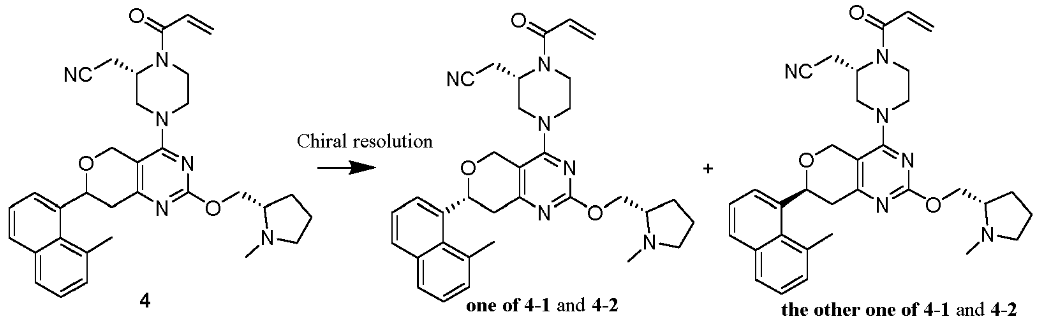


4 one of **4-1** and **4-2** + the other one of **4-1** and **4-2**

According to the method for synthesizing compound **4,** compound **4** (140 mg) was synthesized and purified by chiral resolution to give compound **4-1** (30 mg, 21% yield) as a white solid and compound **4-2** (40 mg, 29% yield) as a white solid.

| Chiral analysis conditions | Chiral preparation conditions |
|---|---|
| equipment: SFC Method Station (Thar, | equipment: SFC-150 (Thar, Waters) |
| Waters)<br>chromatographic column: OJ-H 4.6 * 100 mm, 5 μm (Daicel)<br>column temperature: 40 °C<br>mobile phase: $CO_2$/Methanol (0.1% TEA) = 60/40<br>flow rate: 4.0 ml/min<br>wavelength: 254 nm<br>back pressure: 120 bar | chromatographic column: OJ 20 * 250 mm, 10 μm (Daicel)<br>column temperature: 35 °C<br>mobile phase: $CO_2$/Methanol (0.1% TEA) = 45/55<br>flow rate: 120 g/min<br>column pressure: 100 bar<br>wavelength: 214 nm<br>cycling time: 6 min |
| 4-1: retention time: 0.97 min, d.e.% = 100.0%;<br>4-2: retention time: 1.94 min, d.e.% = 98.1%. | |

**4-1:** LC-MS (ESI): m/z = 567.3 $[M+H]^+$; $^1H$ NMR (400 MHz, $CDCl_3$) δ 7.86 (d, *J*= 8 Hz, 1H), 7.80-7.74 (m, 2H), 7.47 (t, *J*= 8 Hz, 1H), 7.41-7.34 (m, 2H), 6.65-6.50 (m, 1H), 6.40 (d, *J* = 16.4 Hz, 1H), 6.01 (dd, *J* = 8.8, 3.6 Hz, 1H), 5.84 (d, *J* = 10.4 Hz, 1H), 5.17-4.98 (m, 1H), 4.87 (d, *J*= 13.6 Hz, 1H), 4.71 (d, *J*= 13.2 Hz, 1H), 4.43 (dd, *J*= 10.8, 4.8 Hz, 1H), 4.21 (dd, *J* = 10, 6.4 Hz, 1H), 4.03-3.38 (m, 3H), 3.34-3.05 (m, 5H), 3.03-2.97 (m, 1H), 2.95 (s, 3H), 2.84-2.70 (m, 2H), 2.52 (s, 3H), 2.34-2.29 (m, 1H), 2.14-2.00 (m, 2H), 1.92-1.82 (m, 2H), 1.39-1.32 (m, 1H).

**4-2:** LC-MS (ESI): m/z = 567.3 $[M+H]^+$; $^1H$ NMR (400 MHz, $CDCl_3$) δ 7.85 (d, *J*= 7.6 Hz, 2H), 7.76 (d, *J* = 6.4 Hz, 1H), 7.51 (t, *J* = 7.6 Hz, 1H), 7.39-7.35 (m, 2H), 6.68-6.52 (m, 1H), 6.40 (d, *J*= 16.4 Hz, 1H), 6.01 (dd, *J* = 10.4, 2.8 Hz, 1H), 5.84 (d, *J*= 10 Hz, 1H), 5.11-4.92 (m, 2H), 4.80 (d, *J*= 13.6 Hz, 1H), 4.49-4.38 (m, 1H), 4.23-4.15 (m, 1H), 4.04-3.68 (m, 3H), 3.58-3.45 (m, 1H), 3.36-3.00 (m, 5H), 2.92 (s, 3H), 2.78-2.69 (m, 2H), 2.51 (s, 3H), 2.38-2.28 (m, 1H), 2.16-2.01 (m, 2H), 1.91-1.81 (m, 2H), 1.38-1.33 (m, 1H).

**Example 5** Synthetic route of compound **5**

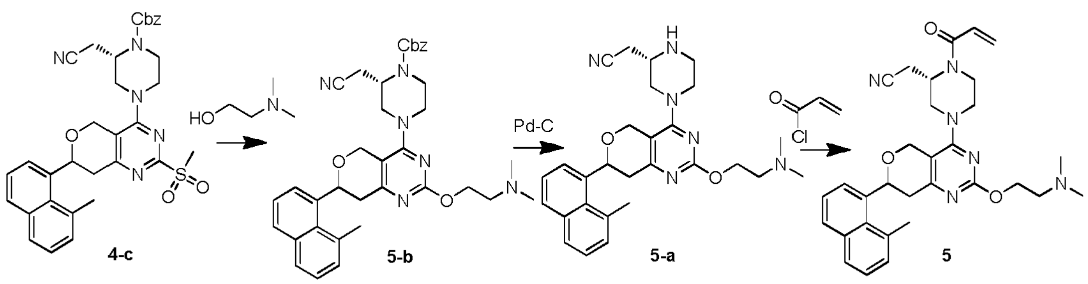


4-c 5-b 5-a 5

Synthesis of compound **5-b**

Compound **4-c** (100 mg, 0.164 mmol) was dissolved in toluene (5 mL); under ice-water bath cooling, 2-dimethylaminoethanol (29 μL, 0.29 mmol) and t-BuONa (32 mg, 0.33 mmol) were successively added. Under nitrogen atmosphere, the mixture was stirred in an ice-water bath for 0.5 hours and then quenched with water (10 mL) and extracted with ethyl acetate (30 mL * 2). The organic phase was dried over anhydrous sodium sulfate, filtered and concentrated to give a crude product; the crude product was separated and purified through a flash column chromatography (MeOH/DCM = 1/10) to give compound **5-b** (100 mg, 98% yield) as a white solid. LC-MS (ESI): m/z = 621.4 $[M+1]^+$.

Synthesis of compound **5-a**

Compound **5-b** (100 mg, 0.161 mmol) was dissolved in methanolic ammonia (7 M, 50 mL), cooled to -78 °C, replaced twice with nitrogen, and then added Pd-C (75 mg). After replaced three times with hydrogen, the reaction mixture was warmed to room temperature and stirred under hydrogen for 2 hours. The reaction mixture was filtered and concentrated to give compound **5-a** (80 mg, 97% yield) as a white solid. LC-MS (ESI): m/z = 487.3 $[M+1]^+$.

Synthesis of compound **5**

At room temperature, compound **5-a** (80 mg, 0.165 mmol) was dissolved in DCM (10 mL), and DIPEA (82 μL, 0.495 mmol) and acryloyl chloride (30 μL, 0.25 mmol) were successively added. Under nitrogen atmosphere, the mixture was stirred at room temperature for 20 hours and then quenched with water (10 mL) and extracted with DCM (50 mL * 2); the organic phase was dried over anhydrous sodium sulfate, filtered and concentrated to give a crude product; the crude product was separated and purified through a flash column chromatography (MeOH/DCM = 10%) to give compound **5** (55 mg, 62% yield) as a white solid. LC-MS (ESI): m/z = 541.3 $[M+1]^+$; $^1H$ NMR(400 MHz, $CDCl_3$): $\delta$ 7.88-7.65 (m, 3H), 7.56-7.40 (m, 1H), 7.38-7.12 (m, 2H), 6.68-6.25 (m, 2H), 6.05-5.66 (m, 2H), 5.44-4.54 (m, 3H), 4.44 (s, 2H), 4.05-3.79 (m, 2H), 3.77-3.33 (m, 3H), 3.29-2.99 (m, 4H), 2.91 (d, 3H, *J*= 12.4 Hz), 2.83-2.56(m, 3H), 2.36 (s, 6H).

**Example 6** Synthetic route of compound **6**

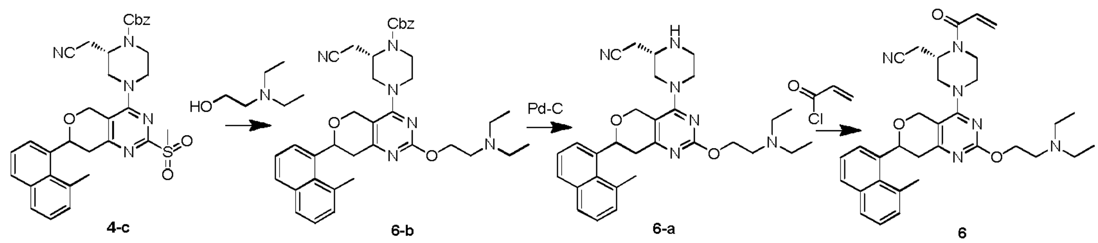


Synthesis of compound **6-b**

Compound **4-c** (62 mg, 0.10 mmol) was dissolved in toluene (5 mL); under ice-water bath cooling, 2-diethylaminoethanol (24 μL, 0.18 mmol) and t-BuONa (20 mg, 0.20 mmol) were successively added. Under nitrogen atmosphere, the mixture was stirred in an ice-water bath for 0.5 hours and then quenched with water (10 mL) and extracted with ethyl acetate (25 mL * 2). The organic phase was dried over anhydrous sodium sulfate, filtered and concentrated to give a crude product; the crude product was separated and purified through a flash column chromatography (MeOH/DCM = 1/10) to give compound **6-b** (65 mg, 99% yield) as a white solid. LC-MS (ESI): m/z = 649.4 $[M+1]^+$.

Synthesis of compound **6-a**

Compound **6-b** (65 mg, 0.1 mmol) was dissolved in methanolic ammonia (7 M, 50 mL), cooled to -78 °C, replaced twice with nitrogen, and then added Pd/C (30 mg). After replaced three times with hydrogen, the reaction mixture was warmed to room temperature and stirred under hydrogen for 1 hour. The reaction mixture was filtered and concentrated to give compound **6-a** (51 mg, 99% yield) as a white solid. LC-MS (ESI): m/z = 515.3 $[M+1]^+$.

Synthesis of compound **6**

At room temperature, compound **6-a** (51 mg, 0.10 mmol) was dissolved in DCM (10 mL), and DIPEA (82 μL, 0.50 mmol) and acryloyl chloride (13.6 mg, 0.15 mmol) were successively added. After completion of the addition, under nitrogen atmosphere, the mixture was stirred at room temperature for 20 hours and then quenched with water (10 mL) and extracted with DCM (50 mL * 2). The organic phase was dried over anhydrous sodium sulfate, filtered and concentrated to give a crude product; the crude product was separated and purified through a flash column chromatography (MeOH/DCM = 1/10) to give compound **6** (50 mg, 68% yield) as a white solid. LC-MS (ESI): m/z = 569.3 $[M+1]^+$; $^1H$ NMR (400 MHz, $CDCl_3$) $\delta$ 7.85-7.71 (m, 3H), 7.52-7.43 (m, 1H), 7.39-7.31 (m, 2H), 6.67-6.47 (m, 1H), 6.42-6.24 (m, 1H), 6.17-5.95 (m, 1H), 5.85-5.66 (m, 1H), 5.08-4.92 (m, 1H), 4.89-4.66 (m, 4H), 4.08-3.85 (m, 2H), 3.80-3.53 (m, 2H), 3.32-3.15 (m, 4H), 3.12-2.97 (m, 6H), 2.92 (d, 3H, *J*= 12.8 Hz), 2.85-2.62 (m, 2H), 1.34-1.27 (m, 6H).

Synthesis of compounds **6-1** and **6-2**

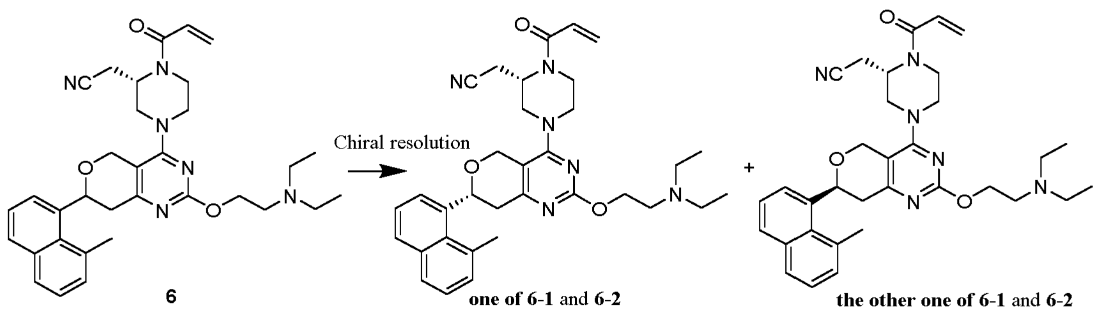


**6** **one of 6-1 and 6-2** **the other one of 6-1 and 6-2**

Compound **6** (40 mg, 0.07 mmol) was subjected to chiral resolution to give compound **6-1** (12 mg, 30% yield) as a white solid and compound **6-2** (11 mg, 28% yield) as a white solid.

| Chiral analysis conditions | Chiral preparation conditions |
|---|---|
| instrument: SFC Method Station (Thar, Waters)<br>chromatographic column: CHIRALCEL OJ-H 4.6 * 100 mm, 5 μm (Daicel)<br>column temperature: 40 °C<br>mobile phase: $CO_2$/MeOH (0.1% TEA) = 65/35<br>back pressure: 120 bar<br>flow rate: 1.0 ml/min | instrument: SFC-80 (Thar, Waters)<br>chromatographic column: CHIRALCEL OJ-H 20 * 250 mm, 5 μm (Daicel)<br>column temperature: 35 °C<br>mobile phase: $CO_2$/MeOH (0.1% TEA) = 65/35<br>flow rate: 80 g/min<br>back pressure: 100 bar<br>detection wavelength: 214 nm |
| 6-1: retention time: 1.22, d.e.% = 100%;<br>6-2: retention time: 2.67, d.e.% = 96.7%. | |

**6-1:** LC-MS (ESI): m/z = 569.3 $[M+1]^+$; $^1H$ NMR (500 MHz, $CDCl_3$): $\delta$ 7.83 (d, 1H, *J*= 8 Hz), 7.75 (t, 2H, *J*= 7 Hz), 7.45 (t, 1H, *J*= 8 Hz), 7.39-7.31 (m, 2H), 6.63-6.49 (m, 1H), 6.38 (d, 1H, *J*= 17 Hz), 6.00 (dd, 1H, $J_1$ = 4 Hz, $J_2$ = 9.5 Hz), 5.81 (d, 1H, *J* = 11 Hz), 5.17-4.92 (m, 1H), 4.86 (d, 1H, *J*= 14 Hz), 4.71 (d, 1H, *J*= 14 Hz), 4.39 (t, 2H, *J*= 6.5 Hz), 4.11-3.78 (m, 2H), 3.70 (d, 1H, *J*= 12 Hz), 3.53-3.33 (m, 1H), 3.25 (dt, 2H, $J_1$ = 3.5 Hz, $J_2$ = 18.5 Hz), 3.17-3.03 (m, 2H), 3.02-2.95 (m, 1H), 2.94 (s, 3H), 2.87 (t, 2H, *J* = 6.5 Hz), 2.83-2.71 (m, 1H), 2.63 (q, 4H, *J*= 6.5 Hz), 1.06 (t, 6H, *J*= 7 Hz).

**6-2:** LC-MS (ESI): m/z = 569.3 $[M+1]^+$; $^1H$ NMR (500 MHz, $CDCl_3$): $\delta$ 7.87-7.79 (m, 2H), 7.75 (d, 1H, *J*= 8 Hz), 7.49 (t, 1H, *J*= 7.5 Hz), 7.39-7.30 (m, 2H), 6.65-6.48 (m, 1H), 6.38 (d, 1H, *J*= 16.5 Hz), 5.99 (dd, 1H, $J_1$ = 3.5 Hz, $J_2$ = 10.5 Hz), 5.82 (d, 1H, *J*= 10.5 Hz), 4.99 (d, 2H, *J*= 13.5 Hz), 4.78 (d, 1H, *J*= 14 Hz), 4.73-4.45 (m, 1H), 4.38 (t, 2H, *J*= 6.5 Hz), 3.96 (d, 1H, *J*= 14 Hz), 3.92-3.78 (m, 1H), 3.75-3.57 (m, 1H), 3.55-3.36 (m, 1H), 3.25 (dd, 1H, $J_1$ = 3 Hz, $J_2$ =18.5 Hz), 3.15-2.96 (m, 2H), 2.91 (s, 3H), 2.86 (t, 2H, *J*= 6.5 Hz), 2.80-2.66 (m, 2H), 2.62 (q, 4H, *J*= 7 Hz), 1.05 (t, 6H, *J*= 7 Hz).

**Example 7** Synthetic route of compound **7**

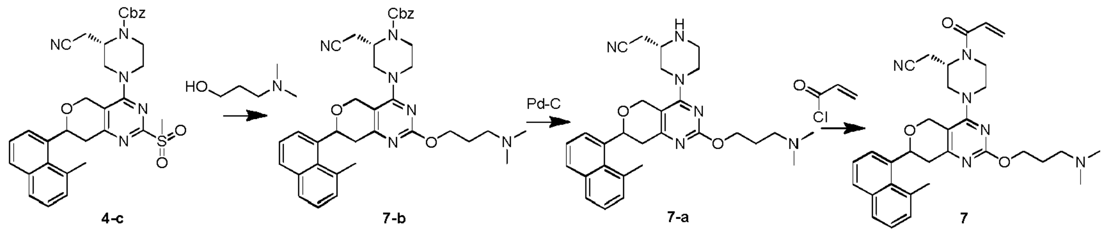


Synthesis of compound **7-b**

Compound **4-c** (62 mg, 0.10 mmol) was dissolved in toluene (5 mL); under ice-water bath cooling, 3-dimethylamino-1-propanol (21 μL, 0.18 mmol) and t-BuONa (20 mg, 0.20 mmol) were successively added. Under nitrogen atmosphere, the mixture was stirred in an ice-water bath for 0.5 hours, warmed to room temperature, stirred for 20 hours and then slowly heated to 100 °C and stirred for about 2 hours. The reaction mixture was cooled to room temperature, quenched with water (50 mL) and extracted with ethyl acetate (50 mL * 2). The organic phase was dried over anhydrous sodium sulfate, filtered and concentrated to give a crude product; the crude product was separated and purified through a flash column chromatography (MeOH/DCM = 1/10) to give compound **7-b** (38 mg, 60% yield) as a white solid. LC-MS (ESI): m/z = 635.4 $[M+1]^+$.

Synthesis of compound **7-a**

Compound **7-b** (38 mg, 0.06 mmol) was dissolved in methanolic ammonia (7 M, 20 mL), cooled to -78 °C, replaced twice with nitrogen, and then added Pd/C (20 mg). After replaced three times with hydrogen, the reaction mixture was warmed to room temperature and stirred under hydrogen for 1 hour; the reaction mixture was filtered and concentrated to give compound **7-a** (35 mg, 99% yield) as a white solid. LC-MS (ESI): m/z = 501.3 $[M+1]^+$.

Synthesis of compound **7**

Compound **7-a** (35 mg, 0.07 mmol) was dissolved in DCM (10 mL), and DIPEA (58 μL, 0.35 mmol) and acryloyl chloride (10 mg, 0.11 mmol) were successively added. Under nitrogen atmosphere, the mixture was stirred at room temperature for 20 hours and then quenched with a saturated sodium bicarbonate solution (20 mL) and extracted with DCM (30 mL * 2). The organic phase was dried over anhydrous sodium sulfate, filtered and concentrated to give a crude product; the crude product was separated and purified through a flash column chromatography (MeOH/DCM = 1/10) to give compound **7** (15 mg, 38% yield) as a white solid. LC-MS (ESI): m/z = 555.3 $[M+1]^+$; $^1H$ NMR (400 MHz, $CDCl_3$): $\delta$ 7.87-7.59 (m, 3H), 7.53-7.41 (m, 1H), 7.41-7.30 (m, 2H), 6.63-6.48 (m, 1H), 6.38 (d, 1H, *J*= 15.6 Hz), 6.19-6.05 (m, 1H), 6.04-5.94 (m, 1H), 5.82 (d, 1H, *J*= 10.4 Hz), 5.59-5.51 (m, 1H), 5.10-4.91 (m, 1H), 4.89-4.62 (m, 2H), 4.42-4.23 (m, 2H), 4.03-3.84 (m, 2H), 3.76-3.58 (m, 1H), 3.56-3.39 (m, 1H), 3.31-3.20 (m, 2H), 3.19-3.00 (m, 5H), 2.77-2.60 (m, 2H), 2.42 (s, 6H), 2.13-2.02 (m, 2H).

**Example 8** Synthetic route of compound **8**

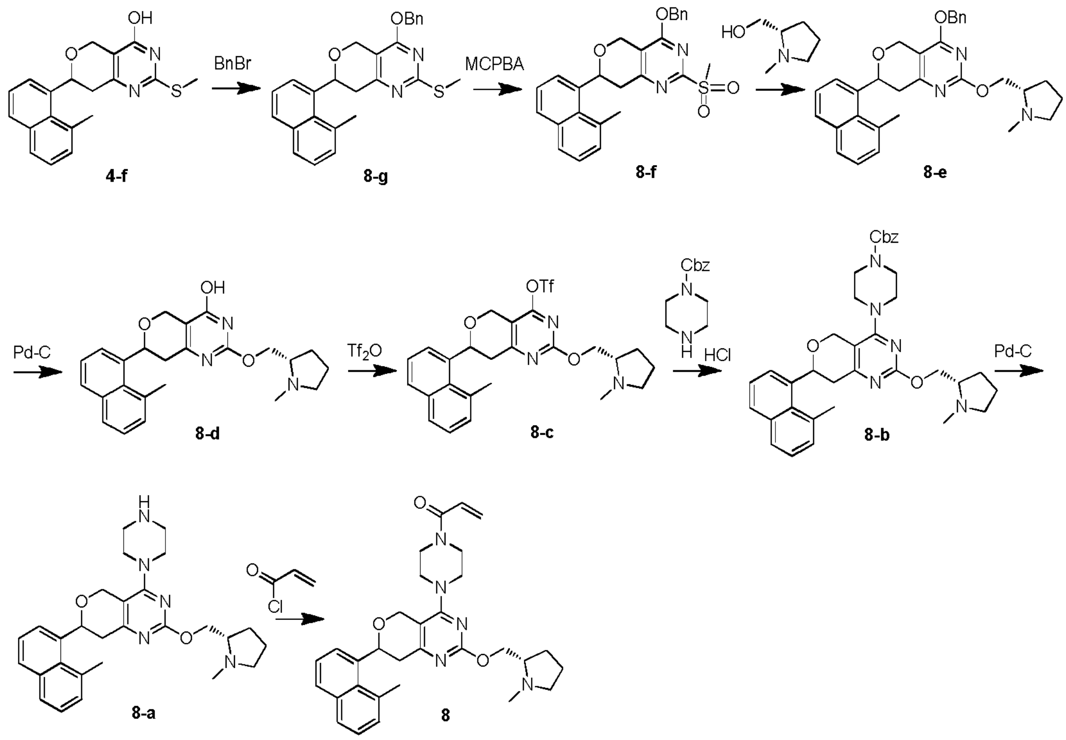


4-f 8-g 8-f 8-e 8-d 8-c 8-b 8-a 8

Synthesis of compound **8-g**

At room temperature, compound **4-f** (338 mg, 1.0 mmol) was dissolved in DMF (10 mL), and potassium carbonate (207 mg, 1.5 mmol) and benzyl bromide (132 μL, 1.1 mmoL) were successively added. After stirred at room temperature for 3 hours, the reaction mixture was poured into 50 mL of water and extracted with ethyl acetate (50 mL * 2). The organic phase was washed with saturated brine (100 mL * 3), dried over anhydrous sodium sulfate, filtered and concentrated to give a crude product. The crude product was separated and purified through a flash column chromatography (PE/EA = 2/1) to give compound (mixture) **8-g** (211 mg, 49% yield) as a white solid. LC-MS (ESI): m/z = 429.2 $[M+1]^+$.

Synthesis of compound **8-f**

Compound **8-g** (211 mg, 0.49 mmol) was dissolved in ethyl acetate (20 mL) and added MCPBA (250 mg, 1.23 mmol) at room temperature. The mixture was stirred at room temperature for 3 hours and then quenched with a saturated sodium bicarbonate solution (50 mL) and extracted with ethyl acetate (50 mL * 2). The organic phase was dried over anhydrous sodium sulfate, filtered and concentrated to give a crude product; the crude product was separated and purified through a flash column chromatography (EA/PE = 1/1) to give compound **8-f** (138 mg, 61% yield) as a white solid. LC-MS (ESI): m/z = 461.0 $[M+1]^+$.

Synthesis of compound **8-e**

Compound **8-f** (138 mg, 0.3 mmol) was dissolved in toluene (10 mL); in an ice-water bath, N-methyl-L-prolinol (65 μL, 0.54 mmol) and t-BuONa (58 mg, 0.6 mmol) were successively added. Under nitrogen atmosphere, the mixture was stirred in an ice-water bath for 0.5 hours and then quenched with water (30 mL) and extracted with ethyl acetate (50 mL * 2). The organic phase was dried over anhydrous sodium sulfate, filtered and concentrated to give a crude product; the crude product was separated and purified through a flash column chromatography (MeOH/DCM = 1/10) to give compound **8-e** (105 mg, 70% yield) as a white solid. LC-MS (ESI): m/z = 496.3 $[M+1]^+$.

Synthesis of compound **8-d**

Compound **8-e** (105 mg, 0.212 mmol) was dissolved in methanol (30 mL), cooled to -78 °C, replaced twice with nitrogen, and then added Pd/C (50 mg). After replaced three times with hydrogen, the reaction mixture was warmed to room temperature and stirred under hydrogen for 3 hours; the reaction mixture was then filtered and concentrated to give

compound **8-d** (91 mg, 100% yield) as a white solid. The crude product was used directly in the next reaction without further purification. LC-MS (ESI): m/z = 406.1 $[M+1]^+$.

Synthesis of compound **8-c**

Compound **8-d** (91 mg, 0.225 mmol) was dissolved in DCM (10 mL); under nitrogen atmosphere, in an ice-water bath, DIPEA (111 μL, 0.68 mmol) and trifluoromethanesulfonic anhydride (57 μL, 0.34 mmol) were successively added. The mixture was stirred in an ice-water bath for 1 hour and then quenched with a saturated sodium bicarbonate solution (20 mL) and extracted with DCM (50 mL * 2). The organic phase was dried over anhydrous sodium sulfate, filtered and concentrated to give a crude product; the crude product was separated and purified through a flash column chromatography (MeOH/DCM = 1/10) to give compound **8-c** (68 mg, 56% yield) as a white solid. LC-MS (ESI): m/z = 538.2 $[M+1]^+$.

Synthesis of compound **8-b**

At room temperature, compound **8-c** (66 mg, 0.123 mmol) was dissolved in DMF (5 mL), and DIPEA (61 μL, 0.37 mmol) and 1-Cbz-piperazine hydrochloride (38 mg, 0.15 mmol) were successively added. Under nitrogen protection, the mixture was stirred at 100 °C for 1 hour and then cooled to room temperature, quenched with saturated brine (50 mL) and extracted with ethyl acetate (50 mL * 2). The organic phase was washed with saturated brine (50 mL * 3) and then dried over anhydrous sodium sulfate, filtered and concentrated to give a crude product; the crude product was separated and purified through a flash column chromatography (MeOH/DCM = 1/10) to give compound **8-b** (62 mg, 83 yield) as a white solid. LC-MS (ESI): m/z = 608.2 $[M+1]^+$.

Synthesis of compound **8-a**

Compound **8-b** (62 mg, 0.102 mmol) was dissolved in methanol (20 mL), cooled to -78 °C, replaced twice with nitrogen and then added Pd/C (30 mg). After replaced three times with hydrogen, the reaction mixture was warmed to room temperature and stirred under hydrogen for 1 hour. The reaction mixture was filtered and concentrated to give compound **8-a** (45 mg, 94% yield) as a white solid. The crude product was used directly in the next reaction without further purification. LC-MS (ESI): m/z = 474.3 $[M+1]^+$.

Synthesis of compound **8**

At room temperature, compound **8-a** (45 mg, 0.095 mmol) was dissolved in DCM (10 mL), and DIPEA (79 μL, 0.48 mmol) and acryloyl chloride (13 mg, 0.143 mmol) were successively added. Under nitrogen atmosphere, the mixture was stirred at room temperature for 20 hours and then quenched with saturated aqueous sodium bicarbonate solution (20 mL) and extracted with DCM (30 mL * 2). The organic phase was dried over anhydrous sodium sulfate, filtered and concentrated to give a crude product; the crude product was subjected to PREP-TLC (MeOH/DCM = 1/10) to give compound 8 (28 mg, 56% yield) as a white solid. LC-MS (ESI): m/z = 528.3 $[M+1]^+$; $^1H$ NMR (400 MHz, $CDCl_3$) $\delta$ 7.89-7.64 (m, 3H), 7.53-7.40 (m, 1H), 7.40-7.16 (m, 2H), 6.65-6.45 (m, 1H), 6.40-6.21 (m, 1H), 6.02-5.89 (m, 1H), 5.79-5.66 (m, 1H), 4.88-4.76 (m, 1H), 4.74-4.60 (m, 1H), 4.45-4.26 (m, 1H), 4.22-4.05 (m, 1H), 3.93-3.17 (m, 10H), 3.13-2.99 (m, 2H), 2.93 (s, 3H), 2.85-2.55 (m, 2H), 2.47 (s, 3H), 2.38-2.25 (m, 1H), 2.10-1.95 (m, 1H).

**Example 9** Synthetic route of compound **9**

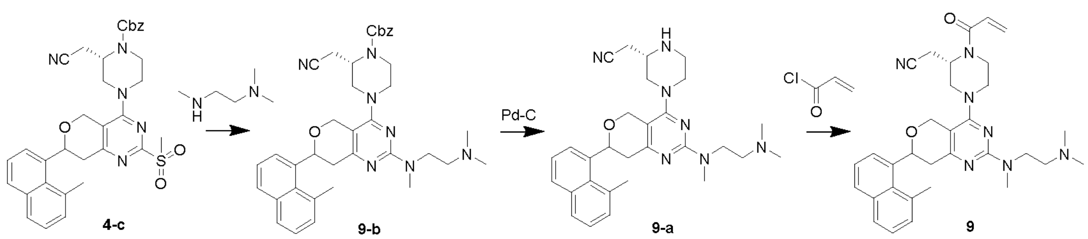


Synthesis of compound **9-b**

At room temperature, compound **4-c** (61 mg, 0.1 mmol) was dissolved in dioxane (5 mL) and then added N,N-dimethyl-N'-methylethylenediamine (255 μL, 2 mmol). Under nitrogen atmosphere, the reaction mixture was stirred at 110 °C for 24 hours, and the reaction mixture was then cooled to room temperature and concentrated to dryness to give a crude product. The crude product was separated and purified through a flash column chromatography (MeOH/DCM = 1/10) to give compound **9-b** (49 mg, 78% yield) as a white solid. LC-MS (ESI): m/z = 634.4 $[M+1]^+$.

Synthesis of compound **9-a**

Compound **9-b** (69 mg, 0.11 mmol) was dissolved in methanolic ammonia (7 M, 50 mL), cooled to -78 °C, replaced twice with nitrogen, and then added Pd/C (50 mg). After replaced three times with hydrogen, the reaction mixture was warmed to room temperature and stirred under hydrogen for 1 hour. The reaction mixture was filtered and concentrated to give compound **9-a** (50 mg, 99% yield) as a white solid. LC-MS (ESI): m/z = 500.5 $[M+1]^+$.

Synthesis of compound **9**

At room temperature, compound **9-a** (50 mg, 0.1 mmol) was dissolved in DCM (10 mL), and DIPEA (83 μL, 0.5 mmol) and acryloyl chloride (15 mg, 0.15 mmol) were successively added. Under nitrogen atmosphere, the mixture was stirred at room temperature for 20 hours and then quenched with a saturated sodium bicarbonate solution (20 mL) and extracted with DCM (50 mL * 2). The organic phase was dried over anhydrous sodium sulfate, filtered and concentrated to give a crude product. The crude product was separated and purified through a flash column chromatography (MeOH/DCM = 1/10) to give compound 9 (27 mg, 49% yield) as a white solid. LC-MS (ESI): m/z = 554.4 $[M+1]^+$; $^1H$ NMR (400 MHz, $CDCl_3$): $\delta$ 7.87-7.69 (m, 3H), 7.55-7.42 (m, 1H), 7.40-7.29 (m, 2H), 6.67-6.45 (m, 1H), 6.37 (d, 1H, *J*= 16.8 Hz), 5.96 (d, 1H, *J*= 7.2 Hz), 5.80 (d, 1H, *J*= 10.4 Hz), 5.20-4.39 (m, 3H), 3.89 (d, 1H, *J* = 13.2 Hz), 3.81-3.65 (m, 2H), 3.65-3.50 (m, 1H), 3.44-3.25 (m, 1H), 3.20-3.03 (m, 4H), 3.01-2.85 (m, 5H), 2.78-2.61 (m, 1H), 2.58-2.41 (m, 2H), 2.31 (s, 6H), 2.14-1.83 (m, 4H).

**Example 10** Synthetic route of compound **10**

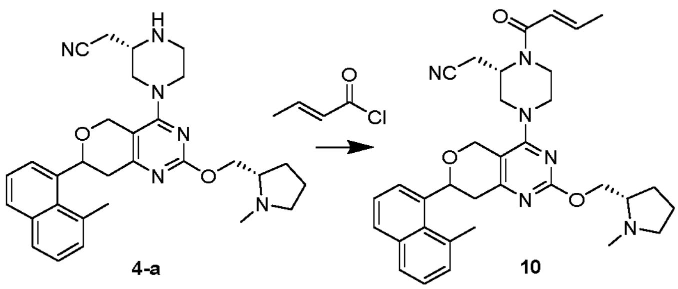

Synthesis of compound **4-a**

Compound **4-a** (61 mg) was prepared according to the synthetic route of compound **4.**

Synthesis of compound **10**

At room temperature, compound **4-a** (61 mg, 0.12 mmol) was dissolved in DCM (10 mL), and DIPEA (99 μL, 0.6 mmol) and 2-butenoyl chloride (17 μL, 0.18 mmol) were successively added. Under nitrogen atmosphere, the mixture was stirred at room temperature for 20 hours and then quenched with a saturated sodium bicarbonate solution (20 mL) and extracted with DCM (50 mL * 2). The organic phase was dried over anhydrous sodium sulfate, filtered and concentrated to give a crude product; the crude product was separated and purified through a flash column chromatography (MeOH/DCM = 1/10) to give compound **10** (55 mg, 78% yield) as a white solid. LC-MS (ESI): m/z = 581.3 $[M+1]^+$; $^1H$ NMR (500 MHz, $CDCl_3$): $\delta$ 7.87-7.68 (m, 3H), 7.54-7.41 (m, 1H), 7.40-7.29 (m, 2H), 7.05-6.90 (m, 1H), 6.30-6.18 (m, 1H), 6.04-5.88 (m, 1H), 5.12-4.91 (m, 1H), 4.89-4.57 (m, 2H), 4.44-4.29 (m, 1H), 4.22-4.10 (m, 1H), 4.02-3.76 (m, 2H), 3.75-3.54 (m, 1H), 3.51-3.34 (m, 1H), 3.31-3.15 (m, 2H), 3.15-2.99 (m, 3H), 2.99-2.82 (m, 4H), 2.81-2.56 (m, 3H), 2.47 (s, 3H), 2.35-2.13 (m, 1H), 2.12-1.97 (m, 1H), 1.91 (s, 3H), 1.85-1.82 (m, 2H).

**Example 11** Synthetic route of compounds **11-1** and **11-2**

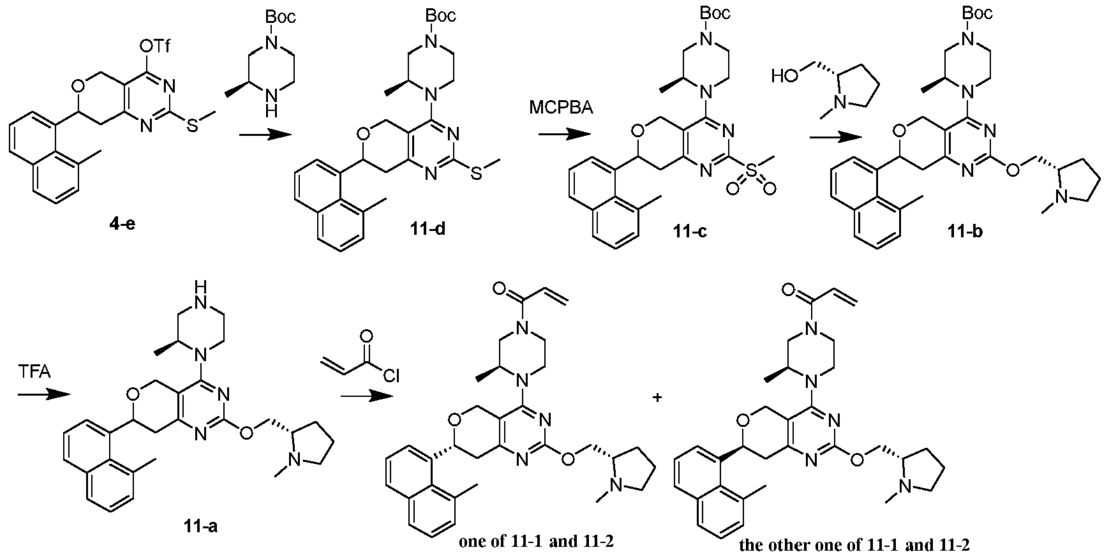


Synthesis of compound **11-d**

At room temperature, to a solution of **4-e** (220 mg, 0.47 mmol) and (S)-4-N-tert-butoxycarbonyl-2-methylpiperazine (112 mg, 0.56 mmol) in DMF (10 mL) was added DIPEA (121 mg, 0.94 mmol). The reaction temperature was increased to 100 °C, and the reaction was stirred at this temperature for 1 hour. The reaction mixture was cooled to room temperature, added water and extracted with ethyl acetate (30 mL * 2); the combined organic phase was dried over anhydrous sodium sulfate, filtered and subjected to rotary evaporation; the crude product was separated and purified through a flash column chromatography (EA : PE = 0 : 100 to 30 :70) to give compound **11-d** (173 mg, 71%) as a white solid. LC-MS (ESI): m/z = 521.3 $[M+H]^+$.

Synthesis of compound **11-c**

In an ice bath, to a solution of **11-d** (173 mg, 0.33 mmol) in ethyl acetate (10 mL) was added 85% m-chloroperoxybenzoic acid (169 mg, 0.83 mmol). The reaction was slowly warmed to room temperature, stirred for 3 hours and then added saturated aqueous sodium bicarbonate solution and extracted with ethyl acetate (50 mL); the organic phase was dried over anhydrous sodium sulfate, filtered and subjected to rotary evaporation; the crude product was separated and purified through a flash column chromatography (EA : PE = 0 : 100 to 50 : 50) to give compound **11-c** (154 mg, 84%) as a white solid. LC-MS (ESI): m/z = 553.2 $[M+H]^+$.

Synthesis of compound **11-b**

At room temperature, to a solution of **11-c** (154 mg, 0.28 mmol) in toluene (6 mL) were respectively added a solution of N-methyl-L-prolinol (48 mg, 0.42 mmol) in toluene (4 mL) and sodium tert-butoxide (53 mg, 0.56 mmol). The mixture was stirred at room temperature for 3 hours and then concentrated, added water and extracted with ethyl acetate (30 mL * 2); the combined organic phase was dried over anhydrous sodium sulfate, filtered and subjected to rotary evaporation; the crude product was separated and purified through a flash column chromatography (DCM : MeOH = 10: 1) to give compound **11-b** (120 mg, 73%) as a white solid. LC-MS (ESI): m/z = 588.3 $[M+H]^+$.

Synthesis of compound **11-a**

At room temperature, to a solution of **11-b** (120 mg, 0.2 mmol) in dichloromethane (8 mL) was added TFA (2 mL). The mixture was stirred at room temperature overnight and then concentrated, added saturated aqueous sodium bicarbonate solution and extracted with ethyl acetate (30 mL * 2); the combined organic phase was dried over anhydrous sodium sulfate, filtered and subjected to rotary evaporation to give compound **11-a** (70 mg, 70%) as a white solid. LC-MS (ESI): m/z = 488.0 $[M+H]^+$.

Synthesis of compounds **11-1** and **11-2**

In an ice bath, to a solution of **11-a** (70 mg, 0.14 mmol) in dichloromethane (5 mL) were respectively added acryloyl chloride (19 mg, 0.22 mmol) and DIPEA (36 mg, 0.28 mmol). The reaction temperature was increased to room temperature, and the reaction was stirred at room temperature for 2 hours and then concentrated, added water and extracted with ethyl acetate (30 mL * 2); the combined organic phase was dried over anhydrous sodium sulfate, filtered and subjected to rotary evaporation; the crude product was subjected to chiral resolution to give compound **11-1** (25 mg, 32%) as a white solid and **11-2** (20 mg, 26%) as a white solid.

| Chiral analysis conditions | Chiral preparation conditions |
|---|---|
| instrument: SFC Method Station (Thar, Waters)<br>chromatographic column: R,R-WHELK-O1 4.6 * 100 mm, 5 μm (REGIS)<br>column temperature: 40 °C<br>mobile phase: COz/(MeOH/CAN = 3 : 2 (0.1% TEA)) = 55/45<br>flow rate: 4.0 ml/min<br>detection wavelength: 254 nm | instrument: SFC-150 (Waters)<br>chromatographic column: R,R-WHELK-O1 20 * 250 mm, 10 um (REGIS)<br>column temperature: 35 °C<br>mobile phase: COz/(MeOH/CAN = 1 : 1(0.1% TEA)) = 40/60<br>flow rate: 120 g/min<br>column pressure: 100 bar<br>detection wavelength: 214 nm<br>cycling time: 7.5 min<br>sample solution: 70 mg dissolved in 20 ml of methanol |
| **11-1:** retention time: 3.26; d.e.% = 100%<br>**11-2:** retention time: 4.16; d.e.% = 98.6% | |

**11-1:** LC-MS (ESI): m/z = 542.3 $[M+H]^+$; $^1H$ NMR (500 MHz, $CDCl_3$): δ 7.85 (t, *J* = 8 Hz, 2H), 7.78 (dd, *J* = 8, 2 Hz, 1H), 7.50 (t, *J* = 7.5 Hz, 1H), 7.40-7.35 (m, 2H), 6.66-6.53 (m, 1H), 6.39 (dd, *J* = 17, 2 Hz, 1H), 6.00 (dd, *J* = 10, 3.5 Hz, 1H), 5.78 (d, *J*= 9 Hz, 1H), 4.85-4.80 (m, 1H),4.77-4.70 (m, 1H), 4.43-4.16 (m, 3H), 4.07-3.96 (m, 1H), 3.77-3.57 (m, 1H), 3.49-3.43 (m, 1H), 3.32-3.24 (m, 2H), 3.14-3.03 (m, 2H), 2.96 (s, 3H), 2.73-2.66 (m, 1H), 2.49 (s, 3H), 2.34-2.24 (m, 1H), 2.11-2.02 (m, 1H), 1.88-1.73 (m, 3H), 1.37-1.31 (m, 1H), 1.24-1.14 (m, 4H).

**11-2:** LC-MS (ESI): m/z = 542.3 $[M+H]^+$; $^1H$ NMR (500 MHz, $CDCl_3$): δ 7.83 (dd, *J* = 17, 8 Hz, 2H), 7.78 (dd, *J* = 7, 2 Hz, 1H), 7.49 (t, *J* = 7.5 Hz, 1H), 7.40-7.35 (m, 2H), 6.66-6.53 (m, 1H), 6.39 (dd, *J*= 17, 1.5 Hz, 1H), 6.00 (d, *J* = 7.5 Hz, 1H), 5.77 (d, *J* = 9.5 Hz, 1H), 4.88 (d, *J*= 14 Hz, 1H), 4.68 (d, *J*= 13.5 Hz, 1H), 4.45-4.34 (m, 1H), 4.20-4.09 (m, 1H), 4.00-3.88 (m, 1H), 3.80-3.69 (m, 1H), 3.62-3.44 (m, 1H), 3.34-3.25 (m, 2H), 3.14-3.03 (m, 3H), 2.96 (s, 3H), 2.73-2.65 (m, 1H), 2.49 (s, 3H), 2.34-2.24 (m, 1H), 2.12-2.02 (m, 1H), 1.88-1.72 (m, 3H), 1.36-1.28 (m, 5H).

**Example 12** Synthetic route of compound 12

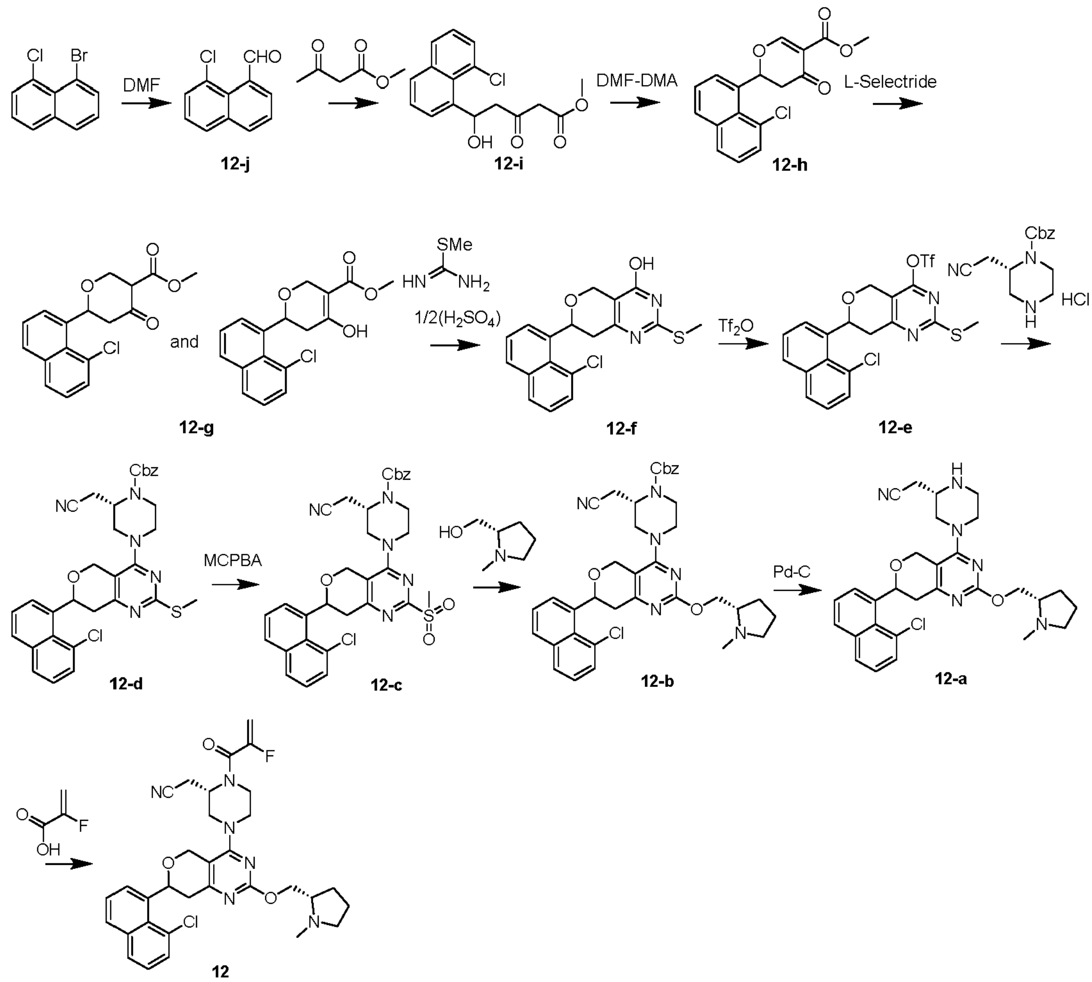


12-j 12-i 12-h

12-g 12-f 12-e

12-d 12-c 12-b 12-a

12

Synthesis of compound **12-j**

Compound 1-bromo-8-chloronaphthalene (500 mg, 2.07 mmol) was dissolved in THF (20 mL), cooled to -78 °C and added dropwise n-BuLi (2.5 M, 1.66 mL, 4.14 mmol) under nitrogen protection. After completion of the dropwise addition, the mixture was stirred at -78 °C for 10 minutes, and then DMF (800 μL, 10.35 mmol) was added dropwise at -78 °C. After completion of the addition, the reaction mixture was stirred at -78 °C for 30 minutes and then warmed to room temperature and stirred for 2 hours; the reaction was quenched with 50 mL of saturated ammonium chloride solutions and extracted with ethyl acetate (50 mL * 2). The organic phase was washed with saturated brine (50 mL * 2), dried over anhydrous sodium sulfate, filtered and concentrated to give a crude product. The crude product was separated and purified through a flash column chromatography (EA/PE = 1/10) to give compound 12-j (330 mg, 84% yield) as a white solid. LC-MS (ESI): m/z = 191.0 $[M+H]^+$; $^1H$ NMR (400 MHz, $CDCL_3$): δ 11.31 (s, 1H), 8.03 (dd, 1H, $J_1$ = 1.2 Hz, $J_2$ = 8.4 Hz), 7.92 (dd, 1H, $J_1$ = 1.2 Hz, $J_2$ = 7.2 Hz), 7.86 (1H, $J$= 8.4 Hz), 7.70 (dd, 1H, $J_1$ = 1.2 Hz, $J_2$ = 7.6 Hz), 7.59 (t, 1H, $J$= 7.6 Hz), 7.47 (t, 1H, $J$= 8 Hz).

Synthesis of compound **12-i**

At room temperature, NaH (60%, 242 mg, 6.05 mmol) was added to 6 mL of THF. Under nitrogen atmosphere, methyl acetoacetate (543 μL, 5.04 mmol) was then added at room temperature; under nitrogen atmosphere, the mixture was stirred at room temperature for 30 minutes and then added dropwise n-BuLi (2.5 M, 2.4 mL, 6.05 mmol) at -15 °C to -10 °C. After completion of the addition, the mixture was kept at this temperature for 30 minutes, and then a solution of compound **12-j** (320 mg, 1.68 mmol) in THF (10 mL) was added dropwise. After completion of the addition, the mixture was stirred at low temperature (-10 °C to 0 °C) for 2 hours; the reaction was then quenched with a saturated ammonium chloride solution (50 mL) and extracted with ethyl acetate (50 mL * 2). The organic phase was washed with saturated brine (50 mL * 2), dried over anhydrous sodium sulfate, filtered and concentrated to give a crude product; the crude product was separated and purified through a flash column chromatography (EA/DCM = 1/10) to give compound **12-i** (510 mg, 99%

yield) as a white solid. LC-MS (ESI): m/z = 329.1 [M+Na]$^+$; $^1$H NMR (400 MHz, $CDCl_3$): δ 8.06 (d, 1H, $J$ = 6.4 Hz), 7.79 (d, 2H, $J$ = 8 Hz), 7.58 (dd, 1H, $J_1$ = 7.6 Hz, $J_2$ = 1.6 Hz), 7.53 (t, 1H, $J$ = 7.6 Hz), 7.34 (t, 1H, $J$ = 7.6 Hz), 6.91 (dd, 1H, $J_1$ = 9.2 Hz, $J_2$ = 2.4 Hz), 3.74 (s, 3H), 3.54 (s, 2H), 3.36 (dd, 1H, $J_1$ = 18 Hz, $J_2$ = 1.6 Hz), 3.24 (d, 1H, $J$ = 3.6 Hz), 2.85-2.75 (m, 1H).

Synthesis of compound **12-h**

At room temperature, compound **12-i** (510 mg, 1.66 mmol) was dissolved in DCM (18 mL), and then under nitrogen atmosphere, DMF-DMA (245 μL, 1.83 mmol) was added at room temperature. At room temperature, the reaction mixture was stirred for 45 minutes and then was added $BF_3.Et_2O$ (232 μL, 1.83 mmol). After completion of the addition, the mixture was stirred at room temperature for 1 hour and then diluted with 100 mL of ethyl acetate. The organic phase was washed successively with a saturated $NaHCO_3$ solution (100 mL) and saturated brine (100 mL * 2), dried over anhydrous sodium sulfate, filtered and concentrated to give crude product compound **12-h** (520 mg). The crude product was used directly in the next reaction without purification. LC-MS (ESI): m/z = 317.1 [M+1]$^+$.

Synthesis of compound **12-g**

At room temperature, compound **12-h** (520 mg, 1.64 mmol) was dissolved in THF (20 mL); at -78 °C, under nitrogen atmosphere, tri-sec-butyl lithium borohydride (1 M, 1.64 mL, 1.64 mmol) was then added dropwise. After completion of the addition, the mixture was stirred at -78 °C for 1 hour; the reaction was then quenched by adding a saturated ammonium chloride solution (50 mL) and extracted with ethyl acetate (50 mL * 2); the organic phase was washed with saturated brine (50 mL * 2), dried over anhydrous sodium sulfate, filtered and concentrated to give a crude product; the crude product was separated and purified through a flash column chromatography (PE/EA = 4/1) to give compound **12-g** (338 mg, 65% yield) as a yellow oil. LC-MS (ESI): m/z = 319.0 [M+1]$^+$.

Synthesis of compound **12-f**

At room temperature, compound **12-g** (338 mg, 1.06 mmol) was dissolved in methanol (20 mL); at 0 °C, under nitrogen atmosphere, sodium methylate (286 mg, 5.3 mmol) and compound 2-methyl-2-thiourea sulfate (265 mg, 0.954 mmol) were successively added. After completion of the addition, the mixture was warmed to room temperature and stirred for 20 hours. The pH of the reaction mixture was adjusted to 5 with 1 N dilute hydrochloric acid; the solid was precipitated out and filtered; the filter cake was washed with water (5 mL * 2) to collect solid, which was dried in vacuum to give crude product **12-f** (313 mg) as a white solid. LC-MS (ESI): m/z = 359.1 [M+1]$^+$.

Synthesis of compound **12-e**

At room temperature, compound **12-f** (313 mg, 0.87 mmol) was dissolved in DCM (10 mL); in an ice-water bath, under nitrogen atmosphere, DIPEA (431 μL, 2.61 mmol) and trifluoromethanesulfonic anhydride (219 μL, 1.31 mmol) were then successively added. After completion of the addition, the reaction mixture was stirred in an ice-water bath for 2 hours and then quenched with a saturated sodium bicarbonate solution (50 mL) and extracted with DCM (50 mL * 2); the organic phase was dried over anhydrous sodium sulfate, filtered and concentrated to give a crude product; the crude product was separated and purified through a flash column chromatography (EA/PE = 1/10) to give compound 12-e (83 mg, 16% yield for two steps) as a white solid. LC-MS (ESI): m/z = 491.0 [M+1]$^+$.

Synthesis of compound **12-d**

At room temperature, compound **12-e** (83 mg, 0.169 mmol) was dissolved in DMF (10 mL), and then DIPEA (84 μL, 0.507 mmol) and benzyl (S)-2-cyanomethylpiperazine-1-carboxylate hydrochloride (59.9 mg, 0.203 mmol) were successively added. After completion of the addition, under nitrogen protection, the mixture was stirred at 100 °C for 1 hour and then cooled to room temperature; the reaction was quenched with saturated brine (50 mL) and extracted with ethyl acetate (50 mL * 2). The organic phase was washed with saturated brine (50 mL * 3) and then dried over anhydrous sodium sulfate, filtered and concentrated to give a crude product; the crude product was separated and purified through a flash column chromatography (EA/PE = 1/1) to give compound **12-d** (101 mg, 99% yield) as a white solid. LC-MS (ESI): m/z = 600.2 [M+1]$^+$.

Synthesis of compound **12-c**

At room temperature, compound 12-d (101 mg, 0.168 mmol) was dissolved in ethyl acetate (10 mL), and MCPBA (85%, 88.4 mg, 0.437 mmol) was then added at room temperature. After completion of the addition, the mixture was stirred

at room temperature for 2 hours and then quenched with a saturated sodium bicarbonate solution (20 mL) and extracted with ethyl acetate (25 mL * 2); the organic phase was dried over anhydrous sodium sulfate, filtered and concentrated to give a crude product; the crude product was separated and purified through a flash column chromatography (EA/PE = 1/4) to give compound **12-c** (88 mg, 82% yield) as a white solid. LC-MS (ESI): m/z = 632.1 $[M+1]^+$.

Synthesis of compound **12-b**

At room temperature, compound **12-c** (88 mg, 0.139 mmol) was dissolved in toluene (10 mL), and then the reaction mixture was cooled to 0 °C; N-methylprolinol (29 μL, 0.243 mmol) and t-BuONa (27 mg, 0.278 mmol) were successively added. After completion of the addition, under nitrogen atmosphere, the reaction mixture was stirred in an ice-water bath for 0.5 hours and then quenched with water (20 mL) and extracted with ethyl acetate (30 mL * 2). The organic phase was dried over anhydrous sodium sulfate, filtered and concentrated to give a crude product; the crude product was separated and purified through a flash column chromatography (MeOH/DCM = 1/10) to give compound **12-b** (78 mg, 84% yield) as a white solid. LC-MS (ESI): m/z = 667.3 $[M+1]^+$;

Synthesis of compound **12-a**

At room temperature, compound **12-b** (72 mg, 0.108 mmol) was dissolved in methanol (50 mL); the reaction mixture was then cooled to -78 °C, replaced twice with nitrogen, and then added Pd/C (150 mg) and $ZnBr_2$ (24.3 mg, 0.108 mmol) and replaced three times with hydrogen; the reaction mixture was warmed to room temperature and stirred under hydrogen for 5 hours. The reaction mixture was filtered and concentrated to give a crude product; the crude product was separated and purified through a flash column chromatography (MeOH/DCM = 1 : 4) to give compound **12-a** (20 mg, 35% yield) as a white solid. LC-MS (ESI): m/z = 533.0 $[M+1]^+$.

Synthesis of compound 12

At room temperature, compound 2-fluoroacrylic acid (5.1 mg, 0.0563 mmol) was dissolved in DMF (2 mL); at 0 °C, HATU (25.6 mg, 0.0675 mmol) and DIPEA (18.6 μL, 0.113 mmol) were then successively added; after completion of the addition, under nitrogen atmosphere, the reaction mixture was stirred at 0 °C for 20 minutes, and a solution of compound **12-a** (20 mg, 0.0375 mmol) in DMF (3 mL) was then added to the above-mentioned reaction mixture, which was warmed to room temperature and continuously stirred for 5 hours. The reaction was quenched with saturated brine (20 mL) and extracted with ethyl acetate (25 mL * 2); the organic phase was washed with saturated brine (50 mL * 3), dried over anhydrous sodium sulfate, filtered and concentrated to give a crude product; the crude product was separated and purified by PREP-TLC (MeOH/DCM = 1/10) to give compound **12** (6 mg, 26% yield) as a white solid. LC-MS (ESI): m/z = 605.2 $[M+1]^+$; $^1H$ NMR (400 MHz, $CDCl_3$): δ 7.99-7.93 (m, 1H), 7.83 (t, 2H, *J* = 8.8 Hz), 7.62-7.49 (m, 2H), 7.36 (t, 1H, *J* = 7.6 Hz), 6.55-6.44 (m, 1H), 5.51-5.31m 1H), 5.25 (d, 1H, *J* = 16.8 Hz), 5.02-4.93 (m, 1H), 4.82 (dd, 1H, $J_1$ = 2.4 Hz, $J_2$ = 13.6 Hz), 4.48-4.38 (m, 1H), 4.32-4.19 (m, 1H), 4.17-4.04 (m, 1H), 4.00 (d, 1H, *J* = 14 Hz), 3.87-3.70 (m, 1H), 3.66-3.36 (m, 2H), 3.31-3.16 (m, 2H), 3.14-2.98 (m, 1H), 2.96-2.69 (m, 4H), 2.59 (d, 3H, *J* = 18 Hz), 2.52-2.34 (m, 1H), 2.15-2.06 (m, 1H), 1.87-1.74 (m, 2H), 0.93-0.76 (m, 2H).

Synthesis of compounds **12-1** and **12-2**

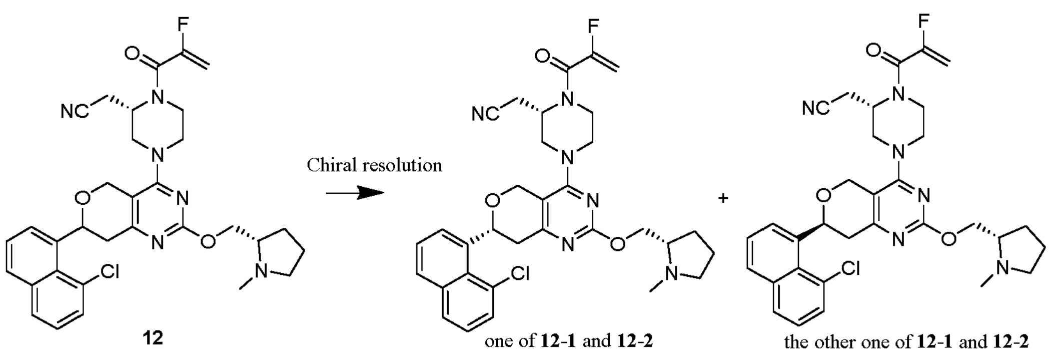


12 — one of **12-1** and **12-2** — the other one of **12-1** and **12-2**

The newly-prepared compound **12** (260 mg, 0.43 mmol) was subjected to chiral resolution to give compound **12-1** (76 mg, 29% yield) as a white solid and compound **12-2** (67 mg, 26% yield) as a white solid.

| Chiral analysis conditions | Chiral preparation conditions |
|---|---|
| equipment: SFC Method Station (Thar, Waters)<br>chromatographic column: OJ-H 4.6 * 100 mm, 5 μm (Daicel)<br>column temperature: 40 °C<br>mobile phase: $CO_2$/MeOH (0.1% TEA) = 60/40<br>flow rate: 4.0 ml/min<br>wavelength: 254 nm<br>back pressure: 120 bar | instrument: SFC-150 (Thar, Waters)<br>chromatographic column: OJ 20 * 250 mm, 10 μm (Daicel)<br>column temperature: 35 °C<br>mobile phase: $CO_2$/MEOH (0.1% TEA) = 40/60<br>flow rate: 120 g/min<br>back pressure: 100 bar<br>detection wavelength: 214 nm<br>cycling time: 6.0 min |
| **12-1:** retention time: 1.36 min; d.e.% = 100%;<br>**12-2:** retention time: 2.77 min; d.e.% = 97.5%. | |

**12-1:** LC-MS (ESI): m/z = 605.3 $[M+1]^+$; $^1$HNMR (400 MHz, $CDCl_3$) δ 7.96 (d, 1H, *J* = 7.2 Hz), 7.83 (t, 2H, *J* = 8.4 Hz), 7.65-7.50 (m, 2H), 7.36 (t, 1H, *J* = 8.0 Hz), 6.47 (dd, 1H, $J_1$ = 10.8 Hz, $J_2$ = 3.2 Hz), 5.42 (d, 1H, *J* = 49.2 Hz), 5.26 (dd, 1H, $J_1$ = 3.6 Hz, $J_2$ = 16.8 Hz), 5.05-4.76 (m, 1H), 4.97 (d, 1H, *J* = 13.6 Hz), 4.84 (d, 1H, *J* = 13.6 Hz), 4.36 (dd, 1H, $J_1$ = 4.8 Hz, $J_2$ = 10.4 Hz), 4.17 (dd, 1H, $J_1$ = 6.8 Hz, $J_2$ = 10.8 Hz), 4.06-3.87 (m, 1H), 3.77 (d, 1H, *J* = 10 Hz), 3.59 (dd, 1H, $J_1$ = 2.4 Hz, $J_2$ = 17.6 Hz), 3.50-3.15 (m, 3H), 3.14-2.99 (m, 2H), 2.96-2.82 (m, 2H), 2.72-2.59 (m, 1H), 2.47 (s, 3H), 2.32-2.21 (m, 1H), 2.10-1.98 (m, 1H), 1.89-1.67 (m, 4H).

**12-2:** LC-MS (ESI): m/z = 605.2 $[M+1]^+$; $^1$HNMR (400 MHz, $CDCl_3$) δ 7.97 (d, 1H, *J* = 7.2 Hz), 7.83 (t, 2H, *J* = 9.2 Hz), 7.63-7.51(m, 2H), 7.36 (t, 1H, *J* = 7.6 Hz), 6.52 (dd, 1H, $J_1$ = 3.2 Hz, $J_2$ = 10.8 Hz), 5.42 (d, 1H, *J* = 47.2 Hz), 5.25 (dd, 1H, $J_1$ = 3.6 Hz, $J_2$ = 16.4 Hz), 4.99 (d, 1H, *J* = 14.0 Hz), 4.82 (d, 1H, *J* = 13.6 Hz), 5.05-4.72 (m, 1H), 4.38 (dd, 1H, $J_1$ = 4.8 Hz, $J_2$ = 10.4 Hz), 4.15 (dd, 1H, $J_1$ = 6.8 Hz, $J_2$ = 10.8 Hz), 3.98 (d, 1H, *J* = 14 Hz), 3.87-3.73 (m, 1H), 3.60 (dd, 1H, $J_1$ = 2.4 Hz, $J_2$ = 18.4 Hz), 3.66-3.54 (m, 1H), 3.54-3.41 (m, 1H), 3.16-2.98 (m, 2H), 2.95-2.71 (m, 3H), 2.71-2.61 (m, 1H), 2.46 (s, 3H), 2.33-2.19 (m, 1H), 2.10-1.98 (m, 1H), 1.90-1.66 (m, 4H).

**Example 13** Synthetic route of compound **13**

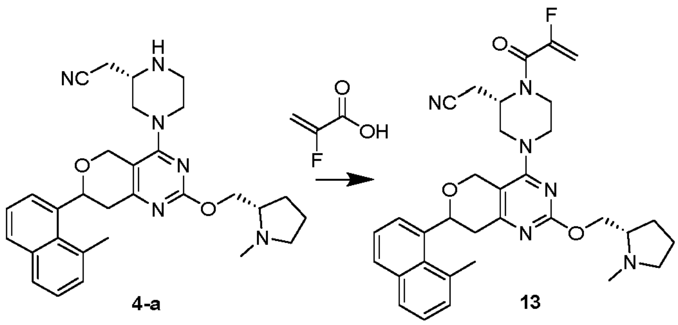

4-a

13

Synthesis of compound **13**

At room temperature, to a solution of **4-a** (240 mg, 0.47 mmol) in DMF (5 mL) were respectively added HATU (356 mg, 0.94 mmol), N,N-diisopropylethylamine (0.23 mL, 1.40 mmol) and 2-fluoroacrylic acid (63.2 mg, 0.70 mmol). The reaction mixture was stirred at room temperature overnight. After completion of the reaction, the solid was precipitated out by adding water and filtered; the filter cake was washed with water and dried to give a crude product, which was then purified by silica gel column chromatography (DCM/MeOH 10/1) to give product 13 (220 mg, 80%) as a white foamy solid. LC-MS (ESI): m/z = 585.0 $[M+H]^+$; $^1$H NMR (400 MHz, $CDCl_3$): δ 7.78-7.83 (m, 2H), 7.71-7.77 (m, 1H), 7.45-7.50 (m, 1H),7.33-7.38 (m, 2H), 6.00-6.07 (m, 1H), 5.19-5.25 (m, 1H), 4.89-4.97 (m, 1H), 4.66-4.72 (m, 2H), 4.54-4.60 (m, 2H), 3.98-4.08 (m, 1H), 3.90-3.98 (m, 1H), 3.75-3.89 (m, 1H), 3.58 -3.75 (m, 2H), 3.44-3.58 (m, 1H), 3.04 (d, 3H, *J* = 4.4 Hz), 2.96-3.37 (m, 6H), 2.93 (s, 3H), 2.65-2.86 (m, 2H), 2.26-2.42 (m, 2H), 2.07-2.24 (m, 2H).

Synthesis of compounds **13-1** and **13-2**

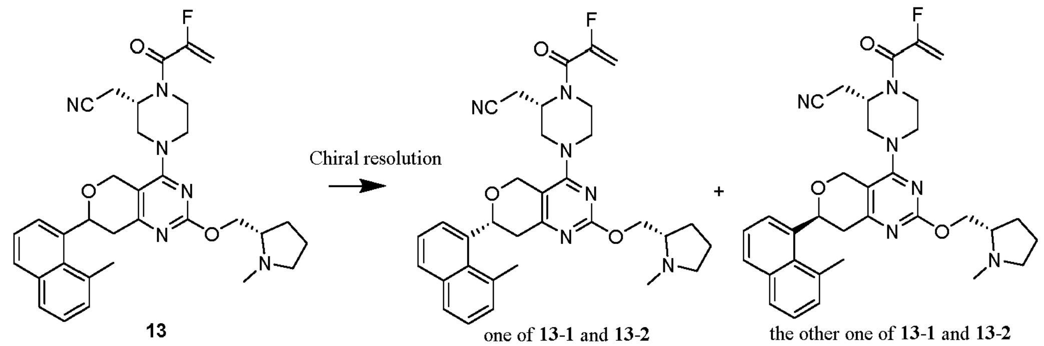


**13** one of **13-1** and **13-2** the other one of **13-1** and **13-2**

Compound **13** (200 mg, 0.34 mmol) was purified by chiral resolution to give compound **13-1** (70 mg, 35%) as a white solid and compound **13-2** (71 mg, 36%) as a white solid.

| Chiral analysis conditions | Chiral preparation conditions |
|---|---|
| equipment: SFC Method Station (Thar, Waters)<br>chromatographic column: OJ-H 4.6 * 100 mm, 5 μm (Daicel)<br>column temperature: 40 °C<br>mobile phase: $CO_2$/MeOH (0.1% TEA) = 60/40<br>flow rate: 4.0 ml/min<br>wavelength: 254 nm<br>back pressure: 120 bar | instrument: SFC-150 (Thar, Waters)<br>chromatographic column: OJ 20 * 250 mm, 10 μm (Daicel)<br>column temperature: 35 °C<br>mobile phase: $CO_2$/[MeOH : CAN = 1 : 1(0.1% TEA)] = 60/40<br>flow rate: 100 g/min<br>back pressure: 100 bar<br>detection wavelength: 214 nm<br>cycling time: 4.0 min |
| **13-1:** retention time: 1.17 min; d.e.% = 100%;<br>**13-2:** retention time: 2.76 min; d.e.% = 99.4%. | |

**13-1:** LC-MS (ESI): m/z = 585.0 $[M+1]^+$; $^1$H NMR (400 MHz, $CDCl_3$): δ 7.83 (d, 1H, *J* = 8.4 Hz), 7.74-7.77 (m, 2H), 7.46 (t, 1H, *J* = 8.0 Hz), 7.34-7.39 (m, 2H), 6.00 (dd, 1H, *J* = 9.6, 4.0 Hz), 5.41 (d, 1H, *J* = 46.8 Hz), 5.25 (dd, 1H, *J* = 16.8, 3.6 Hz), 4.84 (d, 1H, *J* = 14.0 Hz), 4.69 (d, 1H, *J* = 14.0 Hz), 4.51-5.08 (m, 1H), 4.38 (dd, 1H, *J* = 10.4, 5.2 Hz), 4.19 (dd, 1H, *J* = 10.4, 6.4 Hz), 3.81-4.15 (m, 2H), 3.69 (d, 1H, *J* = 11.2 Hz), 2.97-3.31 (m, 6H), 2.94(s, 3H), 2.77-2.89 (m, 1H), 2.64-2.74 (m, 1H), 2.49 (s, 3H), 2.25-2.34 (m, 1H), 2.01-2.11 (m, 1H), 1.68-1.89 (m, 4H).

**13-2:** LC-MS (ESI): m/z = 585.0 $[M+1]^+$; $^1$H NMR (400 MHz, $CDCl_3$): δ 7.82-7.84 (m, 2H), 7.75 (dd, 1H, *J* = 7.2, 2.0 Hz), 7.50 (t, 1H, *J* = 7.6 Hz), 7.33-7.38 (m, 2H), 6.00 (dd, 1H, *J* = 10.4, 3.2 Hz), 5.48 (d, 1H, *J* = 47.2 Hz), 5.25 (dd, 1H, *J* = 16.8, 3.6 Hz), 4.97 (d, 1H, *J* = 13.6 Hz), 4.76 (d, 1H, *J* = 13.6 Hz), 4.52-5.05 (m, 1H), 4.41 (dd, 1H, *J* = 10.8, 4.8 Hz), 4.21 (dd, 1H, *J* = 10.8, 6.0 Hz), 3.98 (d, 1H, *J* = 14.8 Hz), 3.91 (d, 1H, *J* = 12.8 Hz), 3.37-3.78 (m, 2H), 2.97-3.31 (m, 4H), 2.91(s, 3H), 2.69-2.84 (m, 3H), 2.54 (s, 3H), 2.33-2.40 (m, 1H), 2.03-2.14 (m, 1H), 1.76-1.93 (m, 4H).

**Example 14** Synthetic route of compound **14**

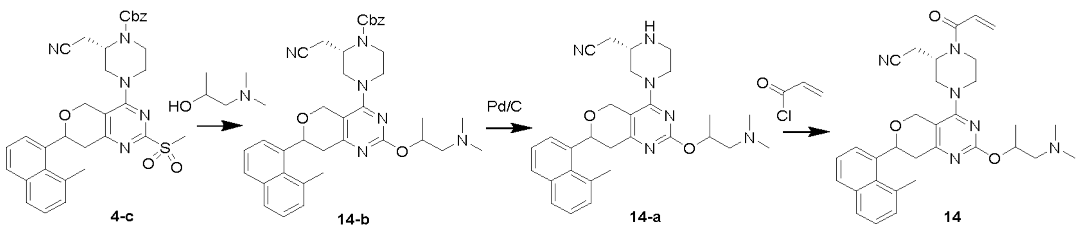


**4-c** **14-b** **14-a** **14**

Synthesis of compound **14-b**

Compound **4-c** (100 mg, 0.164 mmol) was dissolved in toluene (10 mL); in an ice bath, N,N-dimethyl isopropanolamine (33.7 mg, 0.327 mmol) and sodium tert-butoxide (31.4 mg, 0.327 mmol) were added; under nitrogen atmosphere, the mixture was stirred at this temperature for 30 minutes, and TLC monitoring indicated that the reaction was incomplete; N,N-dimethyl isopropanolamine (33.7 mg, 0.327 mmol) and sodium tert-butoxide (31.4 mg, 0.327 mmol) were then supplemented; the mixture was continuously stirred for 30 minutes and TLC monitoring indicated that the reaction was complete; the product was concentrated and subjected to column (biotage, 12 g, silica gel, UV254, MeOH : DCM = 0 to 8%) to give **14-b** (82 mg, 79%) as a white solid. LC-MS (ESI): m/z = 635.3 $[M+H]^+$.

Synthesis of compound **14-a**

**14-b** (82 mg, 0.129 mmol) was dissolved in methanol (10 mL) and ethyl acetate (10 mL), added 10% palladium-carbon (80 mg), replaced three times with hydrogen and stirred at room temperature for 2 hours; TLC monitoring indicated that the reaction was complete; the reaction was filtered and concentrated to give **14-a** (53 mg, 82%) as a white solid. LC-MS (ESI): m/z = 501.3 $[M+H]^+$.

Synthesis of compound **14**

**14-a** (53 mg, 0.106 mmol) was dissolved in dichloromethane (20 mL), and acryloyl chloride (14.3 mg, 0.159 mmol) and DIPEA (41 mg, 0.318 mmol) were successively added; under nitrogen atmosphere, the mixture was stirred at room temperature for 1 hour, and LCMS monitoring indicated that the reaction was complete; the reaction was then quenched with a saturated sodium bicarbonate solution, extracted with dichloromethane (30 mL X 3), dried, concentrated and subjected to column (biotage, 25 g, silica gel, UV254, MeOH : DCM = 0 to 10%) to give **14** (44 mg, 75%) as a white solid. LC-MS (ESI): m/z = 555.3 $[M+H]^+$; $^1H$ NMR (400 MHz, $CDCl_3$): δ 7.74-7.84 (m, 3H), 7.44-7.52 (m, 1H), 7.32-7.39 (m, 2H), 6.51-6.63 (m, 1H), 6.35-6.42 (m, 1H), 5.96-6.02 (m, 1H), 5.79-5.85 (m, 1H), 5.41-5.52 (m, 1H), 4.94-5.03 (m, 1H), 4.68-4.90 (m, 1H), 3.84-4.02 (m, 1H), 3.48-3.75 (m, 1H), 3.19-3.30 (m, 1H), 2.97-3.17 (m, 2H), 2.93 (d, 3H, *J* = 12.4 Hz), 2.68-2.87 (m, 2H), 2.56-2.65 (m, 1H), 2.42 (s, 6H), 1.96-2.33 (m, 5H), 1.33-1.38 (m, 3H).

**Example 15** Synthetic route of compound **15**

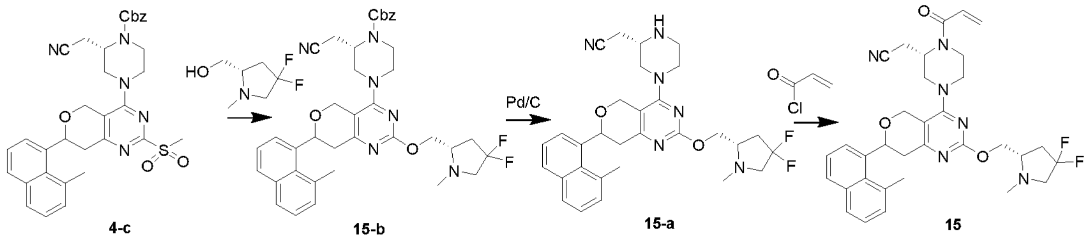


4-c 15-b 15-a 15

Synthesis of compound **15-b**

**4-c** (100 mg, 0.164 mmol) was dissolved in toluene (10 mL); in an ice bath, S-4,4-difluoro-1-methylpyrrolidin-2-methanol (49.4 mg, 0.327 mmol) and sodium tert-butoxide (31.4 mg, 0.327 mmol) were added; under nitrogen atmosphere, the mixture was stirred at this temperature for 30 minutes; LCMS monitoring indicated that the reaction was complete; the reaction was then quenched with water, extracted with ethyl acetate (50 mL X 2), dried, concentrated and subjected to column (biotage, 40 g, silica gel, UV254, EA : PE = 0 to 100%) to give **15-b** (97 mg, 87%) as a colorless gum. LC-MS (ESI): m/z = 683.3 $[M+H]^+$.

Synthesis of compound **15-a**

**15-b** (97 mg, 0.142 mmol) was dissolved in ethyl acetate (10 mL) and methanol (10 mL), added 10% palladium-carbon (97 mg), replaced three times with hydrogen and stirred at room temperature for 2 hours; TLC monitoring indicated that the reaction was complete; the product was filtered through diatomite and subjected to rotary evaporation to give **15-a** (70 mg, 90%) as a solid, which was used directly in the next reaction. LC-MS (ESI): m/z = 549.0 $[M+H]^+$.

Synthesis of compound **15**

**15-a** (70 mg, 0.128 mmol) was dissolved in dichloromethane (15 mL), and acryloyl chloride (17.2 mg, 0.192 mmol) and DIPEA (82.4 mg, 0.639 mmol) were successively added; under nitrogen atmosphere, the mixture was stirred at room temperature for 1 hour; LCMS monitoring indicated that the reaction was complete; the reaction was then quenched with a saturated sodium bicarbonate solution, extracted with dichloromethane (30 mL X 3), dried, concentrated and subjected to column (biotage, 12 g, silica gel, UV254, EA : PE = 0 to 100%) to give **15** (51 mg, 66%) as a white solid. LC-MS (ESI): m/z = 603.3 $[M+H]^+$; $^1H$ NMR (400 MHz, $CDCl_3$): δ 7.73-7.86 (m, 3H), 7.44-7.52 (m, 1H), 7.32-7.40 (m, 2H), 6.51-6.62 (m, 1H), 6.36-6.43 (m, 1H), 5.96-6.03 (m, 1H), 5.83 (d, 1H), 4.92-5.07 (m, 1H), 4.67-4.89 (m, 2H), 4.41-4.48 (m, 1H), 4.22-4.32 (m, 1H), 3.85-4.03 (m, 2H), 3.64-3.75 (m, 1H), 3.36-3.57 (m, 2H), 2.89-3.18 (m, 8H), 2.61-2.85 (m, 3H), 2.43-2.59 (m, 4H), 2.18-2.39 (m, 1H).

**Example 16** Synthetic route of compound **16**

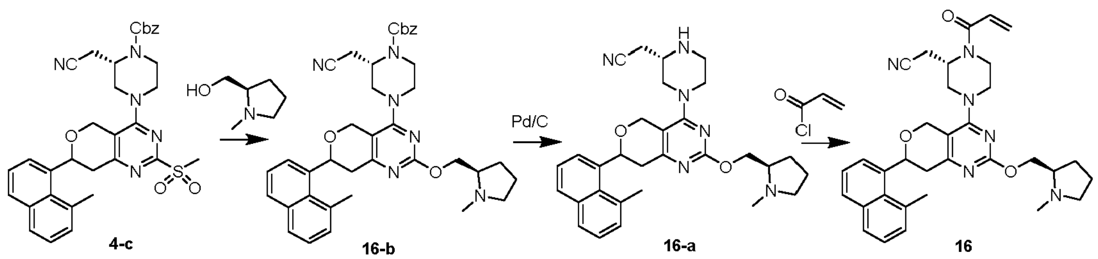


4-c 16-b 16-a 16

Synthesis of compound **16-b**

**4-c** (100 mg, 0.164 mmol) was dissolved in toluene (10 mL); in an ice bath, N-methyl-D-prolinol (37.6 mg, 0.327 mmol) and sodium tert-butoxide (31.4 mg, 0.327 mmol) were added; under nitrogen atmosphere, the mixture was stirred at this temperature for 30 minutes; TLC monitoring indicated that the reaction was complete; the reaction was then quenched with water, extracted with ethyl acetate (50 mL X 2), dried, concentrated and subjected to column (ISCO, 12 g, silica gel, UV254, MeOH : DCM = 0 to 7%) to give **16-b** (92 mg, 87%) as a white solid. LC-MS (ESI): m/z = 647.3 $[M+H]^+$.

Synthesis of compound **16-a**

**16-b** (92 mg, 0.142 mmol) was dissolved in ethyl acetate (10 mL) and methanol (10 mL), added 10% palladium-carbon (90 mg), replaced three times with hydrogen and stirred at room temperature for 2 hours; LCMS monitoring indicated that the reaction was complete; the product was filtered through diatomite and subjected to rotary evaporation to give **16-a** (62 mg, 85%) as a white solid, which was used directly in the next reaction. LC-MS (ESI): m/z = 513.3 $[M+H]^+$.

Synthesis of compound **16**

**16-a** (62 mg, 0.121 mmol) was dissolved in dichloromethane (20 mL), and acryloyl chloride (16.3 mg, 0.182 mmol) and DIPEA (46.9 mg, 0.363 mmol) were successively added; under nitrogen atmosphere, the mixture was stirred at room temperature for 1 hour; LCMS monitoring indicated that the reaction was complete; the reaction was then quenched with a saturated sodium bicarbonate solution, extracted with dichloromethane (40 mL X 4), dried, concentrated and subjected to column (biotage, 25 g, silica gel, UV254, MeOH : DCM = 0 to 15%) to give **16** (60 mg, 87%) as a pale brown solid. LC-MS (ESI): m/z = 567.3 $[M+H]^+$; $^1H$ NMR (400 MHz, $CDCl_3$): δ 7.74-7.84 (m, 3H),7.44-7.52 (m, 1H), 7.32-7.39 (m, 2H), 6.51-6.53 (m, 1H), 6.24-6.41 (m, 1H), 5.97-6.16 (m, 1H), 5.68-5.84 (m, 1H), 4.95-5.12 (m, 1H), 4.68-4.88 (m, 3H), 4.30-4.39 (m, 1H), 3.36-3.75 (m, 3H), 3.01-3.31 (m, 5H), 2.93 (d, 3H, *J* = 15.6 Hz), 2.61-2.83 (m, 4H), 2.51-2.61 (m, 1H), 1.84-2.31 (m, 7H).

**Example 17** Synthetic route of compound 17

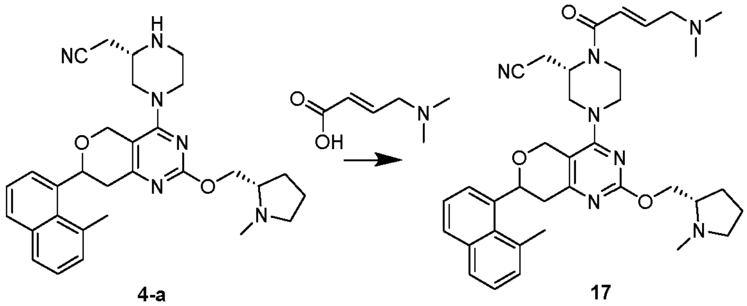

To a solution of **4-a** (50 mg, 0.1 mmol) and HATU (74.2 mg, 0.2 mmol) in DMF (5 mL) were respectively added DIPEA (0.05 mL, 0.3 mmol) and (2E)-4-(dimethylamino)-2-butenoic acid (18.9 mg, 0.015 mmol). The reaction mixture was heated to 60 °C and stirred overnight. After completion of the reaction, the solid was precipitated out by slowly adding water, collected by filtration and washed with water. After drying, a crude product was obtained and purified by silica gel column chromatography (DCM/MeOH 4/1) to give product 17 (17 mg, 28%) as a light brown solid. LC-MS (ESI): m/z = 624.3 $[M+H]^+$; $^1$H NMR (400 MHz, $CDCl_3$): δ 7.74-7.84 (m, 3H), 7.46-7.52 (m, 1H), 7.34-7.37 (m, 2H), 6.89-7.00 (m, 1H), 6.38-6.63 (m, 1H), 5.96-6.00 (m, 1H), 4.89-5.12 (m, 1H), 4.48-4.89 (m, 3H), 4.21-4.35 (m, 1H), 3.86-4.08 (m, 1H), 3.56-3.78 (m, 1H), 3.30-3.55 (m, 2H), 2.85 -3.29 (m, 8H), 2.92 (d, 3H, *J* = 9.6 Hz), 2.66 (d, 3H, *J* = 8.8 Hz), 2.41-2.57 (m, 1H), 2.32 (s, 6H), 2.04-2.22 (m, 2H), 1.76-2.03 (m, 4H).

**Example 18** Synthetic route of compound **18**

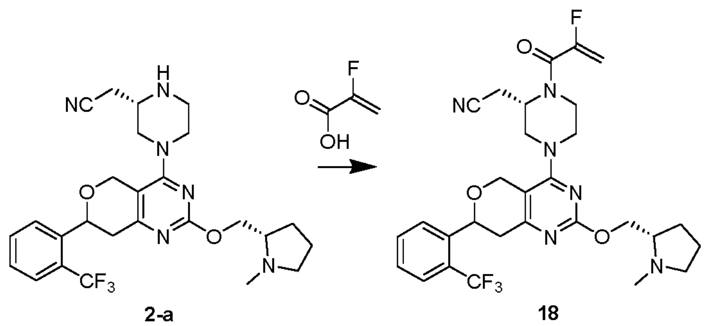

Synthesis of compound **18**

At room temperature, compound **2-a** (0.3 g, 0.58 mmol) was dissolved in DMF (10 mL), and DIPEA (0.15 g, 1.16 mmol), 2-fluoroacrylic acid (0.105 g, 1.16 mmol) and HATU (0.44 g, 1.16 mmol) were successively added. Under nitrogen atmosphere, the mixture was stirred at room temperature for 20 hours, and then the solid was precipitated out by adding water (100 mL), filtered and dried in vacuum to give compound 18 (300 mg, 88%) as a white solid. LC-MS (ESI): m/z = 589.2 $[M+1]^+$; $^1$H NMR (500 MHz, $CD_3OD$): δ 7.82-7-87 (m, 1H), 7.66-7.78 (m, 2H), 7.54 (t, 1H, *J* = 9.5 Hz), 5.33-5.41 (m, 1H), 5.27-5.32 (m, 1H), 5.18-5.23 (m, 1H), 5.03 (d, 1H, *J* = 20.0 Hz), 4.92-4.98 (m, 1H), 4.75-4.88 (m, 2H), 4.52 (t, 1H, *J* = 8.5 Hz), 4.20 (d, 1H, *J* = 9.5 Hz), 3.80-4.05 (m, 2H), 3.60-3.77 (m, 1H), 3.35-3.59 (m, 2H), 3.05-3.32 (m, 3H), 3.07 (d, 3H, *J* = 9.0 Hz), 2.83-3.03 (m, 3H), 2.30-2.43 (m, 1H), 2.15-2.26 (m, 1H), 1.98-2.15 (m, 2H), 1.28-1.41 (m, 1H).

Synthesis of compounds **18-1** and **18-2**

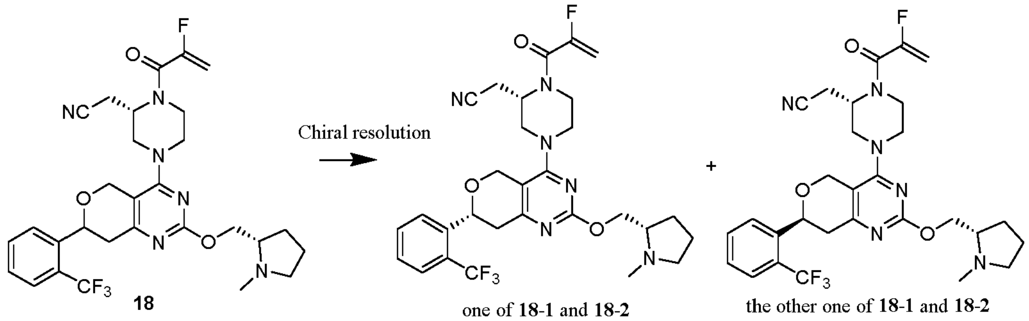


18

one of **18-1** and **18-2**

the other one of **18-1** and **18-2**

Compound **18** (280 mg, 0.48 mmol) was purified by chiral resolution to give compound **18-1** (96 mg, 34%) and compound **18-2** (68 mg, 24%).

| Chiral analysis conditions | Chiral preparation conditions |
|---|---|
| equipment: SFC Method Station (Thar, Waters)<br>chromatographic column: OD-H 4.6 * 100 mm, 5 μm (Daicel)<br>column temperature: 40 °C<br>mobile phase: CO2/MeOH (1% TEA) = 65/35<br>flow rate: 4.0 ml/min<br>wavelength: 254 nm<br>back pressure: 120 bar | instrument: SFC-150 (Thar, Waters)<br>chromatographic column: OD 20 * 250 mm, 10 μm (Daicel)<br>column temperature: 35 °C<br>mobile phase: CO2/MeOH (0.1% TEA) = 50/50<br>flow rate: 120 g/min<br>back pressure: 100 bar<br>detection wavelength: 214 nm<br>cycling time: 4.5 min |
| **18-1:** retention time: 0.78 min, d.e.% = 100%;<br>**18-2:** retention time: 2.42 min, d.e.% = 99.2% | |

18-1: LC-MS (ESI): m/z = 589.2 $[M+1]^+$; $^1H$ NMR (500 MHz, $CD_3OD$): δ 7.84 (d, 1H, *J* = 10.0 Hz), 7.68-7.78 (m, 2H), 7.54 (t, 1H, *J* = 10.0 Hz), 5.34 (d, 1H, *J* = 55.5 Hz), 5.33 (dd, 1H, $J_1$ = 4.5 Hz, $J_2$ = 21.0 Hz), 5.20 (dd, 1H, $J_1$ = 4.5 Hz, $J_2$ = 13.5 Hz), 5.03 (d, 1H, *J* = 17.5 Hz), 4.85 (d, 1H, *J* = 17.0 Hz), 4.73 (dd, 1H, $J_1$ = 4.5 Hz, $J_2$ = 16.0 Hz), 4.51 (dd, 1H, $J_1$ = 8.5 Hz, $J_2$ = 15.5 Hz), 4.47 (d, 1H, *J* = 15.0 Hz), 3.91 (d, 1H, *J* = 12.5 Hz), 3.70-3.86 (m, 1H), 3.60-3.70 (m, 1H), 3.30-3.42 (m, 2H), 3.10-3.28 (m, 3H), 3.01 (s, 3H), 2.96-3.07 (m, 1H), 2.80-2.94 (m, 1H), 2.30-2.43 (m, 1H), 1.97-2.21 (m, 3H).

18-2: LC-MS (ESI): m/z = 589.2 $[M+1]^+$; $^1H$ NMR (500 MHz, $CDCl_3$): δ 7.75 (d, 1H, *J* = 10.0 Hz), 7.60-7.70 (m, 2H), 7.45 (t, 1H, *J* = 9.5 Hz), 5.42 (d, 1H, *J* = 59.5 Hz), 5.25 (dd, 1H, $J_1$ = 4.0 Hz, $J_2$ = 21.0 Hz), 5.17 (dd, 1H, $J_1$ = 4.0 Hz, $J_2$ = 14.0 Hz), 4.88 (d, 1H, *J* = 17.0 Hz), 4.77 (d, 1H, *J* = 16.5 Hz), 4.39 (dd, 1H, $J_1$ = 6.5 Hz, $J_2$ = 13.0 Hz), 4.16 (dd, 1H, $J_1$ = 9.0 Hz, $J_2$ = 13.0 Hz), 3.96 (d, 1H, *J* = 17.5 Hz), 3.74 (d, 1H, *J* = 16.5 Hz), 3.48 (d, 1H, *J*= 14.0 Hz), 2.97-3.13 (m, 3H), 2.60-2.96 (m, 4H), 2.49 (s, 3H), 2.26-2.34 (m, 1H), 1.97-2.10 (m, 1H), 1.60-1.88 (m, 6H).

**Example 19** Synthesis of compounds **19-1** and **19-2**

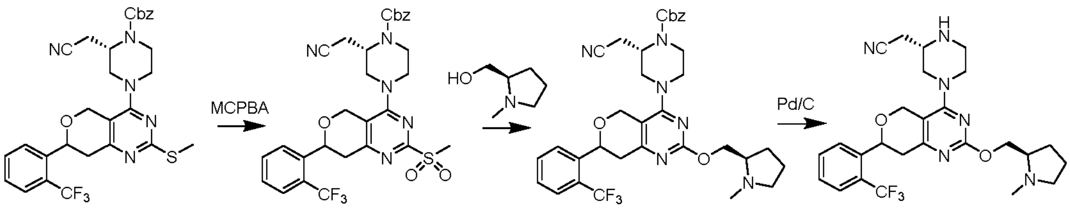


2-d 19-c 19-b 19-a

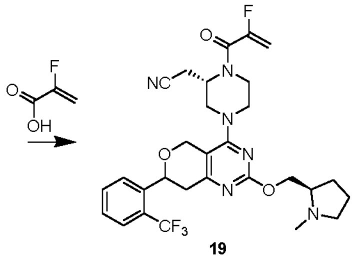

19

Synthesis of compound **19-c**

**2-d** (500 mg, 0.858 mmol) was dissolved in ethyl acetate (25 mL), added MCPBA (434 mg, 2.145 mmol) and stirred at room temperature for 2 hours; LCMS monitoring indicated that the reaction was complete; the reaction was then quenched with a saturated sodium bicarbonate solution, extracted with ethyl acetate (30 mL X 3), dried and concentrated to give **19-c** (527 mg, 100%) as a pale yellow solid, which was used directly in the next reaction without purification. LC-MS (ESI): m/z = 616.2 $[M+H]^+$.

Synthesis of compound **19-b**

**19-c** (527 mg, 0.857 mmol) was dissolved in toluene (25 mL); in an ice bath, N-methyl-D-prolinol (197 mg, 1.71 mmol) and sodium tert-butoxide (165 mg, 1.72 mmol) were added; under nitrogen atmosphere, the mixture was stirred at this temperature for 30 minutes; TLC monitoring indicated that the reaction was complete; the reaction was then quenched with water, extracted with ethyl acetate (50 mL X 2), dried, concentrated and subjected to column (biotage, 40 g, silica gel, UV254, MeOH : DCM = 0 to 10%) to give 19-b (452 mg, 81%) as a white solid. LC-MS (ESI): m/z = 651.2 $[M+H]^+$.

Synthesis of compound **19-a**

**19-b** (452 mg, 0.695 mmol) was dissolved in 7 N methanolic ammonia solution (50 mL) and added 10% palladium-carbon (250 mg) in an dry ice-acetone bath; the mixture was replaced three times with hydrogen and stirred at room temperature for 1 hour; TLC monitoring indicated that the reaction was complete; the product was filtered through diatomite and subjected to rotary evaporation to give **19-a** (359 mg, 100%) as a white solid, which was used directly in the next reaction. LC-MS (ESI): m/z = 517.2 $[M+H]^+$.

Synthesis of compound **19**

**19-a** (359 mg, 0.696 mmol) was dissolved in DMF (15 mL), and 2-fluoroacrylic acid (125 mg, 1.39 mmol), HATU (529 mg, 1.39 mmol) and DIPEA (449 mg, 3.48 mmol) were successively added; under nitrogen atmosphere, the mixture was stirred at room temperature for 1.5 hours; LCMS monitoring indicated that the reaction was complete; the reaction was then quenched with a saturated sodium bicarbonate solution and extracted with ethyl acetate (40 mL X 3); the organic phase was washed with saturated brine (50 mL X 3), dried, concentrated and subjected to column (biotage, 40 g, silica gel, UV254, MeOH : DCM = 0 to 10%) to give **19** (346 mg, 85%) as a pale brown solid. LC-MS (ESI): m/z = 589.0 $[M+H]^+$.

Synthesis of compounds **19-1** and **19-2**

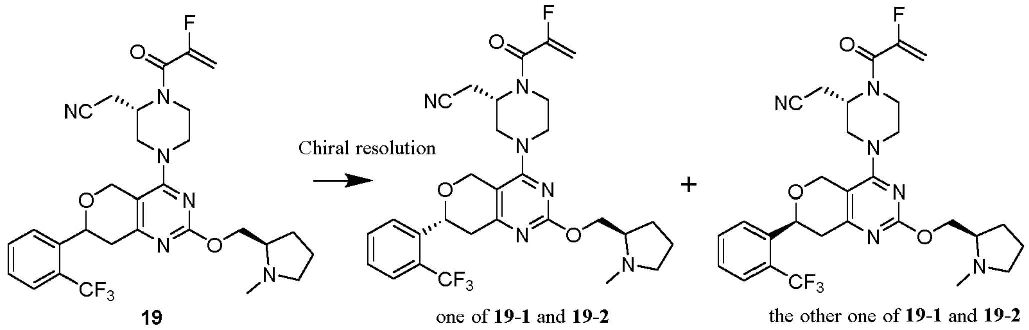


**19** (346 mg, 0.588 mmol) was subjected to resolution using chiral preparation and then subjected to rotary evaporation and lyophilized to give **19-1** (136 mg, 39%) and **19-2** (98 mg, 28%) as a white solid.

| Chiral analysis conditions | Chiral preparation conditions |
|---|---|
| equipment: SFC Method Station (Thar, Waters)<br>chromatographic column: OD-H 4.6 * 100 mm, 5 μm (Daicel)<br>column temperature: 40 °C<br>mobile phase: $CO_2$/MeOH (0.1% TEA) = 65/35<br>flow rate: 4.0 ml/min<br>wavelength: 254 nm<br>back pressure: 120 bar | instrument: SFC-150 (Thar, Waters)<br>chromatographic column: OD 20 * 250 mm, 10 μm (Daicel)<br>column temperature: 35 °C<br>mobile phase: $CO_2$/Methanol (0.1% TEA) = 40/60<br>flow rate: 120 g/min<br>back pressure: 100 bar<br>detection wavelength: 214 nm<br>cycling time: 3.0 min |
| **19-1:** retention time: 0.79 min; d.e.% = 100%;<br>**19-2:** retention time: 2.29 min; d.e.% = 100%. | |

19-1: LC-MS (ESI): m/z = 589.0 $[M+H]^+$; $^1$H NMR (400 MHz, $CDCl_3$): δ 7.74 (d, 1H, *J* = 8.0 Hz), 7.67 (d, 1H, *J*= 8.0 Hz), 7.63 (t, 1H, *J* = 7.6 Hz), 7.44 (t, 1H, *J* = 7.6 Hz), 5.38 (d, 1H, *J* = 48.0 Hz), 5.24 (dd, 1H, *J* = 3.6, 16.8 Hz), 5.19 (dd, 1H, *J* = 4.0, 11.2 Hz), 4.89 (d, 1H, *J* = 13.6 Hz), 4.72 (d, 1H, *J* = 13.6 Hz), 4.51-4.65 (m, 3H), 3.74-3.82 (m, 1H), 3.65-3.72 (m, 1H), 3.56-3.64 (m, 1H), 3.26-3.43 (m, 2H), 2.99-3.17 (m, 5H), 2.83-2.95 (m, 2H), 2.68-2.79 (m, 2H), 2.08-2.37 (m, 4H).

19-2: LC-MS (ESI): m/z = 589.0 $[M+H]^+$; $^1$H NMR (400 MHz, $CDCl_3$): δ 7.76 (d, 1H, J = 7.6 Hz), 7.67 (d, 1H, J = 8.0 Hz), 7.63 (t, 1H, J = 7.6 Hz), 7.44 (t, 1H, J = 8.0 Hz), 5.41 (d, 1H, J = 47.6 Hz), 5.25 (dd, 1H, J = 3.6, 16.8 Hz), 5.17 (dd, 1H, J = 3.2, 11.2 Hz), 4.88 (d, 1H, J = 14.0 Hz), 4.77 (d, 1H, J = 13.6 Hz), 4.41 (dd, 1H, J = 5.2, 10.8 Hz), 4.18 (dd, 1H, J = 6.4, 10.4 Hz), 3.98 (d, 1H, J = 14.4 Hz), 3.70-3.78 (m, 1H), 3.44-3.54 (m, 1H), 2.99-3.17 (m, 3H), 2.67-2.97 (m, 4H), 2.50 (s, 3H), 2.25-2.37 (m, 1H), 2.02-2.12 (m, 1H), 1.68-1.89 (m, 5H).

**Example 20** Synthetic route of compound **20**

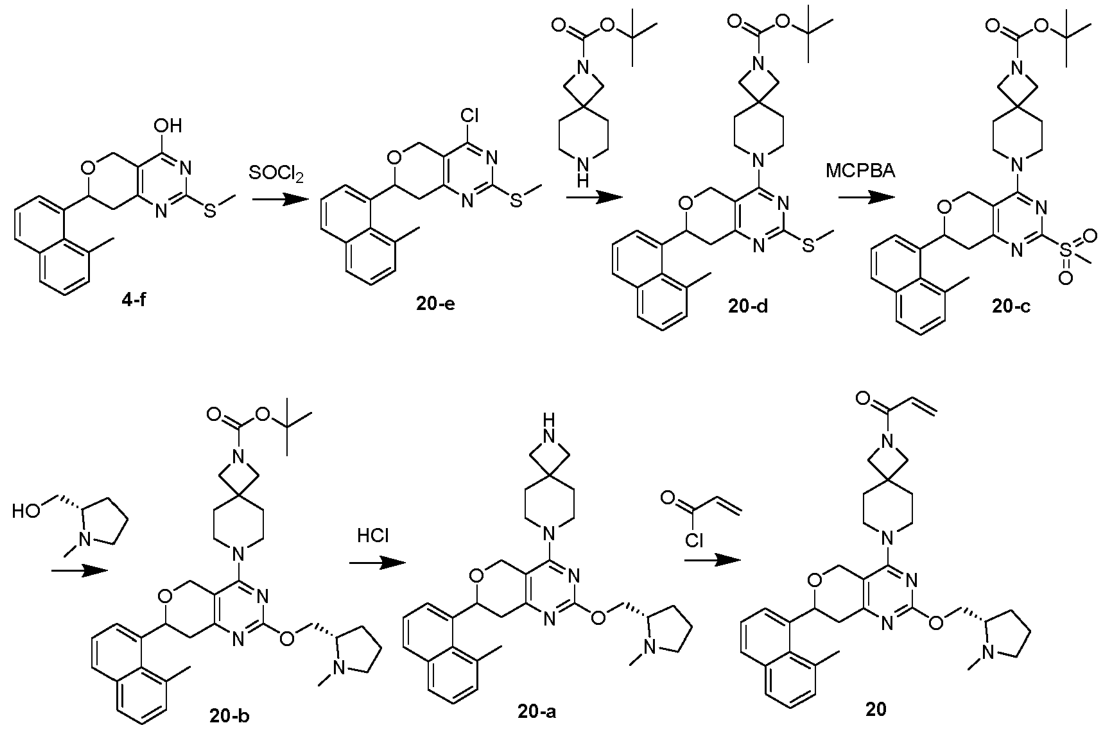

4-f 20-e 20-d 20-c 20-b 20-a 20

Synthesis of compound **20-e**

Compound **4-f** (1.7 g, 5.0 mmol) was dissolved in DMF (20 mL) and DCM (10 mL); at 0 °C, under nitrogen atmosphere, thionyl chloride (2.5 mL, 34.4 mmol) was added dropwise; and the mixture was stirred at 0 °C for 3 hours. The reaction was quenched by adding ice water (80 mL, 120 mmol), extracted with dichloromethane (80 mL * 2) and concentrated. The crude product was separated and purified through a flash column chromatography (petroleum ether/ethyl acetate = 25/1) to give compound **20-e** (1.1 g, 62%) as a pale yellow solid. LC-MS (ESI): m/z = 357.1 $[M+1]^+$.

Synthesis of compound **20-d**

At room temperature, compound **20-e** (0.25 g, 0.70 mmol) was dissolved in DMF (20 mL), and DIPEA (0.135 g, 1.05 mmol), 2-tert-butoxycarbonyl-2,7-diazaspiro[3.5]nonane (0.24 g, 1.05 mmol) were successively added. The mixture was stirred at 100 °C for 2 hours and then quenched by adding water (100 mL); the solid was precipitated out by filtration and dried to give compound **20-d** (0.29 g, 76%) as a grey solid. LC-MS (ESI): m/z = 547.3 $[M+1]^+$.

Synthesis of compound **20-c**

Compound **20-d** (0.29 g, 0.53 mmol) was dissolved in ethyl acetate (20 mL), and MCPBA (0.28 g, 1.36 mmol) was added at room temperature. The mixture was stirred at room temperature for 1 hour and then quenched by adding saturated sodium bicarbonate solution (500 mL) and extracted with ethyl acetate (50 mL * 2); the organic phase was concentrated, and the crude product was separated and purified through a flash column chromatography (DCM/MeOH = 20/1) to give compound **20-c** (0.31 g, 98%) as a white solid. LC-MS (ESI): m/z = 579.3 $[M+1]^+$.

Synthesis of compound **20-b**

Under ice-water bath cooling, to compound **20-c** (0.31 g, 0.536 mmol) in toluene (15 mL), N-methyl-L-prolinol (0.111 g, 0.97 mmol) and t-BuONa (0.103 g, 1.07 mmol) were successively added. Under nitrogen atmosphere, the mixture was stirred in an ice-water bath for 0.5 hours and then quenched by adding water (10 mL) and extracted with ethyl acetate (30 mL * 2); the organic phase was concentrated and the crude product was separated and purified through a flash column chromatography (MeOH/DCM = 1/9) to give compound **20-b** (0.28 g, 85%) as a white solid. LC-MS (ESI): m/z = 614.4 $[M+1]^+$.

Synthesis of compound **20-a**

Compound **20-b** (0.28 g, 0.46 mmol) was added to a mixture of dichloromethane (15 mL) and trifluoroacetic acid (15 mL), stirred for 2 hours under nitrogen atmosphere and concentrated to give compound **20-a** (0.26 g). LC-MS (ESI): m/z = 514.3 $[M+1]^+$.

Synthesis of compound **20**

At room temperature, compound **20-a** (0.26 g, 0.51 mmol) was dissolved in DCM (20 mL) and successively added DIPEA (0.15 g, 1.16 mmol) and acryloyl chloride (0.068 g, 0.76 mmol); under nitrogen atmosphere, the mixture was stirred at room temperature for 2 hours and then extracted by adding water (10 mL) and dichloromethane (30 mL * 2); the organic phase was concentrated; the crude product was separated and purified through a flash column chromatography (MeOH/DCM = 1/20) to give compound **20** (0.06 g, 23% for two reaction steps) as a white solid. LC-MS (ESI): m/z = 568.3 $[M+1]^+$; $^1H$ NMR (500 MHz, $CD_3OD$): δ 7.85 (d, 1H, *J* = 10.0 Hz), 7.83 (d, 1H, *J*= 9.5 Hz), 7.60-7.80 (m, 1H), 7.48 (t, 1H, *J* = 9.5 Hz), 7.33-7.41 (m, 2H), 6.20-6.43 (m, 2H), 6.06 (dd, 1H, $J_1$ = 5.0 Hz, $J_2$ = 12.5 Hz), 5.76 (dd, 1H, $J_1$ = 2.0 Hz, $J_2$ = 12.5 Hz), 5.01 (d, 1H, *J* = 17.5 Hz), 4.68 (d, 1H, *J* = 17.5 Hz), 4.41-4.59 (m, 2H), 4.08 (dd, 2H, $J_1$ = 10.5 Hz, $J_2$ = 16.0 Hz), 3.84 (dd, 2H, $J_1$ = 13.0 Hz, $J_2$ = 16.0 Hz), 3.46-3.60 (m, 2H), 3.34-3.45 (m, 4H), 3.10-3.22 (m, 1H), 2.94 (s, 3H), 2.80-2.92 (m, 1H), 2.82 (s, 3H), 2.20-2.31 (m, 1H), 1.79-2.10 (m, 8H).

**Example 21** Synthetic route of compound **32**

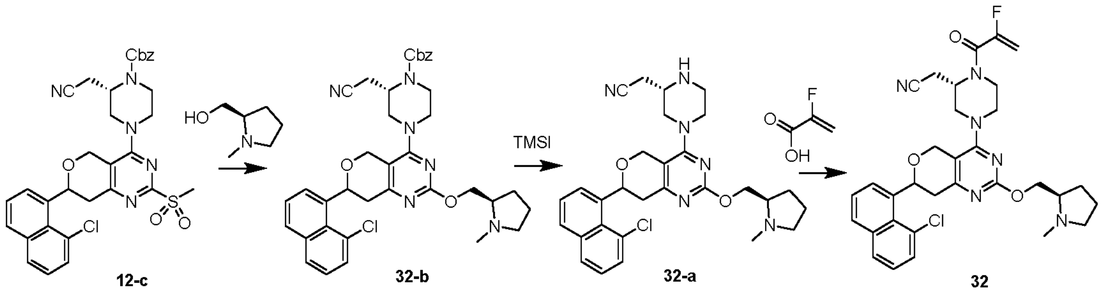


12-c 32-b 32-a 32

Synthesis of compound **32-b**

**12-c** (527 mg, 0.333 mmol) was dissolved in toluene (15 mL); in an ice bath, N-methyl-D-prolinol (76.5 mg, 0.666 mmol) and sodium tert-butoxide (64 mg, 0.667 mmol) were added; under nitrogen atmosphere, the mixture was stirred at this temperature for 30 minutes; TLC monitoring indicated that the reaction was complete; the reaction was then directly mixed with silica gel and subjected to column (biotage, 25 g, silica gel, UV254, MeOH : DCM = 0 to 10%) to give **32-b** (202 mg, 91%) as a white solid. LC-MS (ESI): m/z = 667.3 $[M+H]^+$.

Synthesis of compound **32-a**

**32-b** (202 mg, 0.303 mmol) was dissolved in acetonitrile (20 mL) and added trimethyliodosilane (200 uL, 1.4 mmol); under nitrogen atmosphere, at 30 °C, the mixture was stirred for 2 hours; LCMS monitoring indicated that the reaction was incomplete, and trimethyliodosilane (827 mg, 4.13 mmol) was then supplemented; the mixture was continuously stirred for 2 hours; LCMS monitoring indicated that the reaction was complete, and then 1 mL of triethylamine was added; the mixture was subjected to rotary evaporation to give **32-a** (crude product) as a dark solid, which was used directly in the next reaction without further purification. LC-MS (ESI): m/z = 533.0 $[M+H]^+$.

Synthesis of compound 32

**32-a** (crude product) was dissolved in DMF (10 mL), and 2-fluoroacrylic acid (54.6 mg, 0.607 mmol), HATU (231 mg, 0.608 mmol) and DIPEA (196 mg, 1.52 mmol) were successively added; under nitrogen atmosphere, the mixture was stirred at room temperature for 1.5 overnight; LCMS monitoring indicated that the reaction was complete; the reaction was then quenched with a saturated sodium bicarbonate solution and extracted with ethyl acetate (30 mL X 3); the organic phase was washed with saturated brine (50 mL X 3), dried, concentrated and subjected to column (biotage, 40 g, silica gel,

UV254, MeOH : DCM = 0 to 10%) to give **32** (151 mg, 82%). LC-MS (ESI): m/z = 605.0 [M+H]$^+$.

Synthesis of compounds **32-1** and **32-2**

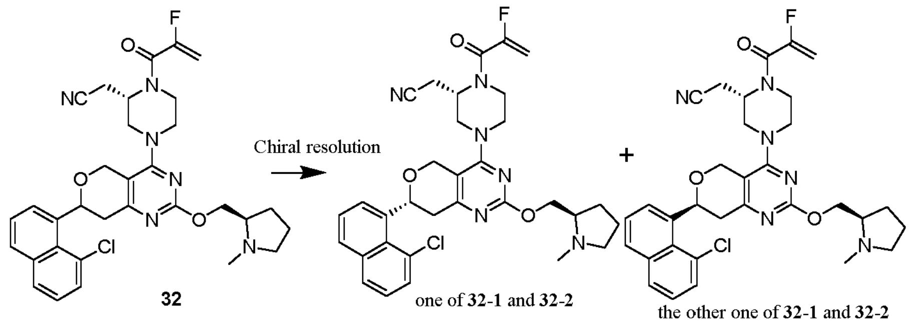


32 one of **32-1** and **32-2** + the other one of **32-1** and **32-2**

**32** (151 mg, 0.25 mmol) was subjected to resolution using chiral preparation and then subjected to rotary evaporation and lyophilized to give **32-1** (46 mg, 30%) and **32-2** (51 mg, 34%) as a white solid.

| Chiral analysis conditions | Chiral preparation conditions |
|---|---|
| equipment: SFC Method Station (Thar, Waters)<br>chromatographic column: OJ-H 4.6 * 100 mm, 5 μm (Daicel)<br>column temperature: 40 °C<br>mobile phase: $CO_2$/MeOH (0.1% TEA) = 60/40<br>flow rate: 4.0 ml/min<br>wavelength: 254 nm<br>back pressure: 120 bar | instrument: SFC-150 (Thar, Waters)<br>chromatographic column: OJ 20 * 250 mm, 10 μm (Daicel)<br>column temperature: 35 °C<br>mobile phase: $CO_2$/Methanol (0.1% TEA) = 40/60<br>flow rate: 120 g/min<br>back pressure: 100 bar<br>detection wavelength: 214 nm<br>cycling time: 5.0 min |
| **32-1:** retention time: 1.45 min; d.e.% = 100%;<br>**32-2:** retention time: 2.81 min; d.e.% = 100%. | |

**32-1:** LC-MS (ESI): m/z = 604.9 [M+H]$^+$; $^1$H NMR (400 MHz, $CDCl_3$): δ 7.96 (d, 1H, *J* = 7.2 Hz), 7.83 (t, 2H, *J* = 8.8 Hz), 7.60 (d, 1H, *J* = 7.2 Hz), 7.55 (t, 1H, *J* = 7.6 Hz), 7.37 (t, 1H, *J* = 7.6 Hz), 6.48 (dd, 1H, *J* = 2.8, 10.8 Hz), 5.42 (d, 1H, *J* = 47.6 Hz), 5.26 (dd, 1H, *J* = 2.8, 16.8 Hz), 4.97 (d, 1H, *J* = 13.6 Hz), 4.84 (d, 1H, *J* = 14.0 Hz), 4.38 (dd, 1H, *J* = 4.8, 10.8 Hz), 4.18 (dd, 1H, *J* = 6.8, 10.8 Hz), 3.98 (d, 1H, *J* = 10.0 Hz), 3.77 (d, 1H, *J* = 10.4 Hz), 3.58 (d, 1H, *J* = 16.8 Hz), 3.18-3.31 (m, 2H), 3.02-3.13 (m, 2H), 2.84-2.94 (m, 2H), 2.63-2.72 (m, 1H), 2.48 (s, 3H), 2.23-2.33 (m, 1H), 1.96-2.10 (m, 1H), 1.69-1.90 (m, 6H).

**32-2:** LC-MS (ESI): m/z = 605.0 [M+H]$^+$; $^1$H NMR (400 MHz, $CDCl_3$): δ 7.97 (d, 1H, *J* = 6.8 Hz), 7.84 (t, 2H, *J* = 9.2 Hz), 7.49-7.67 (m, 2H), 7.37 (t, 1H, *J* = 8.4 Hz), 6.51 (d, 1H, *J* = 10.8 Hz), 5.40 (d, 1H, *J* = 50.0 Hz), 5.25 (d, 1H, *J* = 15.6 Hz), 4.99 (d, 1H, *J* = 14.0 Hz), 4.83 (d, 1H, *J* = 13.6 Hz), 4.54-4.66 (m, 1H), 4.42-4.54 (m, 1H), 4.08-4.17 (m, 1H), 3.76-3.91 (m, 1H), 3.50-3.60 (m, 3H), 3.33-3.47 (m, 1H), 3.00-3.33 (m, 5H), 2.80-2.92 (m, 3H), 1.98-2.41 (m, 7H).

**Example 22** Synthetic route of compound **22**

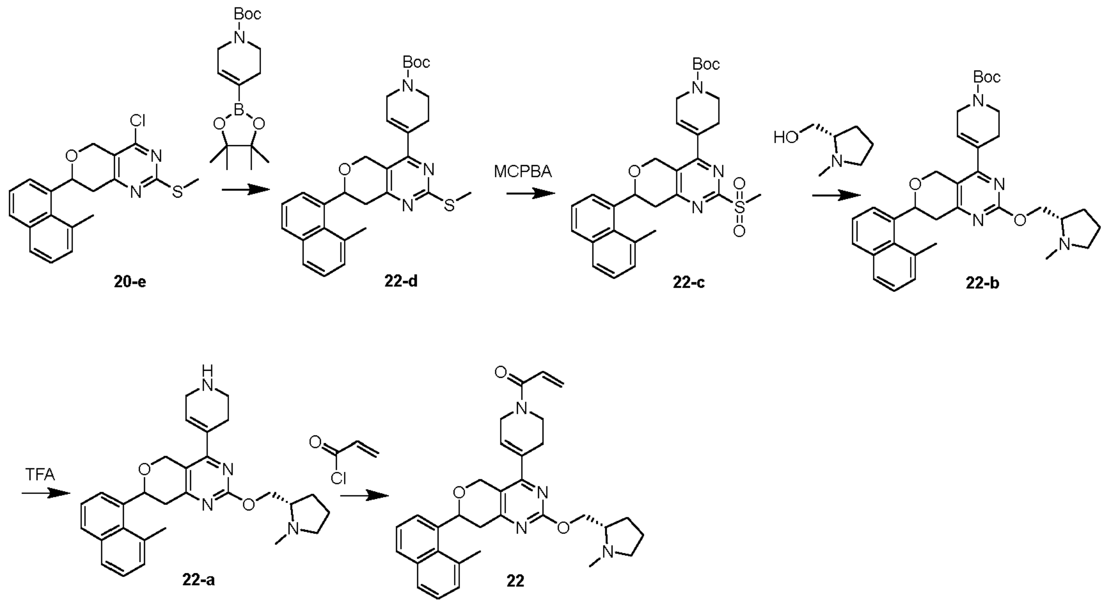


Synthesis of compound **22-d**

**20-e** (100 mg, 0.281 mmol) and N-Boc-1,2,5,6-tetrahydropyridine-4-boronic acid pinacol ester (104 mg, 0.337 mmol) were dissolved in 1,4- dioxane (10 mL), and water (1 mL), potassium carbonate (116 mg, 0.84 mmol) and tetrakis(triphenylphosphine)palladium (32 mg, 0.028 mmol) were successively added; the mixture was replaced three times with nitrogen and stirred at 100 °C overnight; LCMS monitoring indicated that the reaction was complete; the reaction was directly subjected to rotary evaporation, mixed with silica gel and subjected to column (biotage, 25 g, silica gel, UV254, EA : PE = 0 to 25%) to give **22-d** (137 mg, 97%) as a white solid. LC-MS (ESI): m/z = 504.3 $[M+H]^+$.

Synthesis of compound **22-c**

**22-d** (137 mg, 0.272 mmol) was dissolved in ethyl acetate (20 mL), added MCPBA (138 mg, 0.682 mmol) and stirred at room temperature for 2 hours; LCMS monitoring indicated that the reaction was complete; the reaction was then quenched with a saturated sodium bicarbonate solution, extracted with ethyl acetate (30 mL X 2), dried, concentrated and subjected to column (biotage, 25 g, silica gel, UV254, EA : PE = 0 to 50%) to give **22-c** (130 mg, 89%) as a white solid. LC-MS (ESI): m/z = 558.2 $[M+Na]^+$.

Synthesis of compound **22-b**

**22-c** (130 mg, 0.243 mmol) was dissolved in toluene (15 mL); in an ice bath, N-methyl-L-prolinol (55.9 mg, 0.486 mmol) and sodium tert-butoxide (47 mg, 0.489 mmol) were added; under nitrogen atmosphere, the mixture was stirred at this temperature for 30 minutes; LCMS monitoring indicated that the reaction was complete; and then, the reaction was directly mixed with silica gel and subjected to column (biotage, 25 g, silica gel, UV254, MeOH : DCM = 0 to 10%) to give **22-b** (65 mg, 47%) as a white solid. LC-MS (ESI): m/z = 571.3 $[M+H]^+$.

Synthesis of compound **22-a**

**22-b** (65 mg, 0.114 mmol) was dissolved in dichloromethane (10 mL), added trifluoroacetic acid (0.5 mL) and stirred at room temperature for 6 hours; LCMS monitoring indicated that the reaction was complete; trifluoroacetic acid was then removed, and saturated sodium bicarbonate solution was added; the mixture was extracted with dichloromethane (20 mL X 2), dried, and filtered to give the filtrate, i.e., **22-a** (solution), which was used directly in the next reaction. LC-MS (ESI): m/z = 471.3 $[M+H]^+$.

Synthesis of compound **22**

To **22-a** solution, acryloyl chloride (15.4 mg, 0.171 mmol) and DIPEA (73.6 mg, 0.57 mmol) were added; under nitrogen atmosphere, the mixture was stirred at room temperature for 2 hours; LCMS monitoring indicated that the reaction

was complete; the reaction was then quenched with a saturated sodium bicarbonate solution, extracted with dichloromethane (40 mL X 2), dried, concentrated and subjected to column (ISCO, 25 g, silica gel, UV254, MeOH : DCM = 0 to 10%) to give **22** (20 mg, 33% yield for two steps) as a solid. LC-MS (ESI): m/z = 525.3 $[M+H]^+$; $^1H$ NMR (400 MHz, $CD_3OD$): $\delta$ 7.73-7.87 (m, 3H), 7.48 (t, 1H, *J* = 7.2 Hz), 7.33-7.40 (m, 2H), 6.52-6.69 (m, 1H), 6.31-6.39 (m, 1H), 5.87-6.01 (m, 2H), 5.71-5.80 (m, 1H), 4.85-4.97 (m, 2H), 4.65-4.78 (m, 1H), 4.23-4.48 (m, 2H), 3.73-3.93 (m, 2H), 3.39-3.52 (m, 1H), 3.26-3.36 (m, 1H), 3.10-3.21 (m, 1H), 2.92 (d, 3H, *J* = 2.0 Hz), 2.75 (s, 3H), 2.56-2.68 (m, 1H), 2.43-2.55 (m, 1H), 1.87-2.27 (m, 7H).

**Example 23** Synthetic route of compound **23**

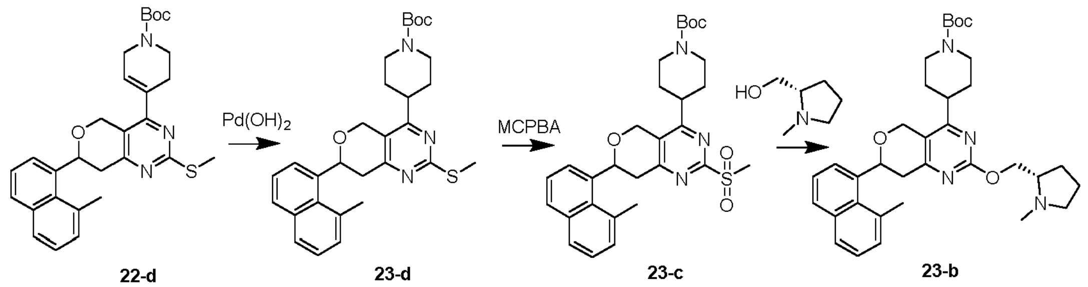


**22-d** **23-d** **23-c** **23-b**

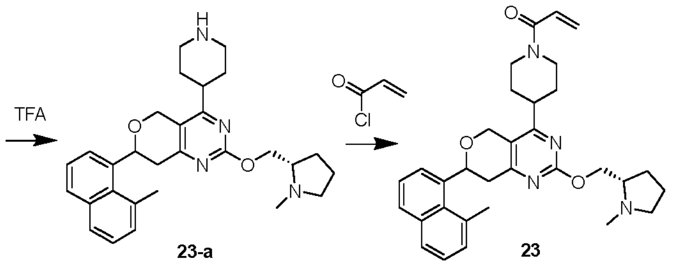


**23-a** **23**

Synthesis of compound **23-d**

**22-d** (200 mg, 0.398 mmol) was dissolved in methanol (100 mL), added palladium hydroxide (200 mg), replaced three times with hydrogen and stirred at 50 °C for 2 hours; LCMS monitoring the reaction was incomplete, and palladium hydroxide (200 mg) was then supplemented; the mixture was continuously reacted for 2 hours, and the reaction was incomplete; palladium hydroxide (200 mg) was then supplemented, and the reaction was continued for 2 hours; LCMS monitoring indicated that the reaction was complete; the reaction was then filtered, concentrated and subjected to column (biotage, 25 g, silica gel, UV254, EA : PE = 0 to 20%) to give **23-d** (80 mg, 40%) as a white solid. LC-MS (ESI): m/z = 506.3 $[M+H]^+$.

Synthesis of compound **23-c**

**23-d** (80 mg, 0.158 mmol) was dissolved in ethyl acetate (30 mL), added MCPBA (80 mg, 0.395 mmol) and stirred at room temperature for 2 hours; LCMS monitoring indicated that the reaction was complete; the reaction was then quenched with a saturated sodium bicarbonate solution, extracted with ethyl acetate (30 mL X 2), dried and concentrated to give **23-c** (85 mg, 100%) as a white solid, which was used directly in the next reaction.

Synthesis of compound **23-b**

**23-c** (85 mg, 0.158 mmol) was dissolved in toluene (20 mL); in an ice bath, N-methyl-L-prolinol (36.4 mg, 0.316 mmol) and sodium tert-butoxide (30 mg, 0.313 mmol) were added; under nitrogen atmosphere, the mixture was stirred at this temperature for 30 minutes; LCMS monitoring indicated that the reaction was complete; the reaction was then quenched with water, extracted with ethyl acetate (30 mL X 2), dried, concentrated and subjected to column (biotage, 12 g, silica gel, UV254, MeOH : DCM = 0 to 10%) to give **23-b** (52 mg, 57%) as a white solid. LC-MS (ESI): m/z = 573.3 $[M+H]^+$.

Synthesis of compound **23-a**

**23-b** (52 mg, 0.091 mmol) was dissolved in dichloromethane (10 mL), added trifluoroacetic acid (1 mL) and stirred at room temperature for 2 hours; LCMS monitoring indicated that the reaction was complete; and then trifluoroacetic acid was removed at room temperature, and saturated sodium bicarbonate solution was added; the mixture was extracted with dichloromethane (30 mL X 3), dried, filtered and concentrated to give **23-a** (43 mg, 100%) as a solid, which was used directly in the next reaction. LC-MS (ESI): m/z = 473.3 $[M+H]^+$.

Synthesis of compound **23**

**23-a** (43 mg, 0.091 mmol) was dissolved in dichloromethane (20 mL) and added acryloyl chloride (12.3 mg, 0.137 mmol) and DIPEA (58.8 mg, 0.456 mmol); under nitrogen atmosphere, the mixture was stirred at room temperature for 2 hours; LCMS monitoring indicated that the reaction was complete; the reaction was then quenched with a saturated sodium bicarbonate solution, extracted with dichloromethane (30 mL X 3), dried, concentrated and subjected to column (ISCO, 12 g, silica gel, UV214, MeOH : DCM = 0 to 10%) to give 23 (26 mg, 54%) as a pale brown solid. LC-MS (ESI): m/z = 527.3 $[M+H]^+$; $^1H$ NMR (400 MHz, $CDCl_3$): δ 7.74-7.87 (m, 3H), 7.49 (t, 1H, *J* = 7.2 Hz), 7.32-7.39 (m, 2H), 6.63 (dd, 1H, *J* = 16.8, 10.4 Hz), 6.31 (dd, 1H, *J* = 16.8, 1.60 Hz), 5.87-5.93 (m, 1H), 5.72 (dd, 1H, *J* = 10.4, 1.6 Hz), 4.91-5.02 (m, 2H), 4.61-4.88 (m, 2H), 4.36-4.48 (m, 1H), 4.11-4.21 (m, 1H), 3.40-3.58 (m, 1H), 3.10-3.27 (m, 4H), 2.89 (d, 3H, *J* = 3.2 Hz), 2.58-2.84 (m, 6H), 1.75-2.26 (m, 8H).

**Example 24** Synthetic route of compound **24**

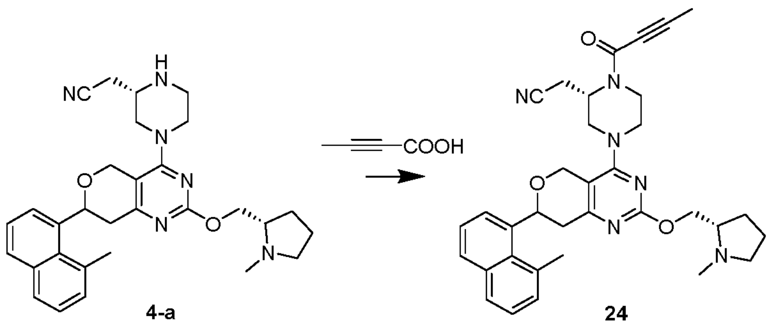


4-a

24

Synthesis of compound **24**

In an ice bath, to a solution of **4-a** (50 mg, 0.1 mmol) in DMF (5 mL) were successively added triethylamine (0.034 mL, 0.24 mmol), 2-tetrolic acid (12.3 mg, 0.15 mmol) and 1-propyl phosphoric anhydride (46.5 mg, 0.073 mmol). After completion of the addition, the reaction mixture was warmed to room temperature and stirred overnight. After completion of the addition, the solid was precipitated out by slowly adding water, collected by filtration, washed with water and dried to give a crude product, which was then purified by silica gel column chromatography (DCM/MeOH 10/1) to give product **24** (20 mg, 35% yield) as a white solid. LC-MS (ESI): m/z = 579.0 $[M+H]^+$; $^1H$ NMR (400 MHz, $CDCl_3$): δ 7.74-7.84 (m, 3H), 7.44-7.52 (m, 1H), 7.31-7.40 (m, 2H), 5.98-6.01 (m, 1H), 4.65-5.03 (m, 3H), 4.44-4.56 (m, 1H), 4.31-4.38 (m, 1H), 4.10-4.30 (m, 1H), 3.90-4.08 (m, 1H), 3.61-3.84 (m, 1H), 3.36-3.53 (m, 1H), 2.96-3.32 (m, 5H), 2.92 (d, 3H, *J* = 12.8 Hz), 2.67-2.88 (m, 2H), 2.57 (s, 3H), 2.28-2.65 (m, 2H), 2.00-2.17 (m, 4H), 1.69-1.99 (m, 3H).

**Example 25** Synthetic route of compound **25**

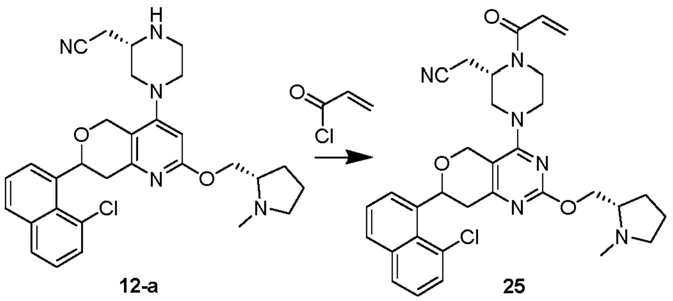

12-a 25

Synthesis of compound **25**

At room temperature, compound **12-a** (55 mg, 0.103 mmol) was dissolved in DCM (10 mL), and then DIPEA (85 uL, 0.515 mmol) and acryloyl chloride (14 mg, 0.155 mmol) were successively added. After completion of the addition, under nitrogen atmosphere, the reaction mixture was stirred at room temperature for 3 hours and then quenched with a saturated sodium bicarbonate solution (50 mL) and extracted with DCM (50 mL * 2). The organic phase was dried over anhydrous sodium sulfate, filtered and concentrated to give a crude product; the crude product was separated and purified through a flash column chromatography (MeOH/DCM = 1/10) to give compound **25** (30 mg, 50% yield) as a white solid. LC-MS (ESI): m/z = 587.3 $[M+1]^+$; $^1$H NMR (400 MHz, $CDCl_3$): $\delta$ 7.96 (d, 1H, *J* = 7.2 Hz), 7.83 (t, 2H, *J* = 9.2 Hz), 7.64-7.49 (m, 2H), 7.36 (t, 1H, *J* = 7.6 Hz), 6.68-6.64 (m, 2H), 6.39 (d, 1H, *J* = 16.8 Hz), 6.31-6.04 (m, 1H), 5.83 (d, 1H, *J* = 10.4 Hz), 5.73-4.90 (m, 2H), 4.89-4.79 (m, 1H), 4.79-4.68 (m, 1H), 4.45-4.31 (m, 1H), 4.16-3.64 (m, 3H), 3.66-3.43 (m, 3H), 3.30-3.16 (m, 2H), 3.00-2.79 (m, 3H), 2.74 (d, 3H, *J* = 8.4 Hz), 2.63 (t, 1H, *J* = 8.4 Hz), 2.26-2.13 (m, 1H), 2.13-2.00 (m, 1H), 2.00-1.84 (m, 2H).

**Example 26** Synthetic route of compound 30

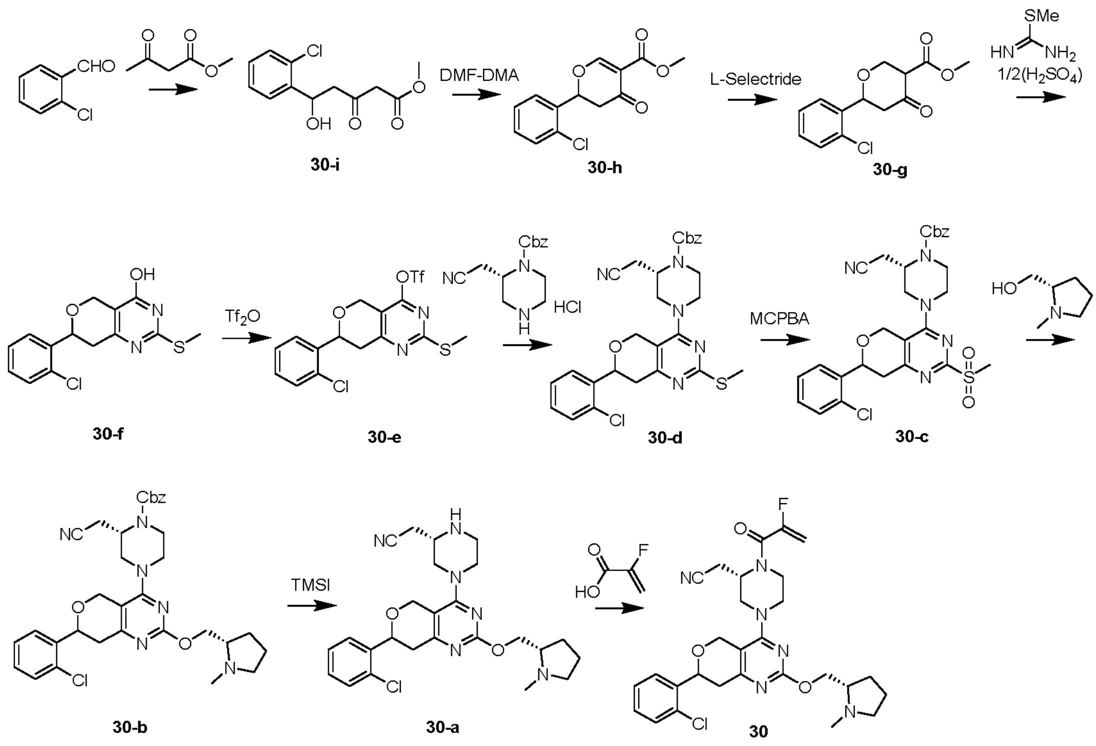


30-i 30-h 30-g 30-f 30-e 30-d 30-c 30-b 30-a 30

Synthesis of compound 30-i

At room temperature, NaH (60%, 4.26 g, 106.54 mmol) was added to 150 mL of THF, and then under nitrogen atmosphere, methyl acetoacetate (11.49 mL, 106.54 mmol) was added. Under nitrogen atmosphere, the mixture was

stirred at room temperature for 30 minutes, and then n-BuLi (2.5 M, 42.62 mL, 106.54 mmol) was added dropwise at -15 °C to -10 °C; the mixture was kept at this temperature for 30 minutes, and a solution of compound o-chlorobenzaldehyde (4.0 mL, 35.51 mmol) in THF (20 mL) was then added dropwise. The mixture was stirred at low temperature (-10 °C to 0 °C) for 2 hours; the reaction was then quenched with a saturated ammonium chloride solution (100 mL) and extracted with ethyl acetate (100 mL * 3); the organic phase was washed with saturated brine (100 mL), dried over anhydrous sodium sulfate, filtered and concentrated to give a crude product; the crude product was purified through a flash column chromatography to give **30-i** (7.9 g, 87%) as a pale yellow oil.

Synthesis of compound **30-h**

Compound **30-i** (7.9 g, 30.78 mmol) was dissolved in DCM (250 mL); under nitrogen atmosphere, DMF-DMA (4.9 mL, 36.93 mmol) was then added at room temperature; the mixture was stirred at room temperature for 45 minutes and then added $BF_3.Et_2O$ (4.6 mL, 36.93 mmol); and then the mixture was stirred at room temperature for 1 hour and then diluted with 200 mL of dichloromethane; the organic phase was washed successively with a saturated $NaHCO_3$ solution (400 mL) and saturated brine (200 mL), dried over anhydrous sodium sulfate, filtered and concentrated to give a crude product. The crude product was purified through a flash column chromatography to give **30-h** (7.5 g, 91%) as a yellow oil. LC-MS (ESI): m/z = 267.0 $[M+1]^+$.

Synthesis of compound **30-g**

Compound **30-h** (7.5 g, 28.12 mmol) was dissolved in THF (200 mL); at -78 °C, under nitrogen atmosphere, a solution of lithium tri-sec-butyl borohydride in tetrahydrofuran (1 M, 30.94 mL, 30.94 mmol) was then added dropwise; the mixture was stirred at this temperature for 1 hour, and then the reaction was quenched with saturated ammonium chloride (100 mL) and extracted with ethyl acetate (100 mL * 3); the organic phase was concentrated to give a crude product, which was purified through a flash column chromatography to give **30-g** (5 g, 66%) as a yellow oil. LC-MS (ESI): m/z = 269.0 $[M+1]^+$.

Synthesis of compound **30-f**

Compound **30-g** (5 g, 18.6 mmol) was dissolved in methanol (30 mL), and then at room temperature, sodium carbonate (13.8 g, 111.7 mmol) and compound 2-methyl-2-thiourea sulfate (10.4 g, 37.2 mmol) were successively added. The mixture was stirred at room temperature overnight. After completion of the reaction, the pH was adjusted to 5 with 1 N dilute hydrochloric acid, and then white solid was precipitated out, filtered, washed with water, dried over anhydrous sodium sulfate, filtered and concentrated to give **30-f** (4 g, 70%) as a white solid. LC-MS (ESI): m/z = 309.1 $[M+1]^+$.

Synthesis of compound **30-e**

In an ice bath, to a solution of compound **30-f** (4.0 g, 12.9 mmol) in DCM (100 mL) were respectively added DIPEA (4.28 mL, 25.9 mmol) and trifluoromethanesulfonic anhydride (3.3 mL, 19.4 mmol). After completion of the addition, the reaction mixture was slowly warmed to room temperature and stirred for 2 hours. The reaction mixture was quenched by adding saturated aqueous sodium bicarbonate solution and extracted with ethyl acetate. The organic phase was washed with saturated aqueous sodium chloride solution, dried over anhydrous sodium sulfate, filtered and subjected to rotary evaporation to give a crude product compound, which was purified through a flash column chromatography to give **30-e** (4 g, 70%) as a white solid. LC-MS (ESI): m/z = 441.0 $[M+1]^+$.

Synthesis of compound **30-d**

At room temperature, to a solution of **30-e** (4.0 g, 9.07 mmol) in DMF (10 mL) were respectively added N,N-diisopropylethylamine (4.5 mL, 27.2 mmol) and benzyl (S)-2-cyanomethylpiperazine-1-carboxylate hydrochloride (2.95 g, 9.98 mmol). After completion of the addition, the reaction mixture was heated to 100 °C and stirred for two hours. After completion of the reaction, the reaction mixture was diluted by adding ethyl acetate, washed successively with water and saturated brine, dried over anhydrous sodium sulfate, filtered and subjected to rotary evaporation. The crude product was separated and purified through a flash column chromatography to give **30-d** (4 g, 80%) as a white solid. LC-MS (ESI): m/z = 550.2 $[M+H]^+$.

Synthesis of compound **30-c**

In an ice bath, compound **30-d** (0.4 g, 0.73 mmol) was dissolved in ethyl acetate (20 mL) and then added m-chloroperoxybenzoic acid (369.1 mg, 1.82 mmol); the mixture was slowly warmed to room temperature and stirred for 2 hours. After completion of the reaction, the reaction mixture was neutralized by adding saturated aqueous sodium bicarbonate solution and extracted with dichloromethane (15 mL * 2); the organic phase was combined, dried over anhydrous sodium sulfate, filtered and concentrated; the crude product was purified through a flash column chromatography (DCM/MeOH = 10/1) to give **30-c** (0.35 g, 83%) as a white solid. LC-MS (ESI): m/z = 582.2 $[M+1]^+$.

Synthesis of compound **30-b**

In an ice bath, to a solution of **30-c** (350 mg, 0.6 mmol) and N-methyl-L-prolinol (138.5 mg, 1.2 mmol) in toluene (10 mL) was added sodium tert-butoxide (115.6 mg, 1.2 mmol). After completion of the addition, the reaction mixture was stirred in an ice bath for 10 minutes. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **30-b** (300 mg, 80%) as a tawny oil. LC-MS (ESI): m/z = 617.2 $[M+H]^+$.

Synthesis of compound **30-a**

At room temperature, to a solution of **30-b** (300 mg, 0.49 mmol) in acetonitrile (40 mL) was added trimethyliodosilane (0.35 mL, 2.43 mmol). After completion of the addition, the reaction mixture was heated to 30 °C and stirred for 5 hours. After completion of the addition, the reactant was neutralized with triethylamine (10 mL) and concentrated under reduced pressure to give **30-a** (200 mg, 85%) as a dark grey solid. LC-MS (ESI): m/z = 483.1 $[M+H]^+$.

Synthesis of compound **30**

At room temperature, to a solution of 2-fluoroacrylic acid (55.9 mg, 0.62 mmol) and HATU (314.9 mg, 0.83 mmol) in DMF (5 mL) were added N,N-diisopropylethylamine (0.21 mL, 1.24 mmol) and **30-a** (200 mg, 0.41 mmol). The reaction mixture was stirred at room temperature overnight. After completion of the reaction, the solid was precipitated out by slowly adding water, collected by filtration, washed with water and dried to give a crude product, which was then purified through a flash column chromatography (DCM/MeOH 10/1) to give product 30 (100 mg, 44%) as a white solid. LC-MS (ESI): m/z = 555.0 $[M+H]^+$; $^1$H NMR (400 MHz, $CDCl_3$): δ 7.55 (d, 1H, *J* = 7.2 Hz), 7.36-7.39 (m, 1H), 7.30-7.35 (m, 1H), 7.24-7.28 (m, 1H), 5.36 (d, 1H, *J* = 49.6 Hz), 5.15-5.26 (m, 2H), 4.40-4.92 (m, 5H), 3.45-4.02 (m, 6H), 3.08-3.38 (m, 5H), 3.03 (d, 3H, *J* = 13.2 Hz), 2.27-2.86 (m, 2H), 2.32-2.38 (m, 1H), 2.03-2.20 (m, 3H).

**Example 27** Synthetic route of compound **31**

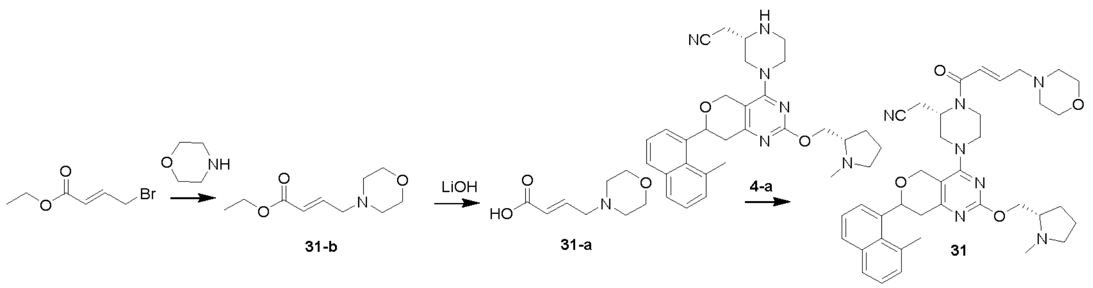


Synthesis of compound **31-b**

In an ice bath, to a solution of compound ethyl 4-bromocrotonate (0.5 mL, 4.25 mmol) in DCM (10 mL) were respectively added potassium carbonate (1.18 g, 8.5 mmol), potassium iodide (40 mg, 0.21 mmol) and morpholine (0.56 mL, 6.38 mmol). After completion of the addition, the reaction mixture was stirred at 0 °C for 30 minutes and then slowly warmed to room temperature and stirred for 4 hours. After completion of the addition, the reaction was quenched by adding water and extracted with ethyl acetate; the organic phase was washed with saturated aqueous sodium chloride solution, dried over anhydrous sodium sulfate, filtered and subjected to rotary evaporation to give a crude product compound, which was purified through a flash column chromatography to give **31-b** (0.75 g, 95%) as a tawny oil. LC-MS (ESI): m/z = 200.0 $[M+1]^+$.

Synthesis of compound **31-a**

To a reaction flask, compound **31-b** (750 mg, 3.76 mmol), lithium hydroxide (632 mg, 15.1 mmol), tetrahydrofuran (12 ml), methanol (6 ml) and water (6 ml) were added. The mixture was stirred at room temperature overnight. After completion of the addition, the organic solvent was removed under reduced pressure; the aqueous layer was added dilute hydrochloric acid to adjust pH to 6-7 and extracted with ethyl acetate; the organic phase was washed successively with water and saturated aqueous sodium chloride solution, dried over anhydrous sodium sulfate, filtered and subjected to rotary evaporation to give crude product compound **31-a** (500 mg, 78%) as a grey solid. LC-MS (ESI): m/z = 172.0 $[M+H]^+$.

Synthesis of compound **31**

At room temperature, to a solution of **31-a** (25 mg, 0.15 mmol) and HATU (74.2 mg, 0.2 mmol) in DMF (5 mL) were added N,N-diisopropylethylamine (37.8 mg, 0.3 mmol) and **4-a** (50 mg, 0.1 mmol). The reaction mixture was stirred at room temperature overnight. After completion of the reaction, the solid was precipitated out by slowly adding water, collected by filtration, washed with water and dried to give a crude product. The crude product was purified through a flash column chromatography (DCM/MeOH 5/1) to give product 31 (15 mg, 23%) as a white foamy solid. LC-MS (ESI): m/z = 666.0 $[M+H]^+$; $^1$H NMR (400 MHz, $CDCl_3$): $\delta$ 7.74-7.84 (m, 3H), 7.45-7.52 (m, 1H), 7.32-7.39 (m, 2H), 6.87-6.99 (m, 1H), 6.36-6.52 (m, 1H), 5.97-6.07 (m, 1H), 4.93-5.11 (m, 1H), 4.64-4.93 (m, 2H), 4.32-4.60 (m, 2H), 3.80-4.28 (m, 2H), 3.32-3.81 (m, 6H), 2.97-3.31 (m, 6H), 2.93 (d, 3H, $J$ = 8.4 Hz), 2.56-2.83 (m, 6H), 2.48 (s, 3H), 2.34-2.55 (m, 1H), 2.14-2.76 (m, 1H), 1.86-2.11 (m, 4H).

**Example 28** Synthetic route of compound **34**

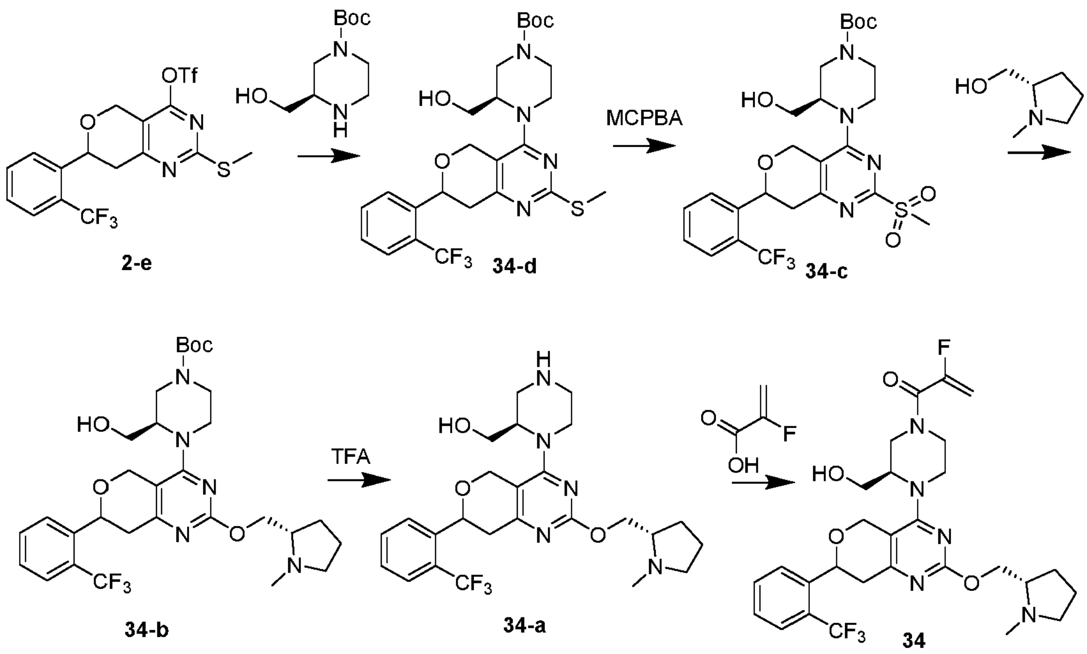

2-e
34-d
34-c
34-b
34-a
34

Synthesis of compound **34-d**

At room temperature, to a solution of **2-e** (205 mg, 0.43 mmol) and (R)-1-BOC-3-hydroxymethylpiperazine (140 mg, 0.65 mmol) in DMF (8 mL) was added DIPEA (111 mg, 0.86 mmol). After completion of the addition, the reaction temperature was slowly increased to 100 °C; the reaction mixture was stirred at this temperature for 1 hour and then cooled to room temperature, added water and extracted with ethyl acetate (50 mL); the organic phase was washed with brine, dried over anhydrous sodium sulfate, filtered and subjected to rotary evaporation; the crude product was separated and purified through a flash column chromatography (0-50%, EA/PE) to give compound **34-d** (111 mg, 47%) as a white solid. LC-MS (ESI): m/z = 541.2 $[M+H]^+$.

Synthesis of compound **34-c**

In an ice bath, to a solution of **34-d** (111 mg, 0.21 mmol) in ethyl acetate (8 mL) was added 85% m-chloroperoxybenzoic acid (104 mg, 0.51 mmol). The reaction was slowly warmed to room temperature, stirred for 3 hours and then added saturated aqueous sodium bicarbonate solution and extracted with ethyl acetate (30 mL * 2); the combined organic phase was dried over anhydrous sodium sulfate, filtered and subjected to rotary evaporation to give crude product compound **34-c** (118 mg, 100%) as a white solid. LC-MS (ESI): m/z = 572.8 $[M+H]^+$.

Synthesis of compound **34-b**

At room temperature, to a solution of **34-c** (118 mg, 0.21 mmol) in toluene (8 mL) were added N-methyl-L-prolinol (36 mg, 0.31 mmol) and then sodium tert-butoxide (40 mg, 0.41 mmol); the mixture was stirred at room temperature for 3 hours and then concentrated; the crude product was separated and purified by Pre-TLC (DCM : MeOH = 10 : 1) to give compound **34-b** (56 mg, 45%) as a yellow solid. LC-MS (ESI): m/z = 608.3 $[M+H]^+$.

Synthesis of compound **34-a**

At room temperature, to a solution of **34-b** (56 mg, 0.09 mmol) in dichloromethane (3 mL) was added TFA (1 mL); the mixture was stirred at room temperature for 1 hour and then concentrated, added saturated aqueous sodium bicarbonate solution, extracted with ethyl acetate (30 mL); the organic phase was dried over anhydrous sodium sulfate, filtered and subjected to rotary evaporation to give crude product compound **34-a** (47 mg, 100%) as a yellow solid. LC-MS (ESI): m/z = 508.3 $[M+H]^+$.

Synthesis of compound **34**

At room temperature, to a solution of **34-a** (47 mg, 0.09 mmol) and 2-fluoroacrylic acid (13 mg, 0.14 mmol) in DMF (5 mL) were added HATU (68 mg, 0.18 mmol) and DIPEA (35 mg, 0.27 mmol); the reaction temperature was increased to room temperature; the mixture was stirred at room temperature for 1 hour and then concentrated, added water and extracted with ethyl acetate (30 mL * 2); the combined organic phase was dried over anhydrous sodium sulfate, filtered and subjected to rotary evaporation; the crude product was separated and purified by Pre-HPLC to give compound **34** (12 mg, 22%) as a white solid. LC-MS (ESI): m/z = 580.2 $[M+H]^+$; $^1H$ NMR (400 MHz, $CDCl_3$): δ 7.75 (d, *J* = 7.6 Hz, 1H), 7.69-7.62 (m, 2H), 7.45 (t, *J* = 7.6 Hz, 1H), 5.40 (d, *J* = 46 Hz, 1H), 5.28-5.14 (m, 2H), 4.86 (dd, *J* = 14, 8 Hz, 1H), 4.74 (dd, *J* = 13.6, 5.6 Hz, 1H), 4.62-4.36 (m, 2H), 4.23-4.13 (m, 2H), 4.09-3.94 (m, 1H), 3.84 (dd, *J* = 11.6, 7.2 Hz, 1H), 3.72-3.22 (m, 4H), 3.17-2.86 (m, 4H), 2.77-2.66 (m, 1H), 2.48 (d, *J* = 4.8 Hz, 3H), 2.31 (dd, *J* = 16.4, 9.2 Hz, 1H), 2.15-1.98 (m, 2H), 1.91-1.66 (m, 3H).

**Example 29** Synthesis of comparative compound **1'**

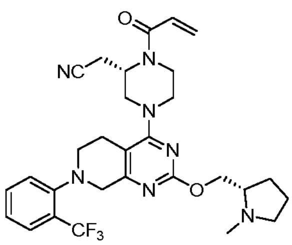

Comparative compound **1'** was synthesized with reference to the method in WO 2017201161 A1.

**Example 30** Synthesis of comparative compound **2'**

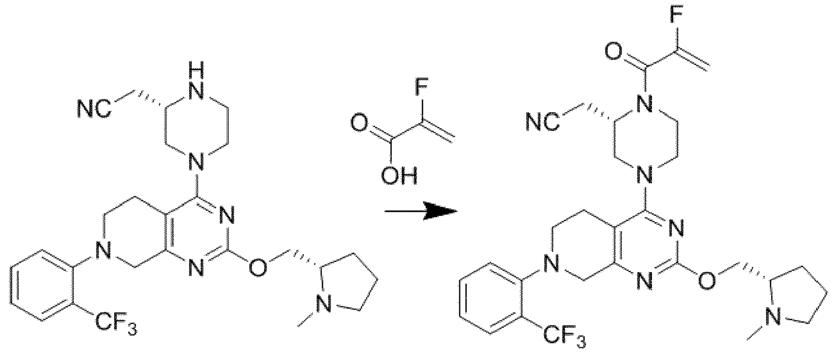

2'-a Comparative compound 2'

Compound **2'-a** was synthesized with reference to the method in WO 2017201161 A1.

**2'-a** (44 mg, 0.085 mmol) was dissolved in DMF (10 mL), and 2-fluoroacrylic acid (15.4 mg, 0.171 mmol), HATU (65 mg, 0.171 mmol) and DIPEA (70.5 μL, 0.427 mmol) were successively added; under nitrogen atmosphere, the mixture was stirred at room temperature overnight. LCMS monitoring indicated that the reaction was complete; the reaction was then quenched with a saturated sodium bicarbonate solution and extracted with ethyl acetate (30 mL X 3); the organic phase was washed with saturated brine (30 mL X 3), dried, concentrated and subjected to column (biotage, 25 g, silica gel, UV254, MeOH : DCM = 0 to 10%) to give comparative compound **2'** (49 mg, 98%) as a pale yellow solid. LC-MS (ESI): m/z = 587.9 $[M+H]^+$; $^1H$ NMR (400 MHz, $CDCL_3$): δ 7.67 (d, 1H, *J* = 8.4 Hz), 7.59 (t, 1H, *J* = 7.6 Hz), 7.44 (d, 1H, *J* = 8.0 Hz), 7.30 (t, 1H, *J* = 8.0 Hz), 5.38 (d, 1H, *J* = 47.2 Hz), 5.24 (dd, 1H, *J* = 4.0, 16.8 Hz), 4.54-4.63 (m, 2H), 4.24-4.34 (m, 1H), 4.00-4.10 (m, 3H), 3.71-3.79 (m, 1H), 3.60-3.68 (m, 1H), 3.33-3.43 (m, 1H), 3.05-3.24 (m, 4H), 3.01 (s, 3H), 2.93-2.98 (m, 1H), 2.72-2.86 (m, 4H), 2.26-2.41 (m, 2H), 2.03-2.21 (m, 4H).

**Example 31** Synthetic route of compound **35**

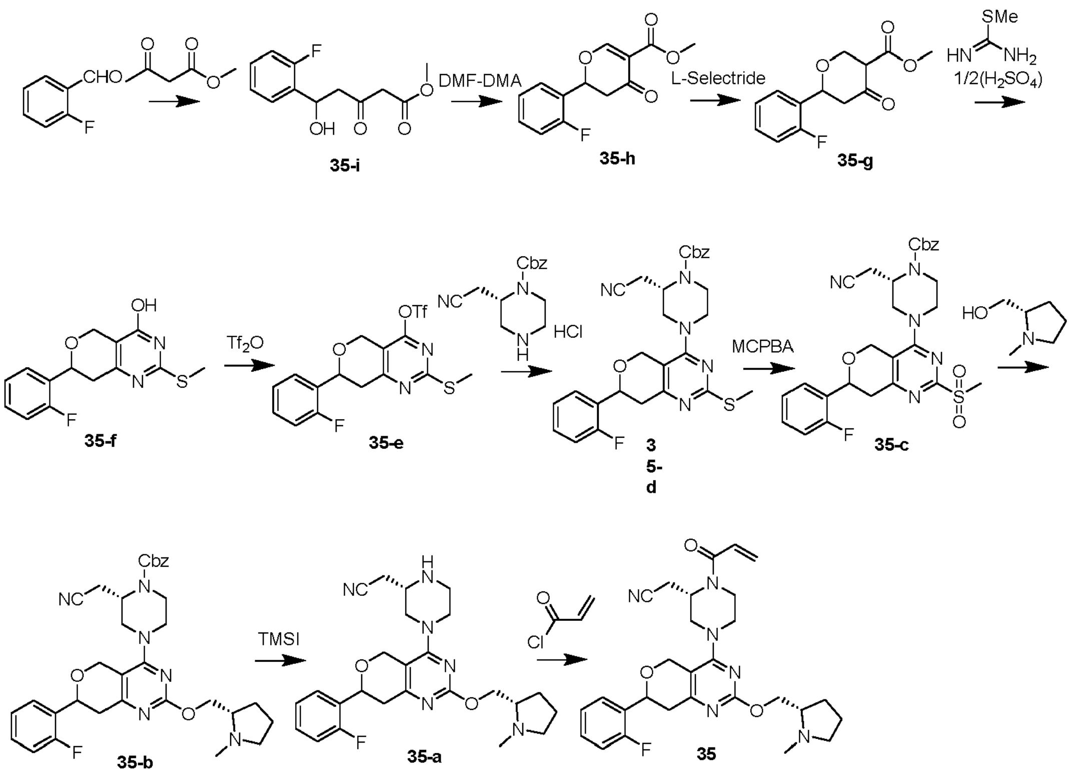


35-i 35-h 35-g 35-f 35-e 35-d 35-c 35-b 35-a 35

Synthesis of compound **35-i**

At room temperature, NaH (60%, 0.97 g, 24.17 mmol) was suspended in 30 mL of THF, and then under nitrogen atmosphere, methyl acetoacetate (2.61 mL, 24.17 mmol) was added. Under nitrogen atmosphere, the mixture was stirred at room temperature for 30 minutes, and then n-BuLi (2.5 M, 9.67 mL, 24.17 mmol) was added dropwise at -15 °C to -10 °C; the mixture was kept at this temperature for 30 minutes, and then a solution of compound o-fluorobenzaldehyde (1.0 g, 8.06 mmol) in THF (10 mL) was added dropwise. The mixture was stirred at low temperature (-10 °C to 0 °C) for 2 hours; the reaction was then quenched with a saturated ammonium chloride solution (20 mL) and extracted with ethyl acetate (20 mL * 3); the organic phase was washed with saturated brine (20 mL), dried over anhydrous sodium sulfate, filtered and concentrated to give a crude product; the crude product was purified through a flash column chromatography to give **35-i** (1.2 g, 62%) as a pale yellow oil. LC-MS (ESI): m/z = 263.1 $[M+Na]^+$.

Synthesis of compound **35-h**

Compound **35-i** (1.2 g, 5.0 mmol) was dissolved in DCM (50 mL); under nitrogen atmosphere, DMF-DMA (0.8 mL, 6.0 mmol) was then added at room temperature; the mixture was stirred at room temperature for 45 minutes and then added $BF_3 \cdot Et_2O$ (0.74 mL, 6.0 mmol); and then the mixture was stirred at room temperature for 1 hour and then diluted with 20 mL of dichloromethane; the organic phase was washed successively with a saturated $NaHCO_3$ solution (40 mL) and saturated brine (20 mL), dried over anhydrous sodium sulfate, filtered and concentrated to give a crude product. The crude product was purified through a flash column chromatography to give **35-h** (1 g, 80%) as a tawny oil. LC-MS (ESI): m/z = 251.1 $[M+1]^+$.

Synthesis of compound **35-g**

Compound **35-h** (1.0 g, 4.0 mmol) was dissolved in THF (30 mL); at -78 °C, under nitrogen atmosphere, lithium tri-sec-butyl borohydride (1 M, 4.4 mL, 4.4 mmol) was then added dropwise; the mixture was stirred at this temperature for 1 hour, and then the reaction was quenched with saturated ammonium chloride (20 mL) and extracted with ethyl acetate (20 mL * 3); the organic phase was concentrated to give a crude product, which was purified through a flash column chromatography to give **35-g** (0.6 g, 60%) as a yellow oil. LC-MS (ESI): m/z = 253.2 $[M+1]^+$.

Synthesis of compound **35-f**

Compound **35-g** (0.6 g, 2.4 mmol) was dissolved in methanol (15 mL), and then at room temperature, sodium carbonate (1.77 g, 14.3 mmol) and compound 2-methyl-2-thiourea sulfate (1.3 g, 4.8 mmol) were successively added. The mixture was stirred at room temperature overnight. After completion of the reaction, the pH was adjusted to 5 with 1 M dilute hydrochloric acid, and then white solid was precipitated out, filtered, washed with water, dried over anhydrous sodium sulfate, filtered and concentrated to give **35-f** (0.3 g, 43%) as a white solid. LC-MS (ESI): m/z = 293.1 $[M+1]^+$.

Synthesis of compound **35-e**

In an ice bath, to a solution of compound **35-f** (0.3 g, 1.0 mmol) in DCM (20 mL) were respectively added DIPEA (265.3 mg, 2.0 mmol) and trifluoromethanesulfonic anhydride (434.3 mg, 1.54 mmol). After completion of the addition, the reaction mixture was slowly warmed to room temperature and stirred for 2 hours. The reaction mixture was quenched by adding saturated aqueous sodium bicarbonate solution and extracted with ethyl acetate. The organic phase was washed with saturated aqueous sodium chloride solution, dried over anhydrous sodium sulfate, filtered and subjected to rotary evaporation to give a crude product compound, which was purified through a flash column chromatography to give **35-e** (260 mg, 60%) as a white solid. LC-MS (ESI): m/z = 425.1 $[M+1]^+$.

Synthesis of compound **35-d**

At room temperature, to a solution of 35-e (0.26 g, 0.61 mmol) in DMF (10 mL) were respectively added DIPEA (0.3 mL, 1.84 mmol) and benzyl (S)-2-cyanomethylpiperazine-1-carboxylate hydrochloride (199.3 mg, 0.67 mmol). After completion of the addition, the reaction mixture was heated to 100 °C and stirred for two hours. After completion of the reaction, the reaction mixture was diluted by adding ethyl acetate, washed successively with water and saturated brine, dried over anhydrous sodium sulfate, filtered and subjected to rotary evaporation. The crude product was separated and purified through a flash column chromatography to give 35-d (0.28 g, 86%) as a white solid. LC-MS (ESI): m/z = 534.2 $[M+H]^+$.

Synthesis of compound 35-c

In an ice bath, compound 35-d (0.28 g, 0.52 mmol) was dissolved in ethyl acetate (20 mL), and then MCPBA (266.2 mg, 1.31 mmol) was added; the mixture was slowly warmed to room temperature and stirred for 2 hours. After completion of the reaction, the reaction mixture was neutralized by adding saturated aqueous sodium bicarbonate solution and extracted with dichloromethane (15 mL * 2); the organic phase was combined, dried over anhydrous sodium sulfate, filtered and concentrated; the crude product was purified through a flash column chromatography (DCM/MeOH = 10/1) to give **35-c** (0.26 g, 88%) as a white solid. LC-MS (ESI): m/z = 566.3 $[M+1]^+$.

Synthesis of compound **35-b**

In an ice bath, to a solution of **35-c** (260 mg, 0.46 mmol) and N-methyl-L-prolinol (105.6 mg, 0.92 mmol) in toluene (10 mL) was added sodium tert-butoxide (88.4 mg, 0.92 mmol). After completion of the addition, the reaction mixture was stirred in an ice bath for 10 minutes. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **35-b** (80 mg, 29%) as a tawny oil. LC-MS (ESI): m/z = 601.0 $[M+H]^+$.

Synthesis of compound **35-a**

At room temperature, to a solution of **35-b** (80 mg, 0.13 mmol) in acetonitrile (20 mL) was added trimethyliodosilane (0.1 mL, 0.67 mmol). After completion of the addition, the reaction mixture was heated to 30 °C and stirred for 5 hours. After completion of the addition, the reactant was neutralized with triethylamine (10 mL) and concentrated under reduced pressure to give **35-a** (20 mg, 32%) as a dark grey solid. LC-MS (ESI): m/z = 467.0 $[M+H]^+$.

Synthesis of compound **35**

At room temperature, to a solution of compound **35-a** (20 mg, 0.043 mmol) in dichloromethane (10 ml) were added acryloyl chloride (7.8 mg, 0.086 mmol) and DIPEA (16.6 mg, 0.13 mmol). The reaction mixture was stirred at room temperature overnight. After completion of the addition, a crude product was obtained by concentration. The crude product was purified by preparative flash column chromatography (DCM/MeOH 10/1) to give compound **35** (5 mg, 22%) as a tawny oil. LC-MS (ESI): m/z = 521.2 $[M+H]^+$; $^1$H NMR (400 MHz, $CDCl_3$): $\delta$ 7.48-7.52 (m, 1H), 7.29-7.33 (m, 1H), 7.20 (t, 1H, *J* = 7.6 Hz), 7.08 (t, 1H, *J* = 8.0 Hz), 6.49-6.78 (m, 1H), 6.39 (d, 1H, *J* = 16.8 Hz), 5.82 (d, 1H, *J* = 10.4 Hz), 4.72-5.31 (m, 4H), 4.49-4.61 (m, 1H), 3.45-4.08 (m, 5H), 2.66-3.44 (m, 10H), 2.26-2.41 (m, 1H), 2.05-2.21 (m, 1H), 1.38-1.59 (m, 4H).

**Example 32** Synthetic route of compound **36**

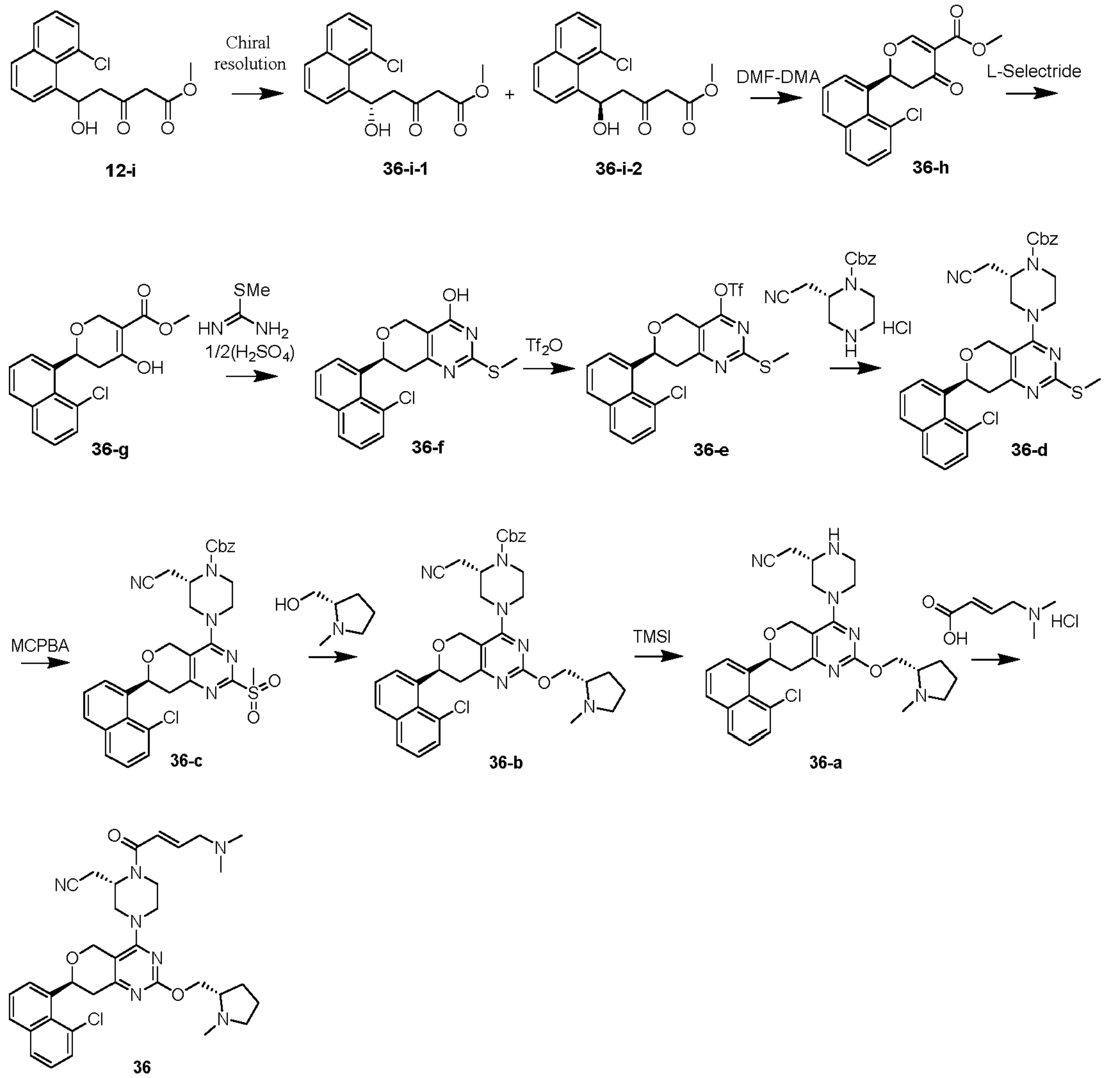


12-i, 36-i-1, 36-i-2, 36-h, 36-g, 36-f, 36-e, 36-d, 36-c, 36-b, 36-a, 36

Synthesis of compounds **36-i-1** and **36-i-2**

Compound **12-i** (8.5 g, 27.8 mmol) was prepared (scale-up) and subjected to chiral resolution to give compound **36-i-1** (2.5 g, 29%) as a white solid and compound **36-i-2** (2.6 g, 31%) as a white solid.

| Chiral analysis conditions | Chiral preparation conditions |
|---|---|
| equipment: SFC Method Station (Thar, Waters)<br>chromatographic column: AD-H 4.6 * 100 mm, 5 um (Daicel)<br>column temperature: 40 °C<br>mobile phase: CO2/Ethanol (1% methanol ammonia) = 75/25<br>flow rate: 4.0 ml/min<br>wavelength: 254 nm<br>back pressure: 120 bar | instrument: SFC-150 (Thar, Waters)<br>chromatographic column: AD 20 * 250 mm, 10 um (Daicel)<br>column temperature: 35 °C<br>mobile phase: CO2/Ethanol (0.2% methanol ammonia) = 65/35<br>flow rate: 100 g/min<br>back pressure: 100 bar<br>detection wavelength: 214 nm<br>cycling time: 5.0 min<br>sample solution: 8.5 g dissolved in 150 ml of methanol and dichloromethane |
| **36-i-1:** retention time: 1.57 min; e.e.% = 100.0%; | |

(continued)

| Chiral analysis conditions | Chiral preparation conditions |
|---|---|
| **36-i-2:** retention time: 2.33 min; e.e.% = 99.12%. | |
| **36-i-1:** LC-MS (ESI): m/z = 329.1 $[M+Na]^+$.<br>**36-i-2:** LC-MS (ESI): m/z = 329.1 $[M+Na]^+$. | |

Synthesis of compound **36-h**

At room temperature, compound **36-i-1** (2.3 g, 7.5 mmol) was dissolved in DCM (80 mL), and then under nitrogen atmosphere, DMF-DMA (1.2 mL, 9.0 mmol) was added at room temperature. At room temperature, the reaction mixture was stirred for 45 minutes and then was added $BF_3 \cdot Et_2O$ (1.2 mL, 9.0 mmol). After completion of the addition, the mixture was stirred at room temperature for 1 hour and then quenched with a saturated sodium bicarbonate solution and extracted with dichloromethane (100 mL * 2); the organic phase was washed with saturated brine (100 mL * 2), dried over anhydrous sodium sulfate, filtered and concentrated to give crude product compound **36-h** (2.0 g, 84%), which was used directly in the next reaction. LC-MS (ESI): m/z = 317.1 $[M+1]^+$.

Synthesis of compound **36-g**

At room temperature, compound **36-h** (2.0 g, 6.31 mmol) was dissolved in THF (60 mL), and then at -78 °C, under nitrogen atmosphere, tri-sec-butyl lithium borohydride (1 M in THF, 6.95 mL, 6.95 mmol) was added dropwise. After completion of the addition, the mixture was stirred at -78 °C for 1 hour, and then the reaction was quenched with a 1 M hydrochloric acid solution (20 mL) and extracted with ethyl acetate (100 mL * 2); the organic phase was washed with saturated brine (50 mL * 2), dried over anhydrous sodium sulfate, filtered and concentrated to give a crude product; the crude product was separated and purified through a flash column chromatography (PE/EA = 0 to 15%) to give compound **36-g** (1.8 g, 89%) as a yellow oil. LC-MS (ESI): m/z = 319.0 $[M+1]^+$.

Synthesis of compound **36-f**

At room temperature, compound **36-g** (1.5 g, 4.71 mmol) was dissolved in methanol (30 mL), and then at 0 °C, under nitrogen atmosphere, sodium methylate (1.27 g, 23.5 mmol) and compound 2-methyl-2-thiourea sulfate (1.18 g, 4.24 mmol) were successively added. After completion of the addition, the mixture was warmed to room temperature and stirred for 20 hours. The pH of the reaction mixture was adjusted to 5 with 1 M dilute hydrochloric acid; the solid was precipitated out and filtered; the filter cake was washed with a mixed solution of ethyl acetate (20 mL) and petroleum ether (20 mL); the solid was collected and dried in vacuum to give crude product **36-f** (0.65 g, 39%) as a white solid. LC-MS (ESI): m/z = 359.1 $[M+1]^+$.

Synthesis of compound **36-e**

At room temperature, compound **36-f** (0.6 g, 1.67 mmol) was dissolved in DCM (50 mL), and then in an ice-water bath, under nitrogen atmosphere, DIPEA (0.83 mL, 5.02 mmol) and trifluoromethanesulfonic anhydride (0.51 mL, 3.01 mmol) were successively added. After completion of the addition, the reaction mixture was stirred in an ice-water bath for 2 hours and then quenched with a saturated sodium bicarbonate solution (50 mL) and extracted with DCM (50 mL * 3); the organic phase was dried over anhydrous sodium sulfate, filtered and concentrated to give a crude product; the crude product was separated and purified through a flash column chromatography (EA/PE = 0 to 5%) to give compound **36-e** (380 mg, 463%) as a white solid. LC-MS (ESI): m/z = 491.1 $[M+1]^+$; chiral-HPLC 100% (ee%).

Synthesis of compound **36-d**

At room temperature, compound **36-e** (360 mg, 0.73 mmol) was dissolved in DMF (15 mL), and then DIPEA (0.36 mL, 2.2 mmol) and benzyl (S)-2-cyanomethylpiperazine-1-carboxylate hydrochloride (238.6 mg, 0.81 mmol) were successively added. After completion of the addition, under nitrogen protection, the mixture was stirred at 100 °C for 1 hour and then cooled to room temperature; the reaction was quenched with saturated brine (50 mL) and extracted with ethyl acetate (50 mL * 2). The organic phase was washed with saturated brine (50 mL * 3) and then was dried over anhydrous sodium sulfate, filtered and concentrated to give a crude product; the crude product was separated and purified through a flash column chromatography (EA/PE = 0 to 40%) to give compound **36-d** (360 mg, 80%) as a white solid. LC-MS (ESI): m/z = 600.2 $[M+1]^+$.

Synthesis of compound **36-c**

At room temperature, compound **36-d** (340 mg, 0.57 mmol) was dissolved in ethyl acetate (25 mL), and then at room temperature, MCPBA (85%, 287.5 mg, 1.42 mmol) was added. After completion of the addition, the mixture was stirred at room temperature for 2 hours, and then the reaction was quenched with a saturated sodium bicarbonate solution (20 mL) and extracted with ethyl acetate (50 mL * 2); the organic phase was dried over anhydrous sodium sulfate, filtered and concentrated to give a crude product; the crude product was separated and purified through a flash column chromatography (EA/PE = 0 to 60%) to give compound **36-c** (320 mg, 89%) as a white solid. LC-MS (ESI): m/z = 632.2 $[M+1]^+$.

Synthesis of compound **36-b**

At room temperature, compound **36-c** (300 mg, 0.47 mmol) was dissolved in toluene (10 mL), and then the reaction mixture was cooled to 0 °C; N-methyl-L-prolinol (109.3 mg, 0.95 mmol) and t-BuONa (91.2 mg, 0.95 mmol) were successively added. After completion of the addition under nitrogen atmosphere the reaction mixture was stirred in an ice-water bath for 0.5 hours and then quenched with water (20 mL) and extracted with ethyl acetate (30 mL * 2). The organic phase was dried over anhydrous sodium sulfate, filtered and concentrated to give a crude product; the crude product was separated and purified through a flash column chromatography (MeOH/DCM = 0 to 7%) to give compound **36-b** (290 mg, 91%) as a white solid. LC-MS (ESI): m/z = 667.3 $[M+1]^+$.

Synthesis of compound **36-a**

At room temperature, compound **36-b** (200 mg, 0.3 mmol) was dissolved in acetonitrile (20 mL) and added trimethyliodosilane (0.21 mL, 1.5 mmol) with stirring; the mixture was stirred at 30 °C for 2 hours, quenched with triethylamine (1 mL) and concentrated to give compound **36-a** (crude product) as a brown solid. LC-MS (ESI): m/z = 533.3 $[M+1]^+$.

Synthesis of compound **36**

In an ice bath, to a solution of compound **36-a** (50 mg, 0.094 mmol) and HATU (71.3 mg, 0.19 mmol) in DMF (5 mL) were respectively added DIPEA (60.6 mg, 0.47 mmol) and 4-(dimethylamino)-2-butenoic acid hydrochloride (23.3 mg, 0.14 mmol). The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 5/1) to give **36** (16 mg, 26%) as a white solid. LC-MS (ESI): m/z = 644.3 $[M+H]^+$; $^1H$ NMR (400 MHz, $CDCl_3$): $\delta$ 7.97 (d, 1H, *J* = 6.8 Hz), 7.83 (t, 2H, *J* = 8.4 Hz), 7.60 (d, 1H, *J* = 7.2 Hz), 7.56 (t, 1H, *J* = 7.6 Hz), 7.36 (t, 1H, *J* = 8.0 Hz), 6.91-6.97 (m, 1H), 6.51 (dd, 1H, *J* = 11.2, 3.2 Hz), 6.37-6.55 (m, 1H), 5.00 (d, 1H, *J* = 13.6 Hz), 4.83 (d, 1H, *J* = 14.0 Hz), 4.55-5.10 (m, 1H), 4.47 (dd, 1H, *J* = 10.8, 5.2 Hz), 4.21 (dd, 1H, *J*= 10.8, 6.4 Hz), 3.99 (d, 1H, *J* = 13.6 Hz), 3.78-3.96 (m, 1H), 3.62-3.78 (m, 1H), 3.59 (dd, 1H, *J* = 18.4, 2.8 Hz), 3.39-3.54 (m, 1H), 3.02-3.24 (m, 4H), 2.62-2.95 (m, 5H), 2.53 (s, 3H), 2.32-2.39 (m, 1H), 2.29 (s, 6H), 2.04-2.10 (m, 1H), 1.75-1.90 (m, 3H).

**Example 33** Synthetic route of compound **37**

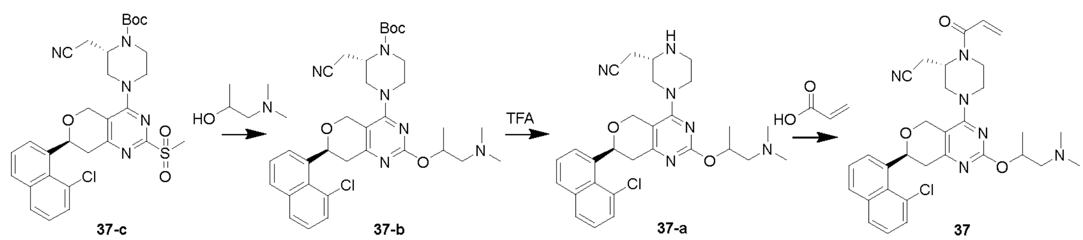


37-c 37-b 37-a 37

Synthesis of compound **37-c**

**37-c** was synthesized with reference to the synthesis of compound 36-c, wherein benzyl (S)-2-cyanomethylpiperazine-1-carboxylate hydrochloride was replaced with tert-butyl (S)-2-cyanomethylpiperazine-1-carboxylate.

Synthesis of compound **37-b**

In an ice bath, to a solution of **37-c** (200 mg, 0.34 mmol) and 1-dimethylamino-2-propanol (68.9 mg, 0.67 mmol) in toluene (10 mL) was added sodium tert-butoxide (64.3 mg, 0.67 mmol). After completion of the addition, the reaction mixture was stirred in an ice bath for 10 minutes. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **37-b** (150 mg, 72%) as a white solid. LC-MS (ESI): m/z = 621.3 $[M+H]^+$.

Synthesis of compound **37-a**

To a solution of **37-b** (150 mg, 0.24 mmol) in dichloromethane (10 mL) was added trifluoroacetic acid (2 mL). The resulting reaction mixture was stirred at room temperature for 4 hours. After completion of the addition, the reaction mixture was concentrated, carefully neutralized to a pH of greater than 7 with saturated sodium bicarbonate solution in an ice bath and extracted with ethyl acetate; the organic layer was combined, washed with saturated brine, dried over anhydrous sodium sulfate, filtered and concentrated to give **37-a** (100 mg, 79%) as an amber-colored oil. LC-MS (ESI): m/z = 521.3 $[M+1]^+$.

Synthesis of compound **37**

At room temperature, to a solution of **37-a** (100 mg, 0.19 mmol) and HATU (145.9 mg, 0.38 mmol) in DMF (5 mL) were respectively added DIPEA (74.4 mg, 0.58 mmol) and acyclic acid (20.7 mg, 0.29 mmol). The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 10/1) to give 37 (25 mg, 23%) as a white solid. LC-MS (ESI): m/z = 575.2 $[M+H]^+$; $^1H$ NMR (400 MHz, $CDCl_3$): $\delta$ 7.97 (d, 1H, $J$ = 7.6 Hz), 7.81-7.86 (m, 2H), 7.54-7.62 (m, 2H), 7.37 (t, 1H, $J$ = 8.0 Hz), 6.48-6.75 (m, 2H), 6.38 (d, 1H, $J$ = 16.8 Hz), 5.83 (d, 1H, $J$ = 10.8 Hz), 5.39-5.53 (m, 1H), 4.48-5.12 (m, 3H), 3.46-4.22 (m, 5H), 2.70-3.35 (m, 7H), 2.55-2.67 (m, 6H), 1.39 (dd, 3H, $J$ = 6.0, 1.6 Hz).

**Example 34** Synthetic route of compound **38**

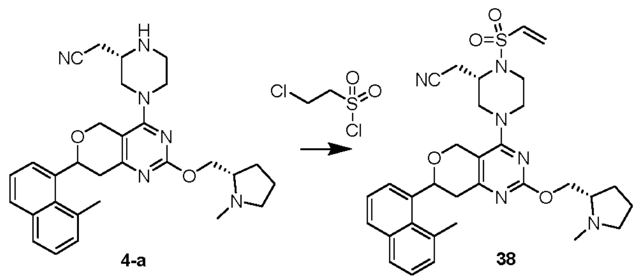

Synthesis of compound **38**

At room temperature, to a solution of compound **4-a** (100 mg, 0.20 mmol) in dichloromethane (10 mL) were respectively added 2-chloroethanesulfonyl chloride (47.7 mg, 0.29 mmol) and triethylamine (59.3 mg, 0.59 mmol). The reaction mixture was stirred at room temperature overnight. After completion of the addition, a crude product was obtained by concentration and then purified by preparative flash column chromatography to give compound **38** (40 mg, 34%) as a white solid. LC-MS (ESI): m/z = 603.3 $[M+H]^+$; $^1H$ NMR (400 MHz, $CDCl_3$): $\delta$ 7.74-7.84 (m, 3H), 7.45-7.49 (m, 1H), 7.34-7.37 (m, 2H), 6.55 (dd, 1H, $J$ = 16.8, 2.8 Hz), 6.34 (dd, 1H, $J$ = 16.4, 2.0 Hz), 6.05 (dd, 1H, $J$ = 9.6, 3.6 Hz), 5.97-6.00 (m, 1H), 4.88 (dd, 1H, $J$ = 54.8, 13.6 Hz), 4.68 (dd, 1H, $J$ = 35.2, 13.2 Hz), 4.44-4.51 (m, 1H), 4.34-4.38 (m, 1H), 4.17-4.25 (m, 1H), 3.42-3.98 (m, 4H), 2.96-3.31 (m, 5H), 2.92 (d, 3H, $J$ = 12.8 Hz), 2.76-2.88 (m, 2H), 2.55 (s, 3H), 2.35-2.41 (m, 1H), 2.02-2.13 (m, 1H), 1.80-1.92 (m, 4H).

**Example 35** Synthetic route of compound **39**

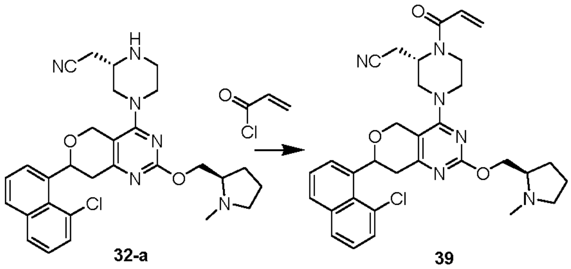

32-a 39

Synthesis of compound **39**

At room temperature, compound **32-a** (55 mg, 0.103 mmol) was dissolved in DCM (10 mL), and then DIPEA (85 μL, 0.515 mmol) and acryloyl chloride (14 mg, 0.155 mmol) were successively added. After completion of the addition, under nitrogen atmosphere, the reaction mixture was stirred at room temperature for 3 hours and then quenched with a saturated sodium bicarbonate solution (50 mL) and extracted with DCM (50 mL * 2). The organic phase was dried over anhydrous sodium sulfate, filtered and concentrated to give a crude product; the crude product was separated and purified through a flash column chromatography (MeOH/DCM = 1/10) to give compound 39 (20 mg, 33% yield). LC-MS (ESI): m/z = 587.3 $[M+1]^+$.

**Example 36** Synthetic route of compound **40**

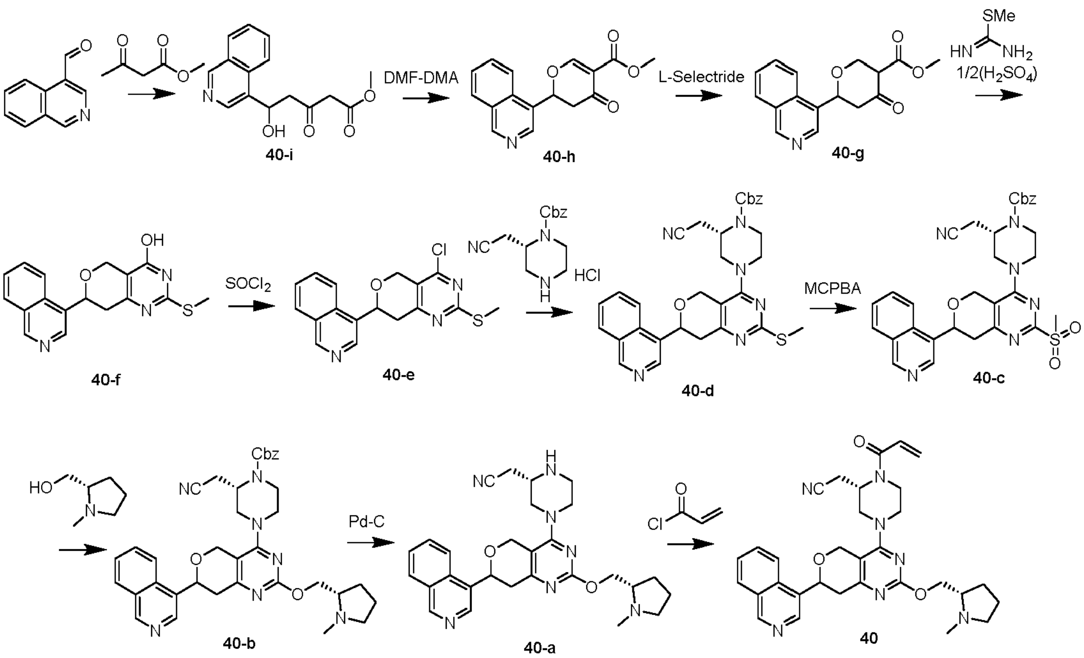


Synthesis of compound **40-i**

At room temperature, NaH (60%, 3.6 g, 90.0 mmol) was added to THF (150 mL); under nitrogen atmosphere, methyl acetoacetate (8 mL, 77.0 mmol) was added at room temperature. Under nitrogen atmosphere, the mixture was stirred at room temperature for 30 minutes, and then n-BuLi (2.5 M, 36.0 mL, 90.0 mmol) was added dropwise at -15 °C to -10 °C. After completion of the addition, the reaction mixture was kept at this temperature and stirred for 30 minutes, and then a solution of compound 4-isoquinolinecarboxaldehyde (5.0 g, 30.0 mmol) in THF (150 mL) was added dropwise. After completion of the addition, the mixture was stirred at low temperature (-10 °C to 0 °C) for 2 hours. The reaction was quenched by adding a saturated ammonium chloride solution (100 mL) and extracted with ethyl acetate (100 mL * 3); the organic phase was washed with saturated brine (150 mL), dried over anhydrous sodium sulfate, filtered and concentrated to give a crude product; the crude product was separated and purified through a flash column chromatography (EA/PE = 1/3) to give compound **40-i** (6.2 g, 75.7%) as a pale yellow liquid. LC-MS (ESI): m/z = 274.1 $[M+H]^+$.

Synthesis of compound **40-h**

Compound **40-i** (6.2 g, 22.7 mmol) was dissolved in DCM (100 mL); under nitrogen atmosphere, DMF-DMA (4.05 g, 34.1 mmol) was added at room temperature. The reaction mixture was stirred at room temperature for 45 minutes, and then $BF_3 \cdot Et2O$ (4.84 g, 34.1 mmol) was added. The mixture was stirred at room temperature for 1 hour and then diluted with dichloromethane (200 mL); the organic phase was washed successively with a saturated $NaHCO_3$ solution (400 mL) and saturated brine (200 mL), dried over anhydrous sodium sulfate, filtered and concentrated to give a crude product, which was separated and purified through a flash column chromatography (EA/PE = 1/3) to give compound **40-h** (5.3 g, 83%) as a pale red liquid. LC-MS (ESI): m/z = 283.9 $[M+H]^+$.

Synthesis of compound **40-g**

At -78 °C, under nitrogen atmosphere, to a solution of compound **40-h** (5.3 g, 18.7 mmol) in THF (100 mL) was added dropwise tri-sec-butyl lithium borohydride tetrahydrofuran (1 M, 28.1 mL, 28.1 mmol). After completion of the addition, the mixture was stirred at this temperature for 1 hour; the reaction was quenched by adding saturated ammonium chloride (20 mL) and extracted with ethyl acetate (100 mL * 3); the organic phase was washed with saturated sodium chloride and concentrated to give a crude product, which was separated and purified through a flash column chromatography (EA/PE = 1/4) to give compound **40-g** (5.3 g, 100%) as a yellow oil. LC-MS (ESI): m/z = 286.2 $[M+H]^+$.

Synthesis of compound **40-f**

In an ice-water bath, to a solution of compound **40-g** (5.3 g, 18.6 mmol) in methanol (150 mL) were successively added sodium methylate (10.0 g, 18.6 mmol) and 2-methyl-2-thiourea sulfate (10.6 g, 37.2 mmol). After completion of the addition, the reaction mixture was warmed to room temperature and stirred overnight. The pH was adjusted to 5 with a 1 M hydrochloric acid solution; the solid was precipitated out, filtered, washed with water (50 mL * 3) and dried to give crude product **40-f** (3.1 g, 51%) as a pale yellow solid. LC-MS (ESI): m/z = 326.1 $[M+H]^+$.

Synthesis of compound **40-e**

In an ice-water bath, at 0 °C, to a solution of compound **40-f** (1.3 g, 4.0 mmol) in DCM (20 mL) and DMF (10 mL) was added thionyl chloride (2.5 mL); the mixture was stirred in an ice-water bath for 3 hours, quenched by adding ice water solution (80 mL) and extracted with DCM (80 mL * 2); the organic phase was dried over anhydrous sodium sulfate, filtered and concentrated; the crude product was separated and purified through a flash column chromatography (EA/PE = 1/25) to give compound **40-e** (0.51 g, 37%). LC-MS (ESI): m/z = 344.1 $[M+H]^+$.

Synthesis of compound **40-d**

At room temperature, compound **40-e** (0.51 g, 1.49 mmol) was dissolved in DMF (12 mL), and DIPEA (0.39 g, 3.0 mmol) and benzyl (S)-2-cyanomethylpiperazine-1-carboxylate hydrochloride (0.48 g, 1.6 mmol) were successively added. After completion of the addition, under nitrogen protection, the mixture was stirred at 100 °C for 1 hour, cooled to room temperature, quenched by adding water (100 mL) and extracted with ethyl acetate (80 mL * 2); the organic phase was washed with saturated brine (100 mL * 3) and concentrated; the crude product was separated and purified through a flash column chromatography (EA/PE = 1/1) to give compound **40-d** (0.7 g, 83%) as a white solid. LC-MS (ESI): m/z = 567.2 [M+H]+.

Synthesis of compound **40-c**

Compound **40-d** (0.7 g, 1.24 mmol) was dissolved in ethyl acetate (20 mL) and MCPBA (0.63 g, 3.1 mmol) was added at room temperature. The mixture was stirred at room temperature for 1 hour and then quenched by adding saturated sodium bicarbonate solution (50 mL), extracted with ethyl acetate (50 mL * 2), filtered and concentrated to give crude product compound **40-c** which was used directly in the next reaction.

Synthesis of compound **40-b**

In an ice-water bath, to a solution of compound **40-c** (0.66 g, 1.1 mmol) in toluene (10 mL) were successively added N-methyl-L-prolinol (0.254 g, 2.2 mmol) and t-BuONa (0.21 g, 2.2 mmol). After completion of the addition, under nitrogen atmosphere, the mixture was stirred in an ice-water bath for 0.5 hours and then quenched by adding water (10 mL) and extracted with ethyl acetate (30 mL * 2); the organic phase was concentrated; the crude product was separated and

purified through a flash column chromatography (MeOH/DCM = 1/9) to give compound **40-b** (0.39 g, 50% yield for two steps). LC-MS (ESI): m/z = 634.0 [M+H]+.

Synthesis of compound **40-a**

A solution of compound **40-b** (0.18 g, 0.28 mmol) in methanolic ammonia (7 M, 50 mL) was cooled to -78 °C, replaced twice with nitrogen, added 10% Pd-C (55 mg) and replaced three times with hydrogen. The reaction mixture was warmed to room temperature and stirred under hydrogen for 2 hours. The reaction mixture was filtered and concentrated to give compound **40-a** (0.093 g, 66%). LC-MS (ESI): m/z = 500.2 [M+H]+.

Synthesis of compound **40**

At room temperature, compound **40-a** (0.09 g, 0.18 mmol) was dissolved in DCM (10 mL), and DIPEA (0.046 g, 0.36 mmol) and acryloyl chloride (27 uL, 0.27 mmol) were successively added. Under nitrogen atmosphere, the mixture was stirred at room temperature overnight, quenched by adding water (10 mL) and extracted with DCM (50 mL * 3); the organic phase was concentrated; the crude product was separated and purified through a flash column chromatography (MeOH/DCM = 1/9) to give compound **40** (13 mg, 7%) as a white solid. LC-MS (ESI): m/z = 554.3 [M+H]+; $^1$H NMR (400 MHz, $CD_3OD$): δ 9.25 (s, 1H), 8.59 (d, 1H, *J* = 6.8 Hz), 8.27 (d, 1H, *J* = 8.8 Hz), 8.20 (d, 1H, *J* = 7.6 Hz), 7.88 (t, 1H, *J* = 7.6 Hz), 7.61 (t, 1H, *J* = 7.6 Hz), 6.72-6.90 (m, 1H), 6.31 (d, 1H, *J* = 15.6 Hz), 5.76-5.95 (m, 1 H), 5.56-5.65 (m, 1H), 5.53 (t, 2H, *J* = 10.0 Hz), 4.43-4.47 (m, 3H), 3.38-4.21 (m, 3H), 3.43-3.52(m, 2H), 3.30-3.36 (m, 1H), 2.93-3.20 (m, 4H), 2.88-2.95 (m, 1H), 2.80-2.83 (m, 1H), 2.51-2.61 (m, 3H), 2.30-2.44 (m, 1H), 2.08-2.22 (m, 1H), 1.81-1.93 (m, 2H), 1.73-1.79 (m, 1H).

**Example 37** Synthetic route of compound **41**

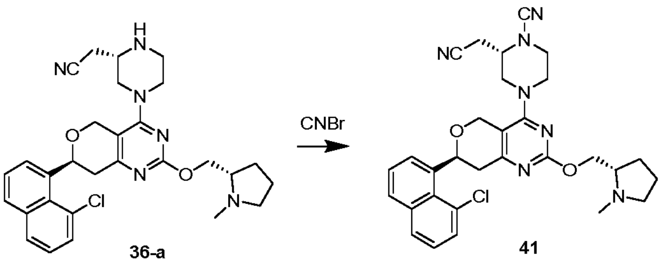


36-a 41

Synthesis of compound **41**

In an ice bath, to a solution of compound **36-a** (50 mg, 0.094 mmol) in DMF (2 mL) were added cesium carbonate (61.1 mg, 0.19 mmol) and cyanogen bromide (10.9 mg, 0.1 mmol). After completion of the addition, the reaction mixture was stirred at 0 °C for 2 hours. After completion of the reaction, the reaction mixture was filtered, and the filtrate was directly purified by Prep-HPLC to give compound **39** (5 mg, 9.6%) as a white solid. LC-MS (ESI): m/z = 558.2 [M+H]+; $^1$H NMR (400 MHz, $CDCl_3$): δ 7.95 (d, 1H, *J* = 7.2 Hz), 7.83 (t, 2H, *J* = 8.8 Hz), 7.60 (dd, 1H, *J* = 7.6, 1.2 Hz), 7.56 (t, 1H, *J* = 8.0 Hz), 7.37 (t, 1H, *J* = 8.0 Hz), 6.44-6.54 (m, 1H), 4.87-4.96 (m, 1H), 4.72-4.80 (m, 1H), 4.36-4.44 (m, 1H), 4.13-4.21 (m, 1H), 3.91-4.00 (m, 1H), 3.75-3.88 (m, 1H), 3.46-3.67 (m, 4H), 3.25-3.44 (m, 2H), 3.06-3.16 (m, 1H), 2.81-2.95 (m, 3H), 2.62-2.74 (m, 1H), 2.48 (s, 3H), 2.21-2.35 (m, 1H), 1.96-2.11 (m, 1H), 1.66-1.92 (m, 3H).

**Example 38** Synthetic route of compound **42**

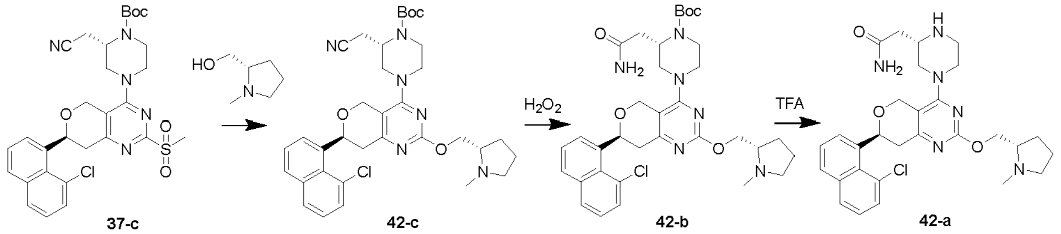


37-c 42-c 42-b 42-a

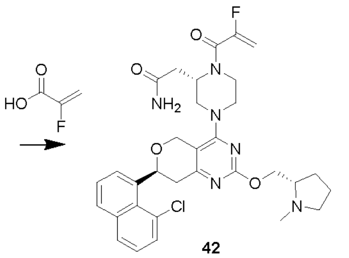

42

Synthesis of compound **42-c**

In an ice bath, to a solution of 37-c (600 mg, 1.0 mmol) and N-methyl-L-prolinol (231 mg, 2.0 mmol) in toluene (10 mL) was added sodium tert-butoxide (192.8 mg, 2.0 mmol). After completion of the addition, the reaction mixture was stirred in an ice bath for 30 minutes. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **42-c** (550 mg, 87%) as a white solid. LC-MS (ESI): m/z = 633.2 $[M+H]^+$.

Synthesis of compound **42-b**

To a reaction flask were added compound **42-c** (150 mg, 0.24 mmol), potassium carbonate (4.91 mg, 0.04 mmol) and dimethylsulfoxide (2 mL). In an ice bath, HzOz in aqueous solution (30%, 107.4 mg, 0.95 mmol) was added dropwise; after completion of the addition, the mixture was warmed to room temperature and stirred overnight. The next day, the solid was precipitated out from the reaction mixture by adding water and then filtered; the filter cake was washed with water and dried to give **42-b** (130 mg, 84%) as a white solid. LC-MS (ESI): m/z = 651.3 $[M+H]^+$.

Synthesis of compound **42-a**

To a solution of **42-b** (130 mg, 0.20 mmol) in dichloromethane (10 mL) was added trifluoroacetic acid (2 mL). The resulting reaction mixture was stirred at room temperature for 4 hours. After completion of the addition, the reaction mixture was concentrated, carefully neutralized to a pH of greater than 7 with saturated sodium bicarbonate solution in an ice bath and extracted with ethyl acetate; the organic layer was combined, washed with saturated brine, dried over anhydrous sodium sulfate, filtered and concentrated to give product **42-a** (100 mg, 90%) as an amber-colored oil. LC-MS (ESI): m/z = 551.2 $[M+1]^+$.

Synthesis of compound **42**

At room temperature, to a solution of **42-a** (100 mg, 0.18 mmol) and HATU (138.0 mg, 0.36 mmol) in DMF (5 mL) were added N,N-diisopropylethylamine (70.4 mg, 0.54 mmol) and 2-fluoroacrylic acid (24.5 mg, 0.27 mmol). The reaction mixture was stirred at room temperature overnight. After completion of the reaction, the solid was precipitated out by slowly adding water, collected by filtration, washed with water and dried to give a crude product, which was then purified by Prep-HPLC to give **42** (60 mg, 53%) as a white solid. LC-MS (ESI): m/z = 623.2 $[M+H]^+$; $^1H$ NMR (400 MHz, $CDCl_3$): δ 7.95 (d, 1H, *J* = 6.8 Hz), 7.83 (t, 2H, *J* = 8.4 Hz), 7.60 (dd, 1H, *J* = 7.2, 1.2 Hz), 7.55 (t, 1H, *J* = 7.6 Hz), 7.36 (t, 1H, *J* = 8.0 Hz), 6.46 (dd, 1H, *J* = 10.8, 3.2 Hz), 6.05-6.21 (m, 1H), 5.36 (s, 1H), 5.32 (dd, 1H, *J* = 47.6, 4.0 Hz), 5.20 (dd, 1H, *J* = 16.8, 3.6 Hz), 4.73-4.96 (m, 3H), 3.80-4.59 (m, 5H), 2.97-3.82 (m, 5H), 2.55 (s, 3H), 2.44-2.95 (m, 4H), 2.28-2.44 (m, 1H), 1.86-2.16 (m, 4H).

**Example 39** Synthetic route of compound **43**

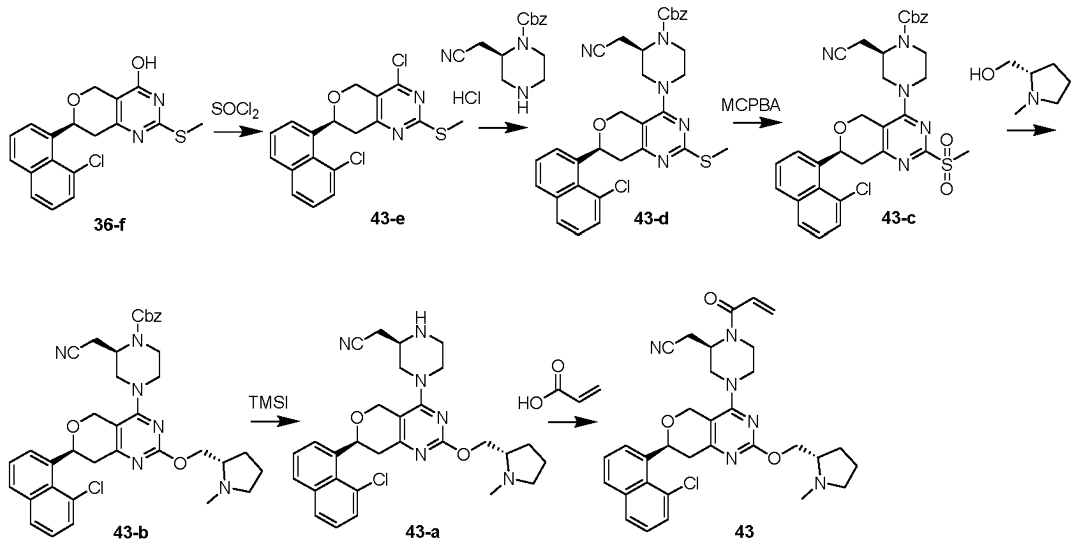


36-f, 43-e, 43-d, 43-c, 43-b, 43-a, 43

Synthesis of compound 43-e

In an ice-water bath, to a solution of **36-f** (4.0 g, 11.1 mmol) in DMF (40 mL) and DCM (20 mL) was added dropwise thionyl chloride (9.3 g, 78.0 mmol). After completion of the addition, the reaction mixture was stirred in an ice-water bath for 4 hours. The reaction mixture was slowly added dropwise to 60 mL of water; the internal temperature was controlled at 0 °C to 10 °C and the reaction mixture was extracted with DCM. The organic phase was washed with saturated sodium bicarbonate and water, concentrated, slurred with n-heptane, cooled to 0 °C to 10 °C, filtered and dried to give compound ’**43-e** (3.2 g, 76%). LC-MS (ESI): m/z = 377.0 $[M+H]^+$. The structure of this compound was confirmed by single crystal analysis.

Synthesis of compound **43-d**

At room temperature, to a solution of **43-e** (150 mg, 0.40 mmol) in DMF (10 mL) were respectively added DIPEA (154.2 mg, 1.19 mmol) and benzyl (R)-2-cyanomethylpiperazine-1-carboxylate hydrochloride (134 mg, 0.52 mmol). After completion of the addition, the reaction mixture was heated to 100 °C and stirred for two hours. After completion of the reaction, the reaction mixture was diluted by adding ethyl acetate, washed successively with water and saturated brine, dried over anhydrous sodium sulfate, filtered and subjected to rotary evaporation. The crude product was separated and purified through a flash column chromatography to give **43-d** (0.2 g, 84%) as a white solid. LC-MS (ESI): m/z = 600.0 $[M+H]^+$.

Synthesis of compound **43-c**

In an ice bath, compound **43-d** (0.2 g, 0.33 mmol) was dissolved in ethyl acetate (20 mL) and then added m-chloroperoxybenzoic acid (143.8 mg, 0.83 mmol); the mixture was slowly warmed to room temperature and stirred for 2 hours. After completion of the reaction, the reaction mixture was neutralized by adding saturated aqueous sodium bicarbonate solution and extracted with ethyl acetate (15 mL * 2); the organic phase was combined, dried over anhydrous sodium sulfate, filtered and concentrated; the crude product was purified through a flash column chromatography (PE/EA = 2/1) to give **43-c** (0.18 g, 85%) as a white solid. LC-MS (ESI): m/z = 632.0 $[M+1]^+$.

Synthesis of compound **43-b**

In an ice bath, to a solution of **43-c** (180 mg, 0.28 mmol) and N-methyl-L-prolinol (65.6 mg, 0.57 mmol) in toluene (10 mL) was added sodium tert-butoxide (54.7 mg, 0.57 mmol). After completion of the addition, the reaction mixture was stirred in an ice bath for 10 minutes. After completion of the addition, the reaction mixture was concentrated under reduced pressure, diluted with ethyl acetate, washed with water and saturated brine, dried over anhydrous sodium sulfate, filtered

and subjected to rotary evaporation to give a brown oil, which was purified through a flash column chromatography to give **43-b** (150 mg, 79%) as a white solid. LC-MS (ESI): m/z = 667.2 $[M+H]^+$.

Synthesis of compound **43-a**

At room temperature, to a solution of **43-b** (150 mg, 0.22 mmol) in acetonitrile (20 mL) was added trimethyliodosilane (224.9 mg, 1.12 mmol). After completion of the addition, the reaction mixture was heated to 30 °C and stirred for 5 hours. After completion of the addition, the reactant was neutralized with triethylamine (5 mL) and concentrated under reduced pressure to give **43-a** (80 mg, 67%) as a dark grey solid. LC-MS (ESI): m/z = 533.2 $[M+H]^+$.

Synthesis of compound **43**

At room temperature, to a solution of **43-a** (80 mg, 0.15 mmol) and HATU (114.1 mg, 0.30 mmol) in DMF (5 mL) were added N,N-diisopropylethylamine (58.2 mg, 0.45 mmol) and acyclic acid (16.2 mg, 0.23 mmol), respectively. The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 10/1) to give 43 (40 mg, 45%) as a white solid. LC-MS (ESI): m/z = 587.3 $[M+H]^+$; $^1H$ NMR (400 MHz, $CDCl_3$): $\delta$ 7.96 (d, 1H, *J* = 7.2 Hz), 7.84 (t, 2H, *J* = 9.2 Hz), 7.61 (d, 1H, *J* = 7.2 Hz), 7.56 (t, 1H, *J* = 8.0 Hz), 7.38 (t, 1H, *J* = 8.0 Hz), 6.53-6.75 (m, 1H), 6.46-6.53 (m, 1H), 6.36 (d, 1H, *J* = 16.4 Hz), 5.83 (d, 1H, *J* = 10.4 Hz), 4.82-5.03 (m, 3H), 3.74-4.75 (m, 5H), 3.23-3.71 (m, 6H), 3.02-3.19 (m, 2H), 2.95 (s, 3H), 2.68-2.90 (m, 2H), 2.01-2.30 (m, 4H).

**Example 40** Synthetic route of compound **44**

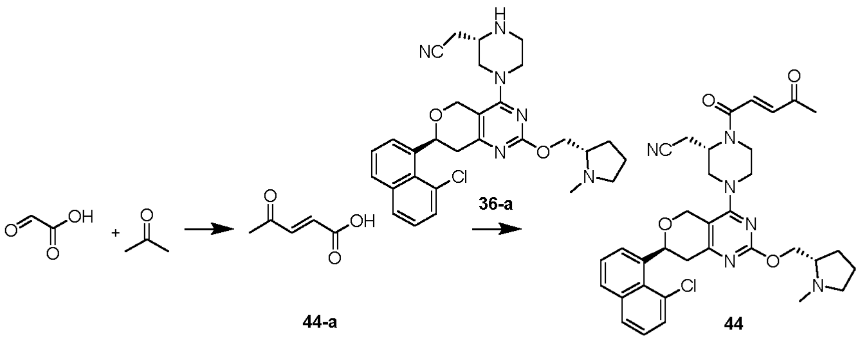

36-a

44-a

44

Synthesis of compound **44-a**

At room temperature, to a solution of compound glyoxylic acid (50% in $H_2O$, 2 g, 13.5 mmol) in acetone (20 mL) was added morpholine hydrochloride (1.67 g, 13.5 mmol). The reaction mixture was stirred at room temperature for 1 hour and then heated to reflux overnight. After completion of the addition, acetone was removed by concentration. The crude product was diluted with water and extracted with ethyl acetate. The organic phase was dried over anhydrous sodium sulfate, filtered and concentrated to give compound **44-a** (0.5 g, 32%) as a white solid. LC-MS (ESI): m/z = 115.1 $[M+H]^+$; $^1H$ NMR (400 MHz, DMSO-$d_6$): $\delta$ 13.12 (bs, 1H), 6.80 (d, 1H, *J* = 16 Hz), 6.66 (d, 1H, *J* = 16 Hz), 2.34 (s, 3H).

Synthesis of compound **44**

To a solution of **36-a** (50 mg, 0.094 mmol) and HATU (71.3 mg, 0.19 mmol) in DMF (5 mL) were added N,N-diisopropylethylamine (36.4 mg, 0.28 mmol) and **44-a** (10.1 mg, 0.14 mmol) respectively. The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 10/1) to give compound **44** (25 mg, 42%) as a white solid. LC-MS (ESI): m/z = 629.2 $[M+H]^+$; $^1H$ NMR (400 MHz, $CDCl_3$): $\delta$ 7.97 (d, 1H, *J* = 6.8 Hz), 7.83 (t, 2H, *J* = 8.8 Hz), 7.60 (d, 1H, *J* = 7.2 Hz), 7.56 (t, 1H, *J* = 4.0 Hz), 7.36 (t, 1H, *J* = 7.6 Hz), 7.07-7.17 (m, 1H), 6.49-6.53 (m, 1H), 4.51-5.14 (m, 3H), 4.44 (dd, 1H, *J* = 10.4, 5.2 Hz), 4.18 (dd, 1H, *J* = 10.8, 2.4 Hz), 3.36-4.02 (m, 5H), 2.64-3.30 (m, 7H), 2.51 (s, 3H), 2.38 (s, 3H), 2.27-2.63 (m, 2H), 2.00-2.13 (m, 1H), 1.70-1.94 (m, 3H).

**Example 41** Synthetic route of compound **45**

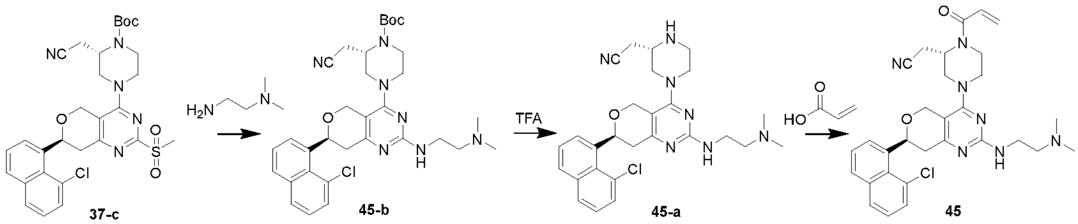


37-c 45-b 45-a 45

Synthesis of compound **45-b**

At room temperature, to a solution of **37-c** (150 mg, 0.25 mmol) in dioxane (20 mL) was added N,N-methylethylenediamine (110.5 mg, 1.25 mmol). The reaction mixture was heated to reflux overnight. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **45-b** (130 mg, 85%) as a white solid. LC-MS (ESI): m/z = 606.2 $[M+H]^+$.

Synthesis of compound **45-a**

To a solution of **45-b** (130 mg, 0.21 mmol) in dichloromethane (10 mL) was added trifluoroacetic acid (2 mL). The resulting reaction mixture was stirred at room temperature for 4 hours. After completion of the addition, the reaction mixture was concentrated, carefully neutralized to a pH of greater than 7 with saturated sodium bicarbonate solution in an ice bath and extracted with ethyl acetate; the organic layer was combined, washed with saturated brine, dried over anhydrous sodium sulfate, filtered and concentrated to give product **45-a** (80 mg, 74%) as an amber-colored oil. LC-MS (ESI): m/z = 506.2 $[M+1]^+$.

Synthesis of compound **45**

At room temperature, to a solution of **45-a** (80 mg, 0.16 mmol) and HATU (120.2 mg, 0.32 mmol) in DMF (5 mL) were added DIPEA (61.3 mg, 0.48 mmol) and acyclic acid (17.1 mg, 0.24 mmol), respectively. The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 10/1) to give **45** (15 mg, 17%) as a white solid. LC-MS (ESI): m/z = 560.2 $[M+H]^+$; $^1H$ NMR (400 MHz, $CDCl_3$): $\delta$ 7.98 (d, 1H, $J$ = 7.6 Hz), 7.82 (t, 2H, $J$ = 7.6 Hz), 7.52-7.61 (m, 2H), 7.36 (t, 1H, $J$ = 7.6 Hz), 6.52-6.66 (m, 1H), 6.48 (dd, 1H, $J$ = 10.8, 3.2 Hz), 6.38 (d, 1H, $J$= 16.4 Hz), 5.77-5.86 (m, 1H), 5.42-5.55 (m, 1H), 4.47-5.15 (m, 3H), 3.25-4.06 (m, 8H), 2.89-3.09 (m, 1H), 2.59-2.88 (m, 5H), 2.41 (s, 6H).

**Example 42** Synthetic route of compound **46**

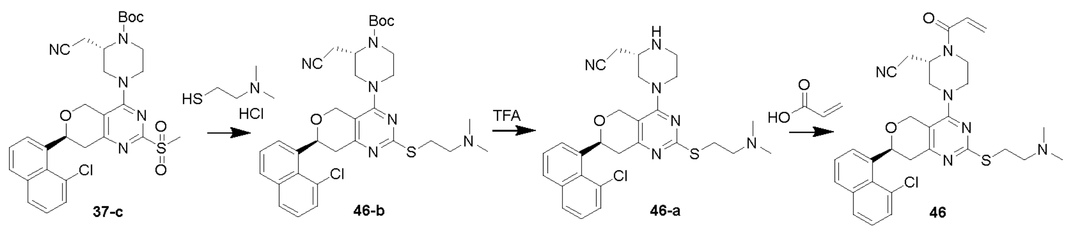


37-c 46-b 46-a 46

Synthesis of compound **46-b**

At room temperature, to a solution of **37-c** (200 mg, 0.33 mmol) in acetonitrile (20 mL) were added 2-dimethylaminoethanethiol hydrochloride (236.8 mg, 1.67 mmol) and triethylamine (338.4 mg, 3.34 mmol). After completion of the addition, the reaction mixture was heated to reflux overnight. After completion of the addition, the reaction mixture was concentrated under reduced pressure, diluted with ethyl acetate, washed with water and saturated brine, dried over anhydrous sodium sulfate, filtered and subjected to rotary evaporation to give a brown oil, which was purified through a flash column chromatography to give **46-b** (100 mg, 48%) as a white solid. LC-MS (ESI): m/z = 623.2 $[M+H]^+$.

Synthesis of compound **46-a**

To a solution of **46-b** (100 mg, 0.16 mmol) in dichloromethane (10 mL) was added trifluoroacetic acid (2 mL). The resulting reaction mixture was stirred at room temperature for 4 hours. After completion of the addition, the reaction mixture was concentrated, carefully neutralized to a pH of greater than 7 with saturated sodium bicarbonate solution in an ice bath and extracted with ethyl acetate; the organic layer was combined, washed with saturated brine, dried over anhydrous sodium sulfate, filtered and concentrated to give product **46-a** (70 mg, 83%) as an amber-colored oil. LC-MS (ESI): m/z = 523.0 $[M+1]^+$.

Synthesis of compound **46**

At room temperature, to a solution of **46-a** (70 mg, 0.16 mmol) and HATU (101.8 mg, 0.27 mmol) in DMF (5 mL) were added DIPEA (51.9 mg, 0.40 mmol) and acyclic acid (14.5 mg, 0.20 mmol). The reaction mixture was stirred at room temperature overnight. After completion of the reaction, the solid was precipitated out by slowly adding water, collected by filtration, washed with water and dried to give a crude product, which was then purified by silica gel column chromatography (DCM/MeOH 10/1) to give **46** (15 mg, 19%) as a white solid. LC-MS (ESI): m/z = 577.2 $[M+H]^+$; $^1H$ NMR (400 MHz, DMSO-$d_6$): $\delta$ 7.95-8.04 (m, 3H), 7.65-7.74 (m, 2H), 7.46-7.55 (m, 1H), 6.78-6.98 (m, 1H), 6.34-6.47 (m, 1H), 5.76-6.21 (m, 1H), 4.54-5.16 (m, 3H), 3.78-4.49 (m, 3H), 3.37-3.77 (m, 6H), 3.02-3.28 (m, 6H), 2.61-3.04 (m, 7H), 2.26-2.43 (m, 1H).

**Example 43** Synthesis of comparative compound **3'**

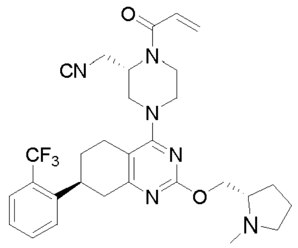

Comparative compound **3'** was synthesized with reference to the method in patent WO 2019155399 A1.

**Effect example 1** Experiments for testing proliferation inhibition of compounds on NCI-H358, A549 and A375 cell lines by CTG method

NCI-H358 was a human non-small cell lung cancer cell with KRAS G12C mutation; A549 was a human non-small cell lung cancer cell with KRAS G12V mutation; and A375 was a wild-type malignant melanoma cell. The inhibitory effects of compounds on different mutations were evaluated by testing the proliferation inhibitory activity of these compounds on the three cell lines.

40 $\mu$L of cell suspension to be tested was added to each well (except for peripheral wells) of three 384-well plates (plate 1: NCI-H358 cell suspension; plate 2: A549 cell suspension; plate 3: A375 cell suspension). The plates were placed in a carbon dioxide incubator overnight. The prepared compounds (3-fold dilutions were made to give 10 concentration gradients of the compounds) were added to each well. The plates were incubated in a carbon dioxide incubator for 120 hours. To the 384-well plates were added 25 $\mu$L of CellTiter Glo reagents, and the mixture was shaken in the dark for 10 minutes and incubated for 10 minutes. The plates were read on the EnVision plate reader. XLFit was used to draw inhibition rate curves of efficacy and $IC_{50}$ values were calculated. Table 1 shows the activity results of representative compounds. "*" represents "$IC_{50}$ > 1 $\mu$M" and "**" represents "$IC_{50} \leq 1$ $\mu$M". "-" represents not determined.

Table 1 Proliferation inhibitory activity of representative compounds of the present disclosure on H358 cells A549 cells and A375 cells

| Compound No. | $IC_{50}$ (μM): (H358) | $IC_{50}$ (μM): (A549) | $IC_{50}$ (μM): (A375) |
|---|---|---|---|
| **1** | * (> 10) | * (> 10) | --- |
| **2** | ** | * | --- |
| **2-1** | ** | * | * |
| **2-2** | ** (0.0025) | * | * |
| **3** | ** | * | --- |
| **3-1** | ** | * | * |
| **3-2** | ** | * | * |
| **4** | ** | * | - |
| **4-1** | ** | * | * |
| **4-2** | ** | * | * |
| **5** | ** | * | - |
| **6-2** | ** | * | - |
| **7** | ** | * | - |
| **8** | ** | * | - |
| **9** | ** | * | - |
| **10** | ** | * | * |
| **11-2** | ** | * | * |
| **12** | ** | * | * |
| **13** | ** | * | * |
| **12-2** | ** | * | * |
| **13-2** | ** | * | * |
| **14** | ** | * | * |
| **15** | ** | * | * |
| **16** | ** | * | * |
| **17** | ** | * | * |
| **18-2** | ** (0.013) | * | * |
| **19-2** | ** | * | * |
| **24** | ** | * | * |
| **25** | ** | * | * |
| **30** | ** | * | * |
| **31** | ** | * | * |
| **32-2** | ** | * | * |
| **Comparative compound 1'** | 0.0073 | --- | --- |
| **Comparative compound 2'** | 0.0394 | --- | --- |

It can be determined from the results of the above-mentioned tests that the compounds of the present disclosure had significant proliferation inhibitory effects on NCI-H358 cells, but had weak proliferation inhibitory activities on A549 cells and A375 cells.

In addition, pyranopyrimidine backbone compounds 2-2 and 18-2 of the present disclosure and the corresponding piperidinopyrimidine backbone compounds, comparative compound 1' and comparative compound 2', were compared in terms of activities. It can be seen from the results that the activity of compound 2-2 was significantly better than that of the corresponding comparative compound 1', and the activity of compound 18-2 was significantly better than that of the corresponding comparative compound 2'.

**Effect example 2**

Experiments for testing proliferation inhibition of compounds on NCI-H358 cell lines by CTG method

The operation was the same as that in effect example 1

Experiments for testing proliferation inhibition of compounds on MIA PaCa-2 cell lines by CTG method

40 μL of MIA PaCa-2 cell suspension was added to each well (except for peripheral wells) of 384-well plates. The plates were placed in a carbon dioxide incubator overnight. The prepared compounds (3-fold dilutions were made to give 10 concentration gradients of the compounds) were added to each well. The plates were incubated in a carbon dioxide incubator for 120 hours. To the 384-well plates were added 25 μL of Cell Titer Glo reagents, and the mixture was shaken in the dark for 10 minutes and incubated for 10 minutes. The plates were read on the EnVision plate reader. XLFit was used to draw inhibition rate curves of efficacy and $IC_{50}$ values were calculated. Table 1 shows the activity results of representative compounds. "*" represents "$IC_{50}$ > 1 μM" and "**" represents "$IC_{50}$ < 1 μM". "---" represents not determined.

MIA PaCa-2 was a human pancreatic cancer cell with KRAS G12C mutation. The activity of compounds can be evaluated by detecting the proliferation inhibitory activity of these compounds on such cell line.

Table 2 Proliferation inhibitory activity of representative compounds of the present disclosure on H358 and MIA PaCa-2 cells

| Compound No. | $IC_{50}$ (nM) H358 | $IC_{50}$ (nM) MIA PaCa-2 |
|---|---|---|
| **2-2** | 2.5 | 1.9 |
| **Comparative compound 1'** | 7.3 | 3.9 |
| **Comparative compound 3'** | --- | 8.3 |
| **18-2** | 13.0 | 9.9 |
| **Comparative compound 2'** | 39.4 | 36.4 |
| **35** | --- | ** |
| **36** | ** | --- |
| **37** | ** | --- |
| **38** | --- | ** |
| **40** | --- | ** |
| **41** | ** | --- |
| **43** | ** | --- |
| **44** | ** | --- |
| **45** | ** | --- |
| **46** | ** | --- |

It can be seen from the results that the activity of compound 2-2 was significantly better than that of the corresponding comparative compounds **1'** and **3',** and the activity of compound **18-2** was significantly better than that of the corresponding comparative compound **2'.**

**Effect example 3** Inhibition activity of metabolic enzymes *in vitro*

Test purpose: In this test, human liver microsomes and CYP enzyme-specific substrates were used to test the effects of different concentrations of compounds to be tested on metabolic rates of enzyme-specific substrates, so as to determine inhibitory effects of these compounds on CYP enzymes in human liver microsomes.

Operation procedure:

1. preparing 100 mM potassium phosphate buffer (pH 7.4);
2. preparing stock solutions of compounds to be tested (10 mM), NADPH solutions (8 mM, which was 4 times the final concentration), positive compound stock solutions, substrate stock solutions and human liver microsome solutions (see the table below);
3. preparing solutions of compounds to be tested: dissolving 8 μL of 10 mM stock solution of compounds to be tested in 12 μL of acetonitrile, and then using a mixed solution of 40% DMSO and 60% acetonitrile to perform a 1 : 3 gradient dilution as follows (wherein the concentration was 400 times the final concentration): 4 mM, 1.333 mM, 0.444 mM, 0.148 mM, 0.0494 mM, 0.0165 mM, 0.00549 mM and 0 mM.
4. preparing positive control inhibitor solutions: dissolving 8 μL of positive control inhibitor stock solutions in 12 μL of acetonitrile, and then using a mixed solution of 40% DMSO and 60% acetonitrile to perform a 1 : 3 gradient dilution as follows (wherein the concentration was 400 times the final concentration, which was described in the table below);
5. preparing mixed solutions of compounds to be tested and human liver microsomes: adding 400 μL of 0.2 mg/mL human liver microsomes to a 96-well plate, and adding 2 μL of solutions of compounds to be tested at a concentration of 400-times higher than that of the microsomes;
6. preparing mixed solutions of positive control compounds and human liver microsomes: adding 200 μL of 0.2 mg/mL human liver microsomes to a 96-well plate, and adding 1 μL of diluted solutions of positive control compounds;
7. dispensing 30 μL of mixed solutions of the compounds and human liver microsomes to a 96-well plate, and then adding 15 μL of substrate solutions; adding 15 μL of preheated NADPH solutions to a preheated reaction plate and mixing evenly to start the reaction;
8. incubating the reaction plate at 37 °C; 3A4 was reacted for 5 minutes; 1A2, 2C9 and 2D6 were reacted for 10 minutes; 2C19 was reacted for 45 minutes;
9. after completion of the addition, adding 120 μL of acetonitrile containing internal standard to terminate the reaction; after terminating the reaction, shaking the sample on a shaker at 600 rpm/min for 10 minutes and centrifuging same at 3220 xg for 15 minutes; after centrifugation, taking out 50 μL of supernatant and mixing same with 50 μL of water for analysis by LC-MS/MS.

| CYP enzyme | Substrate | Concentration of stock solution | Final concentration (μM) | Positive compound | Concentration of stock solution | Initial final concentration (μM) | Human liver microsome concentration (mg/mL) | Incubation time (min) |
|---|---|---|---|---|---|---|---|---|
| **1A2** | Phenacetin | 6 mM in ACN | 30 | α-naphthoflavone | 0.3 mM in DMSO | 0.3 | 0.1 | 10 |
| **2C9** | Diclofenac | 10 mM in $H_2O$ | 10 | Sulfaphenazole | 10 mM in DMSO | 10 | 0.1 | 10 |
| **2C19** | S-Mephenytoin | 35 mM in ACN | 35 | Omeprazole | 100 mM in DMSO | 100 | 0.5 | 45 |
| **2D6** | Bufuralol | 10 mM in $H_2O$ | 10 | Quinidine | 2.5 mM in DMSO | 2.5 | 0.1 | 10 |
| **3A4** | Testosterone | 10 mM in ACN | 80 | Ketoconazole | 2.5 mM in DMSO | 2.5 | 0.1 | 5 |
| | Midazolam | 1 mM in ACN | 5 | | | | | |

Data processing:

According to the following equation, the data was plotted and fitted using a dose-response model in GraphPad Prism software to calculate $IC_{50}$.

$$Y = Bottom + (Top-Bottom)/(1 + 10^{\wedge}((Log\ IC_{50}-X) \times HillSlope))$$

X represents the logarithm concentration of the inhibitor, and Y represents the relative activity of the enzyme at a certain inhibitor concentration (relative to the condition without inhibitor).

Test results:

| Compound | $IC_{50}$ (μM) | | | | | |
|---|---|---|---|---|---|---|
| | CYP1A2 | CYP2C9 | CYP2C19 | CYP2D6 | CYP3A4 (Midazolam) | CYP3A4 (Testosterone) |
| **18-2** | > 10 | >10 | >10 | > 10 | > 10 | >10 |
| **MRTX849** | >10 | 2.29 | >10 | > 10 | 5.92 | 7.85 |
| **AMG-510** | >10 | >10 | >10 | > 10 | 4.46 | >10 |

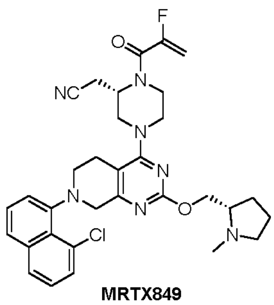

**MRTX849**

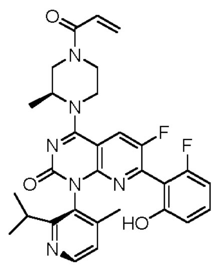

**AMG-510**

The results show that the inhibition IC50 of 18-2 to CYP isoenzymes (1A2, 2C9, 2C19, 2D6 and 3A4) is > 10, > 10, > 10, > 10 and > 10, respectively, indicating that this compound has a weak inhibition on all metabolic enzymes ($IC_{50}$ > 10 μmol/L) and was better than the current clinical compounds MRTX849 and AMG-510.

## Claims

1. An oxygen-containing heterocyclic compound represented by formula **I**, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein:

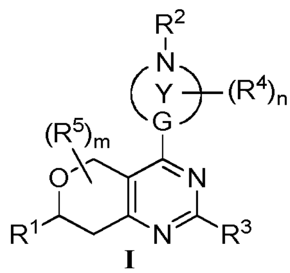

**I**

$R^1$ is $C_{6-20}$ aryl, "5-12 membered heteroaryl containing 1-4 heteroatoms selected from one or more of O, S and N",

$C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, $-C(=O)R^{65}$, $-NR^{63}R^{64}$, $-C(=O)OR^{66}$, $-C(=O)NR^{69}R^{610}$, $C_{1-6}$ alkyl, $C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;
$R^{1-6-1}$ , $R^{1-6-2}$, $R^{1-6-3}$, $R^{1-6-4}$, $R^{1-6-5}$ and $R^{1-6-6}$ are independently cyano, halogen, hydroxyl, $C_{1-6}$ alkoxy, $C_{1-6}$ alkyl, $-C(=O)R^{65-2}$, $-NR^{63-2}R^{64-2}$, $-C(=O)OR^{66-2}$, or $-C(=O)NR^{69-2}R^{610-\mathbf{2}}$;
$R^{65}$, $R^{65-2}$, $R^{63}$, $R^{63-2}$, $R^{64}$, $R^{64-2}$, $R^{66}$ $R^{66-2}$, $R^{69}$, $R^{69-2}$, $R^{610}$ and $R^{610-2}$ are independently hydrogen or $C_{1-6}$ alkyl;
m is 0, 1 or 2;
$R^5$ is independently $C_{1-6}$ alkyl;
$R^3$ is $-OR^{31}$, $-SR^{32}$ or $-NR^{33}R^{34}$;
$R^{31}$, $R^{32}$ and $R^{34}$ are independently $C_{1-6}$ alkyl substituted with one or more $R^{31-1}$, $R^{33}$ is independently H, $C_{1-6}$ alkyl, or $C_{1-6}$ alkyl substituted with one or more $R^{31-1}$,
$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, $-OR^d$, $-SR^{d1}$, $-NR^{e1}R^{e2}$, or $-C(=O)NR^{e3}R^{e4}$;
$R^{d15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;
$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;
$R^{1-8-1}$ and $R^{1-8-2}$ are independently cyano, halogen, hydroxyl, $C_{1-6}$ alkoxy, $-C(=O)R^{e9}$, $-NR^{e10}R^{e11}$, $-C(=O)OR^{e12}$, or $-C(=O)NR^{e13}R^{e14}$;
$R^{e5}$, $R^{e6}$, $R^{e7}$, $R^{e8}$, $R^{e9}$, $R^{e10}$, $R^{e11}$, $R^{e12}$, $R^{e13}$ and $R^{e14}$ are independently hydrogen or $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;
G is N, C or CH;
n is 0, 1, 2 or 3;
$R^4$ is independently $C_{1-6}$ alkyl, $C_{1-6}$ alkyl substituted with one or more $R^{4-1}$, oxo, $-C(=O)OR^{4a}$ or $-C(=O)NR^{4b}R^{4c}$;
$R^{4-1}$ is independently halogen, cyano, hydroxyl, $C_{1-6}$ alkoxy, $-NR^{4i}R^{4j}$, $-C(=O)OR^{4d}$ or $-C(=O)NR^{4e}R^{4f}$, $R^{4a}$, $R^{4e}$, $R^{4f}$, $R^{4i}$ and $R^{4j}$ are independently hydrogen or $C_{1-6}$ alkyl;
$R^{4a}$, $R^{4b}$ and $R^{4c}$ are independently hydrogen or $C_{1-6}$ alkyl;
$R^2$ is CN, $-C(=O)-C(R^a)=C(R^b)(R^f)$, $-C(=O)-C\equiv CR^f$, $-S(=O)_2-C(R^a)=C(R^b)(R^f)$ or $-S(=O)_2-C\equiv CR^f$;
$R^a$ is independently hydrogen, deuterium, halogen or $C_{1-6}$ alkyl;
$R^b$ and $R^f$ are independently hydrogen, deuterium, $C_{1-6}$ alkyl, $C_{1-6}$ alkyl-C(=O)-, or $C_{1-6}$ alkyl substituted with one or more $R^{b-1}$;
$R^{b-1}$ is independently halogen, hydroxyl, $C_{1-6}$ alkoxy, or $-NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N".

**2.** The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, $R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; $R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;

and/or, m is 0;

and/or, $R^3$ is $-OR^{31}$ or $-NR^{33}R^{34}$; $R^{31}$, $R^{33}$ and $R^{34}$ are independently $C_{1-6}$ alkyl substituted with one or more $R^{31-1}$, $R^{31-1}$ is independently "5-7 membered heterocycloalkyl containing 1-3 heteroatoms selected from one or more of O and N" substituted with one or more $R^{d15}$, or $-NR^{e1}R^{e2}$; $R^{a15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$; $R^{1-8-1}$ is independently halogen; $R^{e5}$, $R^{e6}$, $R^{e7}$ and $R^{e8}$ are independently hydrogen or $C_{1-6}$ alkyl; $R^{e1}$ and $R^{e2}$ are independently $C_{1-6}$ alkyl;

and/or, ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring; G is N, C or CH;

and/or, n is 0 or 1; $R^4$ is independently $C_{1-6}$ alkyl, or $C_{1-6}$ alkyl substituted with one or more $R^{4-1}$; $R^{4-1}$ is independently cyano;

and/or, $R^2$ is $-C(=O)-C(R^a)=C(R^b)(R^f)$, $-C( = O)-C \equiv C-Me$ or $-S(=O)_2-C(R^a)=C(R^b)(R^f)$; $R^a$ is independently hydrogen or halogen; $R^b$ and $R^f$ are independently hydrogen, $C_{1-6}$ alkyl, or $C_{1-6}$ alkyl substituted with one or more $R^{b-1}$; $R^{b-1}$ is independently halogen, hydroxyl, $C_{1-6}$ alkoxy, or $-NR^{10j}R^{10k}$; $R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

**3.** The oxygen-containing heterocyclic compound represented by formula **I** as defined in claim 2, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, $R^1$ is $C_{6-20}$ aryl, or $C_{6-20}$ aryl substituted with one or more $R^{1-6}$; $R^{1-6}$ is independently halogen, $C_{1-6}$ alkyl, or $C_{1-6}$ alkyl substituted with one or more $R^{1-6-1}$; $R^{1-6-1}$ is independently halogen;

and/or, $R^3$ is $-OR^{31}$ or $-NR^{33}R^{34}$; $R^{31}$, $R^{33}$ and $R^{34}$ are independently $C_{1-6}$ alkyl substituted with one or more $R^{31-1}$, $R^{31-1}$ is independently "5-7 membered heterocycloalkyl containing 1-3 heteroatoms selected from one or more of O and N" substituted with one or more $R^{d15}$, or $-NR^{e1}R^{e2}$; $R^{e1}$, $R^{e2}$ and $R^{d15}$ are independently $C_{1-6}$ alkyl;

and/or, ring Y is a 4-12 membered heterocyclic ring containing 1-4 N atoms; the heterocyclic ring is a saturated heterocyclic ring or a partly saturated heterocyclic ring; the heterocyclic ring is a monocyclic ring; G is N;

and/or, n is 0 or 1; $R^4$ is independently $C_{1-6}$ alkyl, or $C_{1-6}$ alkyl substituted with one or more $R^{4-1}$; $R^{4-1}$ is independently cyano;

and/or, $R^2$ is $-C(=O)-C(R^a)=C(R^b)(R^f)$; $R^a$ is independently hydrogen or halogen; $R^b$ and $R^f$ are independently hydrogen or $C_{1-6}$ alkyl.

**4.** The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein,

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$; $R^{1-6}$ and $R^{1-7}$ are independently halogen, $C_{1-6}$ alkyl, or $C_{1-6}$ alkyl substituted with one or more $R^{1-6-1}$, $R^{1-6-1}$ is independently halogen;

and/or, $R^3$ is $-OR^{31}$, $-SR^{32}$ or $-NR^{33}R^{34}$; $R^{31}$, $R^{32}$ and $R^{34}$ are independently $C_{1-6}$ alkyl substituted with one or more $R^{31-1}$, $R^{33}$ is independently H, $C_{1-6}$ alkyl, or $C_{1-6}$ alkyl substituted with one or more $R^{31-1}$, $R^{31-1}$ is independently "5-7 membered heterocycloalkyl containing 1-3 heteroatoms selected from one or more of O and N" substituted with one or more $R^{d15}$, or $-NR^{e1}R^{e2}$; $R^{d15}$ is independently $C_{1-6}$ alkyl or halogen; $R^{e1}$ and $R^{e2}$ are independently $C_{1-6}$ alkyl;

and/or, n is 0 or 1; $R^4$ is independently $C_{1-6}$ alkyl, or $C_{1-6}$ alkyl substituted with one or more $R^{4-1}$; $R^{4-1}$ is independently hydroxyl, cyano, or $-C(=O)NR^{4e}R^{4f}$; $R^{4e}$ and $R^{4f}$ are independently hydrogen or $C_{1-6}$ alkyl;

and/or, $R^2$ is CN, $-C(=O)-C(R^a)=C(R^b)(R^f)$, $-C( = O)-C \equiv C-Me$ or $-S(=O)_2-C(R^a)=C(R^b)(R^f)$; $R^a$ is independently hydrogen or halogen; $R^b$ and $R^f$ are independently hydrogen, $C_{1-6}$ alkyl, $C_{1-6}$ alkyl-C(=O)-, or $C_{1-6}$ alkyl substituted with one or more $R^{b-1}$; $R^{b-1}$ is independently $-NR^{10j}R^{10k}$; $R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

**5.** The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein,

$R^3$ is $-OR^{31}$ or $-NR^{33}R^{34}$; $R^{31}$, $R^{33}$ and $R^{34}$ are independently $C_{1-6}$ alkyl substituted with one or more $R^{31-1}$, $R^{31-1}$ is independently "5-7 membered heterocycloalkyl containing 1-3 heteroatoms selected from one or more of O and N" substituted with one or more $R^{d15}$, or $-NR^{e1}R^{e2}$; $R^{d15}$ is independently $C_{1-6}$ alkyl or halogen; $R^{e1}$ and $R^{e2}$ are independently $C_{1-6}$ alkyl;
and/or, n is 0 or 1; $R^4$ is independently $C_{1-6}$ alkyl, or $C_{1-6}$ alkyl substituted with one or more $R^{4-1}$; $R^{4-1}$ is independently hydroxyl or cyano;
and/or, $R^2$ is $-C(=O)-C(R^a)=C(R^b)(R^f)$, $-C( = O)-C \equiv C-Me$ or $-S(=O)_2-C(R^a)=C(R^b)(R^f)$; $R^a$ is independently hydrogen or halogen; $R^b$ and $R^f$ are independently hydrogen, $C_{1-6}$ alkyl, or $C_{1-6}$ alkyl substituted with one or more $R^{b-1}$; $R^{b-1}$ is independently $-NR^{10i}R^{10k}$ ; $R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N".

**6.** The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, the oxygen-containing heterocyclic compound represented by formula **I** is defined as solution 1, solution 2, solution 3, solution 4, solution 5, solution 6 or solution 7:

solution 1:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;
m is 0;
$R^3$ is $-OR^{31}$ or $-NR^{33}R^{34}$;
$R^{31}$, $R^{33}$ and $R^{34}$ are independently $C_{1-6}$ alkyl substituted with one or more $R^{31-1}$,
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;
$R^{1-8-1}$ is independently halogen; $R^{e5}$, $R^{e6}$, $R^{e7}$ and $R^{e8}$ are independently hydrogen or $C_{1-6}$ alkyl;
$R^{e1}$ and $R^{e2}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;
G is N, C or CH;
n is 0 or 1;
$R^4$ is independently $C_{1-6}$ alkyl, or $C_{1-6}$ alkyl substituted with one or more $R^{4-1}$,
$R^{4-1}$ is independently cyano;
$R^2$ is $-C(=O)-C(R^a)=C(R^b)(R^f)$, -C(=O)-C=C-Me or $-S(=O)_2-C(R^a)=C(R^b)(R^f)$;
$R^a$ is independently hydrogen or halogen;
$R^b$ and $R^f$ are independently hydrogen, $C_{1-6}$ alkyl, or $C_{1-6}$ alkyl substituted with one or more $R^{b-1}$;
$R^{b-1}$ is independently halogen, hydroxyl, $C_{1-6}$ alkoxy, or $-NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N";

solution 2:

$R^1$ is $C_{6-20}$ aryl, or $C_{6-20}$ aryl substituted with one or more $R^{1-6}$;
$R^{1-6}$ is independently halogen, $C_{1-6}$ alkyl, or $C_{1-6}$ alkyl substituted with one or more $R^{1-6-1}$,
$R^{1-6-1}$ is independently halogen;
m is 0;
$R^3$ is $-OR^{31}$ or $-NR^{33}R^{34}$;
$R^{31}$, $R^{33}$ and $R^{34}$ are independently $C_{1-6}$ alkyl substituted with one or more $R^{31-1}$,

$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{e1}$, $R^{e2}$ and $R^{d15}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;
GisN;
n is 0 or 1;
$R^4$ is independently $C_{1-6}$ alkyl, or $C_{1-6}$ alkyl substituted with one or more $R^{4-1}$,
$R^{4-1}$ is independently cyano;
$R^2$ is $-C(=O)-C(R^a)=C(R^b)(R^f)$;
$R^a$ is independently hydrogen or halogen;
$R^b$ and $R^f$ are independently hydrogen or $C_{1-6}$ alkyl;

embodiment 3:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;
m is 0;
$R^3$ is $-OR^{31}$ or $-NR^{33}R^{34}$;
$R^{31}$, $R^{33}$ and $R^{34}$ are independently $C_{1-6}$ alkyl substituted with one or more $R^{31-1}$,
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{d15}$ is independently $C_{1-6}$ alkyl, $C_{1-6}$ alkyl substituted with one or more $R^{1-8-1}$, hydroxyl, $C_{1-6}$ alkoxy, halogen, $-NR^{e5}R^{e6}$ or $-C(=O)NR^{e7}R^{e8}$;
$R^{1-8-1}$ is independently halogen; $R^{e5}$, $R^{e6}$, $R^{e7}$ and $R^{e8}$ are independently hydrogen or $C_{1-6}$ alkyl;
$R^{e1}$ and $R^{e2}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;
G is N, C or CH;
n is 0 or 1;
$R^4$ is independently $C_{1-6}$ alkyl, or $C_{1-6}$ alkyl substituted with one or more $R^{4-1}$,
$R^{4-1}$ is independently cyano or hydroxyl;
$R^2$ is $-C(=O)-C(R^a)=C(R^b)(R^f)$, $-C(=O)-C{=}C-Me$ or $-S(=O)_2-C(R^a)=C(R^b)(R^f)$;
$R^a$ is independently hydrogen or halogen;
$R^b$ and $R^f$ are independently hydrogen, $C_{1-6}$ alkyl, or $C_{1-6}$ alkyl substituted with one or more $R^{b-1}$;
$R^{b-1}$ is independently halogen, hydroxyl, $C_{1-6}$ alkoxy, or $-NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N";

solution 4:

$R^1$ is $C_{6-20}$ aryl, or $C_{6-20}$ aryl substituted with one or more $R^{1-6}$;
$R^{1-6}$ is independently halogen, $C_{1-6}$ alkyl, or $C_{1-6}$ alkyl substituted with one or more $R^{1-6-1}$,
$R^{1-6-1}$ is independently halogen;
m is 0;
$R^3$ is $-OR^{31}$ or $-NR^{33}R^{34}$;
$R^{31}$, $R^{33}$ and $R^{34}$ are independently $C_{1-6}$ alkyl substituted with one or more $R^{31-1}$,
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{d15}$ is independently $C_{1-6}$ alkyl or halogen;
$R^{e1}$ and $R^{e2}$ are independently $C_{1-6}$ alkyl;
ring Y is a 4-12 membered heterocyclic ring containing 1-4 N atoms; the heterocyclic ring is a saturated

heterocyclic ring or a partly saturated heterocyclic ring; the heterocyclic ring is a monocyclic ring;
GisN;
n is 0 or 1;
$R^4$ is independently $C_{1-6}$ alkyl, or $C_{1-6}$ alkyl substituted with one or more $R^{4-1}$,
$R^{4-1}$ is independently cyano;
$R^2$ is -C(=O)-C($R^a$)=C($R^b$)($R^f$);
$R^a$ is independently hydrogen or halogen;
$R^b$ and $R^f$ are independently hydrogen or $C_{1-6}$ alkyl, or $C_{1-6}$ alkyl substituted with one or more $R^{b-1}$;
$R^{b-1}$ is independently -$NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N";

solution 5:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, -C(=O)$R^{65}$, -$NR^{63}R^{64}$, -C(=O)O$R^{66}$, -C(=O)$NR^{69}R^{610}$, $C_{1-6}$ alkyl, $C_{1-6}$ alkoxy, $C_{3-10}$ cycloalkyl, "5-7 membered heterocycloalkyl containing 1 or 2 heteroatoms selected from one or more of O and N", $C_{6-20}$ aryl, "5-7 membered heteroaryl containing 1 or 2 heteroatoms selected from one or more of O and N", $C_{1-6}$ alkyl substituted with one or more $R^{1-6-1}$, $C_{1-6}$ alkoxy substituted with one or more $R^{1-6-2}$, $C_{3-10}$ cycloalkyl substituted with one or more $R^{1-6-3}$, "5-7 membered heterocycloalkyl containing 1 or 2 heteroatoms selected from one or more of O and N" substituted with one or more $R^{1-6-4}$, $C_{6-20}$ aryl substituted with one or more $R^{1-6-5}$, or "5-7 membered heteroaryl containing 1 or 2 heteroatoms selected from one or more of O and N" substituted with one or more $R^{1-6-6}$;
$R^{1-6-1}$ , $R^{1-6-2}$, $R^{1-6-3}$, $R^{1-6-4}$, $R^{1-6-5}$ and $R^{1-6-6}$ are independently cyano, halogen, hydroxyl, $C_{1-6}$ alkoxy, $C_{1-6}$ alkyl, -C(=O)$R^{65-2}$, -$NR^{63-2}R^{64-2}$, -C(=O)O$R^{66-2}$, or -C(=O)$NR^{69-2}R^{610-2}$;
$R^{65}$ , $R^{65-2}$ , $R^{63}$ , $R^{63-2}$, $R^{64,}$ $R^{64-2}$, $R^{66}$, $R^{66-2}$, $R^{69}$, $R^{69-2}$, $R^{610}$ and $R^{610-2}$ are independently hydrogen or $C_{1-6}$ alkyl;
m is 0, 1 or 2;
$R^5$ is independently $C_{1-6}$ alkyl;
$R^3$ is -O$R^{31}$, -S$R^{32}$ or -$NR^{33}R^{34}$;
$R^{31}$, $R^{32}$, $R^{33}$ and $R^{34}$ are independently $C_{1-6}$ alkyl substituted with one or more $R^{31-1}$;
$R^{31-1}$ is independently $C_{3-10}$ cycloalkyl, "4-10 membered heterocycloalkyl containing 1-3 heteroatoms selected from one or more of O and N", $C_{3-10}$ cycloalkyl substituted with one or more $R^{d16}$, "4-10 membered heterocycloalkyl containing 1-3 heteroatoms selected from one or more of O and N" substituted with one or more $R^{d15}$, -O$R^d$, -S$R^{d1}$, -$NR^{e1}R^{e2}$, or -C(=O)$NR^{e3}R^{e4}$;
$R^{a15}$ and $R^{d16}$ are independently $C_{1-6}$ alkyl, $C_{1-6}$ alkyl substituted with one or more $R^{1-8-1}$, hydroxyl, $C_{1-6}$ alkoxy, halogen, -$NR^{e5}R^{e6}$ or -C(=O)$NR^{e7}R^{e8}$;
$R^d$, $R^{d1}$, $R^{e1}$, $R^{e2}$, $R^{e3}$ and $R^{e4}$ are independently hydrogen, $C_{1-6}$ alkyl, $C_{3-10}$ cycloalkyl, "4-10 membered heterocycloalkyl containing 1-3 heteroatoms selected from one or more of O and N", or $C_{1-6}$ alkyl substituted with one or more $R^{1-8-2}$;
$R^{1-8-1}$ and $R^{1-8-2}$ are independently cyano, halogen, hydroxyl, $C_{1-6}$ alkoxy, -C(=O)$R^{e9}$, -$NR^{e10}R^{e11}$, -C(=O)O$R^{e12}$, or -C(=O)$NR^{e13}R^{e14}$;
$R^{e5}$, $R^{e6}$, $R^{e7}$, $R^{e8}$, $R^{e9}$, $R^{e10}$, $R^{e11}$, $R^{e12}$, $R^{e13}$ and $R^{e14}$ are independently hydrogen or $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring, a bridged ring or a spiro ring;
G is N, C or CH;
n is 0, 1, 2 or 3;
$R^4$ is independently $C_{1-6}$ alkyl, $C_{1-6}$ alkyl substituted with one or more $R^{4-1}$, oxo, -C(=O)O$R^{4a}$ or -C(=O)$NR^{4b}R^{4c}$;
$R^{4-1}$ is independently halogen, cyano, hydroxyl, $C_{1-6}$ alkoxy, -$NR^{41}R^{4j}$, -C(=O)O$R^{4d}$ or -C(=O)$NR^{4e}R^{4f}$, $R^{4a}$, $R^{4e}$, $R^{4f}$, $R^{41}$ and $R^{4j}$ are independently hydrogen or $C_{1-6}$ alkyl;
$R^{4a}$, $R^{4b}$ and $R^{4c}$ are independently hydrogen or $C_{1-6}$ alkyl;
$R^2$ is -C(=O)-C($R^a$)=C($R^b$)($R^f$), -C(=O)-C≡C$R^f$, -S(=O)$_2$-C($R^a$)=C($R^b$)($R^f$) or -S(=O)$_2$-C≡C$R^f$;
$R^a$ is independently hydrogen, deuterium, halogen or $C_{1-6}$ alkyl;

$R^b$ and $R^f$ are independently hydrogen, deuterium, $C_{1-6}$ alkyl, or $C_{1-6}$ alkyl substituted with one or more $R^{b-1}$;
$R^{b-1}$ is independently halogen, hydroxyl, $C_{1-6}$ alkoxy, or $-NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-10 membered heterocycloalkyl containing 1-3 heteroatoms selected from one or more of O and N";

solution 6:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ and $R^{1-7}$ are independently halogen, hydroxyl, $C_{1-6}$ alkyl, $C_{1-6}$ alkoxy, $C_{3-10}$ cycloalkyl, $C_{1-6}$ alkyl substituted with one or more $R^{1-6-1}$, or $C_{1-6}$ alkoxy substituted with one or more $R^{1-6-2}$; $R^{1-6-1}$ and $R^{1-6-2}$ are independently halogen;
m is 0;
$R^3$ is $-OR^{31}$, $-SR^{32}$ or $-NR^{33}R^{34}$;
$R^{31}$, $R^{32}$ and $R^{34}$ are independently $C_{1-6}$ alkyl substituted with one or more $R^{31-1}$, $R^{33}$ is independently H, $C_{1-6}$ alkyl, or $C_{1-6}$ alkyl substituted with one or more $R^{31-1}$,
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{d15}$ is independently $C_{1-6}$ alkyl or halogen;
$R^{e1}$ and $R^{e2}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring or a spiro ring;
G is N, C or CH;
n is 0 or 1;
$R^4$ is independently $C_{1-6}$ alkyl, or $C_{1-6}$ alkyl substituted with one or more $R^{4-1}$,
$R^{4-1}$ is independently cyano, hydroxyl or $-C(=O)NR^{4e}R^{4f}$, $R^{4e}$ and $R^{4f}$ are independently hydrogen or $C_{1-6}$ alkyl;
$R^2$ is CN, $-C(=O)-C(R^a)=C(R^b)(R^f)$, $-C(=O)-C\equiv C-Me$ or, $-S(=O)_2-C(R^a)=C(R^b)(R^f)$;
$R^a$ is independently hydrogen or halogen;
$R^b$ and $R^f$ are independently hydrogen, $C_{1-6}$ alkyl, $C_{1-6}$ alkyl-C(=O)-, or $C_{1-6}$ alkyl substituted with one or more $R^{b-1}$;
$R^{b-1}$ is independently halogen, hydroxyl, $C_{1-6}$ alkoxy, or $-NR^{10j}R^{10k}$;
$R^{10j}$ and $R^{10k}$ are independently hydrogen or $C_{1-6}$ alkyl, or $R^{10j}$ and $R^{10k}$ taken together with the N atom to which they are attached form "4-6 membered heterocycloalkyl containing 1-3 heteroatoms selected from one or more of O and N";

solution 7:

$R^1$ is $C_{6-20}$ aryl, "5-12 membered heteroaryl containing 1-4 heteroatoms selected from one or more of O, S and N", $C_{6-20}$ aryl substituted with one or more $R^{1-6}$, or "5-12 membered heteroaryl containing 1-4 heteroatoms selected from one or more of O, S and N" substituted with one or more $R^{1-7}$;
$R^{1-6}$ is independently halogen, $C_{1-6}$ alkyl, or $C_{1-6}$ alkyl substituted with one or more $R^{1-6-1}$,
$R^{1-6-1}$ is independently halogen;
m is 0;
$R^3$ is $-OR^{31}$, $-SR^{32}$ or $-NR^{33}R^{34}$;
$R^{31}$, $R^{32}$ and $R^{34}$ are independently $C_{1-6}$ alkyl substituted with one or more $R^{31-1}$, $R^{33}$ is independently H, $C_{1-6}$ alkyl, or $C_{1-6}$ alkyl substituted with one or more $R^{31-1}$,
$R^{31-1}$ is independently "5-7 membered heterocycloalkyl containing 1-3 heteroatoms selected from one or more of O and N" substituted with one or more $R^{d15}$, or $-NR^{e1}R^{e2}$;
$R^{d15}$ is independently $C_{1-6}$ alkyl or halogen;
$R^{e1}$ and $R^{e2}$ are independently $C_{1-6}$ alkyl;
ring Y is a 4-12 membered heterocyclic ring containing 1-4 N atoms; the heterocyclic ring is a saturated heterocyclic ring or a partly saturated heterocyclic ring; the heterocyclic ring is a monocyclic ring;
GisN;
n is 0 or 1;

$R^4$ is independently $C_{1-6}$ alkyl substituted with one or more $R^{4-1}$,
$R^{4-1}$ is independently cyano or $-C(=O)NR^{4e}R^{4f}$, $R^{4e}$ and $R^{4f}$ are independently hydrogen or $C_{1-6}$ alkyl;
$R^2$ is CN, $-C(=O)-C(R^a)=C(R^b)(R^f)$;
$R^a$ is independently hydrogen or halogen;
$R^b$ and $R^f$ are independently hydrogen, $C_{1-6}$ alkyl, or $C_{1-6}$ alkyl-C(=O)-.

**7.** The oxygen-containing heterocyclic compound represented by formula **I** as defined in any one of claims 1-6, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein the oxygen-containing heterocyclic compound represented by formula **I** has a structure as follows:

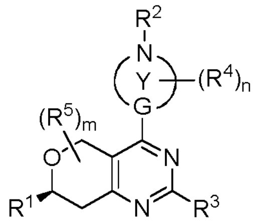
I-2
,
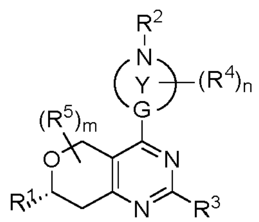
I-3

or "a mixture of

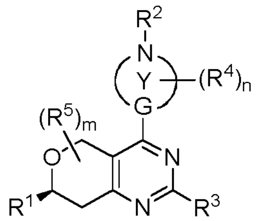
I-2
and
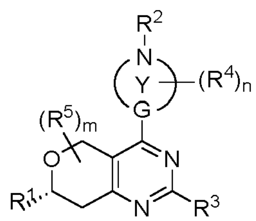
I-3

, with a molar ratio of, for example, 1 : 1";

and/or, when $R^1$ is $C_{6-20}$ aryl, then the $C_{6-20}$ aryl is phenyl or naphthyl;
and/or, when $R^1$ is "5-12 membered heteroaryl containing 1-4 heteroatoms selected from one or more of O, S and N", then the "5-12 membered heteroaryl containing 1-4 heteroatoms selected from one or more of O, S and N" is "9-10 membered heteroaryl containing 1-4 heteroatoms selected from one or more of O, S and N";
and/or, when $R^1$ is $C_{6-20}$ aryl substituted with one or more $R^{1-6}$, then the $C_{6-20}$ aryl is phenyl or naphthyl;
and/or, when $R^1$ is $C_{6-20}$ aryl substituted with one or more $R^{1-6}$, then the more $R^{1-6}$ is two or three $R^{1-6}$;
and/or, when $R^{1-6}$ is independently halogen, then the halogen is fluorine, chlorine, bromine or iodine;
and/or, when $R^{1-6}$ is independently $C_{1-6}$ alkyl, then the $C_{1-6}$ alkyl is $C_{1-4}$ alkyl;
and/or, when $R^{1-6}$ is independently $C_{1-6}$ alkyl substituted with one or more $R^{1-6-1}$, then the $C_{1-6}$ alkyl is $C_{1-4}$ alkyl;
and/or, when $R^{1-6}$ is independently $C_{1-6}$ alkyl substituted with one or more $R^{1-6-1}$, the more $R^{1-6-1}$ is two or three $R^{1-6-1}$,
and/or, when $R^{1-6-1}$ is independently halogen, then the halogen is fluorine, chlorine, bromine or iodine;
and/or, when $R^{31}$, $R^{33}$ and $R^{34}$ are independently $C_{1-6}$ alkyl substituted with one or more $R^{31-1}$, then the $C_{1-6}$ alkyl is $C_{1-4}$ alkyl;
and/or, when $R^{31}$, $R^{33}$ and $R^{34}$ are independently $C_{1-6}$ alkyl substituted with one or more $R^{31-1}$, then the more $R^{31-1}$ is two or three $R^{31-1}$,
and/or, when $R^{31-1}$ is independently "4-10 membered heterocycloalkyl containing 1-3 heteroatoms selected from one or more of O and N" substituted with one or more $R^{d15}$, then the "4-10 membered heterocycloalkyl containing 1-3 heteroatoms selected from one or more of O and N" is "5-7 membered heterocycloalkyl containing 1-3 heteroatoms selected from one or more of O and N";
and/or, when $R^{31-1}$ is independently "4-10 membered heterocycloalkyl containing 1-3 heteroatoms selected from one or more of O and N" substituted with one or more $R^{d15}$, then the more $R^{d15}$ is two or three $R^{d15}$;
and/or, when $R^{d15}$ is independently $C_{1-6}$ alkyl, then the $C_{1-6}$ alkyl is $C_{1-4}$ alkyl;
and/or, when $R^{d15}$ is independently halogen, then the halogen is fluorine, chlorine, bromine or iodine;

and/or, when $R^{e1}$ and $R^{e2}$ are independently $C_{1-6}$ alkyl, then the $C_{1-6}$ alkyl is $C_{1-4}$ alkyl;
and/or, when ring Y is a 4-12 membered heterocyclic ring containing 1-4 N atoms, then the 4-12 membered heterocyclic ring containing 1-4 N atoms is 6-9 membered heterocyclic ring containing 1-2 N atoms;
and/or, when $R^4$ is independently $C_{1-6}$ alkyl, then the $C_{1-6}$ alkyl is $C_{1-4}$ alkyl;
and/or, when $R^4$ is independently $C_{1-6}$ alkyl substituted with one or more $R^{4-1}$, then the $C_{1-6}$ alkyl is $C_{1-4}$ alkyl;
and/or, when $R^4$ is independently $C_{1-6}$ alkyl substituted with one or more $R^{4-1}$, then the more $R^{4-1}$ is two or three $R^{4-1}$;
and/or, when $R^a$ is independently halogen, then the halogen is fluorine, chlorine, bromine or iodine;
and/or, when $R^b$ and $R^f$ are independently $C_{1-6}$ alkyl, then the $C_{1-6}$ alkyl is $C_{1-4}$ alkyl;
and/or, when $R^b$ and $R^f$ are independently $C_{1-6}$ alkyl substituted with one or more $R^{b-1}$, then the $C_{1-6}$ alkyl is $C_{1-4}$ alkyl;
and/or, when $R^b$ and $R^f$ are independently $C_{1-6}$ alkyl substituted with one or more $R^{b-1}$, then the more $R^{b-1}$ is two or three $R^{b-1}$;
and/or, when $R^{10j}$ and $R^{10k}$ are independently $C_{1-6}$ alkyl, then the $C_{1-6}$ alkyl is $C_{1-4}$ alkyl.

**8.** The oxygen-containing heterocyclic compound represented by formula I as defined in claim 7, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, when $R^1$ is "5-12 membered heteroaryl containing 1-4 heteroatoms selected from one or more of O, S and N", then the "5-12 membered heteroaryl containing 1-4 heteroatoms selected from one or more of O, S and N" is isoquinolyl;

and/or, when $R^1$ is $C_{6-20}$ aryl substituted with one or more $R^{1-6}$, then the $C_{6-20}$ aryl is phenyl or 1-naphthyl;
and/or, when $R^{1-6}$ is independently halogen, then the halogen is fluorine or chlorine;
and/or, when $R^{1-6}$ is independently $C_{1-6}$ alkyl, then the $C_{1-6}$ alkyl is methyl, ethyl, n-propyl, isopropyl, n-butyl, isobutyl, sec-butyl or tert-butyl;
and/or, when $R^{1-6}$ is independently $C_{1-6}$ alkyl substituted with one or more $R^{1-6-1}$, then the $C_{1-6}$ alkyl is methyl, ethyl, n-propyl, isopropyl, n-butyl, isobutyl, sec-butyl or tert-butyl;
and/or, when $R^{1-6-1}$ is independently halogen, then the halogen is fluorine;
and/or, when $R^{31}$, $R^{33}$ and $R^{34}$ are independently $C_{1-6}$ alkyl substituted with one or more $R^{31-1}$, then the $C_{1-6}$ alkyl is methyl, ethyl, n-propyl, isopropyl, n-butyl, isobutyl, sec-butyl or tert-butyl;
and/or, when $R^{31-1}$ is independently "4-10 membered heterocycloalkyl containing 1-3 heteroatoms selected from one or more of O and N" substituted with one or more $R^{d15}$, then the "4-10 membered heterocycloalkyl containing 1-3 heteroatoms selected from one or more of O and N" is "5-7 membered heterocycloalkyl containing 1 heteroatom selected from one of O and N";
and/or, when $R^{d15}$ is independently $C_{1-6}$ alkyl, then the $C_{1-6}$ alkyl is methyl, ethyl, n-propyl, isopropyl, n-butyl, isobutyl, sec-butyl or tert-butyl;
and/or, when $R^{d15}$ is independently halogen, then the halogen is fluorine;
and/or, when $R^{e1}$ and $R^{e2}$ are independently $C_{1-6}$ alkyl, then the $C_{1-6}$ alkyl is methyl, ethyl, n-propyl, isopropyl, n-butyl, isobutyl, sec-butyl or tert-butyl;
and/or, when ring Y is a 4-12 membered heterocyclic ring containing 1-4 N atoms, then the 4-12 membered heterocyclic ring containing 1-4 N atoms is

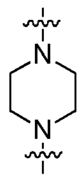 , 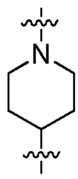 ,

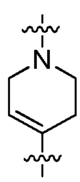 or 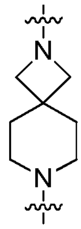 ;

, which, at its upper end, is connected to $R^2$;

and/or, when $R^4$ is independently $C_{1-6}$ alkyl, then the $C_{1-6}$ alkyl is methyl, ethyl, n-propyl, isopropyl, n-butyl, isobutyl, sec-butyl or tert-butyl;

and/or, when $R^4$ is independently $C_{1-6}$ alkyl substituted with one or more $R^{4-1}$, then the $C_{1-6}$ alkyl is methyl, ethyl, n-propyl, isopropyl, n-butyl, isobutyl, sec-butyl or tert-butyl;

and/or, when $R^a$ is independently halogen, then the halogen is fluorine;

and/or, when $R^b$ and $R^f$ are independently $C_{1-6}$ alkyl, then the $C_{1-6}$ alkyl is methyl, ethyl, n-propyl, isopropyl, n-butyl, isobutyl, sec-butyl or tert-butyl;

and/or, when $R^b$ and $R^f$ are independently $C_{1-6}$ alkyl substituted with one or more $R^{b-1}$, then the $C_{1-6}$ alkyl is methyl, ethyl, n-propyl, isopropyl, n-butyl, isobutyl, sec-butyl or tert-butyl;

and/or, when $R^{10j}$ and $R^{10k}$ are independently $C_{1-6}$ alkyl, then the $C_{1-6}$ alkyl is methyl, ethyl, n-propyl, isopropyl, n-butyl, isobutyl, sec-butyl or tert-butyl.

**9.** The oxygen-containing heterocyclic compound represented by formula **I** as defined in claim 8, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, when $R^1$ is "5-12 membered heteroaryl containing 1-4 heteroatoms selected from one or more of O, S and N", then the "5-12 membered heteroaryl containing 1-4 heteroatoms selected from one or more of O, S and N" is

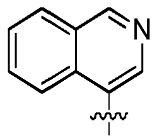
;

and/or, when $R^{1-6}$ is independently $C_{1-6}$ alkyl, then the $C_{1-6}$ alkyl is methyl;

and/or, when $R^{1-6}$ is independently $C_{1-6}$ alkyl substituted with one or more $R^{1-6-1}$, then the $C_{1-6}$ alkyl substituted with one or more $R^{1-6-1}$ is trifluoromethyl;

and/or, when $R^{31}$, $R^{33}$ and $R^{34}$ are independently $C_{1-6}$ alkyl substituted with one or more $R^{31-1}$, then the $C_{1-6}$ alkyl is methyl, ethyl, n-propyl or isopropyl;

and/or, when $R^{31-1}$ is independently "4-10 membered heterocycloalkyl containing 1-3 heteroatoms selected from one or more of O and N" substituted with one or more $R^{d15}$, then the "4-10 membered heterocycloalkyl containing 1-3 heteroatoms selected from one or more of O and N" is tetrahydropyrrolyl;

and/or, when $R^{d15}$ is independently $C_{1-6}$ alkyl, then the $C_{1-6}$ alkyl is methyl;

and/or, when $R^{e1}$ and $R^{e2}$ are independently $C_{1-6}$ alkyl, then the $C_{1-6}$ alkyl is methyl or ethyl;

and/or, the oxygen-containing heterocyclic compound represented by formula **I** has a structure as follows:

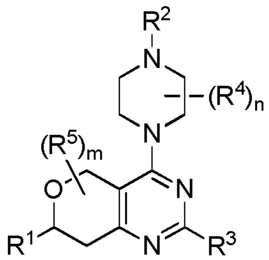
**II**
;

and/or, when $R^4$ is independently $C_{1-6}$ alkyl, then the $C_{1-6}$ alkyl is methyl;

and/or, when $R^4$ is independently $C_{1-6}$ alkyl substituted with one or more $R^{4-1}$, then the $C_{1-6}$ alkyl is methyl;

and/or, when $R^b$ and $R^f$ are independently $C_{1-6}$ alkyl, then the $C_{1-6}$ alkyl is methyl;

and/or, when $R^b$ and $R^f$ are independently $C_{1-6}$ alkyl substituted with one or more $R^{b-1}$, then the $C_{1-6}$ alkyl is methyl;

and/or, when $R^{10j}$ and $R^{10k}$ are independently $C_{1-6}$ alkyl, then the $C_{1-6}$ alkyl is methyl.

**10.** The oxygen-containing heterocyclic compound represented by formula **I** as defined in claim 9, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein,

the oxygen-containing heterocyclic compound represented by formula **I** has a structure as follows:

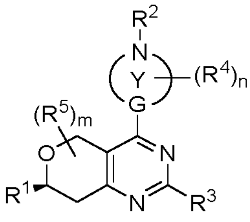

**I-2** ;

and/or, when $R^{33}$ is independently $C_{1-6}$ alkyl, then the $C_{1-6}$ alkyl is methyl, ethyl, n-propyl, isopropyl, n-butyl, isobutyl, sec-butyl or tert-butyl, or may be methyl, ethyl, n-propyl or isopropyl;
and/or, when $R^4$ is independently $C_{1-6}$ alkyl substituted with one or more $R^{4-1}$, then the $C_{1-6}$ alkyl substituted with one or more $R^{4-1}$ is hydroxymethyl, cyanomethyl or

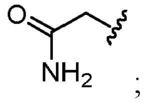 ;

and/or, when $R^b$ and $R^f$ are independently $C_{1-6}$ alkyl-C(=O)-, then the $C_{1-6}$ alkyl in the $C_{1-6}$ alkyl-C(=O)- is methyl, ethyl, n-propyl, isopropyl, n-butyl, isobutyl, sec-butyl or tert-butyl, or may be methyl;
and/or, the $R^2$ is CN,

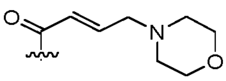, 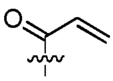, 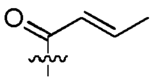, 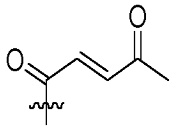,

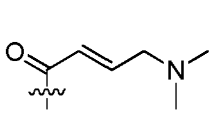, 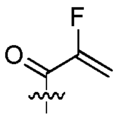, 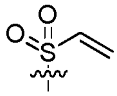 or 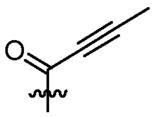 .

**11.** The oxygen-containing heterocyclic compound represented by formula **I** as defined in claim 9, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein,

when $R^1$ is $C_{6-20}$ aryl substituted with one or more $R^{1-6}$, then the $C_{6-20}$ aryl substituted with one or more $R^{1-6}$ is

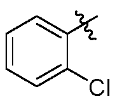, 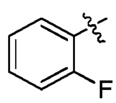, 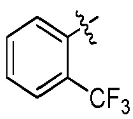, 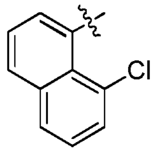

or

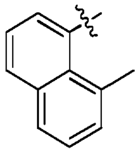 ;

and/or, when $R^4$ is independently $C_{1-6}$ alkyl substituted with one or more $R^{4-1}$, then the $C_{1-6}$ alkyl substituted with

one or more $R^{4-1}$ is hydroxymethyl or cyanomethyl;
and/or, when $R^{10j}$ and $R^{10k}$ taken together with the N atom to which they are attached form "4-10 membered heterocycloalkyl containing 1-3 heteroatoms selected from one or more of O and N", then the "4-10 membered heterocycloalkyl containing 1-3 heteroatoms selected from one or more of O and N" is "5-6 membered heterocycloalkyl containing 2 heteroatoms selected from O and N", or may be

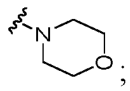;

and/or, $R^2$ is

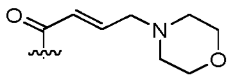, 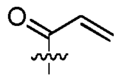, 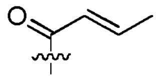, 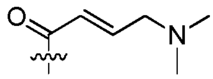,

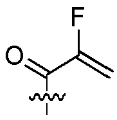, 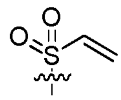 or 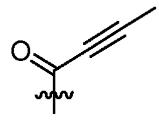.

**12.** The oxygen-containing heterocyclic compound represented by formula **I** as defined in claim 9, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, when $R^1$ is $C_{6-20}$ aryl substituted with one or more $R^{1-6}$, then the $C_{6-20}$ aryl substituted with one or more $R^{1-6}$ is

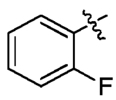, 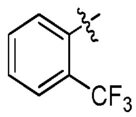, 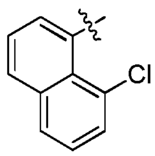 or 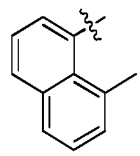;

and/or, when $R^{31}$, $R^{33}$ and $R^{34}$ are independently $C_{1-6}$ alkyl substituted with one or more $R^{31-1}$, then the $C_{1-6}$ alkyl substituted with one or more $R^{31-1}$ is

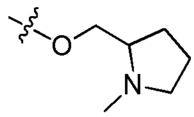,

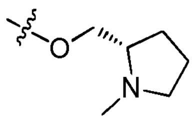, 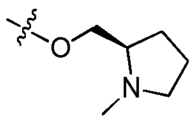, 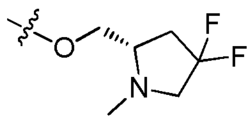, 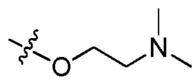,

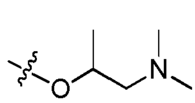, 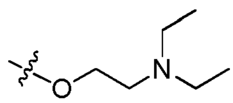, 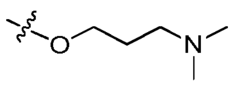 or 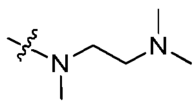;

and/or, when $R^4$ is independently $C_{1-6}$ alkyl substituted with one or more $R^{4-1}$, then the $C_{1-6}$ alkyl substituted with one or more $R^{4-1}$ is cyanomethyl;
and/or, $R^2$ is

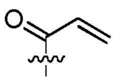,
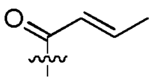,
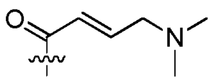,
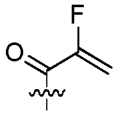,
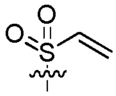

or

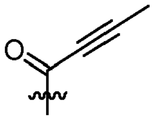.

13. The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, the oxygen-containing heterocyclic compound represented by formula **I** has any one of the following structures:

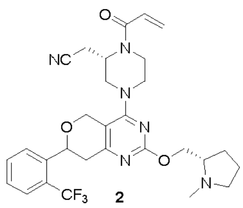
**2**

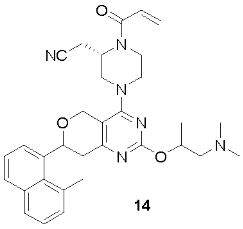
**14**

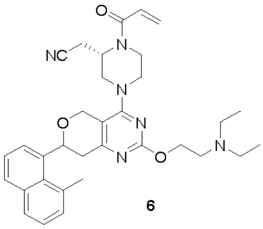
**6**

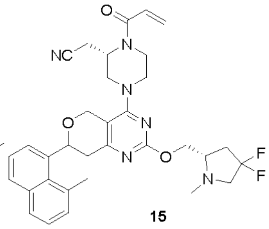
**15**

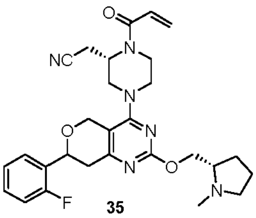
**35**

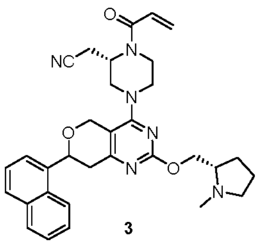
**3**

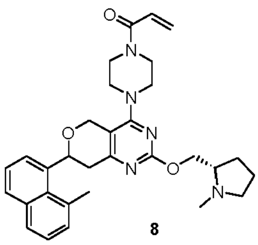
**8**

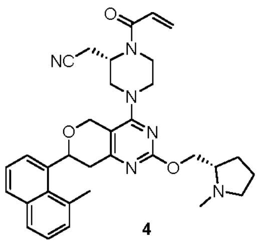
**4**

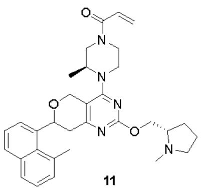
**11**

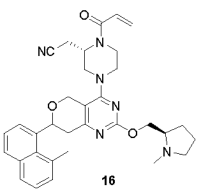
**16**

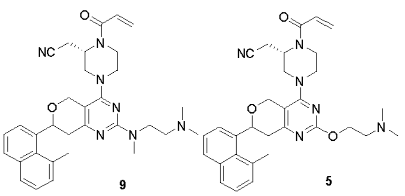
**9**

**5**

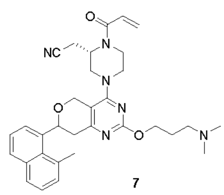
**7**

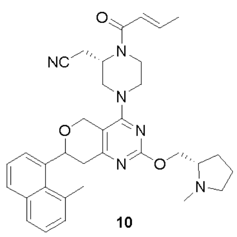
**10**

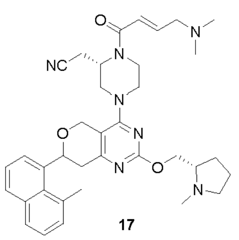
**17**

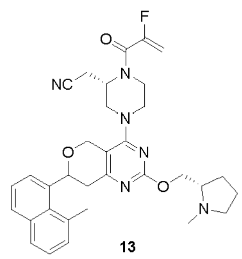
**13**

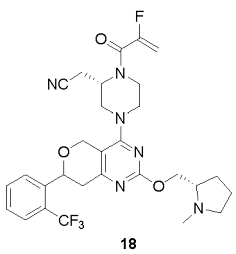
**18**

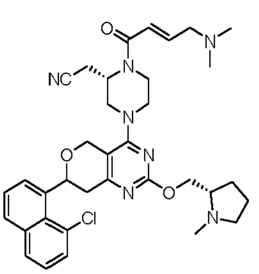

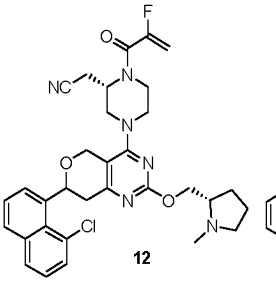

12

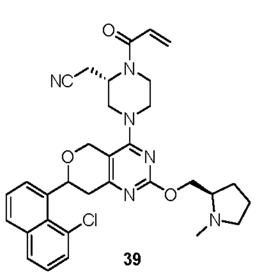

39

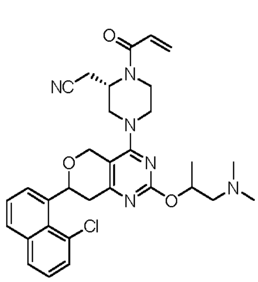

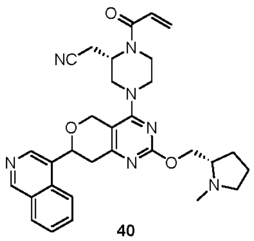

40

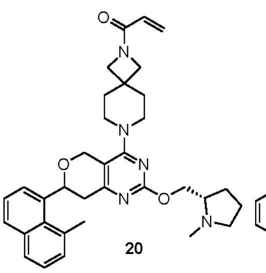

20

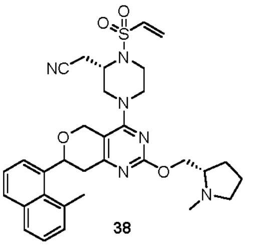

38

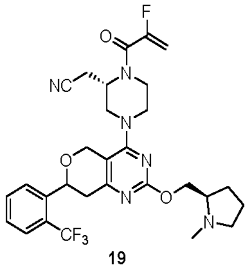

19

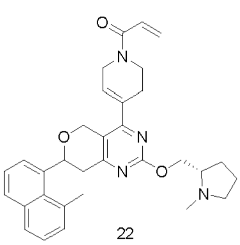

22

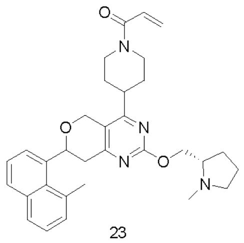

23

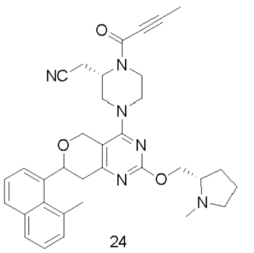

24

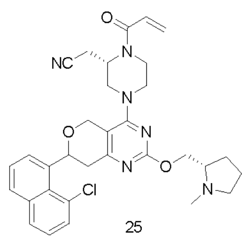

25

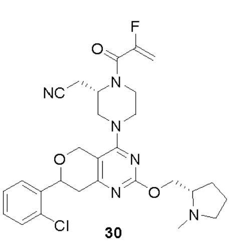

30

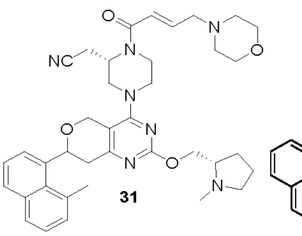

31

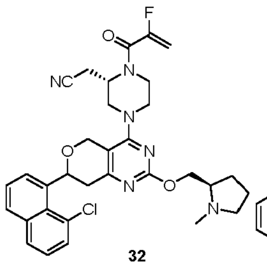

32

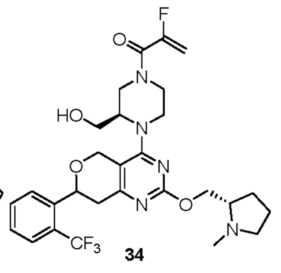

34

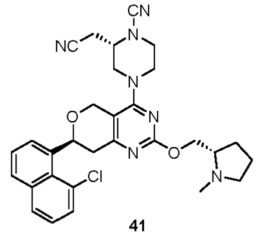

41

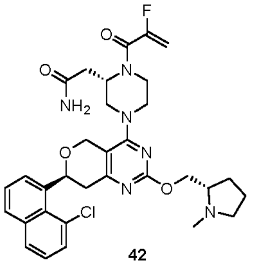

42

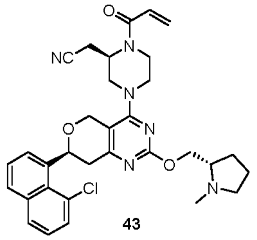

43

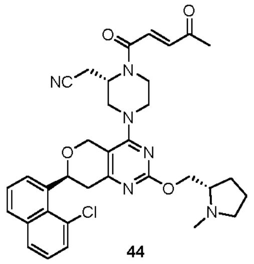

44

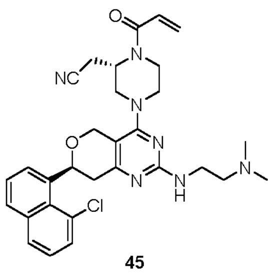
45
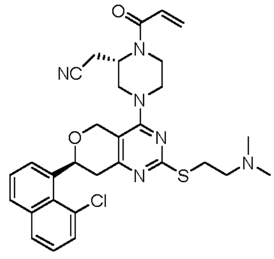
46
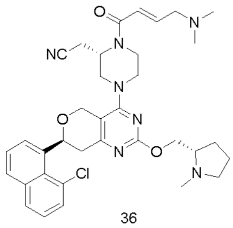
36
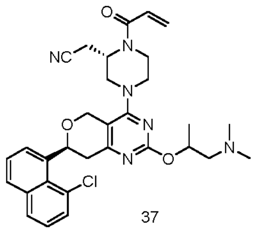
37
.

14. The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein, the oxygen-containing heterocyclic compound represented by formula **I** is any one of the following compounds:

compound

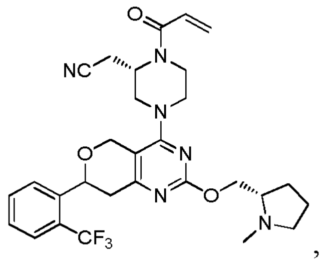
,

which has a retention time of 0.92 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar; compound

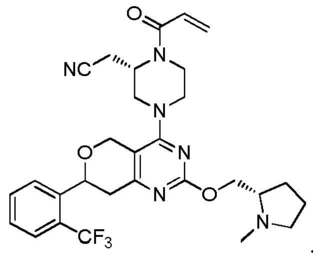
:

, which has a retention time of 2.74 min under the following conditions: equipment: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar; compound

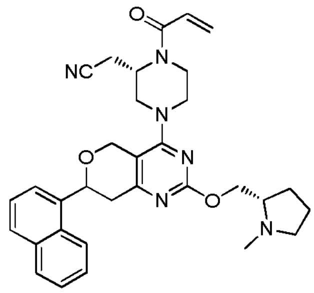

, which has a retention time of 0.97 min under the following conditions: equipment: SFC Method Station (Thar,

Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar; compound

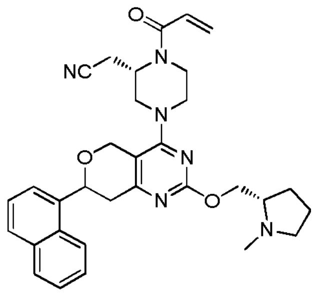

, which has a retention time of 2.40 min under the following conditions: equipment: SFC Method Station (Thar, Waters); chromatographic column: AD-H 4.6 * 100 mm, 5 μm (Daicel); column temperature: 40 °C; mobile phase: $CO_2$/ETOH (0.5% TEA) = 55/45; flow rate: 4.0 ml/min; wavelength: 254 nm; back pressure: 120 bar; compound

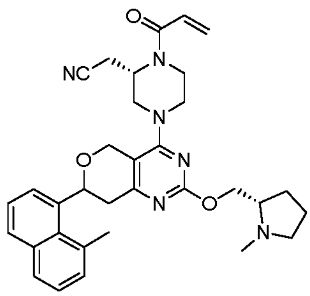

, which has a retention time of 0.97 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar; compound

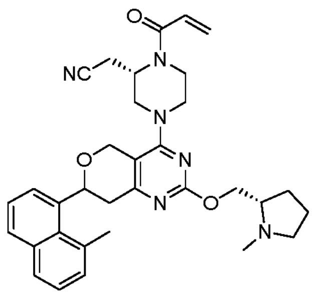

, which has a retention time of 1.94 min under the following conditions: equipment: SFC Method Station (Thar, Waters); chromatographic column: OJ-H 4.6 * 100 mm, 5 μm (Daicel); column temperature: 40 °C; mobile phase: $CO_2$/Methanol (0.1% TEA) = 60/40; flow rate: 4.0 ml/min; wavelength: 254 nm; back pressure: 120 bar; compound

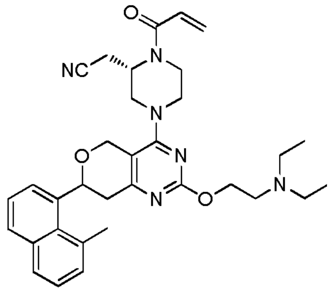

, which has a retention time of 1.22 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;
compound

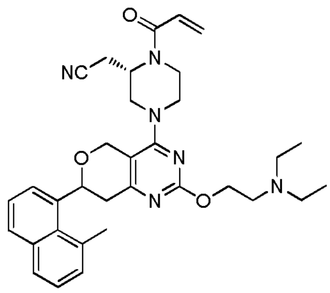

,

, which has a retention time of 2.67 min under the following conditions: equipment: SFC Method Station (Thar, Waters); chromatographic column: CHIRALCEL OJ-H 4.6 * 100 mm, 5 μm (Daicel); column temperature: 40 °C; mobile phase: $CO_2$/MeOH (0.1% TEA) = 65/35; flow rate: 1.0 ml/min; wavelength: 214 nm; back pressure: 120 bar;
compound

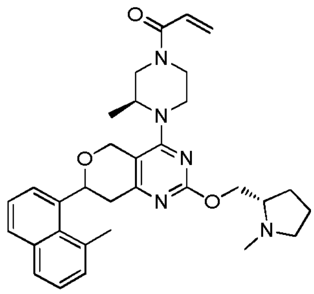

, which has a retention time of 3.26 min under the following conditions: instrument: SFC Method Station (Thar, Waters); chromatographic column: R,R-WHELK-O1 4.6 * 100 mm, 5 μm (REGIS); column temperature: 40 °C; mobile phase: CO2/(MeOH/ACN = 3 : 2 (0.1% TEA)) = 55/45; flow rate: 4.0 ml/min; wavelength: 254 nm; back pressure: 120 bar;
compound

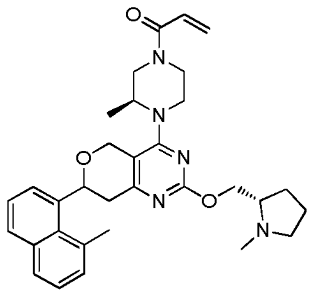

, which has a retention time of 4.16 min under the following conditions: instrument: SFC Method Station (Thar, Waters); chromatographic column: R,R-WHELK-O1 4.6 * 100 mm, 5 μm (REGIS); column temperature: 40 °C; mobile phase: $CO_2$/(MeOH/CAN = 3 : 2 (0.1% TEA)) = 55/45; flow rate: 4.0 ml/min; wavelength: 254 nm; back pressure: 120 bar;
compound

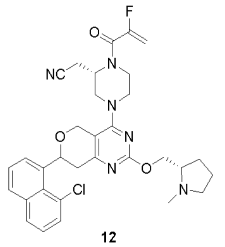

12

, which has a retention time of 1.36 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;
compound

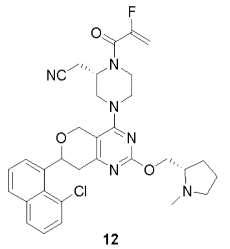

12

;

, which has a retention time of 2.77 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;
compound

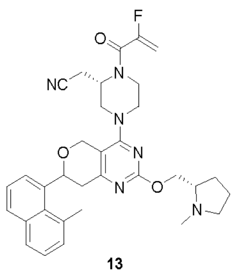

13

, which has a retention time of 1.17 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar;

compound

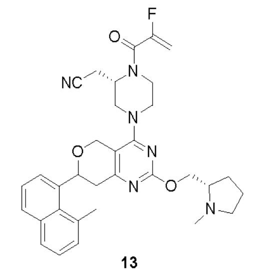

13

;

, which has a retention time of 2.76 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar; compound

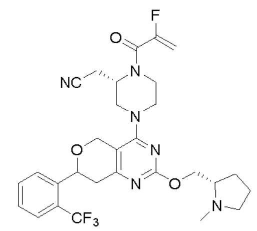

18

, which has a retention time of 0.78 min under the following conditions: instrument: SFC Method Station (Thar, Waters); chromatographic column: AD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar; compound

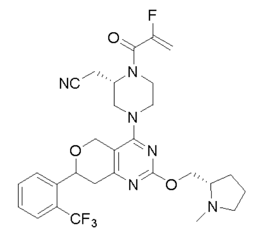

18

;

, which has a retention time of 2.42 min under the following conditions: instrument: SFC Method Station (Thar, Waters); chromatographic column: AD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar; compound

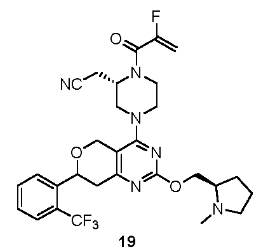

19

, which has a retention time of 0.79 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar;

compound

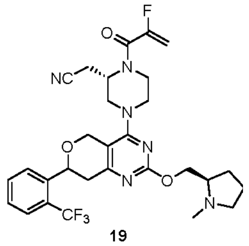

19

, which has a retention time of 2.29 min under the following conditions: instrument: SFC Method Station (Thar, Waters); chromatographic column: OD-H 4.6 * 100 mm, 5 μm (Daicel); column temperature: 40 °C; mobile phase: $CO_2$/MeOH (0.1% TEA) = 65/35; flow rate: 4.0 ml/min; wavelength: 254 nm; back pressure: 120 bar; compound

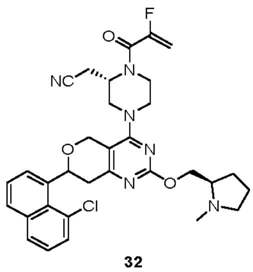

32

, which has a retention time of 1.45 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar; compound

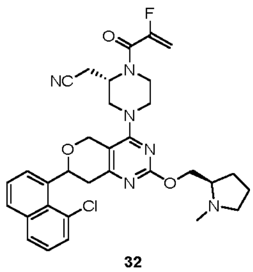

32

, which has a retention time of 2.81 min under the following conditions: instrument: SFC Method Station (Thar, Waters); chromatographic column: OJ-H 4.6 * 100 mm, 5 μm (Daicel); column temperature: 40 °C; mobile phase: $CO_2$/MeOH (0.1% TEA) = 60/40; flow rate: 4.0 ml/min; wavelength: 254 nm; back pressure: 120 bar.

**15.** The oxygen-containing heterocyclic compound represented by formula **I** as defined in claim 1, a pharmaceutically acceptable salt thereof, a solvate thereof, a solvate of the pharmaceutically acceptable salt thereof, a crystal form thereof, a stereoisomer thereof, a tautomer thereof or an isotopic compound thereof, wherein the oxygen-containing heterocyclic compound represented by formula **I** is any one of the following compounds:

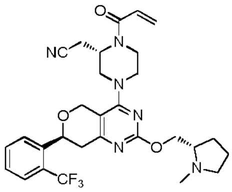 , 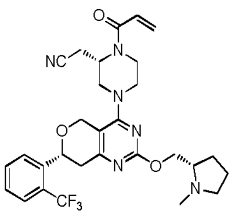 , 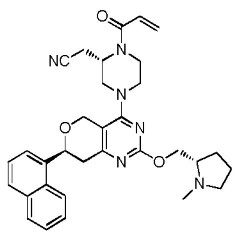 , 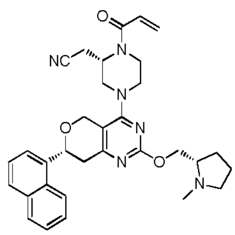 ,

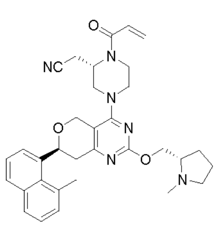, 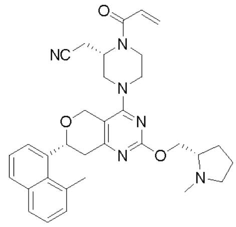, 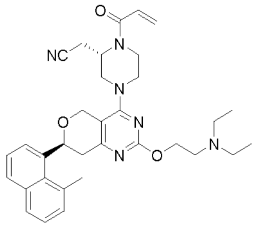, 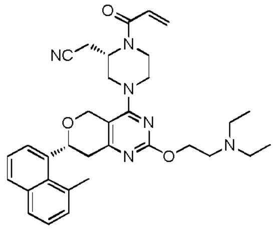,

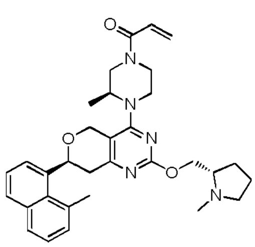, 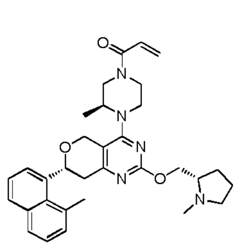, 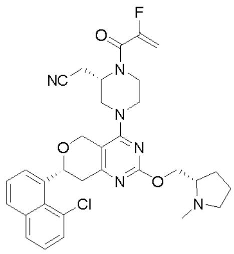, 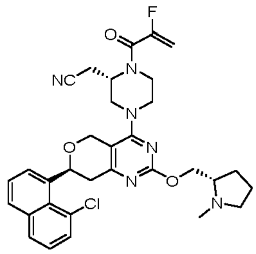,

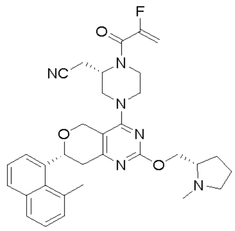, 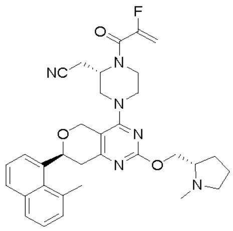, 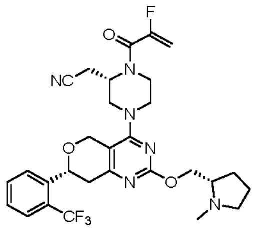, 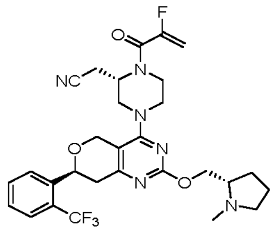,

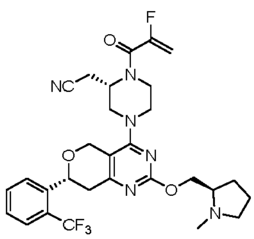, 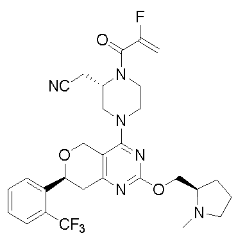, 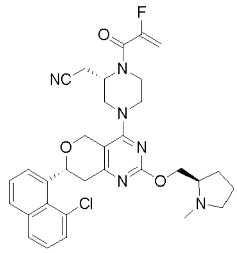, 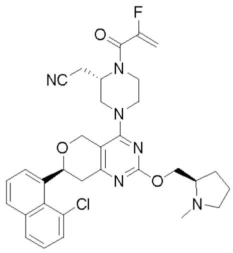.

**16.** A method for preparing the oxygen-containing heterocyclic compound represented by formula **I** as defined in any one of claims 1-15, the method comprising route one or route two:
route one:

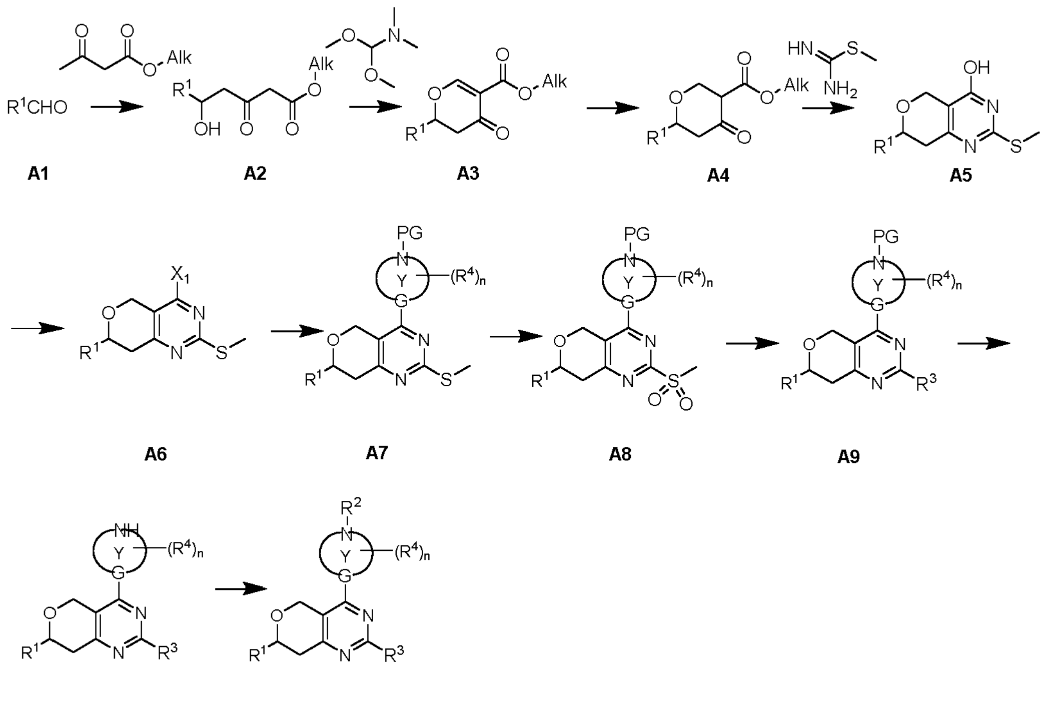

A1 A2 A3 A4 A5

A6 A7 A8 A9

A10 A11

wherein, $X_1$ is a leaving group; Alk is alkyl; and PG is an amino protecting group;
route two:

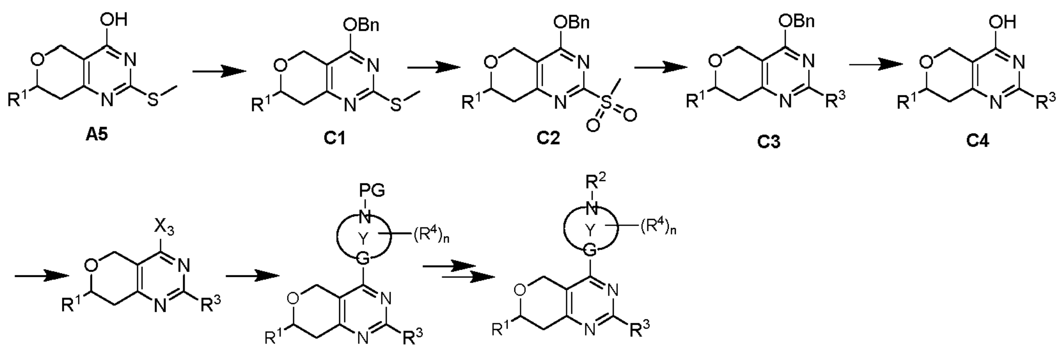

A5 C1 C2 C3 C4

C5 A9 A11

wherein, $X_3$ is a leaving group and PG is an amino protecting group;
the leaving group is OTf or Cl;
the amino protecting group is Boc or Cbz.

**17.** A compound represented by formula A5, A6, A7, A8, A9, A10, C1, C2, C3, C4 or C5;

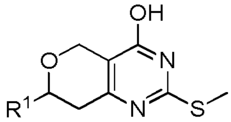

A5

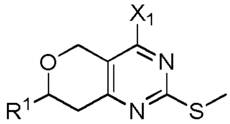

A6

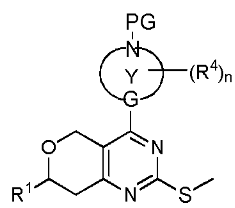

A7

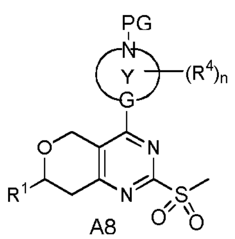

A8

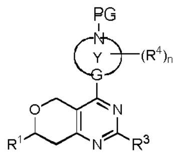
A9

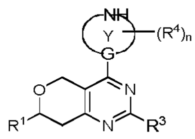
A10

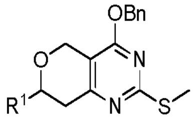
**C1**

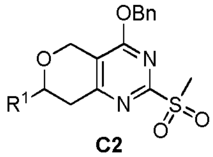
**C2**

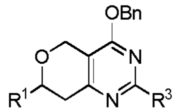
**C3**

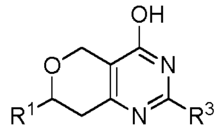
**C4** ,

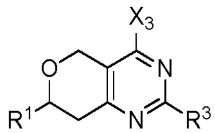
**C5** ;

wherein $R^1$, $R^3$, $R^4$, G, Y and n are as defined in any one of claims 1-15;
$X^1$ and $X^3$ are independently leaving groups and PG is an amino protecting group;
the leaving group is OTf or Cl;
the amino protecting group is Boc or Cbz;
for example, the compound represented by formula A5, A6, A7, A8, A9, A10, C1, C2, C3, C4 or C5 is any one of the following compounds:

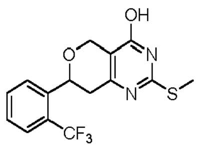
**2-f**

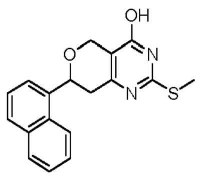
**3-f**

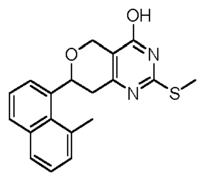
**4-f**

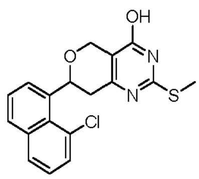
**12-f**

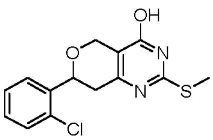
**30-f**

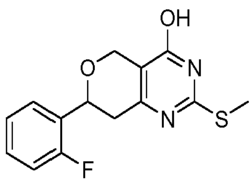
**35-f**

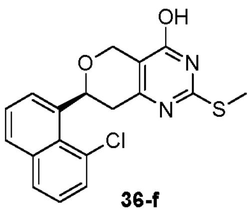
**36-f**

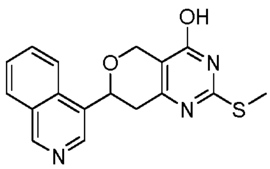
**40-f**

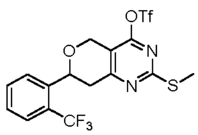
**2-e**

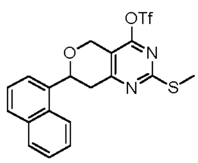
**3-e**

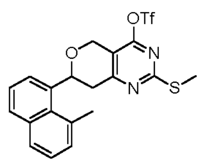
**4-e**

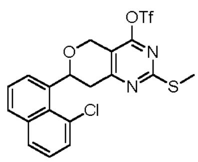
**12-e**

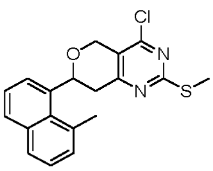
**20-e**

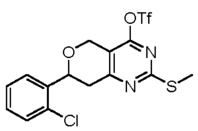
**30-e**

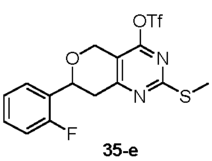
**35-e**

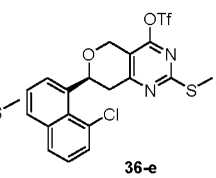
**36-e**

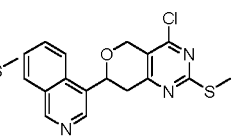
**40-e**

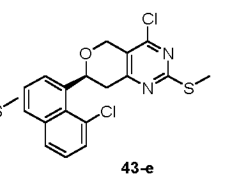
**43-e**

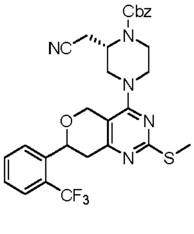
2-d

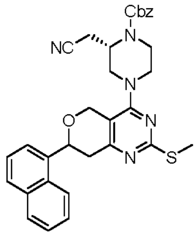
3-d

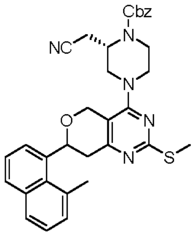
4-d

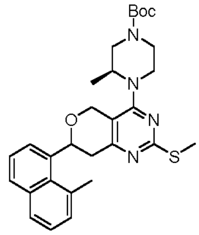
11-d

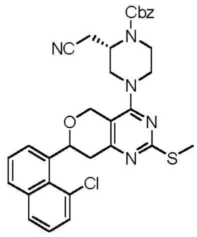
12-d

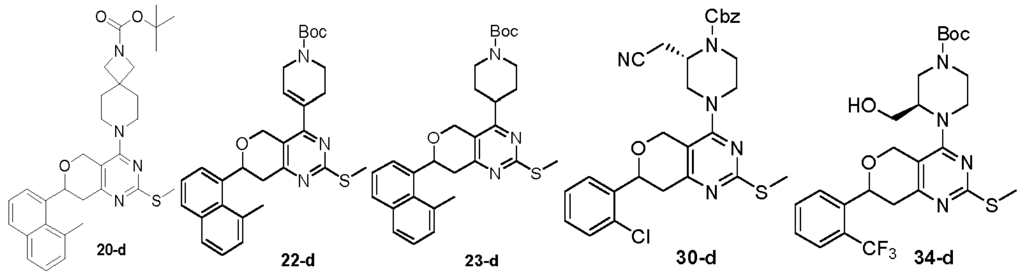
20-d 22-d 23-d 30-d 34-d

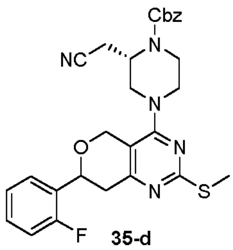
35-d

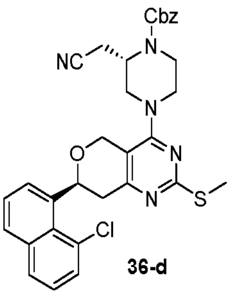
36-d

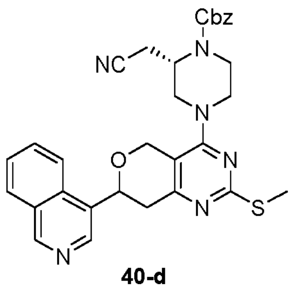
40-d

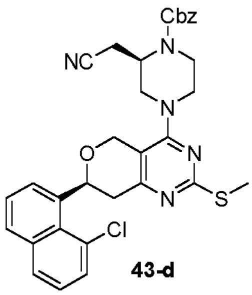
43-d

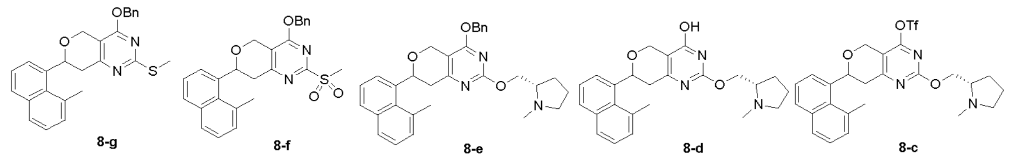
8-g 8-f 8-e 8-d 8-c

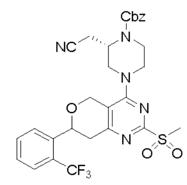
2-c

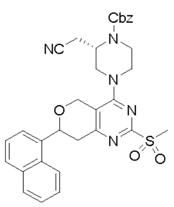
3-c

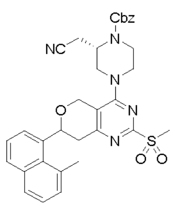
4-c

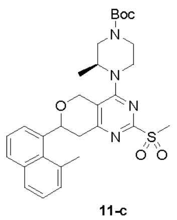
11-c

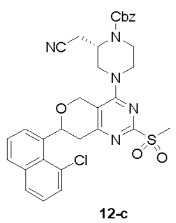
12-c

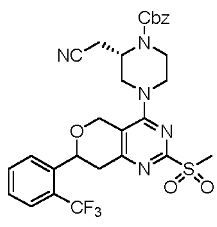
19-c

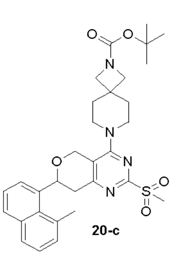
20-c

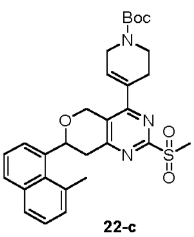
22-c

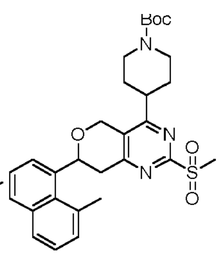
23-c

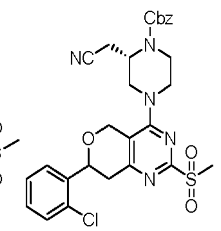
30-c

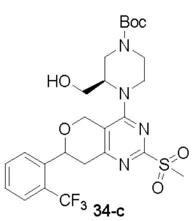
34-c

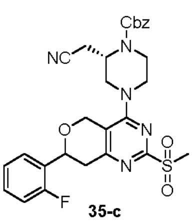
35-c

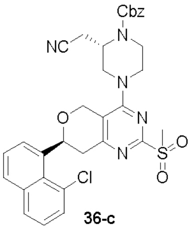
36-c

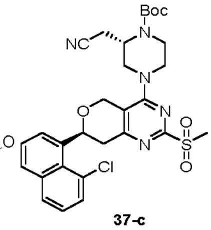
37-c

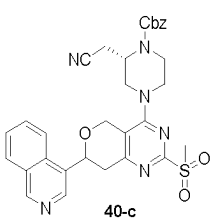
40-c

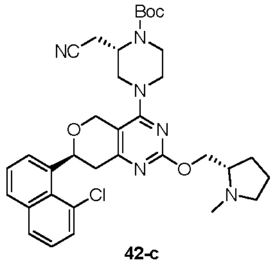
42-c

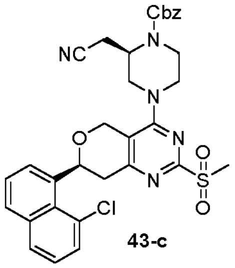
43-c

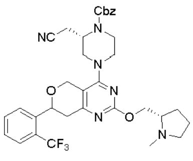
2-b

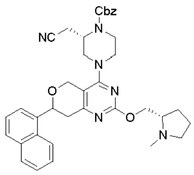
3-b

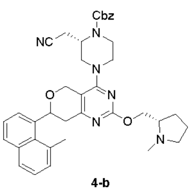
4-b

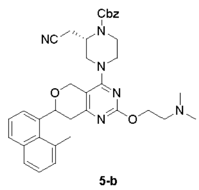
5-b

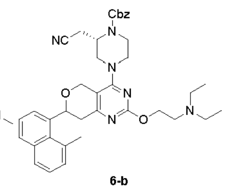
6-b

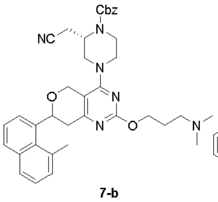
7-b

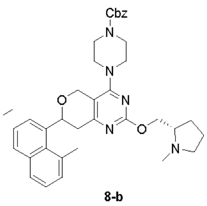
8-b

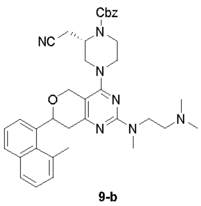
9-b

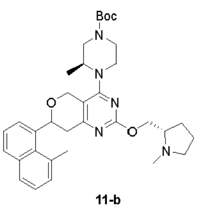
11-b

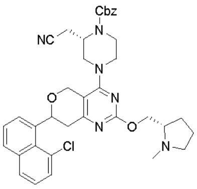
12-b

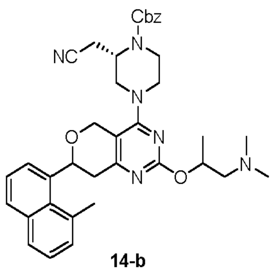
14-b

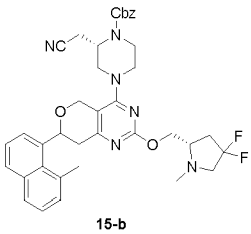
15-b

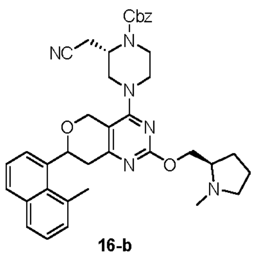
16-b

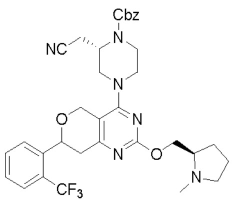
19-b

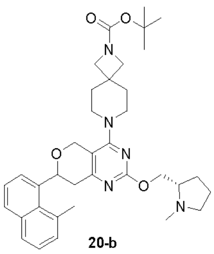
20-b

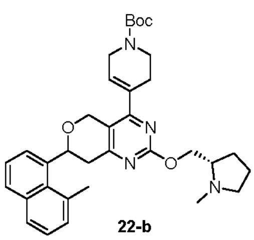
22-b

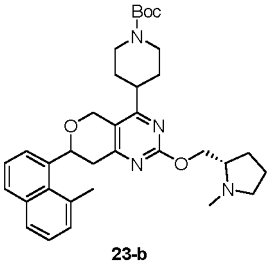
23-b

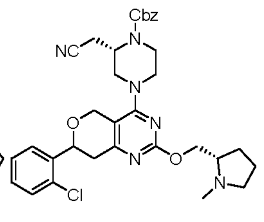
30-b

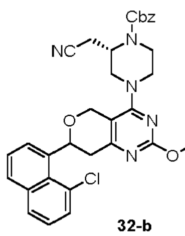
32-b
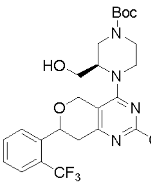
34-b
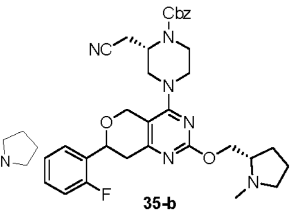
35-b
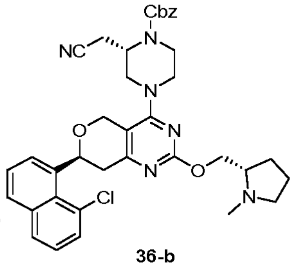
36-b

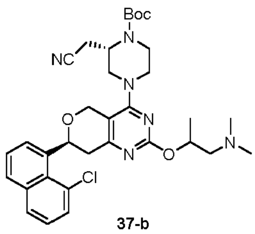
37-b
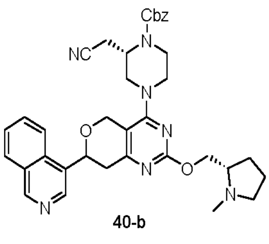
40-b
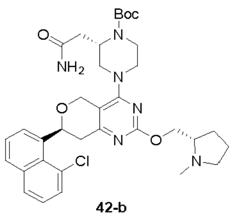
42-b
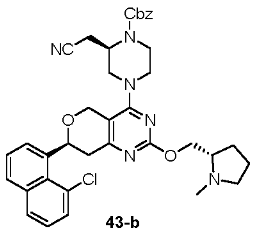
43-b

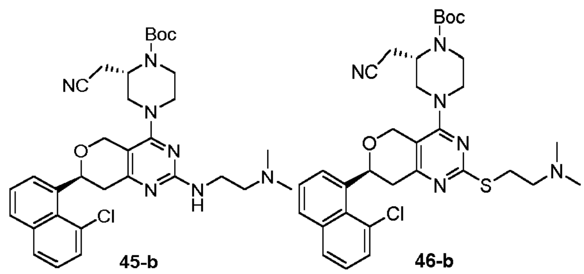
45-b
46-b

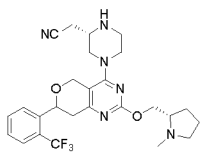
2-a
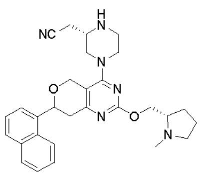
3-a
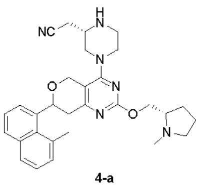
4-a
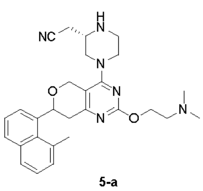
5-a
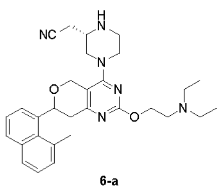
6-a

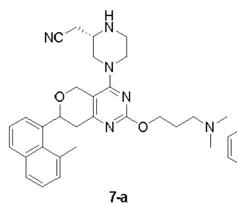
7-a
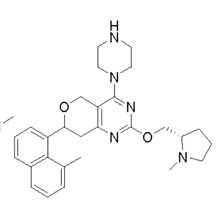
8-a
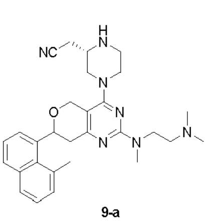
9-a
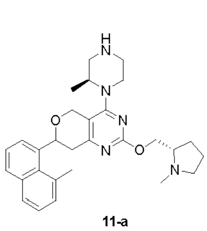
11-a
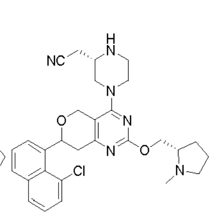
12-a

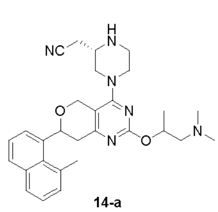
14-a
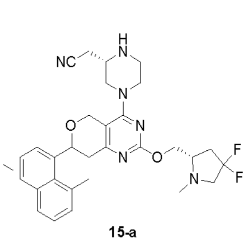
15-a
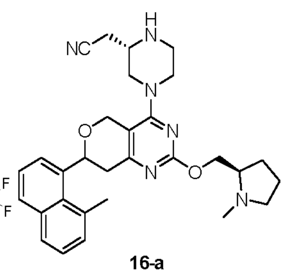
16-a
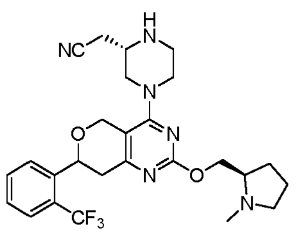
19-a

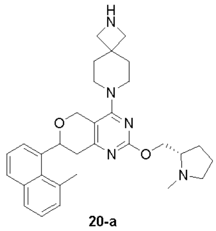
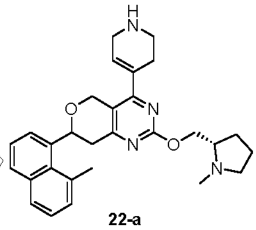
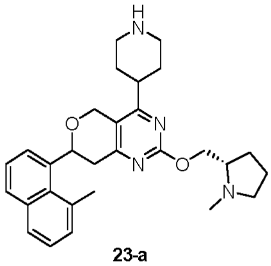
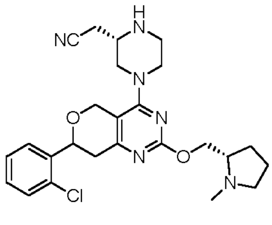

20-a 22-a 23-a 30-a

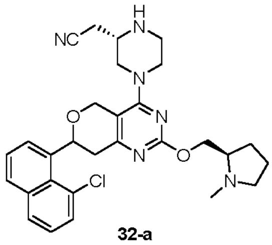
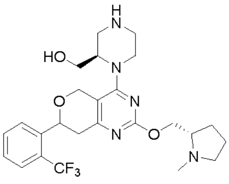
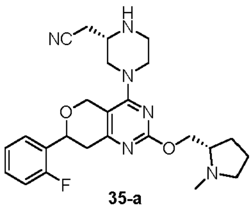
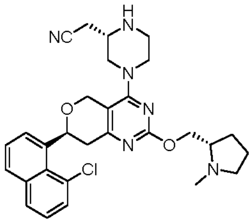

32-a 34-a 35-a 36-a

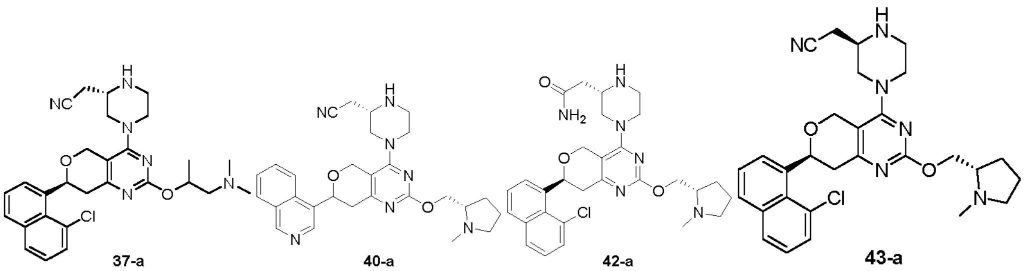

37-a 40-a 42-a 43-a

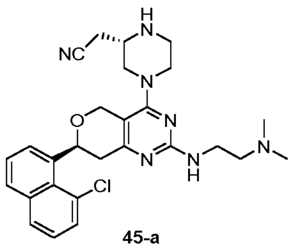
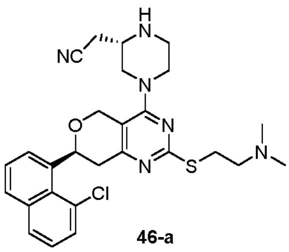

45-a 46-a

.

**18.** A pharmaceutical composition, comprising substance **A** and a pharmaceutical adjuvant; the substance **A** is the oxygen-containing heterocyclic compound represented by formula **I** as defined in any one of claims 1-15, a pharmaceutically acceptable salt thereof, a solvate thereof, a solvate of the pharmaceutically acceptable salt thereof, a crystal form thereof, a stereoisomer thereof, a tautomer thereof or an isotopic compound thereof.

**19.** A substance **A** for use as a medicament, wherein the substance A is the oxygen-containing heterocyclic compound represented by formula I as defined in any one of claims 1-15, a pharmaceutically acceptable salt thereof, a solvate thereof, a solvate of the pharmaceutically acceptable salt thereof, a crystal form thereof, a stereoisomer thereof, a tautomer thereof or an isotopic compound thereof.

**20.** A substance **A** for use as a medicament, wherein the substance A is the oxygen-containing heterocyclic compound represented by formula I as defined in any one of claims 1-15, a pharmaceutically acceptable salt thereof, a solvate thereof, a solvate of the pharmaceutically acceptable salt thereof, a crystal form thereof, a stereoisomer thereof, a tautomer thereof or an isotopic compound thereof;
in the treatment or prevention of cancer, wherein the cancer is one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

21. A substance **A** for use as an RAS inhibitor in *ex vivo*, wherein the substance A is the oxygen-containing heterocyclic compound represented by formula I as defined in any one of claims 1-15, a pharmaceutically acceptable salt thereof, a solvate thereof, a solvate of the pharmaceutically acceptable salt thereof, a crystal form thereof, a stereoisomer thereof, a tautomer thereof or an isotopic compound thereof.

## Patentansprüche

1. Sauerstoffhaltige heterocyclische Verbindung, die durch Formel I dargestellt ist, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei Folgendes gilt:

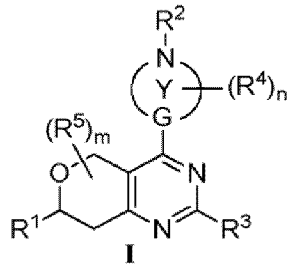

**I**

$R^1$ ist $C_{6-20}$-Aryl, "5- bis 12-gliedriges Heteroaryl, enthaltend 1-4 Heteroatome ausgewählt aus einem oder mehreren von 0, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von O, S und N" substituiert mit einem oder mehreren R1-7;

$R^{1-6}$ und $R^{1-7}$ sind unabhängig Halogen, Hydroxyl, - $C(=O)R^{65}$, $-NR^{63}R^{64}$, $-C(=O)OR^{66}$, $-C(=O)NR^{69}R^{610}$, $C_{1-6}$-Alkyl, $C_{1-6}$-Alkoxy, $C_{3-10}$-Cycloalkyl, "5- bis 7-gliedriges Heterocycloalkyl, enthaltend 1 oder 2 Heteroatome ausgewählt aus einem oder mehreren von 0 und N", $C_{6-20}$-Aryl, "5- bis 7-gliedriges Heteroaryl, enthaltend 1 oder 2 Heteroatome ausgewählt aus einem oder mehreren von 0 und N", $C_{1-6}$-Alkyl substituiert mit einem oder mehreren $R^{1-6-1}$, $C_{1-6}$-Alkoxy substituiert mit einem oder mehreren $R^{1-6-2}$, $C_{3-10}$-Cycloalkyl substituiert mit einem oder mehreren $R^{1-6-3}$, "5- bis 7-gliedriges Heterocycloalkyl, enthaltend 1 oder 2 Heteroatome ausgewählt aus einem oder mehreren von 0 und N" substituiert mit einem oder mehreren $R^{1-6-4}$, $C_{6-20}$-Aryl substituiert mit einem oder mehreren $R^{1-6-5}$ oder "5- bis 7-gliedriges Heteroaryl, enthaltend 1 oder 2 Heteroatome ausgewählt aus einem oder mehreren von 0 und N" substituiert mit einem oder mehreren $R^{1-6-6}$;

$R^{1-6-1}$, $R^{1-6-2}$, $R^{1-6-3}$, $R^{1-6-4}$, $R^{1-6-5}$ und $R^{1-6-6}$ sind unabhängig Cyano, Halogen, Hydroxyl, $C_{1-6}$-Alkoxy, $C_{1-6}$-Alkyl, $-C(=O)R^{65-2}$, $-NR^{63-2}R^{64-2}$, $-C(=O)OR^{66-2}$ oder $-C(=O)NR^{69-2}R^{610-2}$;

$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ und $R^{610-2}$ sind unabhängig Wasserstoff oder $C_{1-6}$-Alkyl;

m ist 0, 1 oder 2;

$R^5$ ist unabhängig $C_{1-6}$-Alkyl;

$R^3$ ist $-OR^{31}$, $-SR^{32}$ oder $-NR^{33}R^{34}$;

$R^{31}$, $R^{32}$ und $R^{34}$ sind unabhängig $C_{1-6}$-Alkyl substituiert mit einem oder mehreren $R^{31-1}$; $R^{33}$ ist unabhängig H, $C_{1-6}$-Alkyl oder $C_{1-6}$-Alkyl substituiert mit einem oder mehreren $R^{31-1}$;

$R^{31-1}$ ist unabhängig $C_{3-10}$-Cycloalkyl, "4- bis 10-gliedriges Heterocycloalkyl, enthaltend 1-3 Heteroatome ausgewählt aus einem oder mehreren von 0 und N", $C_{3-10}$-Cycloalkyl substituiert mit einem oder mehreren $R^{d16}$, "4- bis 10-gliedriges Heterocycloalkyl, enthaltend 1-3 Heteroatome ausgewählt aus einem oder mehreren von 0 und N" substituiert mit einem oder mehreren $R^{d15}$, $-OR^d$, $-SR^{d1}$, $-NR^{e1}R^{e2}$ oder - $C(=O)NR^{e3}R^{e4}$;

$R^{d15}$ und $R^{d16}$ sind unabhängig $C_{1-6}$-Alkyl, $C_{1-6}$-Alkyl substituiert mit einem oder mehreren $R^{1-8-1}$, Hydroxyl, $C_{1-6}$-Alkoxy, Halogen, $-NR^{e5}R^{e6}$ oder $-C(=O)NR^{e7}R^{e8}$;

$R^d$, $R^{d1}$, $R^{e1}$, $R^{e2}$, $R^{e3}$ und $R^{e4}$ sind unabhängig Wasserstoff, $C_{1-6}$-Alkyl, $C_{3-10}$-Cycloalkyl, "4- bis 10-gliedriges Heterocycloalkyl, enthaltend 1-3 Heteroatome ausgewählt aus einem oder mehreren von 0 und N" oder $C_{1-6}$-Alkyl substituiert mit einem oder mehreren $R^{1-8-2}$;

$R^{1-8-1}$ und $R^{1-8-2}$ sind unabhängig Cyano, Halogen, Hydroxyl, $C_{1-6}$-Alkoxy, $-C(=O)R^{e9}$, $-NR^{e10}R^{e11}$, $-C(=O)OR^{e12}$ oder - $C(=O)NR^{e13}R^{e14}$;

$R^{e5}$, $R^{e6}$, $R^{e7}$, $R^{e8}$, $R^{e9}$, $R^{e10}$, $R^{e11}$, $R^{e12}$, $R^{e13}$ und $R^{e14}$ sind unabhängig Wasserstoff oder $C_{1-6}$-Alkyl;

Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring

ist ein monocyclischer Ring, ein verbrückter Ring oder ein Spiroring;
G ist N, C oder CH;
n ist 0, 1, 2 oder 3;
$R^4$ ist unabhängig $C_{1-6}$-Alkyl, $C_{1-6}$-Alkyl substituiert mit einem oder mehreren $R^{4-1}$, Oxo, -C(=O)OR$^{4a}$ oder -C(=O)NR$^{4b}$R$^{4c}$;
$R^{4-1}$ ist unabhängig Halogen, Cyano, Hydroxyl, $C_{1-6}$-Alkoxy, -NR$^{4i}$R$^{4j}$, -C(=O)OR$^{4d}$ oder -C(=O)NR$^{4e}$R$^{4f}$; $R^{4d}$, $R^{4e}$, $R^{4f}$, $R^{4i}$ und $R^{4j}$ sind unabhängig Wasserstoff oder $C_{1-6}$-Alkyl;
$R^{4a}$, $R^{4b}$ und $R^{4c}$ sind unabhängig Wasserstoff oder $C_{1-6}$-Alkyl;
$R^2$ ist CN, -C(=O)-C(R$^a$)=C(R$^b$)(R$^f$), -C(=O)-C≡CR$^f$, -S(=O)$_2$-C(R$^a$)=C(R$^b$)(R$^f$) oder -S(=O)$_2$-C≡CR$^f$;
$R^a$ ist unabhängig Wasserstoff, Deuterium, Halogen oder $C_{1-6}$-Alkyl;
$R^b$ und $R^f$ sind unabhängig Wasserstoff, Deuterium, $C_{1-6}$-Alkyl, $C_{1-6}$-Alkyl-C(=O)- oder $C_{1-6}$-Alkyl substituiert mit einem oder mehreren $R^{b-1}$;
$R^{b-1}$ ist unabhängig Halogen, Hydroxyl, $C_{1-6}$-Alkoxy oder -NR$^{10j}$R$^{10k}$;
$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 10-gliedriges Heterocycloalkyl, enthaltend 1-3 Heteroatome ausgewählt aus einem oder mehreren von 0 und N".

**2.** Sauerstoffhaltige heterocyclische Verbindung, die durch Formel **I** dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei Folgendes gilt: $R^1$ ist $C_{6-20}$-Aryl, "5- bis 12-gliedriges Heteroaryl, enthaltend 1-4 Heteroatome ausgewählt aus einem oder mehreren von 0, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von 0, S und N" substituiert mit einem oder mehreren $R^{1-7}$; $R^{1-6}$ und $R^{1-7}$ sind unabhängig Halogen, Hydroxyl, $C_{1-6}$-Alkyl, $C_{1-6}$-Alkoxy, $C_{3-10}$-Cycloalkyl, $C_{1-6}$-Alkyl substituiert mit einem oder mehreren $R^{1-6-1}$ oder $C_{1-6}$-Alkoxy substituiert mit einem oder mehreren $R^{1-6-2}$; $R^{1-6-1}$ und $R^{1-6-2}$ sind unabhängig Halogen;

und/oder m ist 0;
und/oder $R^3$ ist -OR$^{31}$ oder -NR$^{33}$R$^{34}$; $R^{31}$, $R^{33}$ und $R^{34}$ sind unabhängig $C_{1-6}$-Alkyl substituiert mit einem oder mehreren $R^{31-1}$; $R^{31-1}$ ist unabhängig "5- bis 7-gliedriges Heterocycloalkyl, enthaltend 1-3 Heteroatome ausgewählt aus einem oder mehreren von 0 und N" substituiert mit einem oder mehreren $R^{d15}$ oder -NR$^{e1}$R$^{e2}$; $R^{d15}$ ist unabhängig $C_{1-6}$-Alkyl, $C_{1-6}$-Alkyl substituiert mit einem oder mehreren $R^{1-8-1}$, Hydroxyl, $C_{1-6}$-Alkoxy, Halogen, -NR$^{e5}$R$^{e6}$ oder -C(=O)NR$^{e7}$R$^{e8}$; $R^{1-8-1}$ ist unabhängig Halogen; $R^{e5}$, $R^{e6}$, $R^{e7}$ und $R^{e8}$ sind unabhängig Wasserstoff oder $C_{1-6}$-Alkyl; $R^{e1}$ und $R^{e2}$ sind unabhängig $C_{1-6}$-Alkyl;
und/oder Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring oder ein Spiroring; G ist N, C oder CH;
und/oder n ist 0 oder 1; $R^4$ ist unabhängig $C_{1-6}$-Alkyl oder $C_{1-6}$-Alkyl substituiert mit einem oder mehreren $R^{4-1}$; $R^{4-1}$ ist unabhängig Cyano;
und/oder $R^2$ ist -C(=O)-C(R$^a$)=C(R$^b$)(R$^f$), -C(=O)-C≡C-Me oder -S(=O)$_2$-C(R$^a$)=C(R$^b$)(R$^f$); $R^a$ ist unabhängig Wasserstoff oder Halogen; $R^b$ und $R^f$ sind unabhängig Wasserstoff, $C_{1-6}$-Alkyl oder $C_{1-6}$-Alkyl substituiert mit einem oder mehreren $R^{b-1}$; $R^{b-1}$ ist unabhängig Halogen, Hydroxyl, $C_{1-6}$-Alkoxy oder -NR$^{10j}$R$^{10k}$; $R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von 0 und N".

**3.** Sauerstoffhaltige heterocyclische Verbindung, die durch Formel I dargestellt ist, nach Anspruch 2, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei Folgendes gilt: $R^1$ ist $C_{6-20}$-Aryl oder $C_{6-20}$-Aryl substituiert mit einem oder mehreren $R^{1-6}$; $R^{1-6}$ ist unabhängig Halogen, $C_{1-6}$-Alkyl oder $C_{1-6}$-Alkyl substituiert mit einem oder mehreren $R^{1-6-1}$; $R^{1-6-1}$ ist unabhängig Halogen;

und/oder $R^3$ ist -OR$^{31}$ oder -NR$^{33}$R$^{34}$; $R^{31}$, $R^{33}$ und $R^{34}$ sind unabhängig $C_{1-6}$-Alkyl substituiert mit einem oder mehreren $R^{31-1}$; $R^{31-1}$ ist unabhängig "5- bis 7-gliedriges Heterocycloalkyl, enthaltend 1-3 Heteroatome ausgewählt aus einem oder mehreren von 0 und N" substituiert mit einem oder mehreren $R^{d15}$ oder -NR$^{e1}$R$^{e2}$; $R^{e1}$, $R^{e2}$ und $R^{d15}$ sind unabhängig $C_{1-6}$-Alkyl;
und/oder Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring; G ist N;
und/oder n ist 0 oder 1; $R^4$ ist unabhängig $C_{1-6}$-Alkyl oder $C_{1-6}$-Alkyl substituiert mit einem oder mehreren $R^{4-1}$; $R^{4-1}$ ist unabhängig Cyano;
und/oder $R^2$ ist -C(=O)-C($R^a$)=C($R^b$)($R^f$); $R^a$ ist unabhängig Wasserstoff oder Halogen; $R^b$ und $R^f$ sind unabhängig Wasserstoff oder $C_{1-6}$-Alkyl.

**4.** Sauerstoffhaltige heterocyclische Verbindung, die durch Formel I dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei Folgendes gilt:

$R^1$ ist $C_{6-20}$-Aryl, "5- bis 12-gliedriges Heteroaryl, enthaltend 1-4 Heteroatome ausgewählt aus einem oder mehreren von 0, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von 0, S und N" substituiert mit einem oder mehreren $R^{1-7}$; $R^{1-6}$ und $R^{1-7}$ sind unabhängig Halogen, $C_{1-6}$-Alkyl oder $C_{1-6}$-Alkyl substituiert mit einem oder mehreren $R^{1-6-1}$; $R^{1-6-1}$ ist unabhängig Halogen;
und/oder $R^3$ ist -O$R^{31}$, -S$R^{32}$ oder -N$R^{33}R^{34}$; $R^{31}$, $R^{32}$ und $R^{34}$ sind unabhängig $C_{1-6}$-Alkyl substituiert mit einem oder mehreren $R^{31-1}$; $R^{33}$ ist unabhängig H, $C_{1-6}$-Alkyl oder $C_{1-6}$-Alkyl substituiert mit einem oder mehreren $R^{31-1}$; $R^{31-1}$ ist unabhängig "5- bis 7-gliedriges Heterocycloalkyl, enthaltend 1-3 Heteroatome ausgewählt aus einem oder mehreren von 0 und N" substituiert mit einem oder mehreren $R^{d15}$ oder -N$R^{e1}R^{e2}$; $R^{d15}$ ist unabhängig $C_{1-6}$-Alkyl oder Halogen; $R^{e1}$ und $R^{e2}$ sind unabhängig $C_{1-6}$-Alkyl;
und/oder n ist 0 oder 1; $R^4$ ist unabhängig $C_{1-6}$-Alkyl oder $C_{1-6}$-Alkyl substituiert mit einem oder mehreren $R^{4-1}$; $R^{4-1}$ ist unabhängig Hydroxyl, Cyano oder -C(=O)N$R^{4e}R^{4f}$; $R^{4e}$ und $R^{4f}$ sind unabhängig Wasserstoff oder $C_{1-6}$-Alkyl;
und/oder $R^2$ ist CN, -C(=O)-C($R^a$)=C($R^b$)($R^f$), -C(=O)-C = C-Me oder -S(=O)$_2$-C($R^a$)=C ($R^b$) ($R^f$); $R^a$ ist unabhängig Wasserstoff oder Halogen; $R^b$ und $R^f$ sind unabhängig Wasserstoff, $C_{1-6}$-Alkyl, $C_{1-6}$-Alkyl-C(=O) - oder $C_{1-6}$-Alkyl substituiert mit einem oder mehreren $R^{b-1}$; $R^{b-1}$ ist unabhängig -N$R^{10j}R^{10k}$; $R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von O und N".

**5.** Sauerstoffhaltige heterocyclische Verbindung, die durch Formel I dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei Folgendes gilt:

$R^3$ ist -O$R^{31}$ oder -N$R^{33}R^{34}$; $R^{31}$, $R^{33}$ und $R^{34}$ sind unabhängig $C_{1-6}$-Alkyl substituiert mit einem oder mehreren $R^{31-1}$; $R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder -N$R^{e1}R^{e2}$; $R^{d15}$ ist unabhängig $C_{1-6}$-Alkyl oder Halogen; $R^{e1}$ und $R^{e2}$ sind unabhängig $C_{1-6}$-Alkyl;
und/oder n ist 0 oder 1; $R^4$ ist unabhängig $C_{1-6}$-Alkyl oder $C_{1-6}$-Alkyl substituiert mit einem oder mehreren $R^{4-1}$; $R^{4-1}$ ist unabhängig Hydroxyl oder Cyano;
und/oder $R^2$ ist -C(=O)-C($R^a$)=C($R^b$)($R^f$), -C(=O)-C≡C-Me oder -S(=O)$_2$-C($R^a$)=C($R^b$)($R^f$); $R^a$ ist unabhängig Wasserstoff oder Halogen; $R^b$ und $R^f$ sind unabhängig Wasserstoff, $C_{1-6}$-Alkyl oder $C_{1-6}$-Alkyl substituiert mit einem oder mehreren $R^{b-1}$; $R^{b-1}$ ist unabhängig -N$R^{10j}R^{10k}$; $R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von O und N".

**6.** Sauerstoffhaltige heterocyclische Verbindung, die durch Formel **I** dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei die sauerstoffhaltige heterocyclische Verbindung, die durch Formel **I** dargestellt ist, als Lösung 1, Lösung 2, Lösung 3, Lösung 4, Lösung 5, Lösung 6 oder Lösung 7 definiert ist:

Lösung 1:

$R^1$ ist $C_{6-20}$-Aryl, "5- bis 12-gliedriges Heteroaryl, enthaltend 1-4 Heteroatome ausgewählt aus einem oder mehreren von O, S und N", $C_{6-20}$-Aryl substituiert mit einem oder mehreren $R^{1-6}$ oder "5- bis 12-gliedriges

Heteroaryl, enthaltend 1-4 Heteroatome ausgewählt aus einem oder mehreren von O, S und N" substituiert mit einem oder mehreren $R^{1-7}$;

$R^{1-6}$ und $R^{1-7}$ sind unabhängig Halogen, Hydroxyl, $C_{1-6}$-Alkyl, $C_{1-6}$-Alkoxy, $C_{3-10}$-Cycloalkyl, $C_{1-6}$-Alkyl substituiert mit einem oder mehreren $R^{1-6-1}$ oder $C_{1-6}$-Alkoxy substituiert mit einem oder mehreren $R^{1-6-2}$;

$R^{1-6-1}$ und $R^{1-6-2}$ sind unabhängig Halogen;

m ist 0;

$R^3$ ist -$OR^{31}$ oder -$NR^{33}R^{34}$;

$R^{31}$, $R^{33}$ und $R^{34}$ sind unabhängig $C_{1-6}$-Alkyl substituiert mit einem oder mehreren $R^{31-1}$;

$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder -$NR^{e1}R^{e2}$;

$R^{d15}$ ist unabhängig $C_{1-6}$-Alkyl, $C_{1-6}$-Alkyl substituiert mit einem oder mehreren $R^{1-8-1}$, Hydroxyl, $C_{1-6}$-Alkoxy, Halogen, -$NR^{e5}R^{e6}$ oder -C(=O)$NR^{e7}R^{e8}$;

$R^{1-8-1}$ ist unabhängig Halogen; $R^{e5}$, $R^{e6}$, $R^{e7}$ und $R^{e8}$ sind unabhängig Wasserstoff oder $C_{1-6}$-Alkyl;

$R^{e1}$ und $R^{e2}$ sind unabhängig $C_{1-6}$-Alkyl;

Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring oder ein Spiroring;

G ist N, C oder CH;

n ist 0 oder 1;

$R^4$ ist unabhängig $C_{1-6}$-Alkyl oder $C_{1-6}$-Alkyl substituiert mit einem oder mehreren $R^{4-1}$;

$R^{4-1}$ ist unabhängig Cyano;

$R^2$ ist -C (=O) -C ($R^a$) =C ($R^b$) ($R^f$), -C (=O) -C=C-Me oder - $S(=O)_2$-C($R^a$)=C($R^b$)($R^f$);

$R^a$ ist unabhängig Wasserstoff oder Halogen;

$R^b$ und $R^f$ sind unabhängig Wasserstoff, $C_{1-6}$-Alkyl oder $C_{1-6}$-Alkyl substituiert mit einem oder mehreren $R^{b-1}$;

$R^{b-1}$ ist unabhängig Halogen, Hydroxyl, $C_{1-6}$-Alkoxy oder -$NR^{10j}R^{10k}$;

$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von O und N";

Lösung 2:

$R^1$ ist $C_{6-20}$-Aryl oder $C_{6-20}$-Aryl substituiert mit einem oder mehreren $R^{1-6}$;

$R^{1-6}$ ist unabhängig Halogen, $C_{1-6}$-Alkyl oder $C_{1-6}$-Alkyl substituiert mit einem oder mehreren $R^{1-6-1}$;

$R^{1-6-1}$ ist unabhängig Halogen;

m ist 0;

$R^3$ ist -$OR^{31}$ oder -$NR^{33}R^{34}$;

$R^{31}$, $R^{33}$ und $R^{34}$ sind unabhängig $C_{1-6}$-Alkyl substituiert mit einem oder mehreren $R^{31-1}$;

$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder -$NR^{e1}R^{e2}$;

$R^{e1}$, $R^{e2}$ und $R^{d15}$ sind unabhängig $C_{1-6}$-Alkyl;

Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring;

G ist N;

n ist 0 oder 1;

$R^4$ ist unabhängig $C_{1-6}$-Alkyl oder $C_{1-6}$-Alkyl substituiert mit einem oder mehreren $R^{4-1}$;

$R^{4-1}$ ist unabhängig Cyano;

$R^2$ ist -C (=O) -C ($R^a$) =C ($R^b$) ($R^f$) ;

$R^a$ ist unabhängig Wasserstoff oder Halogen;

$R^b$ und $R^f$ sind unabhängig Wasserstoff oder $C_{1-6}$-Alkyl;

Ausführungsform 3:

$R^1$ ist $C_{6-20}$-Aryl, "5- bis 12-gliedriges Heteroaryl, enthaltend 1-4 Heteroatome ausgewählt aus einem oder mehreren von O, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von O, S und N" substituiert mit einem oder mehreren R1-7;

$R^{1-6}$ und $R^{1-7}$ sind unabhängig Halogen, Hydroxyl, $C_{1-6}$-Alkyl, $C_{1-6}$-Alkoxy, $C_{3-10}$-Cycloalkyl, $C_{1-6}$-Alkyl

substituiert mit einem oder mehreren $R^{1-6-1}$ oder $C_{1-6}$-Alkoxy substituiert mit einem oder mehreren $R^{1-6-2}$;
$R^{1-6-1}$ und $R^{1-6-2}$ sind unabhängig Halogen;
m ist 0;
$R^3$ ist $-OR^{31}$ oder $-NR^{33}R^{34}$;
$R^{31}$, $R^{33}$ und $R^{34}$ sind unabhängig $C_{1-6}$-Alkyl substituiert mit einem oder mehreren $R^{31-1}$;
$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder $-NR^{e1}R^{e2}$;
$R^{d15}$ ist unabhängig $C_{1-6}$-Alkyl, $C_{1-6}$-Alkyl substituiert mit einem oder mehreren $R^{1-8-1}$, Hydroxyl, $C_{1-6}$-Alkoxy, Halogen, $-NR^{e5}R^{e6}$ oder $-C(=O)NR^{e7}R^{e8}$;
$R^{1-8-1}$ ist unabhängig Halogen; $R^{e5}$, $R^{e6}$, $R^{e7}$ und $R^{e8}$ sind unabhängig Wasserstoff oder $C_{1-6}$-Alkyl;
$R^{e1}$ und $R^{e2}$ sind unabhängig $C_{1-6}$-Alkyl;
Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring oder ein Spiroring;
G ist N, C oder CH;
n ist 0 oder 1;
$R^4$ ist unabhängig $C_{1-6}$-Alkyl oder $C_{1-6}$-Alkyl substituiert mit einem oder mehreren $R^{4-1}$;
$R^{4-1}$ ist unabhängig Cyano oder Hydroxyl;
$R^2$ ist $-C (=O) -C (R^a) =C (R^b) (R^f)$, $-C (=O) -C{=}C-Me$ oder $- S(=O)_2-C(R^a)=C(R^b)(R^f)$;
$R^a$ ist unabhängig Wasserstoff oder Halogen;
$R^b$ und $R^f$ sind unabhängig Wasserstoff, $C_{1-6}$-Alkyl oder $C_{1-6}$-Alkyl substituiert mit einem oder mehreren $R^{b-1}$;
$R^{b-1}$ ist unabhängig Halogen, Hydroxyl, $C_{1-6}$-Alkoxy oder $-NR^{10j}R^{10k}$;
$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von O und N";

Lösung 4:

$R^1$ ist $C_{6-20}$-Aryl oder $C_{6-20}$-Aryl substituiert mit einem oder mehreren $R^{1-6}$;
$R^{1-6}$ ist unabhängig Halogen, $C_{1-6}$-Alkyl oder $C_{1-6}$-Alkyl substituiert mit einem oder mehreren $R^{1-6-1}$;
$R^{1-6-1}$ ist unabhängig Halogen;
m ist 0;
$R^3$ ist $-OR^{31}$ oder $-NR^{33}R^{34}$;
$R^{31}$, $R^{33}$ und $R^{34}$ sind unabhängig $C_{1-6}$-Alkyl substituiert mit einem oder mehreren $R^{31-1}$;
$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder $-NR^{e1}R^{e2}$;
$R^{d15}$ ist unabhängig $C_{1-6}$-Alkyl oder Halogen;
$R^{e1}$ und $R^{e2}$ sind unabhängig $C_{1-6}$-Alkyl;
Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring;
G ist N;
n ist 0 oder 1;
$R^4$ ist unabhängig $C_{1-6}$-Alkyl oder $C_{1-6}$-Alkyl substituiert mit einem oder mehreren $R^{4-1}$;
$R^{4-1}$ ist unabhängig Cyano;
$R^2$ ist $-C(=O)-C(R^a)=C(R^b)(R^f)$;
$R^a$ ist unabhängig Wasserstoff oder Halogen;
$R^b$ und $R^f$ sind unabhängig Wasserstoff oder $C_{1-6}$-Alkyl oder $C_{1-6}$-Alkyl substituiert mit einem oder mehreren $R^{b-1}$;
$R^{b-1}$ ist unabhängig $-NR^{10j}R^{10k}$;
$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 10-gliedriges Heterocycloalkyl, enthaltend 1-3 Heteroatome ausgewählt aus einem oder mehreren von O und N";

Lösung 5:

$R^1$ ist $C_{6-20}$-Aryl, "5- bis 12-gliedriges Heteroaryl, enthaltend 1-4 Heteroatome ausgewählt aus einem oder mehreren von O, S und N", $C_{6-20}$-Aryl substituiert mit einem oder mehreren $R^{1-6}$ oder "5- bis 12-gliedriges

Heteroaryl, enthaltend 1-4 Heteroatome ausgewählt aus einem oder mehreren von O, S und N" substituiert mit einem oder mehreren $R^{1-7}$;

$R^{1-6}$ und $R^{1-7}$ sind unabhängig Halogen, Hydroxyl, - $C(=O)R^{65}$, $-NR^{63}R^{64}$, $-C(=O)OR^{66}$, $-C(=O)NR^{69}R^{610}$, $C_{1-6}$-Alkyl, $C_{1-6}$-Alkoxy, $C_{3-10}$-Cycloalkyl, "5- bis 7-gliedriges Heterocycloalkyl, enthaltend 1 oder 2 Heteroatome ausgewählt aus einem oder mehreren von O und N", $C_{6-20}$-Aryl, "5- bis 7-gliedriges Heteroaryl, enthaltend 1 oder 2 Heteroatome ausgewählt aus einem oder mehreren von O und N", $C_{1-6}$-Alkyl substituiert mit einem oder mehreren $R^{1-6-1}$, $C_{1-6}$-Alkoxy substituiert mit einem oder mehreren $R^{1-6-2}$, $C_{3-10}$-Cycloalkyl substituiert mit einem oder mehreren $R^{1-6-3}$, "5- bis 7-gliedriges Heterocycloalkyl, enthaltend 1 oder 2 Heteroatome ausgewählt aus einem oder mehreren von O und N" substituiert mit einem oder mehreren $R^{1-6-4}$, $C_{6-20}$-Aryl substituiert mit einem oder mehreren $R^{1-6-5}$ oder "5- bis 7-gliedriges Heteroaryl, enthaltend 1 oder 2 Heteroatome ausgewählt aus einem oder mehreren von O und N" substituiert mit einem oder mehreren $R^{1-6-6}$;

$R^{1-6-1}$, $R^{1-6-2}$, $R^{1-6-3}$, $R^{1-6-4}$, $R^{1-6-5}$ und $R^{1-6-6}$ sind unabhängig Cyano, Halogen, Hydroxyl, $C_{1-6}$-Alkoxy, $C_{1-6}$-Alkyl, $-C(=O)R^{65-2}$, $-NR^{63-2}R^{64-2}$, $-C(=O)OR^{66-2}$ oder $-C(=O)NR^{69-2}R^{610-2}$;

$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ und $R^{610-2}$ sind unabhängig Wasserstoff oder $C_{1-6}$-Alkyl;

m ist 0, 1 oder 2;

$R^5$ ist unabhängig $C_{1-6}$-Alkyl;

$R^3$ ist $-OR^{31}$, $-SR^{32}$ oder $-NR^{33}R^{34}$;

$R^{31}$, $R^{32}$, $R^{33}$ und $R^{34}$ sind unabhängig $C_{1-6}$-Alkyl substituiert mit einem oder mehreren $R^{31-1}$;

$R^{31-1}$ ist unabhängig $C_{3-10}$-Cycloalkyl, "4- bis 10-gliedriges Heterocycloalkyl, enthaltend 1-3 Heteroatome ausgewählt aus einem oder mehreren von O und N", $C_{3-10}$-Cycloalkyl substituiert mit einem oder mehreren $R^{d16}$, "4- bis 10-gliedriges Heterocycloalkyl, enthaltend 1-3 Heteroatome ausgewählt aus einem oder mehreren von O und N" substituiert mit einem oder mehreren $R^{d15}$, $-OR^d$, $-SR^{d1}$, $-NR^{e1}R^{e2}$ oder - $C(=O)NR^{e3}R^{e4}$;

$R^{d15}$ und $R^{d16}$ sind unabhängig $C_{1-6}$-Alkyl, $C_{1-6}$-Alkyl substituiert mit einem oder mehreren $R^{1-8-1}$, Hydroxyl, $C_{1-6}$-Alkoxy, Halogen, $-NR^{e5}R^{e6}$ oder $-C(=O)NR^{e7}R^{e8}$;

$R^d$, $R^{d1}$, $R^{e1}$, $R^{e2}$, $R^{e3}$ und $R^{e4}$ sind unabhängig Wasserstoff, $C_{1-6}$-Alkyl, $C_{3-10}$-Cycloalkyl, "4- bis 10-gliedriges Heterocycloalkyl, enthaltend 1-3 Heteroatome ausgewählt aus einem oder mehreren von O und N" oder $C_{1-6}$-Alkyl substituiert mit einem oder mehreren $R^{1-8-2}$;

$R^{1-8-1}$ und $R^{1-8-2}$ sind unabhängig Cyano, Halogen, Hydroxyl, $C_{1-6}$-Alkoxy, $-C(=O)R^{e9}$, $-NR^{e10}R^{e11}$, $-C(=O)OR^{e12}$ oder - $C(=O)NR^{e13}R^{e14}$;

$R^{e5}$, $R^{e6}$, $R^{e7}$, $R^{e8}$, $R^{e9}$, $R^{e10}$, $R^{e11}$, $R^{e12}$, $R^{e13}$ und $R^{e14}$ sind unabhängig Wasserstoff oder $C_{1-6}$-Alkyl;

Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring, ein verbrückter Ring oder ein Spiroring;

G ist N, C oder CH;

n ist 0, 1, 2 oder 3;

$R^4$ ist unabhängig $C_{1-6}$-Alkyl, $C_{1-6}$-Alkyl substituiert mit einem oder mehreren $R^{4-1}$, Oxo, $-C(=O)OR^{4a}$ oder $-C(=O)NR^{4b}R^{4c}$;

$R^{4-1}$ ist unabhängig Halogen, Cyano, Hydroxyl, $C_{1-6}$-Alkoxy, $-NR^{4i}R^{4j}$, $-C(=O)OR^{4d}$ oder $-C(=O)NR^{4e}R^{4f}$;

$R^{4d}$, $R^{4e}$, $R^{4f}$, $R^{4i}$ und $R^{4j}$ sind unabhängig Wasserstoff oder $C_{1-6}$-Alkyl;

$R^{4a}$, $R^{4b}$ und $R^{4c}$ sind unabhängig Wasserstoff oder $C_{1-6}$-Alkyl;

$R^2$ ist $-C(=O)-C(R^a)=C(R^b)(R^f)$, $-C(=O)-C\equiv CR^f$, $-S(=O)_2-C(R^a)=C(R^b)(R^f)$ oder $-S(=O)_2-C\equiv CR^f$;

$R^a$ ist unabhängig Wasserstoff, Deuterium, Halogen oder $C_{1-6}$-Alkyl;

$R^b$ und $R^f$ sind unabhängig Wasserstoff, Deuterium, $C_{1-6}$-Alkyl oder $C_{1-6}$-Alkyl substituiert mit einem oder mehreren $R^{b-1}$;

$R^{b-1}$ ist unabhängig Halogen, Hydroxyl, $C_{1-6}$-Alkoxy oder $-NR^{10j}R^{10k}$;

$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 10-gliedriges Heterocycloalkyl, enthaltend 1-3 Heteroatome ausgewählt aus einem oder mehreren von O und N";

Lösung 6:

$R^1$ ist $C_{6-20}$-Aryl, "5- bis 12-gliedriges Heteroaryl, enthaltend 1-4 Heteroatome ausgewählt aus einem oder mehreren von O, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von O, S und N" substituiert mit einem oder mehreren $R^{1-7}$;

$R^{1-6}$ und $R^{1-7}$ sind unabhängig Halogen, Hydroxyl, $C_{1-6}$-Alkyl, $C_{1-6}$-Alkoxy, $C_{3-10}$-Cycloalkyl, $C_{1-6}$-Alkyl substituiert mit einem oder mehreren $R^{1-6-1}$ oder $C_{1-6}$-Alkoxy substituiert mit einem oder mehreren $R^{1-6-2}$;
$R^{1-6-1}$ und $R^{1-6-2}$ sind unabhängig Halogen;
m ist 0;
$R^3$ ist $-OR^{31}$, $-SR^{32}$ oder $-NR^{33}R^{34}$;
$R^{31}$, $R^{32}$ und $R^{34}$ sind unabhängig $C_{1-6}$-Alkyl substituiert mit einem oder mehreren $R^{31-1}$; $R^{33}$ ist unabhängig H, $C_{1-6}$-Alkyl oder $C_{1-6}$-Alkyl substituiert mit einem oder mehreren $R^{31-1}$;
$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder $-NR^{e1}R^{e2}$;
$R^{d15}$ ist unabhängig $C_{1-6}$-Alkyl oder Halogen;
$R^{e1}$ und $R^{e2}$ sind unabhängig $C_{1-6}$-Alkyl;
Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring oder ein Spiroring;
G ist N, C oder CH;
n ist 0 oder 1;
$R^4$ ist unabhängig $C_{1-6}$-Alkyl oder $C_{1-6}$-Alkyl substituiert mit einem oder mehreren $R^{4-1}$;
$R^{4-1}$ ist unabhängig Cyano, Hydroxyl oder $-C(=O)NR^{4e}R^{4f}$; $R^{4e}$ und $R^{4f}$ sind unabhängig Wasserstoff oder $C_{1-6}$-Alkyl;
$R^2$ ist CN, $-C(=O)-C(R^a)=C(R^b)(R^f)$, $-C( = O)-C = C-Me$ oder $-S(=O)_2-C(R^a)=C(R^b)(R^f)$ ;
$R^a$ ist unabhängig Wasserstoff oder Halogen;
$R^b$ und $R^f$ sind unabhängig Wasserstoff, $C_{1-6}$-Alkyl, $C_{1-6}$-Alkyl-C (=O) - oder $C_{1-6}$-Alkyl substituiert mit einem oder mehreren $R^{b-1}$;
$R^{b-1}$ ist unabhängig Halogen, Hydroxyl, $C_{1-6}$-Alkoxy oder $-NR^{10j}R^{10k}$;
$R^{10j}$ und $R^{10k}$ sind unabhängig Wasserstoff oder $C_{1-6}$-Alkyl oder $R^{10j}$ und $R^{10k}$ bilden zusammen mit dem N-Atom, an das sie gebunden sind, "4- bis 6-gliedriges Heterocycloalkyl, enthaltend 1-3 Heteroatome ausgewählt aus einem oder mehreren von O und N";

Lösung 7:

$R^1$ ist $C_{6-20}$-Aryl, "5- bis 12-gliedriges Heteroaryl, enthaltend 1-4 Heteroatome ausgewählt aus einem oder mehreren von O, S und N", $C_{6-20}$-Aryl substituiert mit einem oder mehreren $R^{1-6}$ oder "5- bis 12-gliedriges Heteroaryl, enthaltend 1-4 Heteroatome ausgewählt aus einem oder mehreren von O, S und N" substituiert mit einem oder mehreren $R^{1-7}$;
$R^{1-6}$ ist unabhängig Halogen, $C_{1-6}$-Alkyl oder $C_{1-6}$-Alkyl substituiert mit einem oder mehreren $R^{1-6-1}$;
$R^{1-6-1}$ ist unabhängig Halogen;
m ist 0;
$R^3$ ist $-OR^{31}$, $-SR^{32}$ oder $-NR^{33}R^{34}$;
$R^{31}$, $R^{32}$ und $R^{34}$ sind unabhängig $C_{1-6}$-Alkyl substituiert mit einem oder mehreren $R^{31-1}$; $R^{33}$ ist unabhängig H, $C_{1-6}$-Alkyl oder $C_{1-6}$-Alkyl substituiert mit einem oder mehreren $R^{31-1}$;
$R^{31-1}$ ist unabhängig "5- bis 7-gliedriges Heterocycloalkyl, enthaltend 1-3 Heteroatome ausgewählt aus einem oder mehreren von O und N" substituiert mit einem oder mehreren $R^{d15}$ oder $-NR^{e1}R^{e2}$;
$R^{d15}$ ist unabhängig $C_{1-6}$-Alkyl oder Halogen;
$R^{e1}$ und $R^{e2}$ sind unabhängig $C_{1-6}$-Alkyl;
Ring Y ist ein 4- bis 12-gliedriger heterocyclischer Ring, der 1-4 N-Atome enthält; der heterocyclische Ring ist ein gesättigter heterocyclischer Ring oder ein teilweise gesättigter heterocyclischer Ring; der heterocyclische Ring ist ein monocyclischer Ring;
G ist N;
n ist 0 oder 1;
$R^4$ ist unabhängig $C_{1-6}$-Alkyl substituiert mit einem oder mehreren $R^{4-1}$;
$R^{4-1}$ ist unabhängig Cyano oder $-C(=O)NR^{4e}R^{4f}$; $R^{4e}$ und $R^{4f}$ sind unabhängig Wasserstoff oder $C_{1-6}$-Alkyl;
$R^2$ ist CN, $-C(=O)-C(R^a)=C(R^b)(R^f)$ ;
$R^a$ ist unabhängig Wasserstoff oder Halogen;
$R^b$ und $R^f$ sind unabhängig Wasserstoff, $C_{1-6}$-Alkyl oder $C_{1-6}$-Alkyl-C(=O)-.

**7.** Sauerstoffhaltige heterocyclische Verbindung, die durch Formel I dargestellt ist, nach einem der Ansprüche 1-6, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei die sauerstoffhaltige heterocyclische Verbindung, die durch Formel I dargestellt ist, eine Struktur wie folgt:

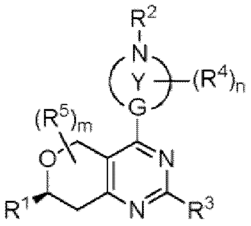
I-2

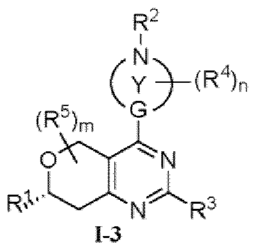
I-3

,

oder "ein Gemisch aus

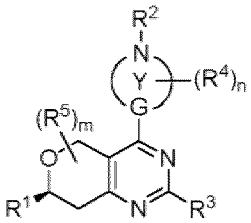
I-2

und

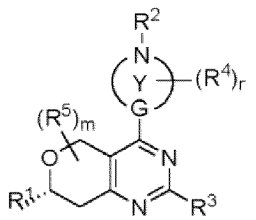
I-3

mit einem Molverhältnis von zum Beispiel 1 : 1" aufweist;

und/oder, wenn $R^1$ $C_{6-20}$-Aryl ist, dann das $C_{6-20}$-Aryl Phenyl oder Naphthyl ist;
und/oder, wenn $R^1$ "5- bis 12-gliedriges Heteroaryl, enthaltend 1-4 Heteroatome ausgewählt aus einem oder mehreren von O, S und N" ist, dann das "5- bis 12-gliedrige Heteroaryl, enthaltend 1-4 Heteroatome ausgewählt aus einem oder mehreren von O, S und N" "9- bis 10-gliedriges Heteroaryl, enthaltend 1-4 Heteroatome ausgewählt aus einem oder mehreren von O, S und N" ist;
und/oder, wenn $R^1$ $C_{6-20}$-Aryl substituiert mit einem oder mehreren $R^{1-6}$ ist, dann das $C_{6-20}$-Aryl Phenyl oder Naphthyl ist;
und/oder, wenn $R^1$ $C_{6-20}$-Aryl substituiert mit einem oder mehreren $R^{1-6}$ ist, dann die mehreren $R^{1-6}$ zwei oder drei $R^{1-6}$ sind;
und/oder, wenn $R^{1-6}$ unabhängig Halogen ist, dann das Halogen Fluor, Chlor, Brom oder Iod ist;
und/oder, wenn $R^{1-6}$ unabhängig $C_{1-6}$-Alkyl ist, dann das $C_{1-6}$-Alkyl $C_{1-4}$-Alkyl ist;
und/oder, wenn $R^{1-6}$ unabhängig $C_{1-6}$-Alkyl substituiert mit einem oder mehreren $R^{1-6-1}$ ist, dann das $C_{1-6}$-Alkyl $C_{1-4}$-Alkyl ist;
und/oder, wenn $R^{1-6}$ unabhängig $C_{1-6}$-Alkyl substituiert mit einem oder mehreren $R^{1-6-1}$ ist, dann die mehreren $R^{1-6-1}$ zwei oder drei $R^{1-6-1}$ sind;
und/oder, wenn $R^{1-6-1}$ unabhängig Halogen ist, dann das Halogen Fluor, Chlor, Brom oder Iod ist;
und/oder, wenn $R^{31}$, $R^{33}$ und $R^{34}$ unabhängig $C_{1-6}$-Alkyl substituiert mit einem oder mehreren $R^{31-1}$ sind, dann das $C_{1-6}$-Alkyl $C_{1-4}$-Alkyl ist;
und/oder, wenn $R^{31}$, $R^{33}$ und $R^{34}$ unabhängig $C_{1-6}$-Alkyl substituiert mit einem oder mehreren $R^{31-1}$ sind, dann die mehreren $R^{31-1}$ zwei oder drei $R^{31-1}$ sind;
und/oder, wenn $R^{31-1}$ unabhängig "4- bis 10-gliedriges Heterocycloalkyl, enthaltend 1-3 Heteroatome ausgewählt aus einem oder mehreren von O und N" substituiert mit einem oder mehreren $R^{d15}$ ist, dann das "4- bis 10-gliedrige Heterocycloalkyl, enthaltend 1-3 Heteroatome ausgewählt aus einem oder mehreren von O und N" "5- bis 7-gliedriges Heterocycloalkyl, enthaltend 1-3 Heteroatome ausgewählt aus einem oder mehreren von O und N" ist;
und/oder, wenn $R^{31-1}$ unabhängig "4- bis 10-gliedriges Heterocycloalkyl, enthaltend 1-3 Heteroatome ausgewählt aus einem oder mehreren von O und N" substituiert mit einem oder mehreren $R^{d15}$ ist, dann die mehreren $R^{d15}$ zwei oder drei $R^{d15}$ sind;
und/oder, wenn $R^{d15}$ unabhängig $C_{1-6}$-Alkyl ist, dann das $C_{1-6}$-Alkyl $C_{1-4}$-Alkyl ist;
und/oder, wenn $R^{d15}$ unabhängig Halogen ist, dann das Halogen Fluor, Chlor, Brom oder Iod ist;
und/oder, wenn $R^{e1}$ und $R^{e2}$ unabhängig $C_{1-6}$-Alkyl sind, dann das $C_{1-6}$-Alkyl $C_{1-4}$-Alkyl ist;

und/oder, wenn Ring Y ein 4- bis 12-gliedriger heterocyclischer Ring ist, der 1-4 N-Atome enthält, dann der 4- bis 12-gliedrige heterocyclische Ring, der 1-4 N-Atome enthält, ein 6- bis 9- gliedriger heterocyclischer Ring ist, der 1-2 N-Atome enthält;
und/oder, wenn $R^4$ unabhängig $C_{1-6}$-Alkyl ist, dann das $C_{1-6}$-Alkyl $C_{1-4}$-Alkyl ist;
und/oder, wenn $R^4$ unabhängig $C_{1-6}$-Alkyl substituiert mit einem oder mehreren $R^{4-1}$ ist, dann das $C_{1-6}$-Alkyl $C_{1-4}$-Alkyl ist;
und/oder, wenn $R^4$ unabhängig $C_{1-6}$-Alkyl substituiert mit einem oder mehreren $R^{4-1}$ ist, dann die mehreren $R^{4-1}$ zwei oder drei $R^{4-1}$ sind;
und/oder, wenn $R^a$ unabhängig Halogen ist, dann das Halogen Fluor, Chlor, Brom oder Iod ist;
und/oder, wenn $R^b$ und $R^f$ unabhängig $C_{1-6}$-Alkyl sind, dann das $C_{1-6}$-Alkyl $C_{1-4}$-Alkyl ist;
und/oder, wenn $R^b$ und $R^f$ unabhängig $C_{1-6}$-Alkyl substituiert mit einem oder mehreren $R^{b-1}$ sind, dann das $C_{1-6}$-Alkyl $C_{1-4}$-Alkyl ist;
und/oder, wenn $R^b$ und $R^f$ unabhängig $C_{1-6}$-Alkyl substituiert mit einem oder mehreren $R^{b-1}$ sind, dann die mehreren $R^{b-1}$ zwei oder drei $R^{b-1}$ sind;
und/oder, wenn $R^{10j}$ und $R^{10k}$ unabhängig $C_{1-6}$-Alkyl sind, dann das $C_{1-6}$-Alkyl $C_{1-4}$-Alkyl ist.

**8.** Sauerstoffhaltige heterocyclische Verbindung, die durch Formel I dargestellt ist, nach Anspruch 7, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei, wenn $R^1$ "5- bis 12-gliedriges Heteroaryl, enthaltend 1-4 Heteroatome ausgewählt aus einem oder mehreren von O, S und N" ist, dann das "5- bis 12-gliedrige Heteroaryl, enthaltend 1-4 Heteroatome ausgewählt aus einem oder mehreren von O, S und N" Isochinolyl ist;

und/oder, wenn $R^1$ $C_{6-20}$-Aryl substituiert mit einem oder mehreren $R^{1-6}$ ist, dann das $C_{6-20}$-Aryl Phenyl oder 1-Naphthyl ist;
und/oder, wenn $R^{1-6}$ unabhängig Halogen ist, dann das Halogen Fluor oder Chlor ist;
und/oder, wenn $R^{1-6}$ unabhängig $C_{1-6}$-Alkyl ist, dann das $C_{1-6}$-Alkyl Methyl, Ethyl, n-Propyl, Isopropyl, n-Butyl, Isobutyl, sec-Butyl oder tert-Butyl ist;
und/oder, wenn $R^{1-6}$ unabhängig $C_{1-6}$-Alkyl substituiert mit einem oder mehreren $R^{1-6-1}$ ist, dann das $C_{1-6}$-Alkyl Methyl, Ethyl, n-Propyl, Isopropyl, n-Butyl, Isobutyl, sec-Butyl oder tert-Butyl ist;
und/oder, wenn $R^{1-6-1}$ unabhängig Halogen ist, dann das Halogen Fluor ist;
und/oder, wenn $R^{31}$, $R^{33}$ und $R^{34}$ unabhängig $C_{1-6}$-Alkyl substituiert mit einem oder mehreren $R^{31-1}$ sind, dann das $C_{1-6}$-Alkyl Methyl, Ethyl, n-Propyl, Isopropyl, n-Butyl, Isobutyl, sec-Butyl oder tert-Butyl ist;
und/oder, wenn $R^{31-1}$ unabhängig "4- bis 10-gliedriges Heterocycloalkyl, enthaltend 1-3 Heteroatome ausgewählt aus einem oder mehreren von O und N" substituiert mit einem oder mehreren $R^{d15}$ ist, dann das "4- bis 10-gliedrige Heterocycloalkyl, enthaltend 1-3 Heteroatome ausgewählt aus einem oder mehreren von O und N" "5- bis 7-gliedriges Heterocycloalkyl, enthaltend 1 Heteroatom ausgewählt aus einem von O und N" ist;
und/oder, wenn $R^{d15}$ unabhängig $C_{1-6}$-Alkyl ist, dann das $C_{1-6}$-Alkyl Methyl, Ethyl, n-Propyl, Isopropyl, n-Butyl, Isobutyl, sec-Butyl oder tert-Butyl ist;
und/oder, wenn $R^{d15}$ unabhängig Halogen ist, dann das Halogen Fluor ist;
und/oder, wenn $R^{e1}$ und $R^{e2}$ unabhängig $C_{1-6}$-Alkyl sind, dann das $C_{1-6}$-Alkyl Methyl, Ethyl, n-Propyl, Isopropyl, n-Butyl, Isobutyl, sec-Butyl oder tert-Butyl ist;
und/oder, wenn Ring Y ein 4- bis 12-gliedriger heterocyclischer Ring ist, der 1-4 N-Atome enthält, dann der 4- bis 12-gliedrige heterocyclische Ring, der 1-4 N-Atome enthält,

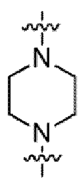 , 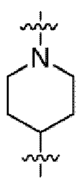 , 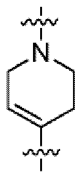 oder 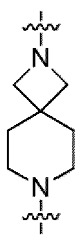

ist, das an seinem oberen Ende mit $R^2$ verbunden ist;
und/oder, wenn $R^4$ unabhängig $C_{1-6}$-Alkyl ist, dann das $C_{1-6}$-Alkyl Methyl, Ethyl, n-Propyl, Isopropyl, n-Butyl, Isobutyl, sec-Butyl oder tert-Butyl ist;
und/oder, wenn $R^4$ unabhängig $C_{1-6}$-Alkyl substituiert mit einem oder mehreren $R^{4-1}$ ist, dann das $C_{1-6}$-Alkyl

Methyl, Ethyl, n-Propyl, Isopropyl, n-Butyl, Isobutyl, sec-Butyl oder tert-Butyl ist;
und/oder, wenn $R^a$ unabhängig Halogen ist, dann das Halogen Fluor ist;
und/oder, wenn $R^b$ und $R^f$ unabhängig $C_{1-6}$-Alkyl sind, dann das $C_{1-6}$-Alkyl Methyl, Ethyl, n-Propyl, Isopropyl, n-Butyl, Isobutyl, sec-Butyl oder tert-Butyl ist;
und/oder, wenn $R^b$ und $R^f$ unabhängig $C_{1-6}$-Alkyl substituiert mit einem oder mehreren $R^{b-1}$ sind, dann das $C_{1-6}$-Alkyl Methyl, Ethyl, n-Propyl, Isopropyl, n-Butyl, Isobutyl, sec-Butyl oder tert-Butyl ist;
und/oder, wenn $R^{10j}$ und $R^{10k}$ unabhängig $C_{1-6}$-Alkyl sind, dann das $C_{1-6}$-Alkyl Methyl, Ethyl, n-Propyl, Isopropyl, n-Butyl, Isobutyl, sec-Butyl oder tert-Butyl ist.

**9.** Sauerstoffhaltige heterocyclische Verbindung, die durch Formel I dargestellt ist, nach Anspruch 8, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei, wenn $R^1$ "5- bis 12-gliedriges Heteroaryl, enthaltend 1-4 Heteroatome ausgewählt aus einem oder mehreren von O, S und N" ist, dann das "5- bis 12-gliedrige Heteroaryl, enthaltend 1-4 Heteroatome ausgewählt aus einem oder mehreren von O, S und N"

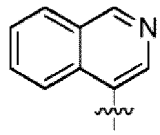

ist;

und/oder, wenn $R^{1-6}$ unabhängig $C_{1-6}$-Alkyl ist, dann das $C_{1-6}$-Alkyl Methyl ist;
und/oder, wenn $R^{1-6}$ unabhängig $C_{1-6}$-Alkyl substituiert mit einem oder mehreren $R^{1-6-1}$ ist, dann das $C_{1-6}$-Alkyl substituiert mit einem oder mehreren $R^{1-6-1}$ Trifluormethyl ist;
und/oder, wenn $R^{31}$, $R^{33}$ und $R^{34}$ unabhängig $C_{1-6}$-Alkyl substituiert mit einem oder mehreren $R^{31-1}$ sind, dann das $C_{1-6}$-Alkyl Methyl, Ethyl, n-Propyl oder Isopropyl ist;
und/oder, wenn $R^{31-1}$ unabhängig "4- bis 10-gliedriges Heterocycloalkyl, enthaltend 1-3 Heteroatome ausgewählt aus einem oder mehreren von O und N" substituiert mit einem oder mehreren $R^{d15}$ ist, dann das "4- bis 10-gliedrige Heterocycloalkyl, enthaltend 1-3 Heteroatome ausgewählt aus einem oder mehreren von O und N" Tetrahydropyrrolyl ist;
und/oder, wenn $R^{d15}$ unabhängig $C_{1-6}$-Alkyl ist, dann das $C_{1-6}$-Alkyl Methyl ist;
und/oder, wenn $R^{e1}$ und $R^{e2}$ unabhängig $C_{1-6}$-Alkyl sind, dann das $C_{1-6}$-Alkyl Methyl oder Ethyl ist;
und/oder die sauerstoffhaltige heterocyclische Verbindung, die durch Formel I dargestellt ist, eine Struktur wie folgt aufweist:

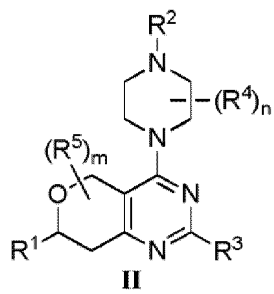

**II**

;

und/oder, wenn $R^4$ unabhängig $C_{1-6}$-Alkyl ist, dann das $C_{1-6}$-Alkyl Methyl ist;
und/oder, wenn $R^4$ unabhängig $C_{1-6}$-Alkyl substituiert mit einem oder mehreren $R^{4-1}$ ist, dann das $C_{1-6}$-Alkyl Methyl ist;
und/oder, wenn $R^b$ und $R^f$ unabhängig $C_{1-6}$-Alkyl sind, dann das $C_{1-6}$-Alkyl Methyl ist;
und/oder, wenn $R^b$ und $R^f$ unabhängig $C_{1-6}$-Alkyl substituiert mit einem oder mehreren $R^{b-1}$ sind, dann das $C_{1-6}$-Alkyl Methyl ist;
und/oder, wenn $R^{10j}$ und $R^{10k}$ unabhängig $C_{1-6}$-Alkyl sind, dann das $C_{1-6}$-Alkyl Methyl ist.

**10.** Sauerstoffhaltige heterocyclische Verbindung, die durch Formel I dargestellt ist, nach Anspruch 9, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei

die sauerstoffhaltige heterocyclische Verbindung, die durch Formel I dargestellt ist, eine Struktur wie folgt aufweist:

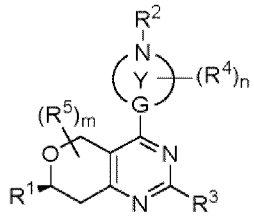

I-2 ;

und/oder, wenn $R^{33}$ unabhängig $C_{1-6}$-Alkyl ist, dann das $C_{1-6}$-Alkyl Methyl, Ethyl, n-Propyl, Isopropyl, n-Butyl, Isobutyl, sec-Butyl oder tert-Butyl ist oder Methyl, Ethyl, n-Propyl oder Isopropyl sein kann;
und/oder, wenn $R^4$ unabhängig $C_{1-6}$-Alkyl substituiert mit einem oder mehreren $R^{4-1}$ ist, dann das $C_{1-6}$-Alkyl substituiert mit einem oder mehreren $R^{4-1}$ Hydroxymethyl, Cyanomethyl oder

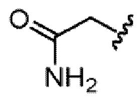

ist;
und/oder, wenn $R^b$ und $R^f$ unabhängig $C_{1-6}$-Alkyl-C(=O) - sind, dann das $C_{1-6}$-Alkyl in dem $C_{1-6}$-Alkyl-C(=O)- Methyl, Ethyl, n-Propyl, Isopropyl, n-Butyl, Isobutyl, sec-Butyl oder tert-Butyl ist oder Methyl sein kann;
und/oder das $R^2$ CN,

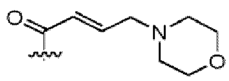, 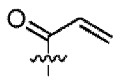, 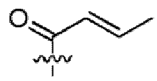, 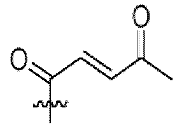,

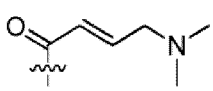, 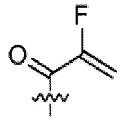, 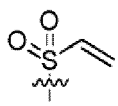 oder 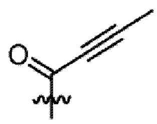

ist.

**11.** Sauerstoffhaltige heterocyclische Verbindung, die durch Formel I dargestellt ist, nach Anspruch 9, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei,

wenn $R^1$ $C_{6-20}$-Aryl substituiert mit einem oder mehreren $R^{1-6}$ ist, dann das $C_{6-20}$-Aryl substituiert mit einem oder mehreren $R^{1-6}$

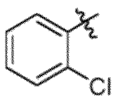, 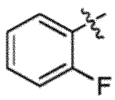, 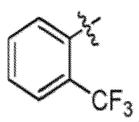, 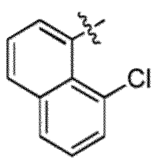 oder 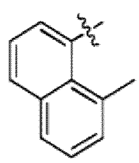

ist;

und/oder, wenn $R^4$ unabhängig $C_{1-6}$-Alkyl substituiert mit einem oder mehreren $R^{4-1}$ ist, dann das $C_{1-6}$-Alkyl substituiert mit einem oder mehreren $R^{4-1}$ Hydroxymethyl oder Cyanomethyl ist;

und/oder, wenn $R^{10j}$ und $R^{10k}$ zusammen mit dem N-Atom, an das sie gebunden sind, "4- bis 10-gliedriges Heterocycloalkyl, enthaltend 1-3 Heteroatome ausgewählt aus einem oder mehreren von O und N" bilden, dann das "4- bis 10-gliedrige Heterocycloalkyl, enthaltend 1-3 Heteroatome ausgewählt aus einem oder mehreren von O und N" "5- bis 6-gliedriges Heterocycloalkyl, enthaltend 2 Heteroatome ausgewählt aus O und N" ist oder

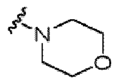

sein kann;

und/oder $R^2$

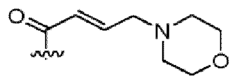, 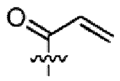, 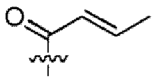, 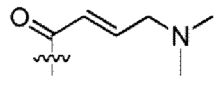, 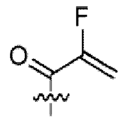, 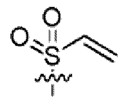

oder

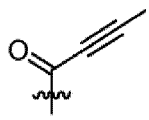

ist.

12. Sauerstoffhaltige heterocyclische Verbindung, die durch Formel I dargestellt ist, nach Anspruch 9, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei, wenn $R^1$ $C_{6-20}$-Aryl substituiert mit einem oder mehreren $R^{1-6}$ ist, dann das $C_{6-20}$-Aryl substituiert mit einem oder mehreren $R^{1-6}$

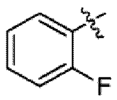, 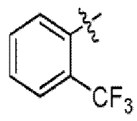, 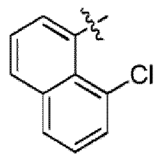 oder 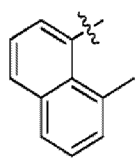

ist;

und/oder, wenn $R^{31}$, $R^{33}$ und $R^{34}$ unabhängig $C_{1-6}$-Alkyl substituiert mit einem oder mehreren $R^{31-1}$ sind, dann das $C_{1-6}$-Alkyl substituiert mit einem oder mehreren $R^{31-1}$

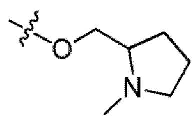,

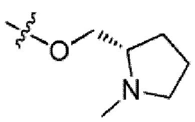, 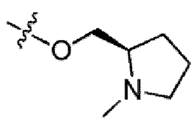, 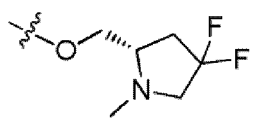, 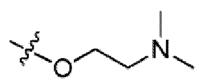,

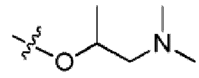, 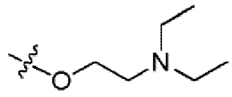, 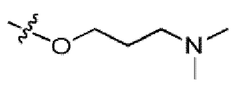 oder 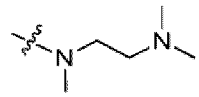

ist;

und/oder, wenn $R^4$ unabhängig $C_{1-6}$-Alkyl substituiert mit einem oder mehreren $R^{4-1}$ ist, dann das $C_{1-6}$-Alkyl substituiert mit einem oder mehreren $R^{4-1}$ Cyanomethyl ist;

und/oder $R^2$

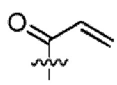, 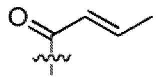, 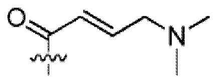, 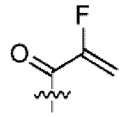, 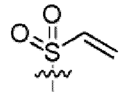 oder 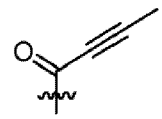

ist.

**13.** Sauerstoffhaltige heterocyclische Verbindung, die durch Formel I dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei die sauerstoffhaltige heterocyclische Verbindung, die durch Formel I dargestellt ist, eine beliebige der folgenden Strukturen aufweist:

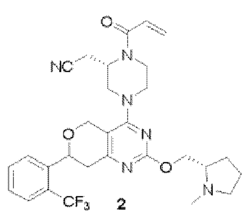
2

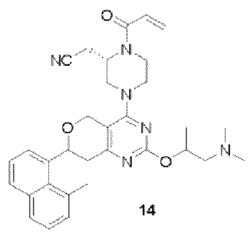
14

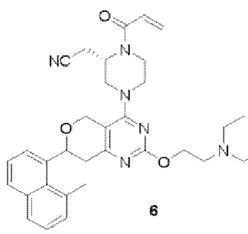
6

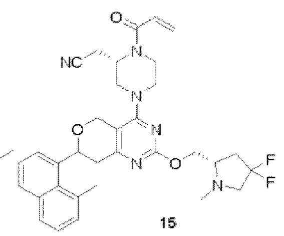
15

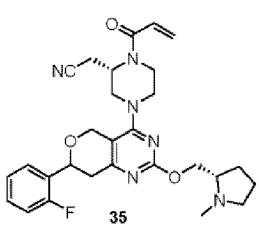
35

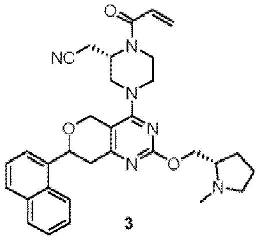
3

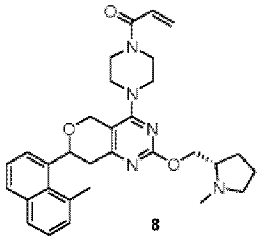
8

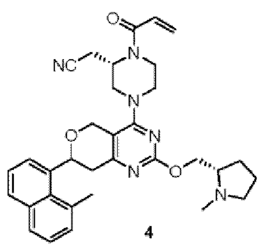
4

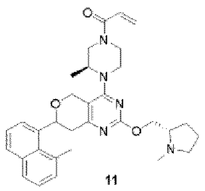
11

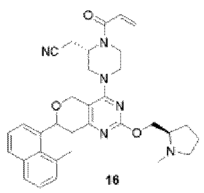
16

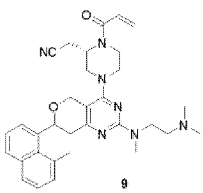
9

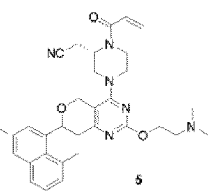
5

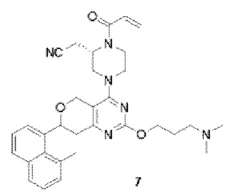
7

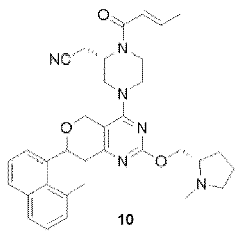
10

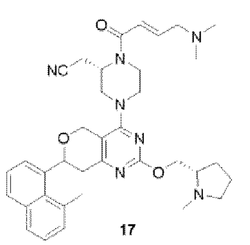
17

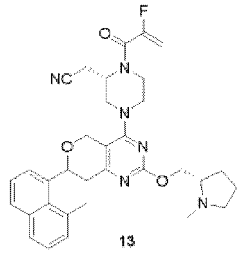
13

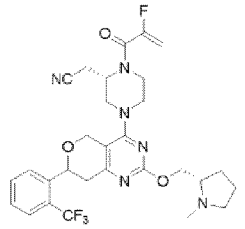
18

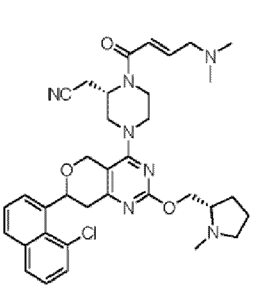

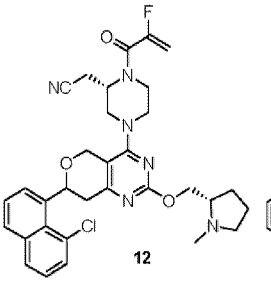

12

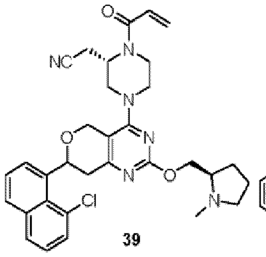

39

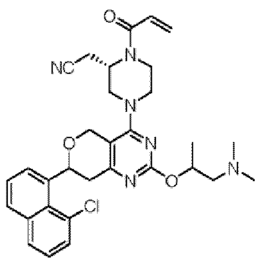

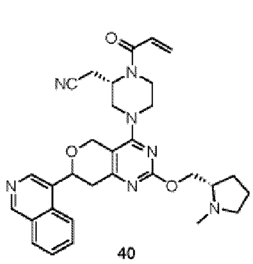

40

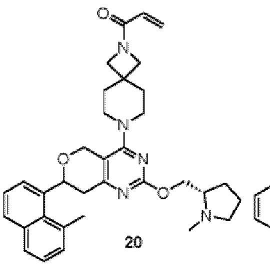

20

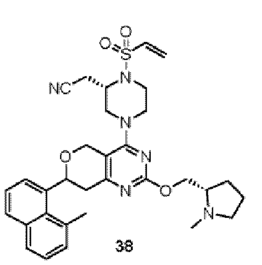

38

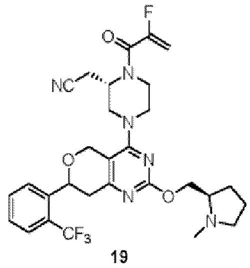

19

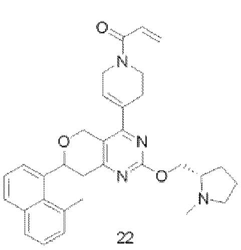

22

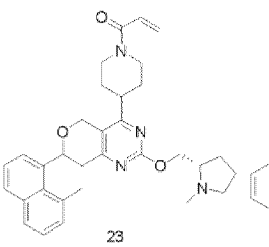

23

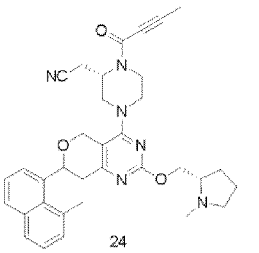

24

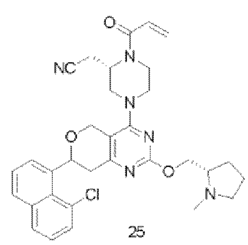

25

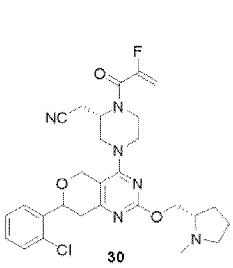

30

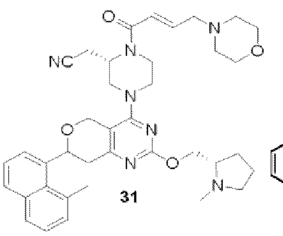

31

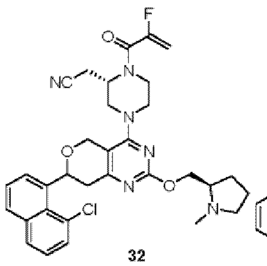

32

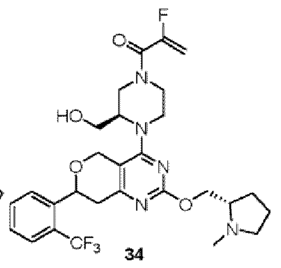

34

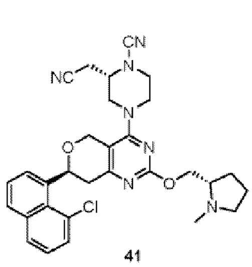

41

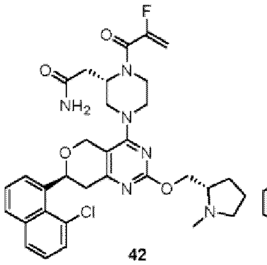

42

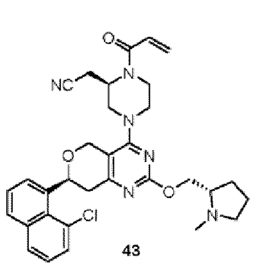

43

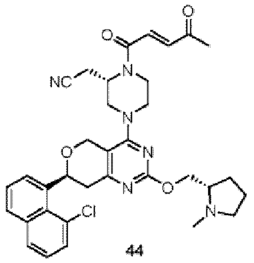

44

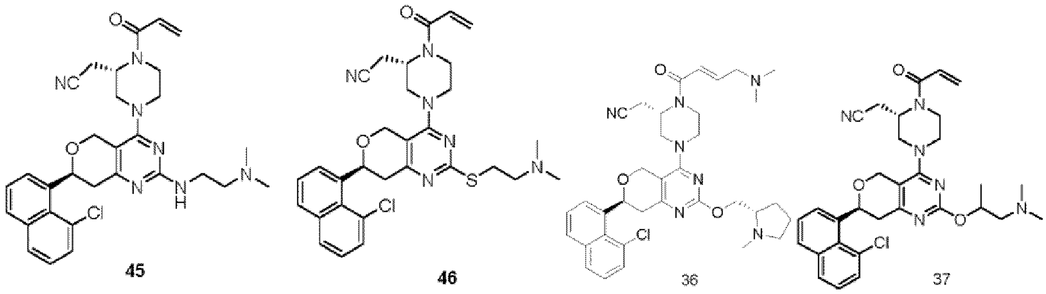

45 46 36 37

**14.** Sauerstoffhaltige heterocyclische Verbindung, die durch Formel I dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei die sauerstoffhaltige heterocyclische Verbindung, die durch Formel I dargestellt ist, eine beliebige der folgenden Verbindungen ist:

Verbindung

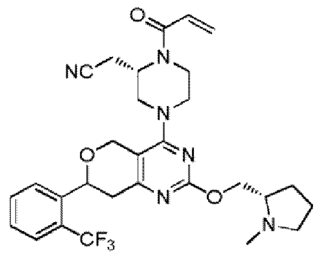

, die eine Retentionszeit von 0,92 min unter den folgenden Bedingungen aufweist: Ausrüstung: SFC Method Station (Thar, Waters); Chromatographiesäule: OD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

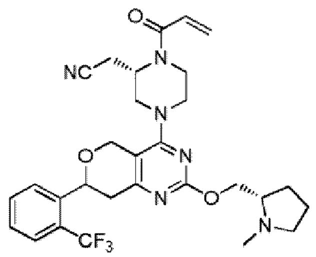

, die eine Retentionszeit von 2,74 min unter den folgenden Bedingungen aufweist: Ausrüstung: SFC Method Station (Thar, Waters); Chromatographiesäule: OD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

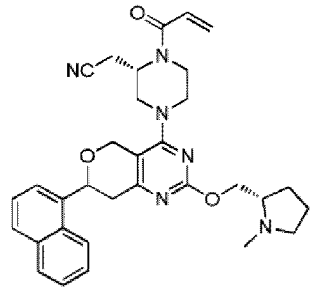

, die eine Retentionszeit von 0,97 min unter den folgenden Bedingungen aufweist: Ausrüstung: SFC Method Station (Thar, Waters); Chromatographiesäule: AD-H 4,6 * 100 mm, 5 um (Daicel); Säulentemperatur: 40 °C; mobile Phase: $CO_2$/ETOH (0,5 % TEA) = 55/45; Durchflussgeschwindigkeit: 4,0 ml/min; Wellenlänge: 254 nm; Gegendruck: 120 bar;
Verbindung

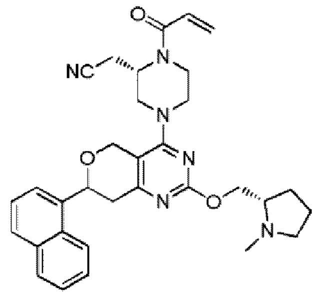

,

, die eine Retentionszeit von 2,40 min unter den folgenden Bedingungen aufweist: Ausrüstung: SFC Method Station (Thar, Waters); Chromatographiesäule: AD-H 4,6 * 100 mm, 5 um (Daicel); Säulentemperatur: 40 °C; mobile Phase: $CO_2$/ETOH (0,5 % TEA) = 55/45; Durchflussgeschwindigkeit: 4,0 ml/min; Wellenlänge: 254 nm; Gegendruck: 120 bar;
Verbindung

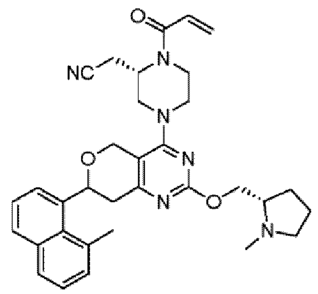

, die eine Retentionszeit von 0,97 min unter den folgenden Bedingungen aufweist: Ausrüstung: SFC Method Station (Thar, Waters); Chromatographiesäule: OJ-H 4,6 * 100 mm, 5 um (Daicel); Säulentemperatur: 40 °C; mobile Phase: $CO_2$/Methanol (0,1 % TEA) = 60/40; Durchflussgeschwindigkeit: 4,0 ml/min; Wellenlänge: 254 nm; Gegendruck: 120 bar;
Verbindung

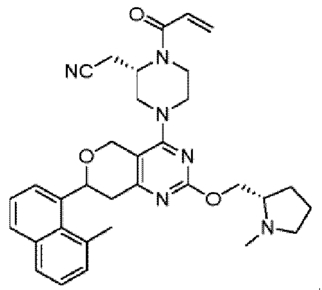

, die eine Retentionszeit von 1,94 min unter den folgenden Bedingungen aufweist: Ausrüstung: SFC Method Station (Thar, Waters); Chromatographiesäule: OJ-H 4,6 * 100 mm, 5 μm (Daicel); Säulentemperatur: 40 °C; mobile Phase: $CO_2$/Methanol (0,1 % TEA) = 60/40; Durchflussgeschwindigkeit: 4,0 ml/min; Wellenlänge: 254 nm; Gegendruck: 120 bar;
Verbindung

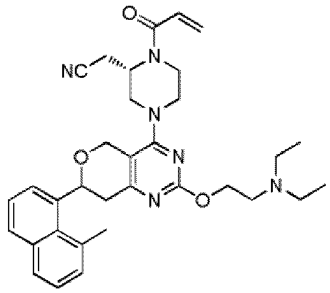

, die eine Retentionszeit von 1,22 min unter den folgenden Bedingungen aufweist: Ausrüstung: SFC Method Station (Thar, Waters); Chromatographiesäule: CHIRALCEL OJ-H 4,6 * 100 mm, 5 um (Daicel); Säulentemperatur: 40 °C; mobile Phase: $CO_2$/MeOH (0,1 % TEA) = 65/35; Durchflussgeschwindigkeit: 1,0 ml/min; Wellenlänge: 214 nm; Gegendruck: 120 bar;
Verbindung

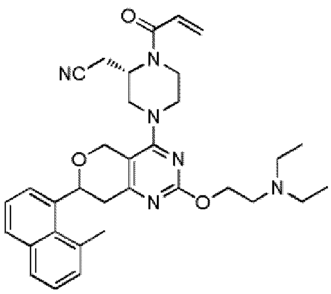

, die eine Retentionszeit von 2,67 min unter den folgenden Bedingungen aufweist: Ausrüstung: SFC Method Station (Thar, Waters); Chromatographiesäule: CHIRALCEL OJ-H 4,6 * 100 mm, 5 um (Daicel); Säulentemperatur: 40 °C; mobile Phase: $CO_2$/MeOH (0,1 % TEA) = 65/35; Durchflussgeschwindigkeit: 1,0 ml/min; Wellenlänge: 214 nm; Gegendruck: 120 bar;
Verbindung

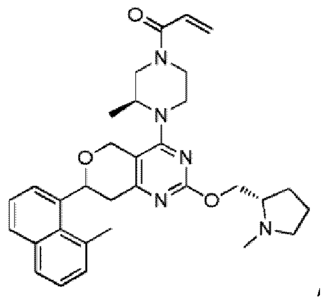

, die eine Retentionszeit von 3,26 min unter den folgenden Bedingungen aufweist: Instrument: SFC Method Station (Thar, Waters); Chromatographiesäule: R,R-WHELK-O1 4,6 * 100 mm, 5 um (REGIS); Säulentemperatur: 40 °C; mobile Phase: CO2/(MeOH/ACN = 3 : 2 (0,1 % TEA)) = 55/45; Durchflussgeschwindigkeit: 4,0 ml/min; Wellenlänge: 254 nm; Gegendruck: 120 bar;
Verbindung

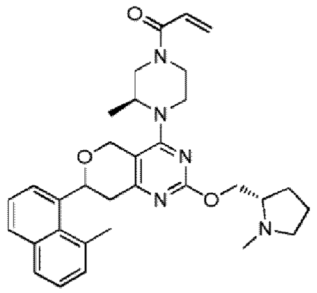

, die eine Retentionszeit von 4,16 min unter den folgenden Bedingungen aufweist: Instrument: SFC Method Station (Thar, Waters); Chromatographiesäule: R,R-WHELK-O1 4,6 * 100 mm, 5 um (REGIS); Säulentemperatur: 40 °C; mobile Phase: $CO_2$/(MeOH/CAN = 3 : 2 (0,1 % TEA)) = 55/45; Durchflussgeschwindigkeit: 4,0 ml/min; Wellenlänge: 254 nm; Gegendruck: 120 bar;
Verbindung

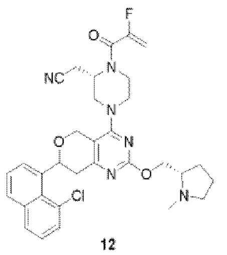

12

, die eine Retentionszeit von 1,36 min unter den folgenden Bedingungen aufweist: Instrument: SFC Method Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm; Gegendruck: 120 bar;
Verbindung

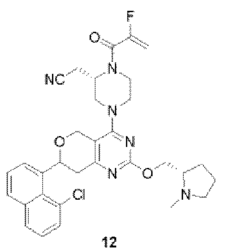

12

, die eine Retentionszeit von 2,77 min unter den folgenden Bedingungen aufweist: Instrument: SFC Method

Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm; Gegendruck: 120 bar;
Verbindung

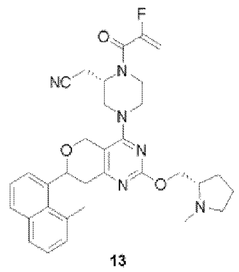

13

, die eine Retentionszeit von 1,17 min unter den folgenden Bedingungen aufweist: Instrument: SFC Method Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm; Gegendruck: 120 bar;
Verbindung

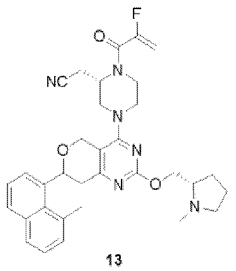

13

, die eine Retentionszeit von 2,76 min unter den folgenden Bedingungen aufweist: Instrument: SFC Method Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm; Gegendruck: 120 bar;
Verbindung

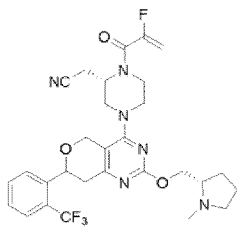

18

, die eine Retentionszeit von 0,78 min unter den folgenden Bedingungen aufweist: Instrument: SFC Method Station (Thar, Waters); Chromatographiesäule: AD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

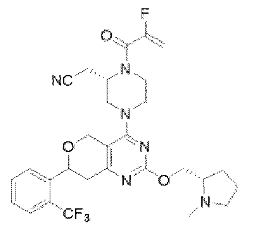

18

, die eine Retentionszeit von 2,42 min unter den folgenden Bedingungen aufweist: Instrument: SFC Method Station (Thar, Waters); Chromatographiesäule: AD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

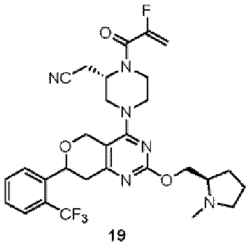

19

, die eine Retentionszeit von 0,79 min unter den folgenden Bedingungen aufweist: Instrument: SFC Method Station (Thar, Waters); Chromatographiesäule: OD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

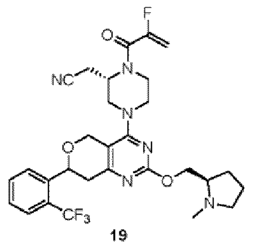

19

, die eine Retentionszeit von 2,29 min unter den folgenden Bedingungen aufweist: Instrument: SFC Method Station (Thar, Waters); Chromatographiesäule: OD-H 4,6 * 100 mm, 5 um (Daicel); Säulentemperatur: 40 °C; mobile Phase: $CO_2$/MeOH (0,1 % TEA) = 65/35; Durchflussgeschwindigkeit: 4,0 ml/min; Wellenlänge: 254 nm; Gegendruck: 120 bar;
Verbindung

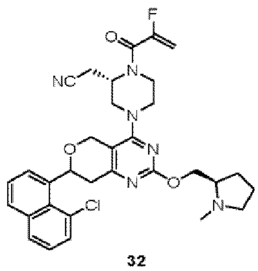

32

, die eine Retentionszeit von 1,45 min unter den folgenden Bedingungen aufweist: Instrument: SFC Method Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm;

Gegendruck: 120 bar;
Verbindung

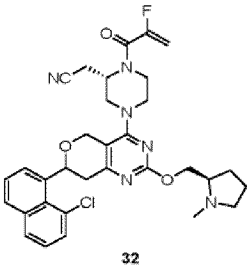

32

, die eine Retentionszeit von 2,81 min unter den folgenden Bedingungen aufweist: Instrument: SFC Method Station (Thar, Waters); Chromatographiesäule: OJ-H 4,6 * 100 mm, 5 um (Daicel); Säulentemperatur: 40 °C; mobile Phase: $CO_2$/MeOH (0,1 % TEA) = 60/40; Durchflussgeschwindigkeit: 4,0 ml/min; Wellenlänge: 254 nm; Gegendruck: 120 bar.

**15.** Sauerstoffhaltige heterocyclische Verbindung, die durch Formel I dargestellt ist, nach Anspruch 1, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon, wobei die sauerstoffhaltige heterocyclische Verbindung, die durch Formel I dargestellt ist, eine beliebige der folgenden Verbindungen ist:

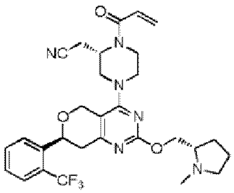 , 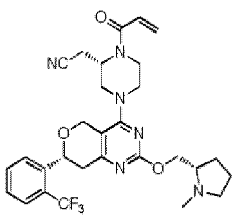 , 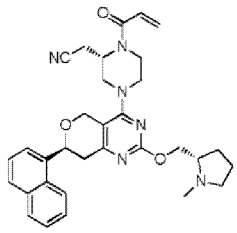 , 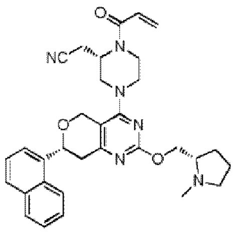 ,

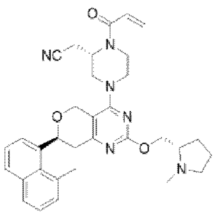 , 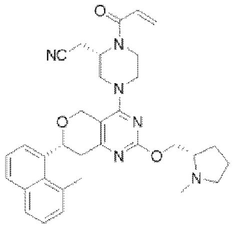 , 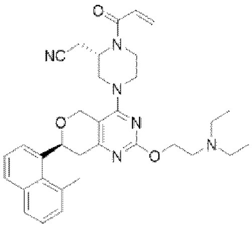 , 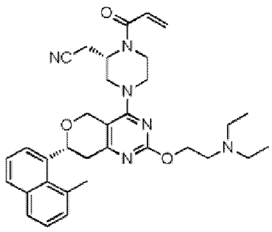 ,

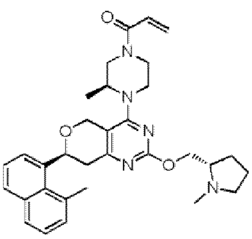 , 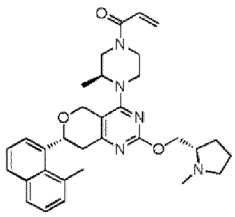 , 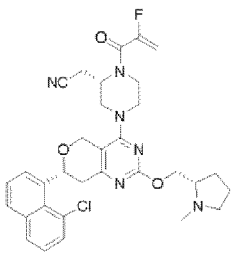 , 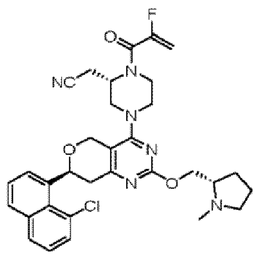 ,

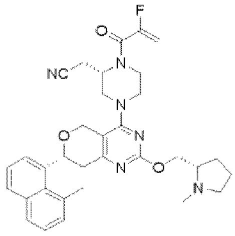, 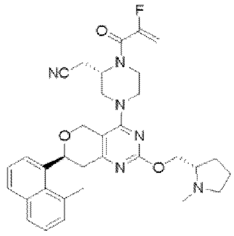, 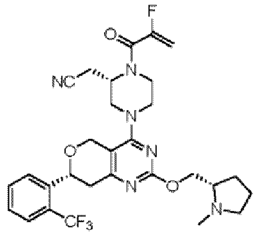, 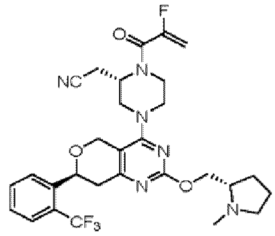,

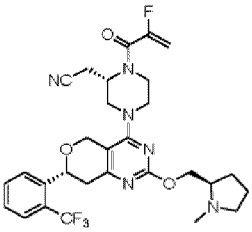, 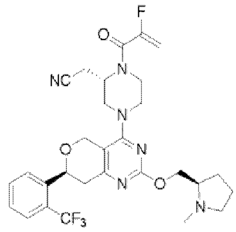, 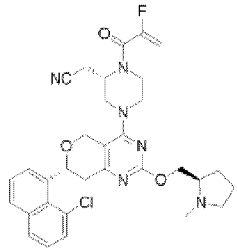, 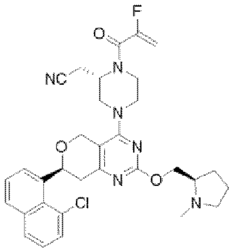.

**16.** Verfahren zum Herstellen der sauerstoffhaltigen heterocyclischen Verbindung, die durch Formel I dargestellt ist, nach einem der Ansprüche 1-15, wobei das Verfahren Route eins oder Route zwei umfasst:
Route eins:

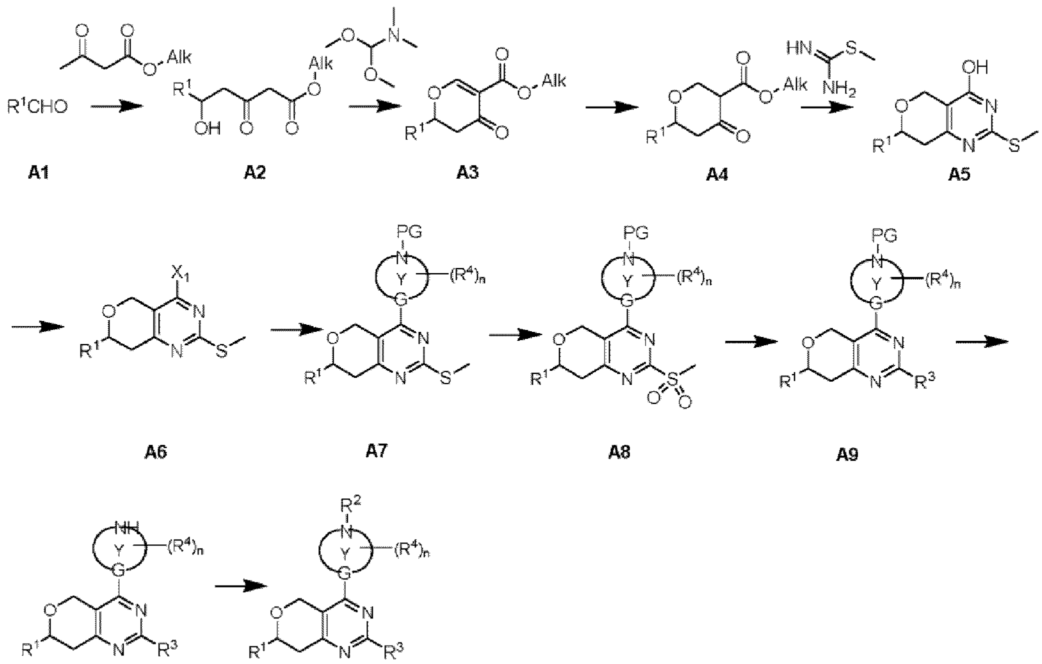
A1 A2 A3 A4 A5
A6 A7 A8 A9

wobei $X_1$ eine Abgangsgruppe ist; Alk Alkyl ist; und PG eine Amino-Schutzgruppe ist;
Route zwei:

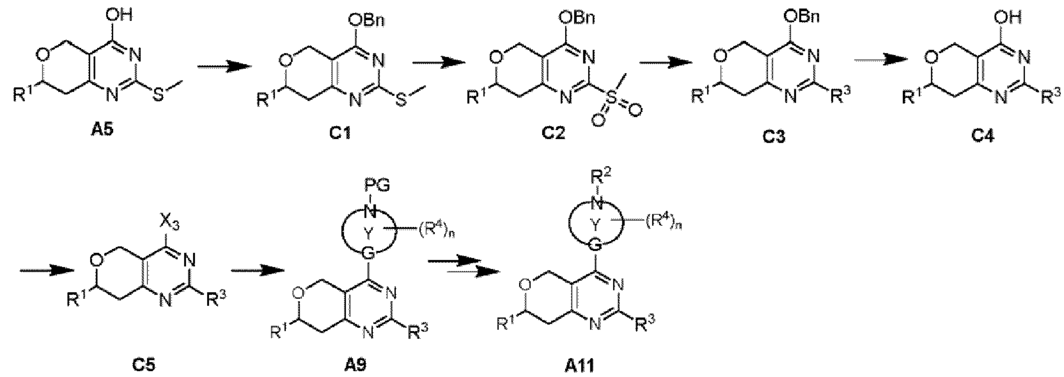

A5 C1 C2 C3 C4

C5 A9 A11

wobei $X_3$ eine Abgangsgruppe ist und PG eine Amino-Schutzgruppe ist;
die Abgangsgruppe OTf oder Cl ist;
die Amino-Schutzgruppe Boc oder Cbz ist.

**17.** Verbindung, die durch Formel A5, A6, A7, A8, A9, A10, C1, C2, C3, C4 oder C5 dargestellt ist;

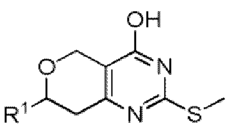

A5

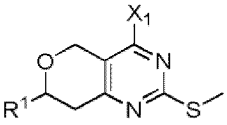

A6

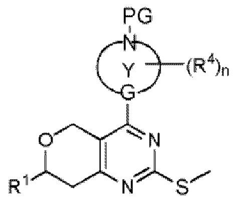

A7

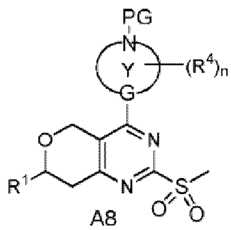

A8

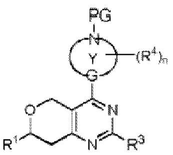

A9

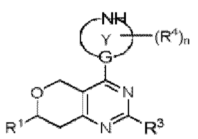

A10

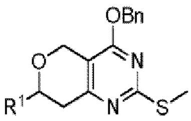

**C1**

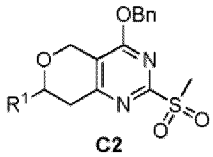

**C2**

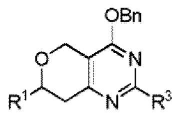

**C3**

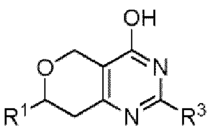

**C4** .

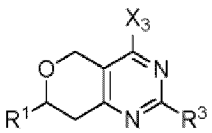

**C5** ;

wobei $R^1$, $R^3$, $R^4$, G, Y und n nach einem der Ansprüche 1-15 sind;
$X^1$ und $X^3$ unabhängig Abgangsgruppen sind und PG eine Amino-Schutzgruppe ist;
die Abgangsgruppe OTf oder Cl ist;
die Amino-Schutzgruppe Boc oder Cbz ist;
zum Beispiel die Verbindung, die durch Formel A5, A6, A7, A8, A9, A10, C1, C2, C3, C4 oder C5 dargestellt ist, eine beliebige der folgenden Verbindungen ist:

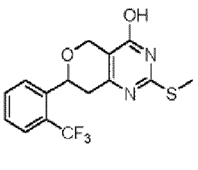

**2-f**

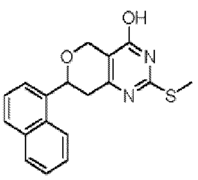

**3-f**

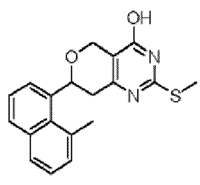

**4-f**

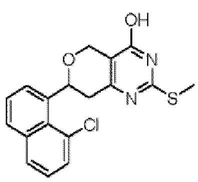

**12-f**

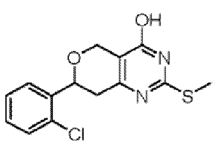

**30-f**

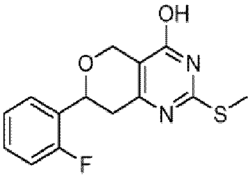
35-f
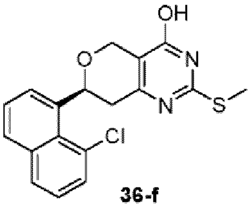
36-f
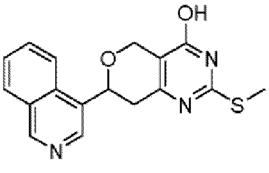
40-f

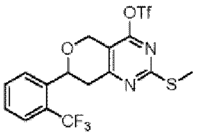
2-e
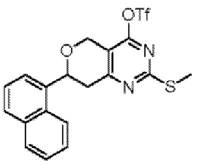
3-e
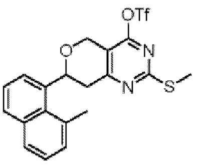
4-e
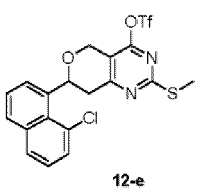
12-e
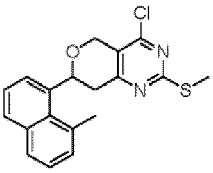
20-e

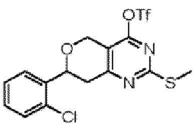
30-e
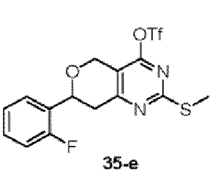
35-e
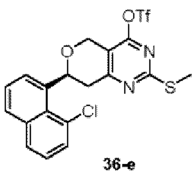
36-e
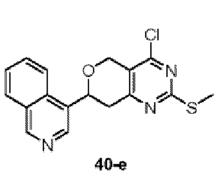
40-e
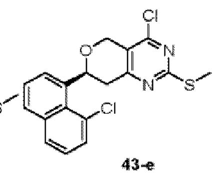
43-e

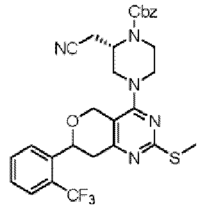
2-d
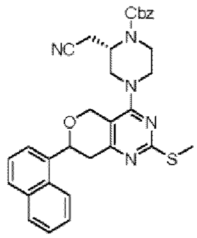
3-d
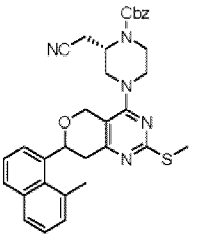
4-d
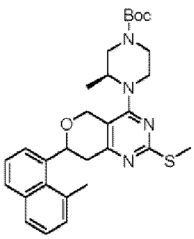
11-d
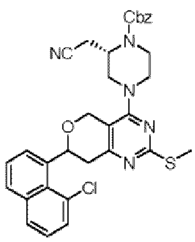
12-d

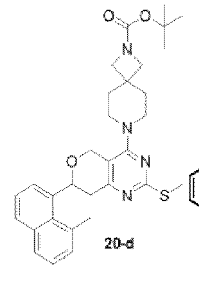
20-d
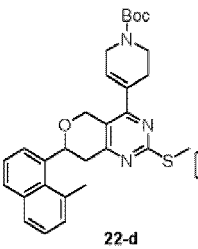
22-d
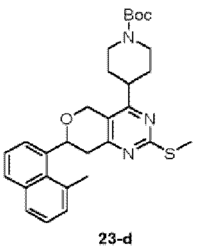
23-d
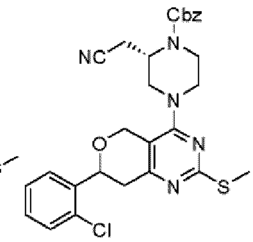
30-d
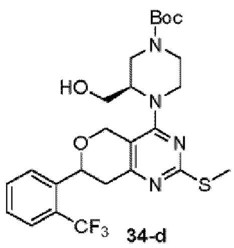
34-d

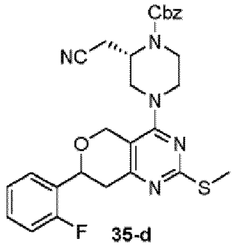
35-d
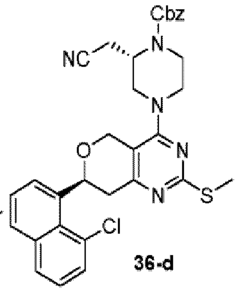
36-d
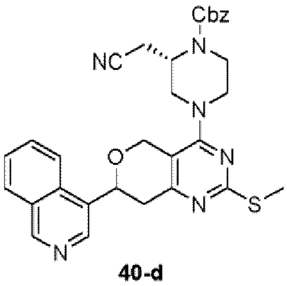
40-d
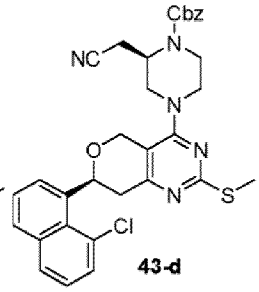
43-d

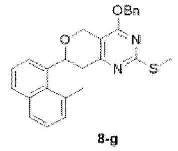
8-g
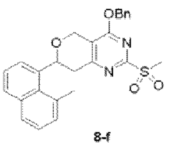
8-f
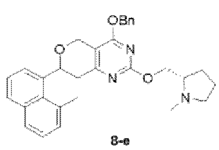
8-e
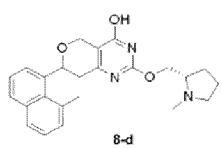
8-d
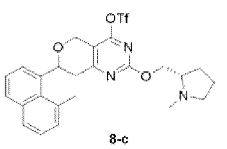
8-c

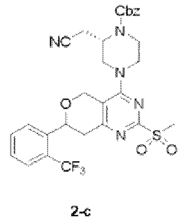
2-c

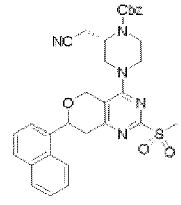
3-c

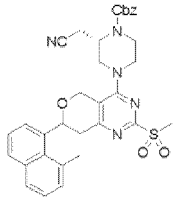
4-c

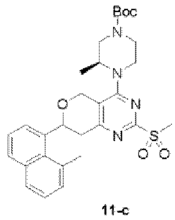
11-c

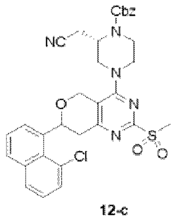
12-c

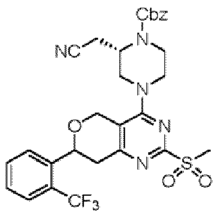
19-c

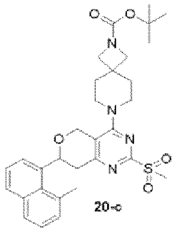
20-c

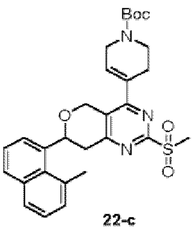
22-c

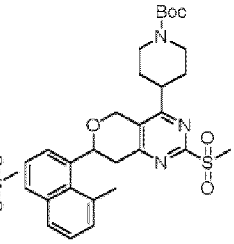
23-c

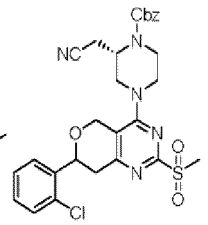
30-c

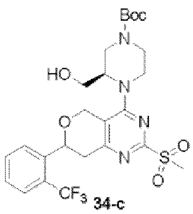
34-c

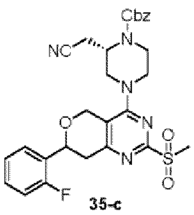
35-c

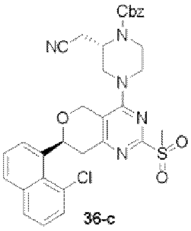
36-c

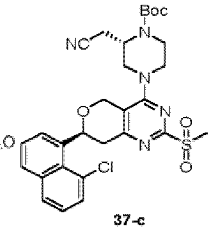
37-c

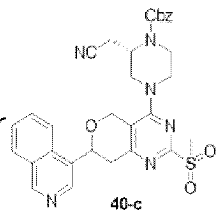
40-c

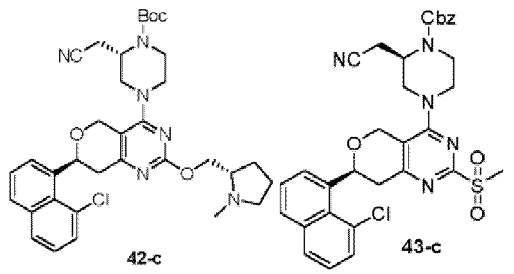
42-c 43-c

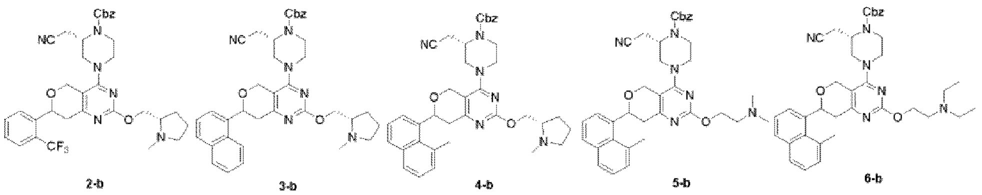
2-b 3-b 4-b 5-b 6-b

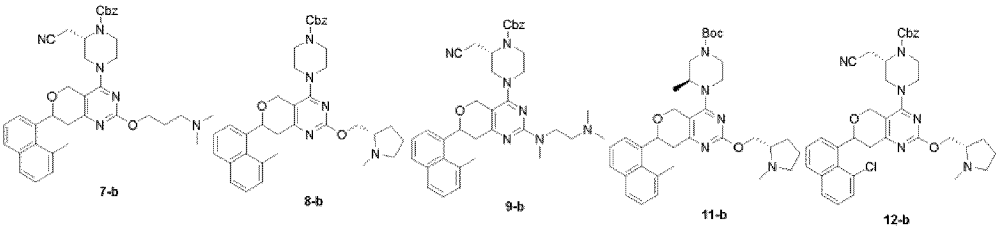
7-b 8-b 9-b 11-b 12-b

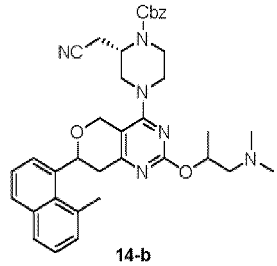
14-b
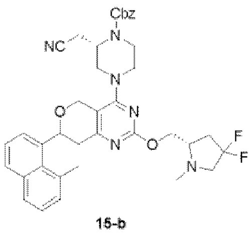
15-b
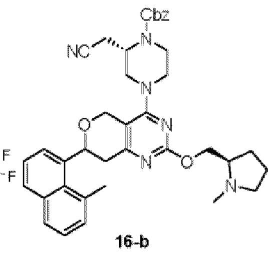
16-b
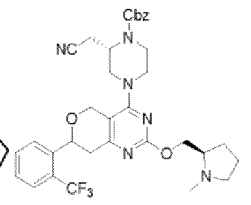
19-b

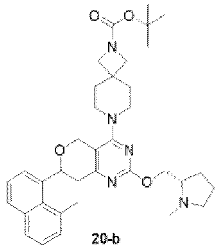
20-b
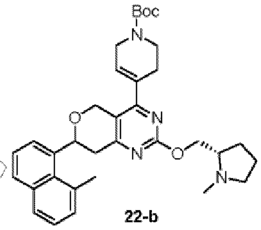
22-b
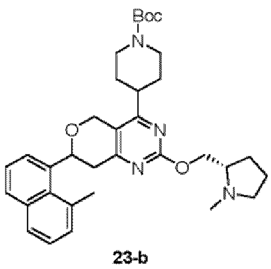
23-b
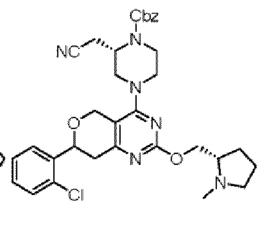
30-b

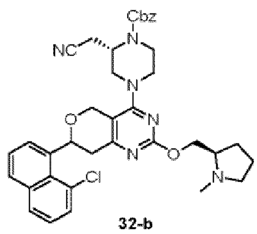
32-b
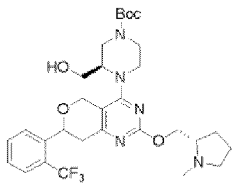
34-b
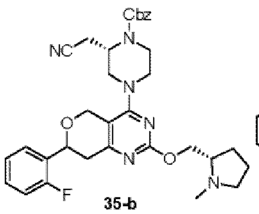
35-b
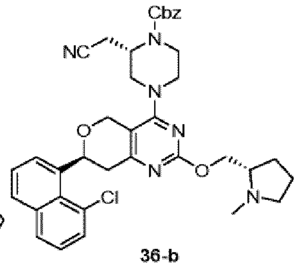
36-b

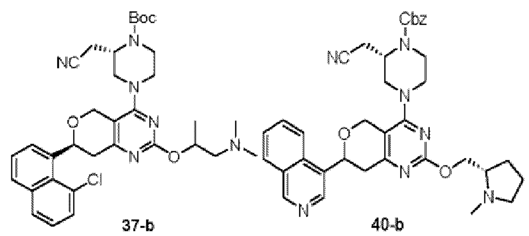
37-b
40-b
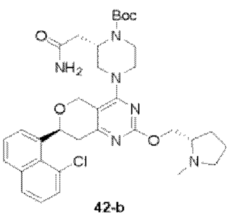
42-b
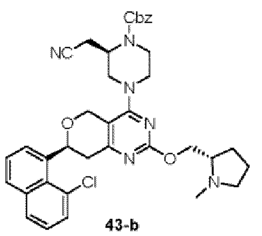
43-b

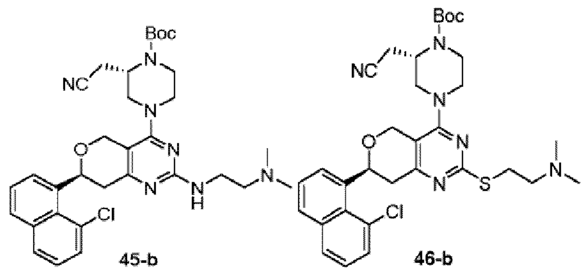
45-b
46-b

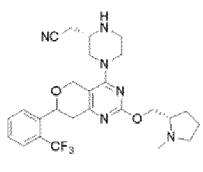
2-a
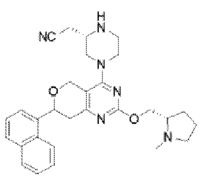
3-a
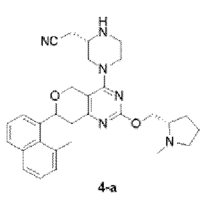
4-a
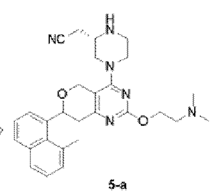
5-a
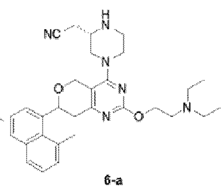
6-a

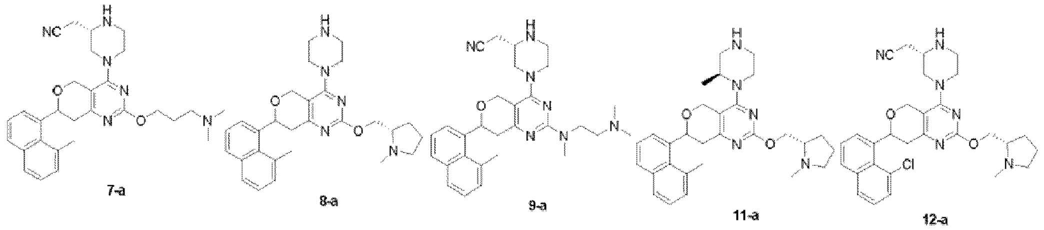

7-a 8-a 9-a 11-a 12-a

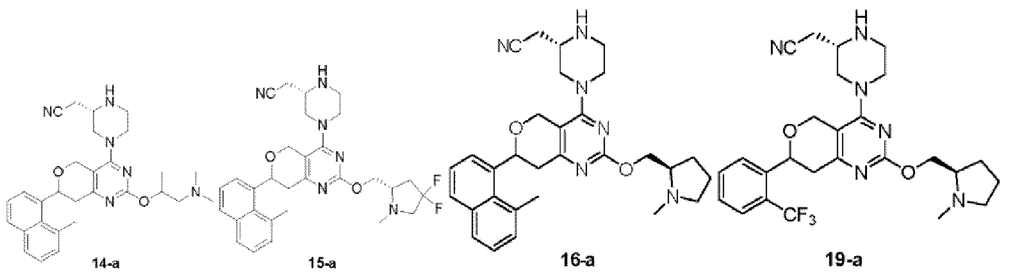

14-a 15-a 16-a 19-a

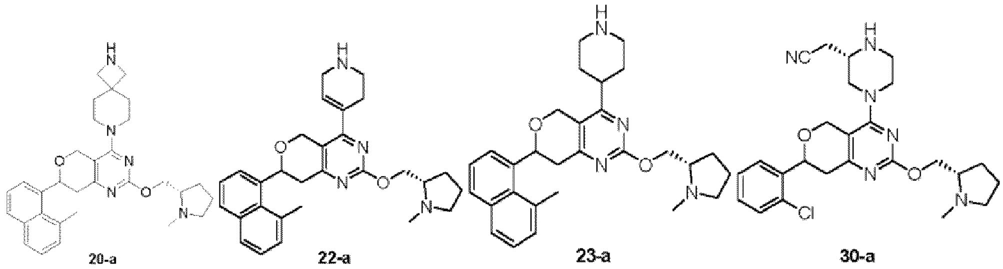

20-a 22-a 23-a 30-a

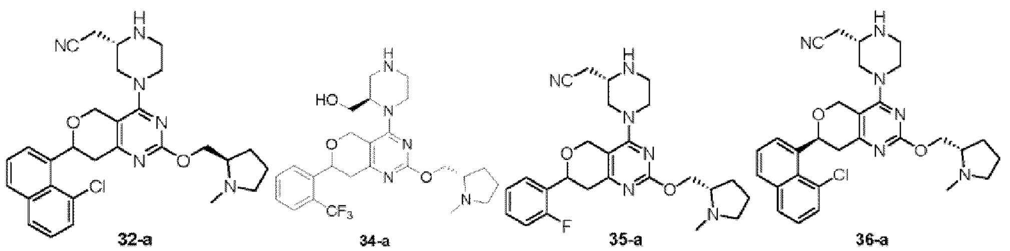

32-a 34-a 35-a 36-a

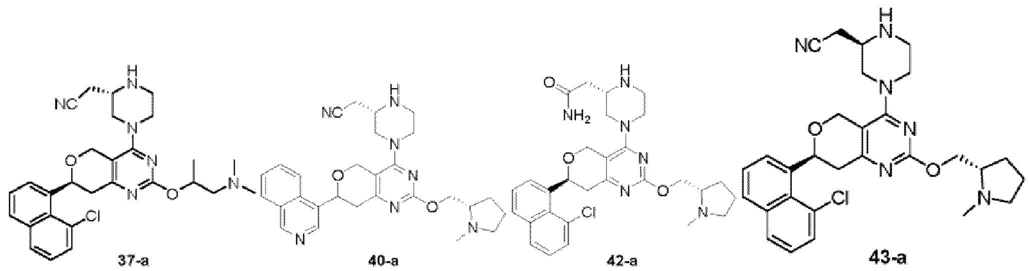

37-a 40-a 42-a 43-a

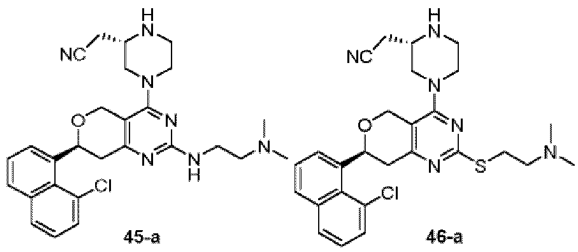

45-a 46-a

**18.** Pharmazeutische Zusammensetzung, umfassend Substanz A und ein pharmazeutisches Adjuvans; wobei die

Substanz A die sauerstoffhaltige heterocyclische Verbindung, die durch Formel I dargestellt ist, nach einem der Ansprüche 1-15, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon ist.

**19.** Substanz A zur Verwendung als Medikament, wobei die Substanz A die sauerstoffhaltige heterocyclische Verbindung, die durch Formel I dargestellt ist, nach einem der Ansprüche 1-15, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon ist.

**20.** Substanz A zur Verwendung als Medikament, wobei die Substanz A die sauerstoffhaltige heterocyclische Verbindung, die durch Formel I dargestellt ist, nach einem der Ansprüche 1-15, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon ist;
bei der Behandlung oder Vorbeugung von Krebs, wobei es sich bei dem Krebs um eines oder mehrere von Kolonkrebs, Bauchspeicheldrüsenkrebs, Brustkrebs, Prostatakrebs, Lungenkrebs, Hirnkrebs, Eierstockkrebs, Gebärmutterhalskrebs, Hodenkrebs, Nierenkarzinom, Kopf- oder Halskrebs, Knochenkrebs, Hautkrebs, Rektalkrebs, Leberkrebs, Kolonkrebs, Speiseröhrenkrebs, Magenkrebs, Bauchspeicheldrüsenkrebs, Schilddrüsenkrebs, Blasenkrebs, Lymphom, Leukämie und Melanom handelt.

**21.** Substanz A zur Verwendung als RAS-Hemmer *ex vivo*, wobei die Substanz A die sauerstoffhaltige heterocyclische Verbindung, die durch Formel I dargestellt ist, nach einem der Ansprüche 1-15, ein pharmazeutisch unbedenkliches Salz davon, ein Solvat davon, ein Solvat des pharmazeutisch unbedenklichen Salzes davon, eine Kristallform davon, ein Stereoisomer davon, ein Tautomer davon oder eine isotopische Verbindung davon ist.

**Revendications**

**1.** Composé hétérocyclique contenant de l'oxygène représenté par la formule I, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel :

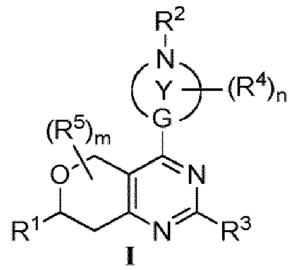

I

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ et $R^{1-7}$ sont indépendamment halogène, hydroxyle, - C(=O)$R^{65}$, -N$R^{63}R^{64}$, -C(=O)O$R^{66}$, -C(=O)N$R^{69}R^{610}$, alkyle en $C_{1-6}$, alcoxy en $C_{1-6}$, cycloalkyle en $C_{3-10}$, « hétérocycloalkyle à 5 à 7 chaînons contenant 1 ou 2 hétéroatomes choisis à partir d'un ou de plusieurs parmi O et N », aryle en $C_{6-20}$, « hétéroaryle à 5 à 7 chaînons contenant 1 ou 2 hétéroatomes choisis à partir d'un ou de plusieurs parmi O et N », alkyle en $C_{1-6}$ substitué par un ou plusieurs $R^{1-6-1}$, alcoxy en $C_{1-6}$ substitué par un ou plusieurs $R^{1-6-2}$, cycloalkyle en $C_{3-10}$ substitué par un ou plusieurs $R^{1-6-3}$, « hétérocycloalkyle à 5 à 7 chaînons contenant 1 ou 2 hétéroatomes choisis à partir d'un ou de plusieurs parmi O et N » substitué par un ou plusieurs $R^{1-6-4}$, aryle en $C_{6-20}$ substitué par un ou plusieurs $R^{1-6-5}$, ou « hétéroaryle à 5 à 7 chaînons contenant 1 ou 2 hétéroatomes choisis à partir d'un ou de plusieurs parmi O et N » substitué par un ou plusieurs $R^{1-6-6}$ ;
$R^{1-6-1}$, $R^{1-6-2}$, $R^{1-6-3}$, $R^{1-6-4}$, $R^{1-6-5}$ et $R^{1-6-6}$ sont indépendamment cyano, halogène, hydroxyle, alcoxy en $C_{1-6}$, alkyle en $C_{1-6}$, -C(=O)$R^{65-2}$, -N$R^{63-2}R^{64-2}$, -C(=O)O$R^{66-2}$ ou - C(=O)N$R^{69-2}R^{610-2}$ ;
$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ et $R^{610-2}$ sont indépendamment hydrogène ou

alkyle en $C_{1-6}$ ;
m vaut 0, 1 ou 2 ;
$R^5$ est indépendamment alkyle en $C_{1-6}$ ;
$R^3$ est -$OR^{31}$, -$SR^{32}$ ou -$NR^{33}R^{34}$ ;
$R^{31}$, $R^{32}$ et $R^{34}$ sont indépendamment alkyle en $C_{1-6}$ substitué par un ou plusieurs $R^{31-1}$ ; $R^{33}$ est indépendamment H, alkyle en $C_{1-6}$ ou alkyle en $C_{1-6}$ substitué par un ou plusieurs $R^{31-1}$ ;
$R^{31-1}$ est indépendamment cycloalkyle en $C_{3-10}$, « hétérocycloalkyle à 4 à 10 chaînons contenant 1 à 3 hétéroatomes choisis à partir d'un ou de plusieurs parmi O et N », cycloalkyle en $C_{3-10}$ substitué par un ou plusieurs $R^{d16}$, « hétérocycloalkyle à 4 à 10 chaînons contenant 1 à 3 hétéroatomes choisis à partir d'un ou de plusieurs parmi O et N » substitué par un ou plusieurs $R^{d15}$, -$OR^d$, -$SR^{d1}$, - $NR^{e1}R^{e2}$, ou -C(=O)$NR^{e3}R^{e4}$ ;
$R^{d15}$ et $R^{d16}$ sont indépendamment alkyle en $C_{1-6}$, alkyle en $C_{1-6}$ substitué par un ou plusieurs $R^{1-8-1}$, hydroxyle, alcoxy en $C_{1-6}$, halogène, -$NR^{e5}R^{e6}$ ou -C(=O)$NR^{e7}R^{e8}$ ;
$R^d$, $R^{d1}$, $R^{e1}$, $R^{e2}$, $R^{e3}$ et $R^{e4}$ sont indépendamment hydrogène, alkyle en $C_{1-6}$, cycloalkyle en $C_{3-10}$, « hétérocycloalkyle à 4 à 10 chaînons contenant 1 à 3 hétéroatomes choisis à partir d'un ou de plusieurs parmi O et N », ou alkyle en $C_{1-6}$ substitué par un ou plusieurs $R^{1-8-2}$ ;
$R^{1-8-1}$ et $R^{1-8-2}$ sont indépendamment cyano, halogène, hydroxyle, alcoxy en $C_{1-6}$, -C(=O)$R^{e9}$, -$NR^{e10}R^{e11}$, -C(=O)$OR^{e12}$ ou -C(=O)$NR^{e13}R^{e14}$ ;
$R^{e5}$, $R^{e6}$, $R^{e7}$, $R^{e8}$, $R^{e9}$, $R^{e10}$, $R^{e11}$, $R^{e12}$, $R^{e13}$ et $R^{e14}$ sont indépendamment hydrogène ou alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique, un cycle ponté ou un cycle spiro ;
G est N, C ou CH ;
n vaut 0, 1, 2 ou 3 ;
$R^4$ est indépendamment alkyle en $C_{1-6}$, alkyle en $C_{1-6}$ substitué par un ou plusieurs $R^{4-1}$, oxo, -C(=O)$OR^{4a}$ ou -C(=O)$NR^{4b}R^{4c}$ ;
$R^{4-1}$ est indépendamment halogène, cyano, hydroxyle, alcoxy en $C_{1-6}$, -$NR^{4i}R^{4j}$, -C(=O)$OR^{4d}$ ou -C(=O)$NR^{4e}R^{4f}$ ; $R^{4d}$, $R^{4e}$, $R^{4f}$, $R^{4i}$ et $R^{4j}$ sont indépendamment hydrogène ou alkyle en $C_{1-6}$ ;
$R^{4a}$, $R^{4b}$ et $R^{4c}$ sont indépendamment hydrogène ou alkyle en $C_{1-6}$ ;
$R^2$ est CN, -C(=O)-C($R^a$)=C($R^b$)($R^f$), -C(=O)-C≡C$R^f$, -$S(=O)_2$-C($R^a$)=C($R^b$)($R^f$) ou -$S(=O)_2$-C≡C$R^f$ ;
$R^a$ est indépendamment hydrogène, deutérium, halogène ou alkyle en $C_{1-6}$ ;
$R^b$ et $R^f$ sont indépendamment hydrogène, deutérium, alkyle en $C_{1-6}$, (alkyl en $C_{1-6}$)-C(=O)-, ou alkyle en $C_{1-6}$ substitué par un ou plusieurs $R^{b-1}$ ;
$R^{b-1}$ est indépendamment halogène, hydroxyle, alcoxy en $C_{1-6}$ ou -$NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 10 chaînons contenant 1 à 3 hétéroatomes choisis à partir d'un ou de plusieurs parmi O et N ».

**2.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel $R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ; $R^{1-6}$ et $R^{1-7}$ sont indépendamment halogène, hydroxyle, alkyle en $C_{1-6}$, alcoxy en $C_{1-6}$, cycloalkyle en $C_{3-10}$, alkyle en $C_{1-6}$ substitué par un ou plusieurs $R^{1-6-1}$, ou alcoxy en $C_{1-6}$ substitué par un ou plusieurs $R^{1-6-2}$ ; $R^{1-6-1}$ et $R^{1-6-2}$ sont indépendamment halogène ;

et/ou, m vaut 0 ;
et/ou, $R^3$ est -$OR^{31}$ ou -$NR^{33}R^{34}$ ; $R^{31}$, $R^{33}$ et $R^{34}$ sont indépendamment alkyle en $C_{1-6}$ substitué par un ou plusieurs $R^{31-1}$ ; $R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou -$NR^{e1}R^{e2}$ ; $R^{d15}$ est indépendamment alkyle en $C_{1-6}$, alkyle en $C_{1-6}$ substitué par un ou plusieurs $R^{1-8-1}$, hydroxyle, alcoxy en $C_{1-6}$, halogène, -$NR^{e5}R^{e6}$ ou -C (=O)$NR^{e7}R^{e8}$ ; $R^{1-8-1}$ est indépendamment halogène ; $R^{e5}$, $R^{e6}$, $R^{e7}$ et $R^{e8}$ sont indépendamment hydrogène ou alkyle en $C_{1-6}$ ; $R^{e1}$ et $R^{e2}$ sont indépendamment alkyle en $C_{1-6}$ ;
et/ou le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ou un cycle spiro ; G est N, C ou CH ;
et/ou, n vaut 0 ou 1 ; $R^4$ est indépendamment alkyle en $C_{1-6}$, ou alkyle en $C_{1-6}$ substitué par un ou plusieurs $R^{4-1}$ ;

$R^{4-1}$ est indépendamment cyano ;

et/ou, $R^2$ est -C(=O)-C($R^a$)=C($R^b$)($R^f$), -C(=O)-C≡C-Me ou - S(=O)$_2$-C($R^a$)=C ($R^b$)($R^f$) ; $R^a$ est indépendamment hydrogène ou halogène ; $R^b$ et $R^f$ sont indépendamment hydrogène, alkyle en $C_{1-6}$ ou alkyle en $C_{1-6}$ substitué par un ou plusieurs $R^{b-1}$ ; $R^{b-1}$ est indépendamment halogène, hydroxyle, alcoxy en $C_{1-6}$ ou - $NR^{10j}R^{10k}$ ; $R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 6 chaînons contenant 1 à 3 hétéroatomes choisis à partir d'un ou de plusieurs parmi O et N ».

**3.** Composé hétérocyclique contenant de l'oxygène représenté par la formule I telle que définie dans la revendication 2, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel $R^1$ est aryle en $C_{6-20}$, ou aryle en $C_{6-20}$ substitué par un ou plusieurs $R^{1-6}$ ; $R^{1-6}$ est indépendamment halogène, alkyle en $C_{1-6}$ ou alkyle en $C_{1-6}$ substitué par un ou plusieurs $R^{1-6-1}$ ; $R^{1-6-1}$ est indépendamment halogène ;

et/ou, $R^3$ est $-OR^{31}$ ou $-NR^{33}R^{34}$ ; $R^{31}$, $R^{33}$ et $R^{34}$ sont indépendamment alkyle en $C_{1-6}$ substitué par un ou plusieurs $R^{31-1}$ ; $R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ; $R^{e1}$, $R^{e2}$ et $R^{d15}$ sont indépendamment alkyle en $C_{1-6}$ ;

et/ou le cycle Y est un cycle hétérocyclique à 4 à 12 chaînons contenant 1 à 4 atomes de N ; le cycle hétérocyclique est un cycle hétérocyclique saturé ou un cycle hétérocyclique partiellement saturé ; le cycle hétérocyclique est un cycle monocyclique ; G est N ;

et/ou, n vaut 0 ou 1 ; $R^4$ est indépendamment alkyle en $C_{1-6}$ ou alkyle en $C_{1-6}$ substitué par un ou plusieurs $R^{4-1}$ ; $R^{4-1}$ est indépendamment cyano ;

et/ou, $R^2$ est -C (=O) -C ($R^a$)=C($R^b$)($R^f$) ; $R^a$ est indépendamment hydrogène ou halogène ; $R^b$ et $R^f$ sont indépendamment hydrogène ou alkyle en $C_{1-6}$.

**4.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel,

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ; $R^{1-6}$ et $R^{1-7}$ sont indépendamment halogène, alkyle en $C_{1-6}$ ou alkyle en $C_{1-6}$ substitué par un ou plusieurs $R^{1-6-1}$ ; $R^{1-6-1}$ est indépendamment halogène ;

et/ou $R^3$ est $-OR^{31}$, $-SR^{32}$ ou $-NR^{33}R^{34}$ ; $R^{31}$, $R^{32}$ et $R^{34}$ sont indépendamment alkyle en $C_{1-6}$ substitué par un ou plusieurs $R^{31-1}$ ; $R^{33}$ est indépendamment H, alkyle en $C_{1-6}$ ou alkyle en $C_{1-6}$ substitué par un ou plusieurs $R^{31-1}$ ; $R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ; $R^{d15}$ est indépendamment alkyle en $C_{1-6}$ ou halogène ; $R^{e1}$ et $R^{e2}$ sont indépendamment alkyle en $C_{1-6}$ ;

et/ou n vaut 0 ou 1 ; $R^4$ est indépendamment alkyle en $C_{1-6}$ ou alkyle en $C_{1-6}$ substitué par un ou plusieurs $R^{4-1}$ ; $R^{4-1}$ est indépendamment hydroxyle, cyano ou $-C(=O)NR^{4e}R^{4f}$ ; $R^{4e}$ et $R^{4f}$ sont indépendamment hydrogène ou alkyle en $C_{1-6}$ ;

et/ou $R^2$ est CN, -C(=O)-C($R^a$)=C($R^b$)($R^f$), -C(=O)-C=C-Me ou -S(=O)$_2$-C($R^a$)=C($R^b$)($R^f$) ; $R^a$ est indépendamment hydrogène ou halogène ; $R^b$ et $R^f$ sont indépendamment hydrogène, alkyle en $C_{1-6}$, (alkyl en $C_{1-6}$)-C(=O)- ou alkyle en $C_{1-6}$ substitué par un ou plusieurs $R^{b-1}$ ; $R^{b-1}$ est indépendamment $-NR^{10j}R^{10k}$ ; $R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 6 chaînons contenant 1 à 3 hétéroatomes choisis à partir d'un ou de plusieurs parmi O et N ».

**5.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel,

$R^3$ est $-OR^{31}$ ou $-NR^{33}R^{34}$ ; $R^{31}$, $R^{33}$ et $R^{34}$ sont indépendamment alkyle en $C_{1-6}$ substitué par un ou plusieurs

$R^{31-1}$ ; $R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ; $R^{d15}$ est indépendamment alkyle en $C_{1-6}$ ou halogène ; $R^{e1}$ et $R^{e2}$ sont indépendamment alkyle en $C_{1-6}$ ;
et/ou n vaut 0 ou 1 ; $R^4$ est indépendamment alkyle en $C_{1-6}$ ou alkyle en $C_{1-6}$ substitué par un ou plusieurs $R^{4-1}$ ; $R^{4-1}$ est indépendamment hydroxyle ou cyano ;
et/ou, $R^2$ est-C(=O)-C($R^a$)=C($R^b$)($R^f$), -C(=O)-C≡C-Me ou -S(=O)$_2$-C($R^a$)=C($R^b$)($R^f$) ; $R^a$ est indépendamment hydrogène ou halogène ; $R^b$ et $R^f$ sont indépendamment hydrogène, alkyle en $C_{1-6}$ ou alkyle en $C_{1-6}$ substitué par un ou plusieurs $R^{b-1}$ ; $R^{b-1}$ est indépendamment $-NR^{10j}R^{10k}$ ; $R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « hétérocycloalkyle à 4 à 6 chaînons contenant 1 à 3 hétéroatomes choisis à partir d'un ou de plusieurs parmi O et N ».

**6.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel le composé hétérocyclique contenant de l'oxygène représenté par la formule I est défini comme solution 1, solution 2, solution 3, solution 4, solution 5, solution 6 ou solution 7 :

solution 1 :

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ et $R^{1-7}$ sont indépendamment halogène, hydroxyle, alkyle en $C_{1-6}$, alcoxy en $C_{1-6}$, cycloalkyle en $C_{3-10}$, alkyle en $C_{1-6}$ substitué par un ou plusieurs $R^{1-6-1}$, ou alcoxy en $C_{1-6}$ substitué par un ou plusieurs $R^{1-6-2}$ ;
$R^{1-6-1}$ et $R^{1-6-2}$ sont indépendamment halogène ;
m vaut 0 ;
$R^3$ est $-OR^{31}$ ou $-NR^{33}R^{34}$ ;
$R^{31}$, $R^{33}$ et $R^{34}$ sont indépendamment alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ;
$R^{d15}$ est indépendamment alkyle en $C_{1-6}$, alkyle en $C_{1-6}$ substitué par un ou plusieurs $R^{1-8-1}$, hydroxyle, alcoxy en $C_{1-6}$, halogène, $-NR^{e5}R^{e6}$ ou $-C(=O)NR^{e7}R^{e8}$ ;
$R^{1-8-1}$ est indépendamment halogène ; $R^{e5}$, $R^{e6}$, $R^{e7}$ et $R^{e8}$ sont indépendamment hydrogène ou alkyle en $C_{1-6}$ ;
$R^{e1}$ et $R^{e2}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ou un cycle spiro ;
G est N, C ou CH ;
n vaut 0 ou 1 ;
$R^4$ est indépendamment alkyle en $C_{1-6}$ ou alkyle en $C_{1-6}$ substitué par un ou plusieurs $R^{4-1}$ ;
$R^{4-1}$ est indépendamment cyano ;
$R^2$ est -C(=O)-C($R^a$)=C($R^b$)($R^f$), -C(=O)-C=C-Me ou -S(=O)$_2$-C($R^a$)=C($R^b$)($R^f$) ;
$R^a$ est indépendamment hydrogène ou halogène ;
$R^b$ et $R^f$ sont indépendamment hydrogène, alkyle en $C_{1-6}$ ou alkyle en $C_{1-6}$ substitué par un ou plusieurs $R^{b-1}$ ;
$R^{b-1}$ est indépendamment halogène, hydroxyle, alcoxy en $C_{1-6}$ ou $-NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 6 chaînons contenant 1 à 3 hétéroatomes choisis à partir d'un ou de plusieurs parmi O et N » ;

solution 2 :

$R^1$ est aryle en $C_{6-20}$, ou aryle en $C_{6-20}$ substitué par un ou plusieurs $R^{1-6}$ ;
$R^{1-6}$ est indépendamment halogène, alkyle en $C_{1-6}$ ou alkyle en $C_{1-6}$ substitué par un ou plusieurs $R^{1-6-1}$ ;
$R^{1-6-1}$ est indépendamment halogène ;
m vaut 0 ;
$R^3$ est $-OR^{31}$ ou $-NR^{33}R^{34}$ ;

$R^{31}$, $R^{33}$ et $R^{34}$ sont indépendamment alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ;
$R^{e1}$, $R^{e2}$ et $R^{d15}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ;
G est N ;
n vaut 0 ou 1 ;
$R^4$ est indépendamment alkyle en $C_{1-6}$ ou alkyle en $C_{1-6}$ substitué par un ou plusieurs $R^{4-1}$ ;
$R^{4-1}$ est indépendamment cyano ;
$R^2$ est -C (=O) -C($R^a$)=C($R^b$)($R^f$) ;
$R^a$ est indépendamment hydrogène ou halogène ;
$R^b$ et $R^f$ sont indépendamment hydrogène ou alkyle en $C_{1-}6$ ;

mode de réalisation 3 :

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ et $R^{1-7}$ sont indépendamment halogène, hydroxyle, alkyle en $C_{1-6}$, alcoxy en $C_{1-6}$, cycloalkyle en $C_{3-10}$, alkyle en $C_{1-6}$ substitué par un ou plusieurs $R^{1-6-1}$, ou alcoxy en $C_{1-6}$ substitué par un ou plusieurs $R^{1-6-2}$ ;
$R^{1-6-1}$ et $R^{1-6-2}$ sont indépendamment halogène ;
m vaut 0 ;
$R^3$ est $-OR^{31}$ ou $-NR^{33}R^{34}$ ;
$R^{31}$, $R^{33}$ et $R^{34}$ sont indépendamment alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ;
$R^{d15}$ est indépendamment alkyle en $C_{1-6}$, alkyle en $C_{1-6}$ substitué par un ou plusieurs $R^{1-8-1}$, hydroxyle, alcoxy en $C_{1-6}$, halogène, $-NR^{e5}R^{e6}$ ou -C (=O)$NR^{e7}R^{e8}$ ;
$R^{1-8-1}$ est indépendamment halogène ; $R^{e5}$, $R^{e6}$, $R^{e7}$ et $R^{e8}$ sont indépendamment hydrogène ou alkyle en $C_{1-6}$ ;
$R^{e1}$ et $R^{e2}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ou un cycle spiro ;
G est N, C ou CH ;
n vaut 0 ou 1 ;
$R^4$ est indépendamment alkyle en $C_{1-6}$ ou alkyle en $C_{1-6}$ substitué par un ou plusieurs $R^{4-1}$ ;
$R^{4-1}$ est indépendamment cyano ou hydroxyle ;
$R^2$ est -C(=O)-C($R^a$)=C($R^b$)($R^f$), -C(=O)-C≡C-Me ou -S(=O)$_2$-C($R^a$)=C($R^b$)($R^f$) ;
$R^a$ est indépendamment hydrogène ou halogène ;
$R^b$ et $R^f$ sont indépendamment hydrogène, alkyle en $C_{1-6}$ ou alkyle en $C_{1-6}$ substitué par un ou plusieurs $R^{b-1}$ ;
$R^{b-1}$ est indépendamment halogène, hydroxyle, alcoxy en $C_{1-6}$ ou $-NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 6 chaînons contenant 1 à 3 hétéroatomes choisis à partir d'un ou de plusieurs parmi O et N » ;

solution 4 :

$R^1$ est aryle en $C_{6-20}$ ou aryle en $C_{6-20}$ substitué par un ou plusieurs $R^{1-6}$ ;
$R^{1-6}$ est indépendamment halogène, alkyle en $C_{1-6}$ ou alkyle en $C_{1-6}$ substitué par un ou plusieurs $R^{1-6-1}$ ;
$R^{1-6-1}$ est indépendamment halogène ;
m vaut 0 ;
$R^3$ est $-OR^{31}$ ou $-NR^{33}R^{34}$ ;
$R^{31}$, $R^{33}$ et $R^{34}$ sont indépendamment alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à

partir d'un ou de plusieurs parmi O et N » substitué par un ou plusieurs $R^{d15}$, ou $-NR^{e1}R^{e2}$ ;
$R^{d15}$ est indépendamment alkyle en $C_{1-6}$ ou halogène ;
$R^{e1}$ et $R^{e2}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ;
G est N ;
n vaut 0 ou 1 ;
$R^4$ est indépendamment alkyle en $C_{1-6}$ ou alkyle en $C_{1-6}$ substitué par un ou plusieurs $R^{4-1}$ ;
$R^{4-1}$ est indépendamment cyano ;
$R^2$ est $-C(=O)-C(R^a)=C(R^b)(R^f)$ ;
$R^a$ est indépendamment hydrogène ou halogène ;
$R^b$ et $R^f$ sont indépendamment hydrogène ou alkyle en $C_{1-6}$, ou alkyle en $C_{1-6}$ substitué par un ou plusieurs $R^{b-1}$ ;
$R^{b-1}$ est indépendamment $-NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 10 chaînons contenant 1 à 3 hétéroatomes choisis à partir d'un ou de plusieurs parmi O et N » ;

solution 5 :

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ et $R^{1-7}$ sont indépendamment halogène, hydroxyle, $-C(=O)R^{65}$, $-NR^{63}R^{64}$, $-C(=O)OR^{66}$, $-C(=O)NR^{69}R^{610}$, alkyle en $C_{1-6}$, alcoxy en $C_{1-6}$, cycloalkyle en $C_{3-10}$, « hétérocycloalkyle à 5 à 7 chaînons contenant 1 ou 2 hétéroatomes choisis à partir d'un ou de plusieurs parmi O et N », aryle en $C_{6-20}$, « hétéroaryle à 5 à 7 chaînons contenant 1 ou 2 hétéroatomes choisis à partir d'un ou de plusieurs parmi O et N », alkyle en $C_{1-6}$ substitué par un ou plusieurs $R^{1-6-1}$, alcoxy en $C_{1-6}$ substitué par un ou plusieurs $R^{1-6-2}$, cycloalkyle en $C_{3-10}$ substitué par un ou plusieurs $R^{1-6-3}$, « hétérocycloalkyle à 5 à 7 chaînons contenant 1 ou 2 hétéroatomes choisis à partir d'un ou de plusieurs parmi O et N » substitué par un ou plusieurs $R^{1-6-4}$, aryle en $C_{6-20}$ substitué par un ou plusieurs $R^{1-6-5}$, ou « hétéroaryle à 5 à 7 chaînons contenant 1 ou 2 hétéroatomes choisis à partir d'un ou de plusieurs parmi O et N » substitué par un ou plusieurs $R^{1-6-6}$ ;
$R^{1-6-1}$, $R^{1-6-2}$, $R^{1-6-3}$, $R^{1-6-4}$, $R^{1-6-5}$ et $R^{1-6-6}$ sont indépendamment cyano, halogène, hydroxyle, alcoxy en $C_{1-6}$, alkyle en $C_{1-6}$, $-C(=O)R^{65-2}$, $-NR^{63-2}R^{64-2}$, $-C(=O)OR^{66-2}$ ou $-C(=O)NR^{69-2}R^{610-2}$ ;
$R^{65}$, $R^{65-2}$, $R^{63}$, $R^{63-2}$, $R^{64}$, $R^{64-2}$, $R^{66}$, $R^{66-2}$, $R^{69}$, $R^{69-2}$, $R^{610}$ et $R^{610-}$sont indépendamment hydrogène ou alkyle en $C_{1-6}$ ;
m vaut 0, 1 ou 2 ;
$R^5$ est indépendamment alkyle en $C_{1-6}$ ;
$R^3$ est $-OR^{31}$, $-SR^{32}$ ou $-NR^{33}R^{34}$ ;
$R^{31}$, $R^{32}$, $R^{33}$ et $R^{34}$ sont indépendamment alkyle en $C_{1-6}$ substitué par un ou plusieurs $R^{31-1}$ ;
$R^{31-1}$ est indépendamment cycloalkyle en $C_{3-10}$, « hétérocycloalkyle à 4 à 10 chaînons contenant 1 à 3 hétéroatomes choisis à partir d'un ou de plusieurs parmi O et N », cycloalkyle en $C_{3-10}$ substitué par un ou plusieurs $R^{d16}$, « hétérocycloalkyle à 4 à 10 chaînons contenant 1 à 3 hétéroatomes choisis à partir d'un ou de plusieurs parmi O et N » substitué par un ou plusieurs $R^{d15}$, $-OR^d$, $-SR^{d1}$, $-NR^{e1}R^{e2}$ ou $-C(=O)NR^{e3}R^{e4}$ ;
$R^{d15}$ et $R^{d16}$ sont indépendamment alkyle en $C_{1-6}$, alkyle en $C_{1-6}$ substitué par un ou plusieurs $R^{1-8-1}$, hydroxyle, alcoxy en $C_{1-6}$, halogène, $-NR^{e5}R^{e6}$ ou $-C(=O)NR^{e7}R^{e8}$ ;
$R^d$, $R^{d1}$, $R^{e1}$, $R^{e2}$, $R^{e3}$ et $R^{e4}$ sont indépendamment hydrogène, alkyle en $C_{1-6}$, cycloalkyle en $C_{3-10}$, « hétérocycloalkyle à 4 à 10 chaînons contenant 1 à 3 hétéroatomes choisis à partir d'un ou de plusieurs parmi O et N », ou alkyle en $C_{1-6}$ substitué par un ou plusieurs $R^{1-8-2}$ ;
$R^{1-8-1}$ et $R^{1-8-2}$ sont indépendamment cyano, halogène, hydroxyle, alcoxy en $C_{1-6}$, $-C(=O)R^{e9}$, $-NR^{e10}R^{e11}$, $-C(=O)OR^{e12}$ ou $-C(=O)NR^{e13}R^{e14}$ ;
$R^{e5}$, $R^{e6}$, $R^{e7}$, $R^{e8}$, $R^{e9}$, $R^{e10}$, $R^{e11}$, $R^{e12}$, $R^{e13}$ et $R^{e14}$ sont indépendamment hydrogène ou alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique, un cycle ponté ou un cycle spiro ;

G est N, C ou CH ;
n vaut 0, 1, 2 ou 3 ;
$R^4$ est indépendamment alkyle en $C_{1-6}$, alkyle en $C_{1-6}$ substitué par un ou plusieurs $R^{4-1}$, oxo, -C (=O) $OR^{4a}$ ou - C(=O)$NR^{4b}R^{4c}$ ;
$R^{4-1}$ est indépendamment halogène, cyano, hydroxyle, alcoxy en $C_{1-6}$, -$NR^{4i}R^{4j}$, -C(=O)$OR^{4d}$ ou -C(=O)$NR^{4e}R^{4f}$ ; $R^{4d}$, $R^{4e}$, $R^{4f}$, $R^{4i}$ et $R^{4j}$ sont indépendamment hydrogène ou alkyle en $C_{1-}6$ ;
$R^{4a}$, $R^{4b}$ et $R^{4c}$ sont indépendamment hydrogène ou alkyle en C1-6 ;
$R^2$ est -C(=O)-C($R^a$)=C($R^b$)($R^f$), -C(=O)-C≡C$R^r$, -S(=O)$_2$-C($R^a$)=C($R^b$)($R^f$) ou -S(=O)$_2$-C≡C$R^f$ ;
$R^a$ est indépendamment hydrogène, deutérium, halogène ou alkyle en $C_{1-6}$ ;
$R^b$ et $R^f$ sont indépendamment hydrogène, deutérium, alkyle en $C_{1-6}$ ou alkyle en $C_{1-6}$ substitué par un ou plusieurs $R^{b-1}$ ;
$R^{b-1}$ est indépendamment halogène, hydroxyle, alcoxy en $C_{1-6}$ ou -$NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 10 chaînons contenant 1 à 3 hétéroatomes choisis à partir d'un ou de plusieurs parmi O et N » ;

solution 6 :

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ et $R^{1-7}$ sont indépendamment halogène, hydroxyle, alkyle en $C_{1-6}$, alcoxy en $C_{1-6}$, cycloalkyle en C3-10, alkyle en $C_{1-6}$ substitué par un ou plusieurs $R^{1-6-1}$, ou alcoxy en $C_{1-6}$ substitué par un ou plusieurs $R^{1-6-2}$ ;
$R^{1-6-1}$ et $R^{1-6-2}$ sont indépendamment halogène ;
m vaut 0 ;
$R^3$ est -$OR^{31}$, -$SR^{32}$ ou -$NR^{33}R^{34}$ ;
$R^{31}$, $R^{32}$ et $R^{34}$ sont indépendamment alkyle en $C_{1-6}$ substitué par un ou plusieurs $R^{31-1}$ ; $R^{33}$ est indépendamment H, alkyle en $C_{1-6}$ ou alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou -$NR^{e1}R^{e2}$ ;
$R^{d15}$ est indépendamment alkyle en $C_{1-6}$ ou halogène ;
$R^{e1}$ et $R^{e2}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ou un cycle spiro ;
G est N, C ou CH ;
n vaut 0 ou 1 ;
$R^4$ est indépendamment alkyle en $C_{1-6}$ ou alkyle en $C_{1-6}$ substitué par un ou plusieurs $R^{4-1}$ ;
$R^{4-1}$ est indépendamment cyano, hydroxyle ou -C(=O)$NR^{4e}R^{4f}$ ; $R^{4e}$ et $R^{4f}$ sont indépendamment hydrogène ou alkyle en $C_{1-6}$ ;
$R^2$ est CN, -C(=O)-C($R^a$)=C($R^b$)($R^f$), -C(=O)-C≡C-Me ou - S(=O)$_2$-C($R^a$)=C($R^b$)($R^f$) ;
$R^a$ est indépendamment hydrogène ou halogène ;
$R^b$ et $R^f$ sont indépendamment hydrogène, alkyle en $C_{1-6}$, alkyle en $C_{1-6}$ -C(=O)- ou alkyle en $C_{1-6}$ substitué par un ou plusieurs $R^{b-1}$ ;
$R^{b-1}$ est indépendamment halogène, hydroxyle, alcoxy en $C_{1-6}$ ou -$NR^{10j}R^{10k}$ ;
$R^{10j}$ et $R^{10k}$ sont indépendamment hydrogène ou alkyle en $C_{1-6}$, ou $R^{10j}$ et $R^{10k}$ pris ensemble avec l'atome N auquel ils sont attachés forment « un hétérocycloalkyle à 4 à 6 chaînons contenant 1 à 3 hétéroatomes choisis à partir d'un ou de plusieurs parmi O et N » ;

solution 7 :

$R^1$ est aryle en $C_{6-20}$, « hétéroaryle à 5 à 12 chaînons contenant 1 à 4 hétéroatomes choisis à partir d'un ou de plusieurs parmi O, S et N », aryle en $C_{6-20}$ substitué par un ou plusieurs $R^{1-6}$, ou « hétéroaryle à 5 à 12 chaînons contenant 1 à 4 hétéroatomes choisis à partir d'un ou de plusieurs parmi O, S et N » substitué par un ou plusieurs $R^{1-7}$ ;
$R^{1-6}$ est indépendamment halogène, alkyle en $C_{1-6}$ ou alkyle en $C_{1-6}$ substitué par un ou plusieurs $R^{1-6-1}$ ;
$R^{1-6-1}$ est indépendamment halogène ;

m vaut 0 ;
$R^3$ est $-OR^{31}$, $-SR^{32}$ ou $-NR^{33}R^{34}$ ;
$R^{31}$, $R^{32}$ et $R^{34}$ sont indépendamment alkyle en $C_{1-6}$ substitué par un ou plusieurs $R^{31-1}$ ; $R^{33}$ est indépendamment H, alkyle en $C_{1-6}$ ou alkyle en $C_{1-6}$ substitué par un ou plusieurs $R^{31-1}$ ;
$R^{31-1}$ est indépendamment « hétérocycloalkyle à 5 à 7 chaînons contenant 1 à 3 hétéroatomes choisis à partir d'un ou de plusieurs parmi O et N » substitué par un ou plusieurs $R^{d15}$, ou $-NR^{e1}R^{e2}$ ;
$R^{d15}$ est indépendamment alkyle en $C_{1-6}$ ou halogène ;
$R^{e1}$ et $R^{e2}$ sont indépendamment alkyle en $C_{1-6}$ ;
le cycle Y est un cycle hétérocyclique à 4 à 12 chaînons contenant 1 à 4 atomes N ; le cycle hétérocyclique est un cycle hétérocyclique saturé ou un cycle hétérocyclique partiellement saturé ; le cycle hétérocyclique est un cycle monocyclique ;
G est N ;
n vaut 0 ou 1 ;
$R^4$ est indépendamment alkyle en $C_{1-6}$ substitué par un ou plusieurs $R^{4-1}$ ;
$R^{4-1}$ est indépendamment cyano ou $-C(=O)NR^{4e}R^{4f}$ ; $R^{4e}$ et $R^{4f}$ sont indépendamment hydrogène ou alkyle en $C_{1-6}$ ;
$R^2$ est CN, $-C(=O)-C(R^a)=C(R^b)(R^f)$ ;
$R^a$ est indépendamment hydrogène ou halogène ;
$R^b$ et $R^f$ sont indépendamment hydrogène, alkyle en $C_{1-6}$ ou alkyle en $C_{1-6}$ -C(=O)-.

7. Composé hétérocyclique contenant de l'oxygène représenté par la formule I telle que définie dans l'une quelconque des revendications 1 à 6, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel le composé hétérocyclique contenant de l'oxygène représenté par la formule I a une

structure comme suit :

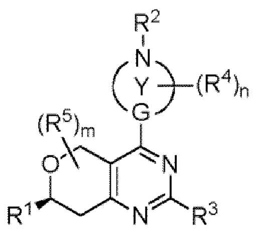
I-2

,

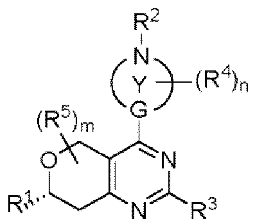
I-3

ou « un mélange de

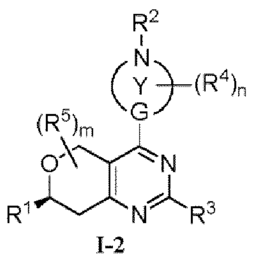
I-2

et

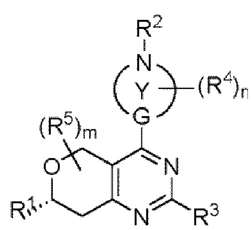
I-3

avec un rapport molaire de, par exemple, 1 : 1 » ;
et/ou, lorsque $R^1$ est aryle en $C_{6-20}$, alors l'aryle en $C_{6-20}$ est phényle ou naphtyle ;
et/ou, lorsque $R^1$ est « hétéroaryle à 5 à 12 chaînons contenant 1 à 4 hétéroatomes choisis à partir d'un ou de plusieurs parmi O, S et N », alors « l'hétéroaryle à 5 à 12 chaînons contenant 1 à 4 hétéroatomes choisis à partir d'un ou de plusieurs parmi O, S et N » est « hétéroaryle à 9 à 10 chaînons contenant 1 à 4 hétéroatomes choisis à partir d'un ou de plusieurs parmi O, S et N » ;
et/ou, lorsque $R^1$ est aryle en $C_{6-20}$ substitué par un ou plusieurs $R^{1-6}$, alors l'aryle en $C_{6-20}$ est phényle ou naphtyle ;

et/ou, lorsque $R^1$ est aryle en $C_{6-20}$ substitué par un ou plusieurs $R^{1-6}$, alors les plusieurs $R^{1-6}$ sont deux ou trois $R^{1-6}$ ;

et/ou, lorsque $R^{1-6}$ est indépendamment halogène, alors l'halogène est le fluor, le chlore, le brome ou l'iode ;

et/ou, lorsque $R^{1-6}$ est indépendamment alkyle en $C_{1-6}$, alors l'alkyle en $C_{1-6}$ est alkyle en $C_{1-4}$ ;

et/ou, lorsque $R^{1-6}$ est indépendamment alkyle en $C_{1-6}$ substitué par un ou plusieurs $R^{1-6-1}$, alors l'alkyle en $C_{1-6}$ est alkyle en $C_{1-4}$ ;

et/ou, lorsque $R^{1-6}$ est indépendamment alkyle en $C_{1-6}$ substitué par un ou plusieurs $R^{1-6-1}$, les plusieurs $R^{1-6-1}$ sont deux ou trois $R^{1-6-1}$ ;

et/ou, lorsque $R^{1-6-1}$ est indépendamment halogène, alors l'halogène est le fluor, le chlore, le brome ou l'iode ;

et/ou, lorsque $R^{31}$, $R^{33}$ et $R^{34}$ sont indépendamment alkyle en $C_{1-6}$ substitué par un ou plusieurs $R^{31-1}$, alors l'alkyle en $C_{1-6}$ est alkyle en $C_{1-4}$ ;

et/ou, lorsque $R^{31}$, $R^{33}$ et $R^{34}$ sont indépendamment alkyle en $C_{1-6}$ substitué par un ou plusieurs $R^{31-1}$, alors les plusieurs $R^{31-1}$ sont deux ou trois $R^{31-1}$ ;

et/ou, lorsque $R^{31-1}$ est indépendamment « hétérocycloalkyle à 4 à 10 chaînons contenant 1 à 3 hétéroatomes choisis à partir d'un ou de plusieurs parmi O et N » substitué par un ou plusieurs $R^{d15}$, alors « l'hétérocycloalkyle à 4 à 10 chaînons contenant 1 à 3 hétéroatomes choisis à partir d'un ou de plusieurs parmi O et N » est « hétérocycloalkyle à 5 à 7 chaînons contenant 1 à 3 hétéroatomes choisis à partir d'un ou de plusieurs parmi O et N » ;

et/ou, lorsque $R^{31-1}$ est indépendamment « hétérocycloalkyle à 4 à 10 chaînons contenant 1 à 3 hétéroatomes choisis à partir d'un ou de plusieurs parmi O et N » substitué par un ou plusieurs $R^{d15}$, alors les plusieurs $R^{d15}$ sont deux ou trois $R^{d15}$ ;

et/ou, lorsque $R^{d15}$ est indépendamment alkyle en $C_{1-6}$, alors l'alkyle en $C_{1-6}$ est alkyle en $C_{1-4}$ ;

et/ou, lorsque $R^{d15}$ est indépendamment halogène, alors l'halogène est le fluor, le chlore, le brome ou l'iode ;

et/ou, lorsque $R^{e1}$ et $R^{e2}$ sont indépendamment alkyle en $C_{1-6}$, alors l'alkyle en $C_{1-6}$ est alkyle en $C_{1-4}$ ;

et/ou, lorsque le cycle Y est un cycle hétérocyclique à 4 à 12 chaînons contenant 1 à 4 atomes N, alors le cycle hétérocyclique à 4 à 12 chaînons contenant 1 à 4 atomes N est un cycle hétérocyclique à 6 à 9 chaînons contenant 1 à 2 atomes N ;

et/ou, lorsque $R^4$ est indépendamment alkyle en $C_{1-6}$, alors l'alkyle en $C_{1-6}$ est alkyle en $C_{1-4}$ ;

et/ou, lorsque $R^4$ est indépendamment alkyle en $C_{1-6}$ substitué par un ou plusieurs $R^{4-1}$, alors l'alkyle en $C_{1-6}$ est alkyle en $C_{1-4}$ ;

et/ou, lorsque $R^4$ est indépendamment alkyle en $C_{1-6}$ substitué par un ou plusieurs $R^{4-1}$, alors les plusieurs $R^{4-1}$ sont deux ou trois $R^{4-1}$ ;

et/ou, lorsque $R^a$ est indépendamment halogène, alors l'halogène est le fluor, le chlore, le brome ou l'iode ;

et/ou, lorsque $R^b$ et $R^f$ sont indépendamment alkyle en $C_{1-6}$, alors l'alkyle en $C_{1-6}$ est alkyle en $C_{1-4}$ ;

et/ou, lorsque $R^b$ et $R^f$ sont indépendamment alkyle en $C_{1-6}$ substitué par un ou plusieurs $R^{b-1}$, alors l'alkyle en $C_{1-6}$ est alkyle en $C_{1-4}$ ;

et/ou, lorsque $R^b$ et $R^f$ sont indépendamment alkyle en $C_{1-6}$ substitué par un ou plusieurs $R^{b-1}$, alors les plusieurs $R^{b-1}$ sont deux ou trois $R^{b-1}$ ;

et/ou, lorsque $R^{10j}$ et $R^{10k}$ sont indépendamment alkyle en $C_{1-6}$, alors l'alkyle en $C_{1-6}$ est alkyle en $C_{1-4}$.

**8.** Composé hétérocyclique contenant de l'oxygène représenté par la formule I telle que définie dans la revendication 7, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel, lorsque $R^1$ est « hétéroaryle à 5 à 12 chaînons contenant 1 à 4 hétéroatomes choisis à partir d'un ou de plusieurs parmi O, S et N », alors « l'hétéroaryle à 5 à 12 chaînons contenant 1 à 4 hétéroatomes choisis à partir d'un ou de plusieurs parmi O, S et N » est isoquinolyle ;

et/ou, lorsque $R^1$ est aryle en $C_{6-20}$ substitué par un ou plusieurs $R^{1-6}$, alors l'aryle en $C_{6-20}$ est phényle ou 1-naphtyle ;

et/ou, lorsque $R^{1-6}$ est indépendamment halogène, alors l'halogène est le fluor ou le chlore ;

et/ou, lorsque $R^{1-6}$ est indépendamment alkyle en $C_{1-6}$, alors l'alkyle en $C_{1-6}$ est méthyle, éthyle, n-propyle, isopropyle, n-butyle, isobutyle, sec-butyle ou tert-butyle ;

et/ou, lorsque $R^{1-6}$ est indépendamment alkyle en $C_{1-6}$ substitué par un ou plusieurs $R^{1-6-1}$, alors l'alkyle en $C_{1-6}$ est méthyle, éthyle, n-propyle, isopropyle, n-butyle, isobutyle, sec-butyle ou tert-butyle ;

et/ou, lorsque $R^{1-6-1}$ est indépendamment halogène, alors l'halogène est le fluor ;

et/ou, lorsque $R^{31}$, $R^{33}$ et $R^{34}$ sont indépendamment alkyle en $C_{1-6}$ substitué par un ou plusieurs $R^{31-1}$, alors l'alkyle en $C_{1-6}$ est méthyle, éthyle, n-propyle, isopropyle, n-butyle, isobutyle, sec-butyle ou tert-butyle ;

et/ou, lorsque $R^{31-1}$ est indépendamment « hétérocycloalkyle à 4 à 10 chaînons contenant 1 à 3 hétéroatomes

choisis à partir d'un ou de plusieurs parmi O et N » substitué par un ou plusieurs $R^{d15}$, alors « l'hétérocycloalkyle à 4 à 10 chaînons contenant 1 à 3 hétéroatomes choisis à partir d'un ou de plusieurs parmi O et N » est « hétérocycloalkyle à 5 à 7 chaînons contenant 1 hétéroatome choisi parmi l'un de O et N » ;
et/ou, lorsque $R^{d15}$ est indépendamment alkyle en $C_{1-6}$, alors l'alkyle en $C_{1-6}$ est méthyle, éthyle, n-propyle, isopropyle, n-butyle, isobutyle, sec-butyle ou tert-butyle ;
et/ou, lorsque $R^{d15}$ est indépendamment halogène, alors l'halogène est le fluor ;
et/ou, lorsque $R^{e1}$ et $R^{e2}$ sont indépendamment alkyle en $C_{1-6}$, alors l'alkyle en $C_{1-6}$ est méthyle, éthyle, n-propyle, isopropyle, n-butyle, isobutyle, sec-butyle ou tert-butyle ;
et/ou, lorsque le cycle Y est un cycle hétérocyclique à 4 à 12 chaînons contenant 1 à 4 atomes N, alors le cycle hétérocyclique à 4 à 12 chaînons contenant 1 à 4 atomes N est

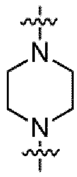 , 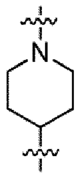 , 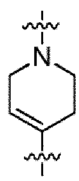 ou 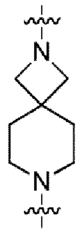

qui, à son extrémité supérieure, est connectée à $R^2$ ;
et/ou, lorsque $R^4$ est indépendamment alkyle en $C_{1-6}$, alors l'alkyle en $C_{1-6}$ est méthyle, éthyle, n-propyle, isopropyle, n-butyle, isobutyle, sec-butyle ou tert-butyle ;
et/ou, lorsque $R^4$ est indépendamment alkyle en $C_{1-6}$ substitué par un ou plusieurs $R^{4-1}$, alors l'alkyle en $C_{1-6}$ est méthyle, éthyle, n-propyle, isopropyle, n-butyle, isobutyle, sec-butyle ou tert-butyle ;
et/ou, lorsque $R^a$ est indépendamment halogène, alors l'halogène est le fluor ;
et/ou, lorsque $R^b$ et $R^f$ sont indépendamment alkyle en $C_{1-6}$, alors l'alkyle en $C_{1-6}$ est méthyle, éthyle, n-propyle, isopropyle, n-butyle, isobutyle, sec-butyle ou tert-butyle ;
et/ou, lorsque $R^b$ et $R^f$ sont indépendamment alkyle en $C_{1-6}$ substitué par un ou plusieurs $R^{b-1}$, alors l'alkyle en $C_{1-6}$ est méthyle, éthyle, n-propyle, isopropyle, n-butyle, isobutyle, sec-butyle ou tert-butyle ;
et/ou, lorsque $R^{10j}$ et $R^{10k}$ sont indépendamment alkyle en $C_{1-6}$, alors l'alkyle en $C_{1-6}$ est méthyle, éthyle, n-propyle, isopropyle, n-butyle, isobutyle, sec-butyle ou tert-butyle.

**9.** Composé hétérocyclique contenant de l'oxygène représenté par la formule I telle que définie dans la revendication 8, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel, lorsque $R^1$ est « hétéroaryle à 5 à 12 chaînons contenant 1 à 4 hétéroatomes choisis à partir d'un ou de plusieurs parmi O, S et N », alors « l'hétéroaryle à 5 à 12 chaînons contenant 1 à 4 hétéroatomes choisis à partir d'un ou de plusieurs parmi O, S et N » est

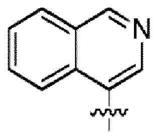 ;

et/ou, lorsque $R^{1-6}$ est indépendamment alkyle en $C_{1-6}$, alors l'alkyle en $C_{1-6}$ est méthyle ;
et/ou, lorsque $R^{1-6}$ est indépendamment alkyle en $C_{1-6}$ substitué par un ou plusieurs $R^{1-6-1}$, alors l'alkyle en $C_{1-6}$ substitué par un ou plusieurs $R^{1-6-1}$ est trifluorométhyle ;
et/ou, lorsque $R^{31}$, $R^{33}$ et $R^{34}$ sont indépendamment alkyle en $C_{1-6}$ substitué par un ou plusieurs $R^{31-1}$, alors l'alkyle en $C_{1-6}$ est méthyle, éthyle, n-propyle ou isopropyle ;
et/ou, lorsque $R^{31-1}$ est indépendamment « hétérocycloalkyle à 4 à 10 chaînons contenant 1 à 3 hétéroatomes choisis à partir d'un ou de plusieurs parmi O et N » substitué par un ou plusieurs $R^{d15}$, alors « l'hétérocycloalkyle à 4 à 10 chaînons contenant 1 à 3 hétéroatomes choisis à partir d'un ou de plusieurs parmi O et N » est tétrahydropyrrolyle ;
et/ou, lorsque $R^{d15}$ est indépendamment alkyle en $C_{1-6}$, alors l'alkyle en $C_{1-6}$ est méthyle ;
et/ou, lorsque $R^{e1}$ et $R^{e2}$ sont indépendamment alkyle en $C_{1-6}$, alors l'alkyle en $C_{1-6}$ est méthyle ou éthyle ;
et/ou, le composé hétérocyclique contenant de l'oxygène représenté par la formule I

a une structure comme suit :

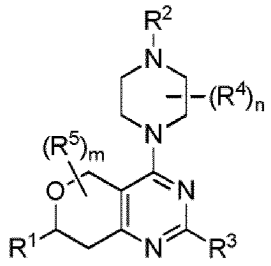

II

;

et/ou, lorsque $R^4$ est indépendamment alkyle en $C_{1-6}$, alors l'alkyle en $C_{1-6}$ est méthyle ;
et/ou, lorsque $R^4$ est indépendamment alkyle en $C_{1-6}$ substitué par un ou plusieurs $R^{4-1}$, alors l'alkyle en $C_{1-6}$ est méthyle ;
et/ou, lorsque $R^b$ et $R^f$ sont indépendamment alkyle en $C_{1-6}$, alors l'alkyle en $C_{1-6}$ est méthyle ;
et/ou, lorsque $R^b$ et $R^f$ sont indépendamment alkyle en $C_{1-6}$ substitué par un ou plusieurs $R^{b-1}$, alors l'alkyle en $C_{1-6}$ est méthyle ;
et/ou, lorsque $R^{10j}$ et $R^{10k}$ sont indépendamment alkyle en $C_{1-6}$, alors l'alkyle en $C_{1-6}$ est méthyle.

**10.** Composé hétérocyclique contenant de l'oxygène représenté par la formule I telle que définie dans la revendication 9, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel,

le composé hétérocyclique contenant de l'oxygène représenté par la formule I a une structure comme suit :

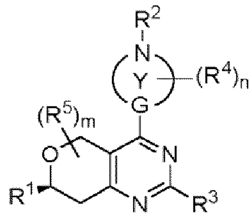

I-2

;

et/ou, lorsque $R^{33}$ est indépendamment alkyle en $C_{1-6}$,
alors l'alkyle en $C_{1-6}$ est méthyle, éthyle, n-propyle, isopropyle, n-butyle, isobutyle, sec-butyle ou tert-butyle, ou peut être méthyle, éthyle, n-propyle ou isopropyle ;
et/ou, lorsque $R^4$ est indépendamment alkyle en $C_{1-6}$ substitué par un ou plusieurs $R^{4-1}$, alors l'alkyle en $C_{1-6}$ substitué par un ou plusieurs $R^{4-1}$ est hydroxyméthyle, cyanométhyle
ou

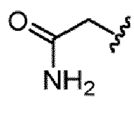

;

et/ou, lorsque $R^b$ et $R^f$ sont indépendamment (alkyl en $C_{1-6}$)-C(=O)-, alors l'alkyle en $C_{1-6}$ dans l'(alkyl en $C_{1-6}$) C(=O)-est méthyle, éthyle, n-propyle, isopropyle, n-butyle, isobutyle, sec-butyle ou tert-butyle, ou peut être méthyle ;
et/ou, $R^2$ est CN,

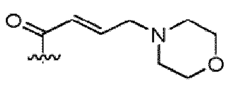, 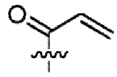, 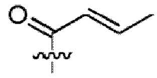, 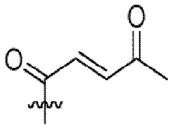,

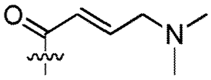, 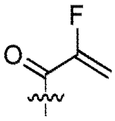, 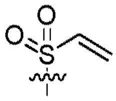 ou 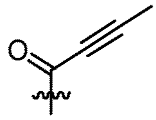.

**11.** Composé hétérocyclique contenant de l'oxygène représenté par la formule I telle que définie dans la revendication 9, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel,

lorsque $R^1$ est aryle en $C_{6-20}$ substitué par un ou plusieurs $R^{1-6}$, alors l'aryle en $C_{6-20}$ substitué par un ou plusieurs $R^{1-6}$ est

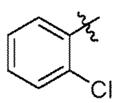, 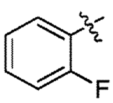, 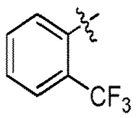, 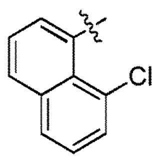 ou 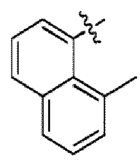;

et/ou, lorsque $R^4$ est indépendamment alkyle en $C_{1-6}$ substitué par un ou plusieurs $R^{4-1}$, alors l'alkyle en $C_{1-6}$ substitué par un ou plusieurs $R^{4-1}$ est hydroxyméthyle ou cyanométhyle ;

et/ou, lorsque $R^{10j}$ et $R^{10k}$ pris ensemble avec l'atome N auquel ils sont attachés forment « hétérocycloalkyle à 4 à 10 chaînons contenant 1 à 3 hétéroatomes choisis à partir d'un ou de plusieurs parmi O et N », alors « l'hétérocycloalkyle à 4 à 10 chaînons contenant 1 à 3 hétéroatomes choisis à partir d'un ou de plusieurs parmi O et N » est « hétérocycloalkyle à 5 à 6 chaînons contenant 2 hétéroatomes choisis parmi O et N », ou peut être

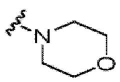;

et/ou, $R^2$ est

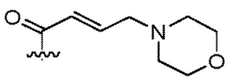, 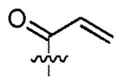, 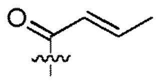, 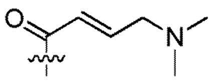,

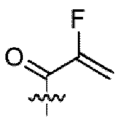, 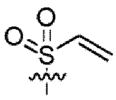 ou 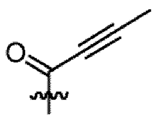.

**12.** Composé hétérocyclique contenant de l'oxygène représenté par la formule I telle que définie dans la revendication 9, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel, lorsque $R^1$ est aryle en $C_{6-20}$ substitué par un ou plusieurs $R^{1-6}$, alors l'aryle en $C_{6-20}$ substitué par un ou plusieurs $R^{1-6}$ est

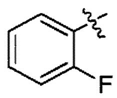, 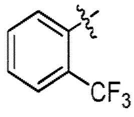, 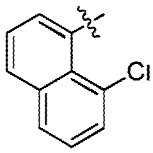 ou 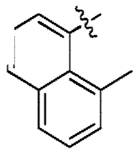 or ;

et/ou, lorsque $R^{31}$, $R^{33}$ et $R^{34}$ sont indépendamment alkyle en $C_{1-6}$ substitué par un ou plusieurs $R^{31-1}$, alors l'alkyle en $C_{1-6}$ substitué par un ou plusieurs $R^{31-1}$ est

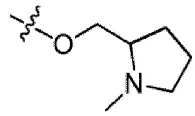,

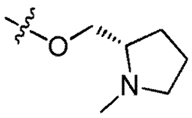, 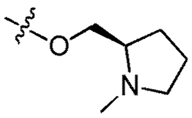, 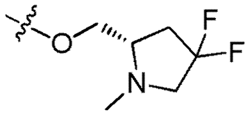, 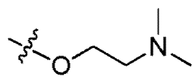,

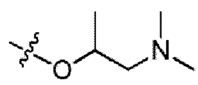, 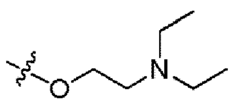, 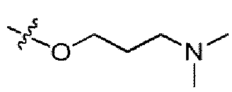 ou 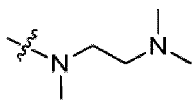;

et/ou, lorsque $R^4$ est indépendamment alkyle en $C_{1-6}$ substitué par un ou plusieurs $R^{4-1}$, alors l'alkyle en $C_{1-6}$ substitué par un ou plusieurs $R^{4-1}$ est cyanométhyle ;
et/ou, $R^2$ est

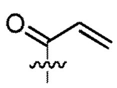, 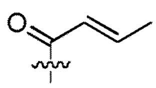, 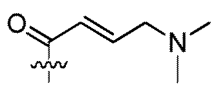, 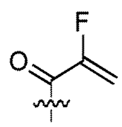, 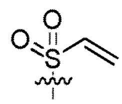 ou 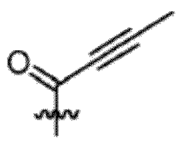.

**13.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel le composé hétérocyclique contenant de l'oxygène représenté par la formule I a l'une quelconque des structures suivantes :

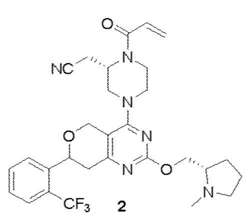
2

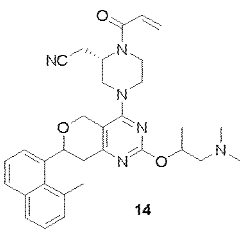
14

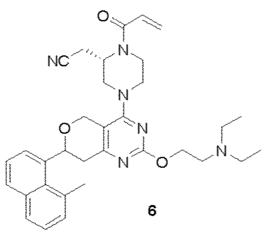
6

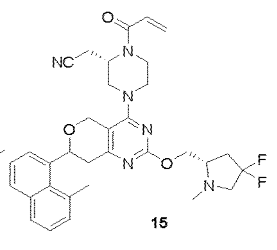
15

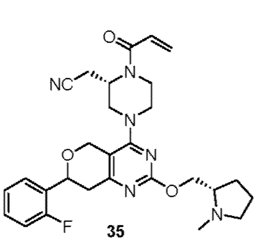
35

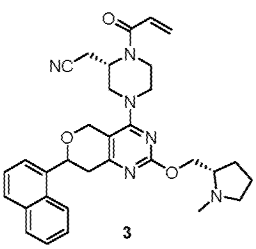
3

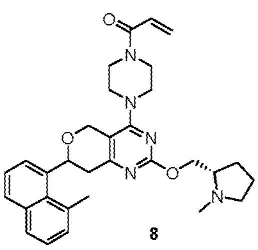
8

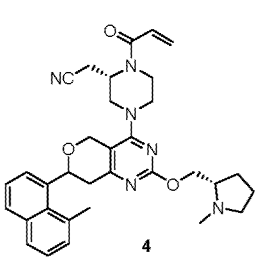
4

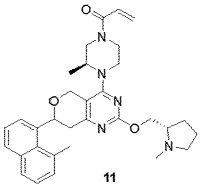
11

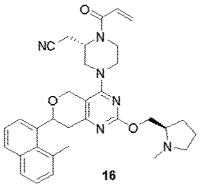
16

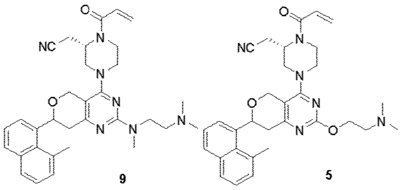
9

5

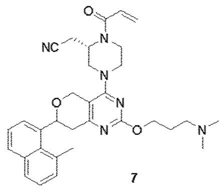
7

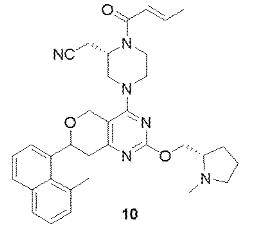
10

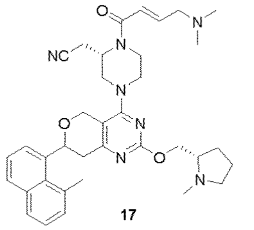
17

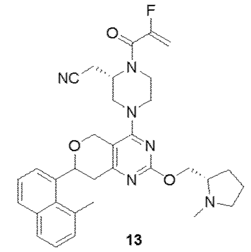
13

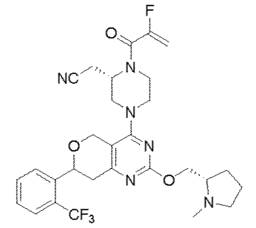
18

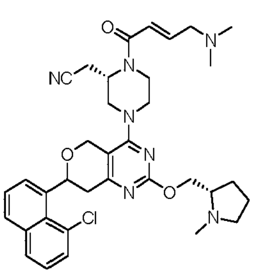

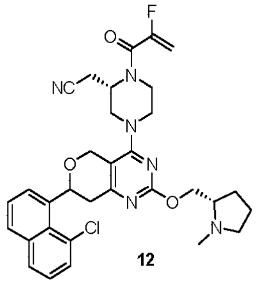
12

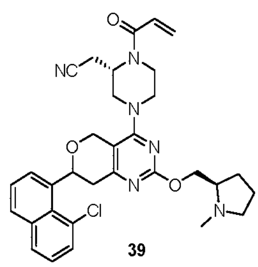
39

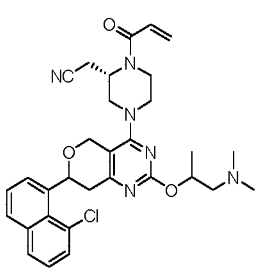

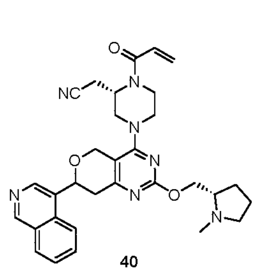
40

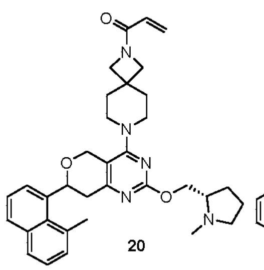
20

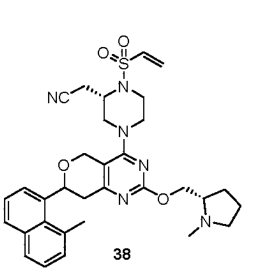
38

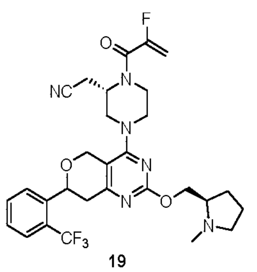
19

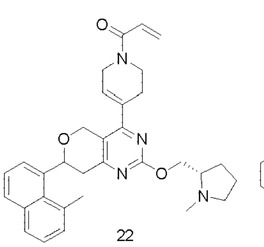
22

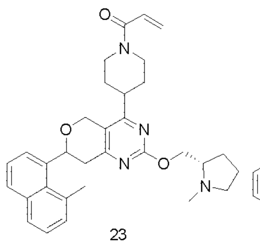
23

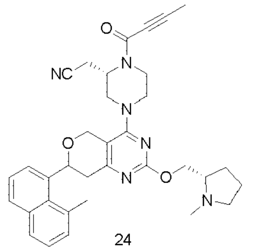
24

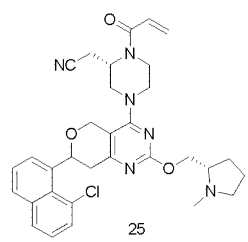
25

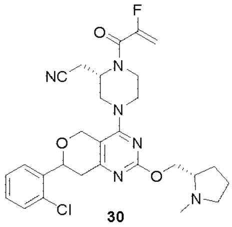
30

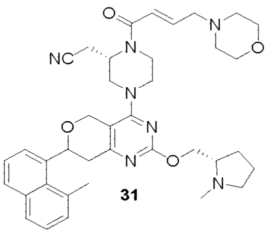
31

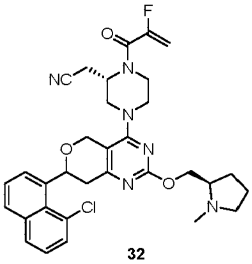
32

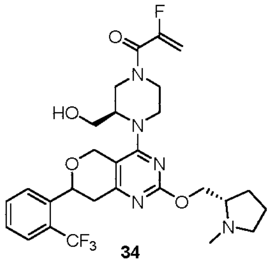
34

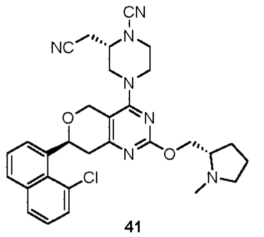
41

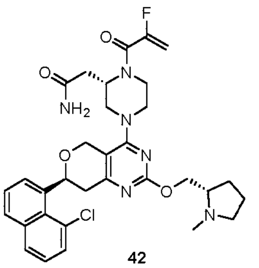
42

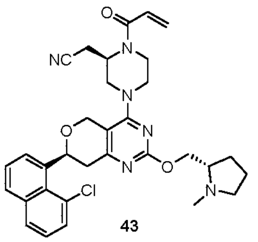
43

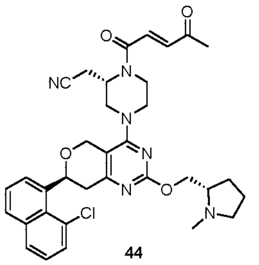
44

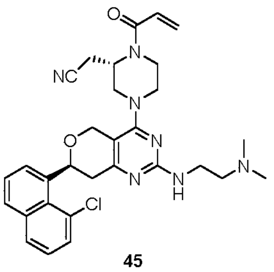
45

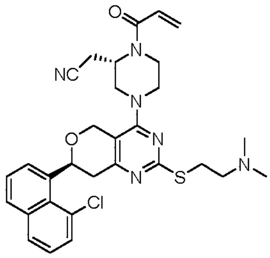
46

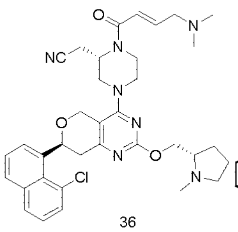
36

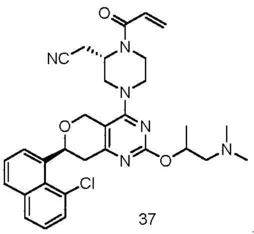
37

**14.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel le composé hétérocyclique contenant de l'oxygène représenté par la formule I est l'un quelconque des composés suivants :

composé

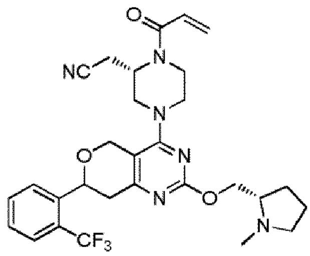

qui a un temps de rétention de 0,92 min dans les conditions suivantes : équipement : Station de méthode SFC (Thar, Waters) ; colonne chromatographique : OD-H 4,6 * 100 mm, 5 um (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

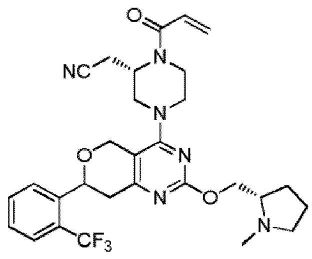

qui a un temps de rétention de 2,74 min dans les conditions suivantes : équipement : Station de méthode SFC (Thar, Waters) ; colonne chromatographique : OD-H 4,6 * 100 mm, 5 um (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

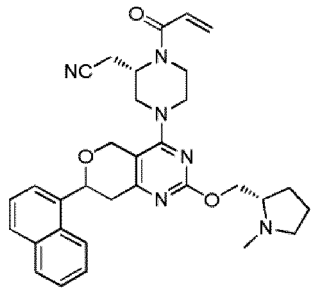

qui a un temps de rétention de 0,97 min dans les conditions suivantes : équipement : Station de méthode SFC (Thar, Waters) ; colonne chromatographique : AD-H 4,6 * 100 mm, 5 um (Daicel) ; température de la colonne : 40 °C ; phase mobile : $CO_2$/ETOH (0,5 % TEA) = 55/45 ; débit : 4,0 ml/min ; longueur d'onde : 254 nm ; contre-pression : 120 bar ;
composé

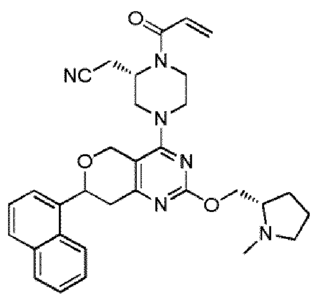

qui a un temps de rétention de 2,40 min dans les conditions suivantes : équipement : Station de méthode SFC (Thar, Waters) ; colonne chromatographique : AD-H 4,6 * 100 mm, 5 um (Daicel) ; température de la colonne : 40 °C ; phase mobile : $CO_2$/ETOH (0,5 % TEA) = 55/45 ; débit : 4,0 ml/min ; longueur d'onde : 254 nm ; contre-pression : 120 bar ;
composé

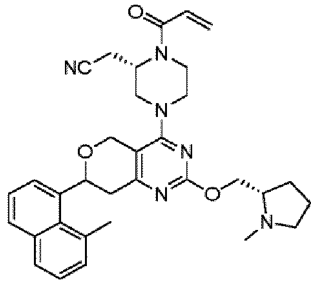

qui a un temps de rétention de 0,97 min dans les conditions suivantes : équipement : Station de méthode SFC (Thar, Waters) ; colonne chromatographique : OJ-H 4,6 * 100 mm, 5 um (Daicel) ; température de la colonne : 40 °C ; phase mobile : $CO_2$/Méthanol (0,1 % TEA) = 60/40 ; débit : 4,0 ml/min ; longueur d'onde : 254 nm ; contre-pression : 120 bar ;
composé

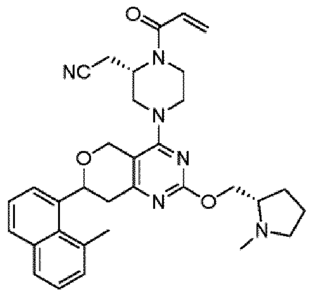

qui a un temps de rétention de 1,94 min dans les conditions suivantes : équipement : Station de méthode SFC (Thar, Waters) ; colonne chromatographique : OJ-H 4,6 * 100 mm, 5 um (Daicel) ; température de la colonne : 40 °C ; phase mobile : $CO_2$/Méthanol (0,1 % TEA) = 60/40 ; débit : 4,0 ml/min ; longueur d'onde : 254 nm ; contre-pression : 120 bar ;
composé

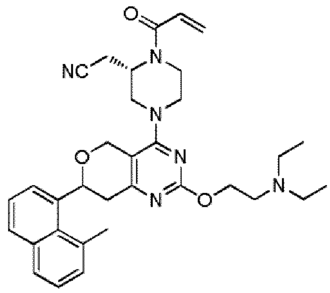

qui a un temps de rétention de 1,22 min dans les conditions suivantes : équipement : Station de méthode SFC (Thar, Waters) ; colonne chromatographique : CHIRALCEL OJ-H 4,6 * 100 mm, 5 um (Daicel) ; température de la colonne : 40 °C ; phase mobile : $CO_2$/MeOH (0,1 % TEA) = 65/35 ; débit : 1,0 ml/min ; longueur d'onde : 214 nm ; contre-pression : 120 bar ;
composé

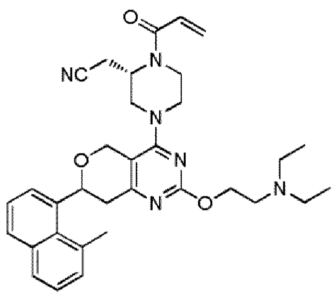

qui a un temps de rétention de 2,67 min dans les conditions suivantes : équipement : Station de méthode SFC (Thar, Waters) ; colonne chromatographique : CHIRALCEL OJ-H 4,6 * 100 mm, 5 um (Daicel) ; température de la colonne : 40 °C ; phase mobile : $CO_2$/MeOH (0,1 % TEA) = 65/35 ; débit : 1,0 ml/min ; longueur d'onde : 214 nm ; contre-pression : 120 bar ;
composé

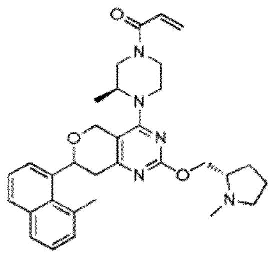

qui a un temps de rétention de 3,26 min dans les conditions suivantes : instrument : Station de méthode SFC (Thar, Waters) ; colonne chromatographique : R,R-WHELK-O1 4,6 * 100 mm, 5 um (REGIS) ; température de la colonne : 40 °C ; phase mobile : CO2/(MeOH/ACN = 3 : 2 (0,1 % TEA)) = 55/45 ; débit : 4,0 ml/min ; longueur d'onde : 254 nm ; contre-pression : 120 bar ;
composé

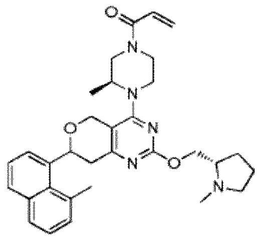

qui a un temps de rétention de 4,16 min dans les conditions suivantes : instrument : Station de méthode SFC (Thar, Waters) ; colonne chromatographique : R,R-WHELK-O1 4,6 * 100 mm, 5 um (REGIS) ; température de la colonne : 40 °C ; phase mobile : CO2/(MeOH/CAN = 3 : 2 (0,1 % TEA)) = 55/45 ; débit : 4,0 ml/min ; longueur d'onde : 254 nm ; contre-pression : 120 bar ;
composé

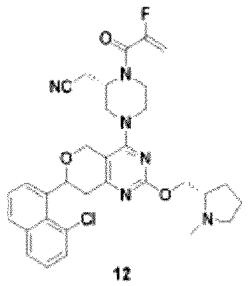

12

qui a un temps de rétention de 1,36 min dans les conditions suivantes : instrument : Station de méthode SFC (Thar, Waters) ; colonne chromatographique : OJ-H 4,6 * 100 mm, 5 um (Daicel) ; température de la colonne : 40 °C ; phase mobile : $CO_2$/MeOH (0,1 % TEA) = 60/40 ; débit : 4,0 ml/min ; longueur d'onde : 254 nm ; contre-pression : 120 bar ;
composé

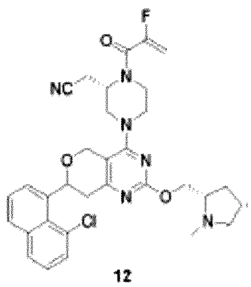

12

qui a un temps de rétention de 2,77 min dans les conditions suivantes : instrument : Station de méthode SFC (Thar, Waters) ; colonne chromatographique : OJ-H 4,6 * 100 mm, 5 um (Daicel) ; température de la colonne : 40

°C ; phase mobile : $CO_2$/MeOH (0,1 % TEA) = 60/40 ; débit : 4,0 ml/min ; longueur d'onde : 254 nm ; contre-pression : 120 bar;
composé

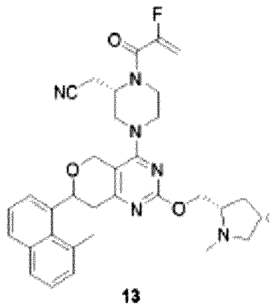

13

qui a un temps de rétention de 1,17 min dans les conditions suivantes : instrument : Station de méthode SFC (Thar, Waters) ; colonne chromatographique : OJ-H 4,6 * 100 mm, 5 um (Daicel) ; température de la colonne : 40 °C ; phase mobile : $CO_2$/MeOH (0,1 % TEA) = 60/40 ; débit : 4,0 ml/min ; longueur d'onde : 254 nm ; contre-pression : 120 bar ;
composé

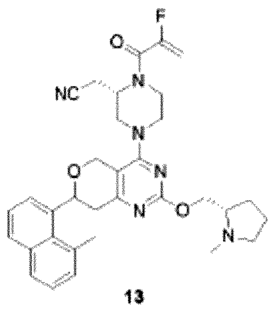

13

qui a un temps de rétention de 2,76 min dans les conditions suivantes : instrument : Station de méthode SFC (Thar, Waters) ; colonne chromatographique : OJ-H 4,6 * 100 mm, 5 μm (Daicel) ; température de la colonne : 40 °C ; phase mobile : $CO_2$/MeOH (0,1 % TEA) = 60/40 ; débit : 4,0 ml/min ; longueur d'onde : 254 nm ; contre-pression : 120 bar ;
composé

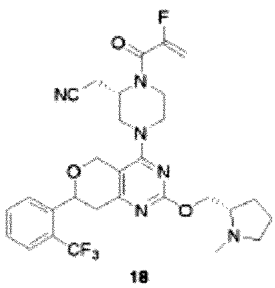

18

qui a un temps de rétention de 0,78 min dans les conditions suivantes : instrument : Station de méthode SFC (Thar, Waters) ; colonne chromatographique : AD-H 4,6 * 100 mm, 5 μm (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

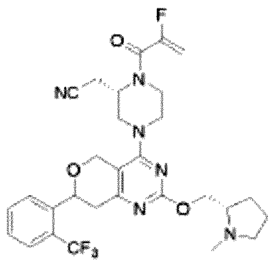

18

qui a un temps de rétention de 2,42 min dans les conditions suivantes : instrument : Station de méthode SFC (Thar, Waters) ; colonne chromatographique : AD-H 4,6 * 100 mm, 5 μm (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

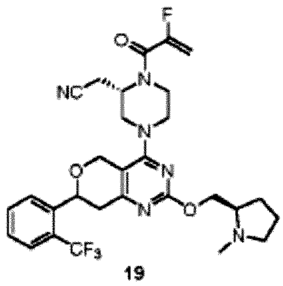

19

qui a un temps de rétention de 0,79 min dans les conditions suivantes : instrument : Station de méthode SFC (Thar, Waters) ; colonne chromatographique : OD-H 4,6 * 100 mm, 5 μm (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

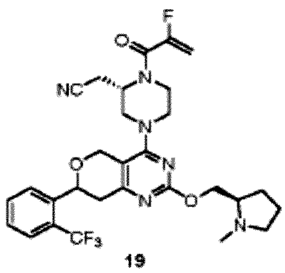

19

qui a un temps de rétention de 2,29 min dans les conditions suivantes : instrument : Station de méthode SFC (Thar, Waters) ; colonne chromatographique : OD-H 4,6 * 100 mm, 5 μm (Daicel) ; température de la colonne : 40 °C ; phase mobile : $CO_2$/MeOH (0,1 % TEA) = 65/35 ; débit : 4,0 ml/min ; longueur d'onde : 254 nm ; contre-pression : 120 bar ;
composé

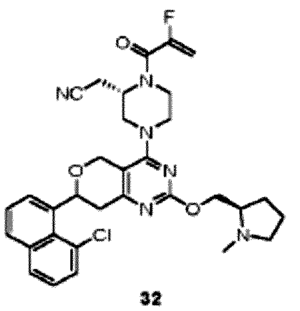

32

qui a un temps de rétention de 1,45 min dans les conditions suivantes : instrument : Station de méthode SFC (Thar, Waters) ; colonne chromatographique : OJ-H 4,6 * 100 mm, 5 μm (Daicel) ; température de la colonne : 40 °C ; phase mobile : $CO_2$/MeOH (0,1 % TEA) = 60/40 ; débit : 4,0 ml/min ;
longueur d'onde : 254 nm ; contre-pression : 120 bar ;
composé

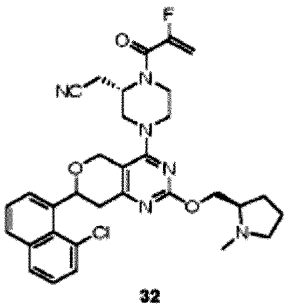

32

qui a un temps de rétention de 2,81 min dans les conditions suivantes : instrument : Station de méthode SFC (Thar, Waters) ; colonne chromatographique : OJ-H 4,6 * 100 mm, 5 μm (Daicel) ; température de la colonne : 40 °C ; phase mobile : $CO_2$/MeOH (0,1 % TEA) = 60/40 ; débit : 4,0 ml/min ; longueur d'onde : 254 nm ; contre-pression : 120 bar.

**15.** Composé hétérocyclique contenant de l'oxygène représenté par la formule I telle que définie dans la revendication 1, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci, dans lequel le composé hétérocyclique contenant de l'oxygène représenté par la formule I est l'un quelconque des composés suivants :

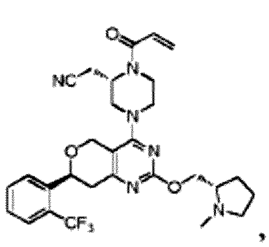 , 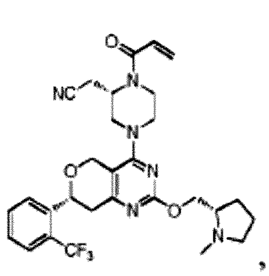 , 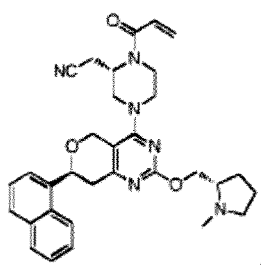 , 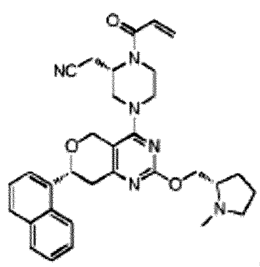 ,

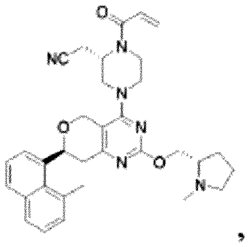 , 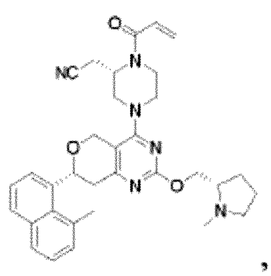 , 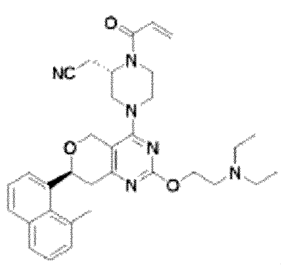 , 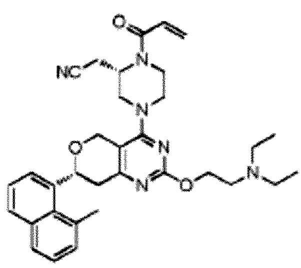 ,

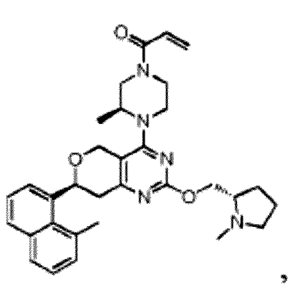, 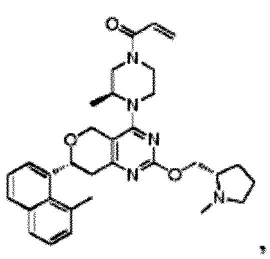, 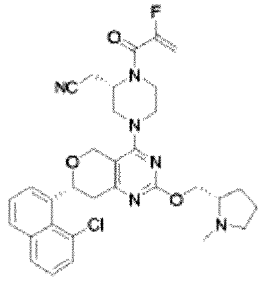, 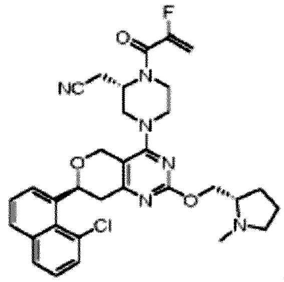,

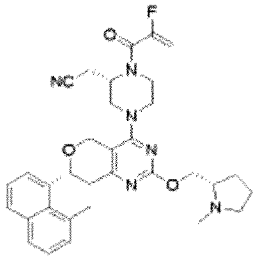, 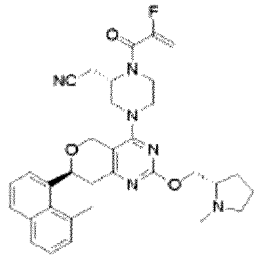, 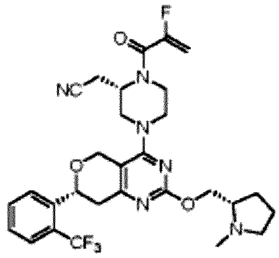, 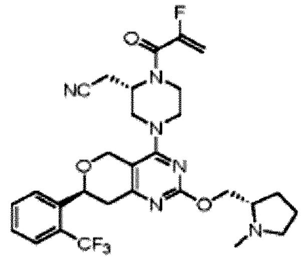,

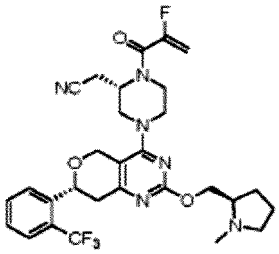, 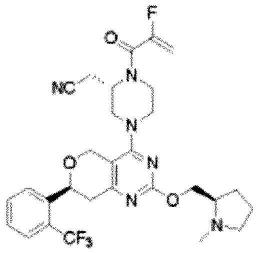, 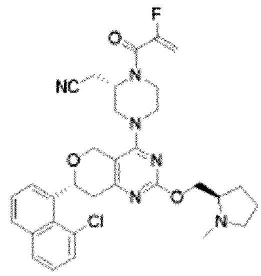, 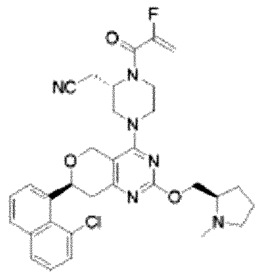.

16. Procédé de préparation du composé hétérocyclique contenant de l'oxygène représenté par la formule I telle que définie dans l'une quelconque des revendications 1 à 15, le procédé comprenant la voie un ou la voie deux :
voie un :

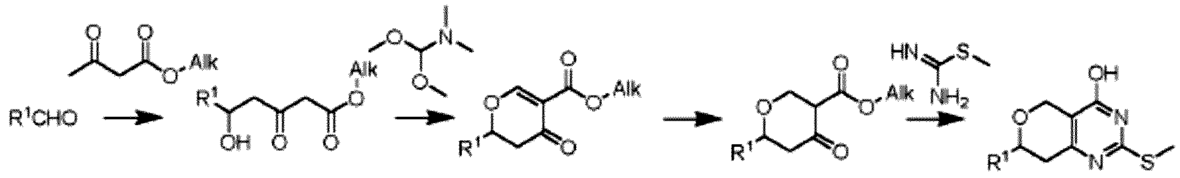

A1 A2 A3 A4 A5

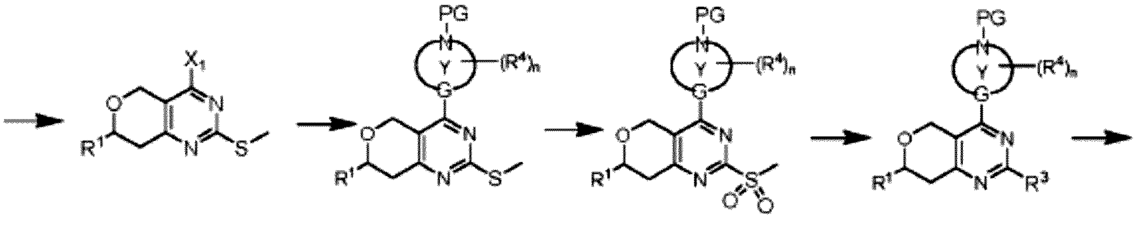

A6 A7 A8 A9

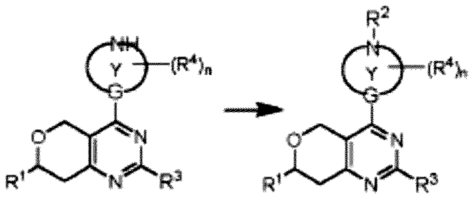

A10 A11

dans lequel $X_1$ est un groupe partant ; Alk est alkyle ; et PG est un groupe amino protecteur ;
voie deux :

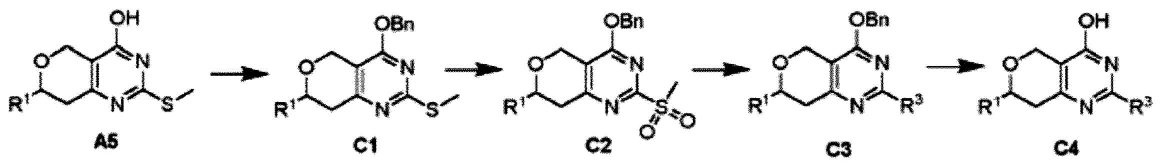

A5 C1 C2 C3 C4

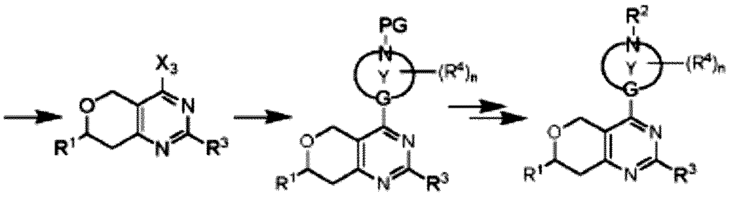

C5 A9 A11

dans lequel $X_3$ est un groupe partant et PG est un groupe amino protecteur ;
le groupe partant est OTf ou Cl ;
le groupe amino protecteur est Boc ou Cbz.

**17.** Composé représenté par la formule A5, A6, A7, A8, A9, A10, C1, C2, C3, C4 ou C5 ;

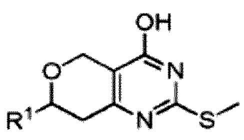
A5

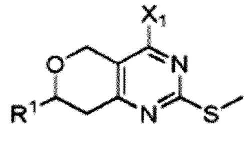
A6

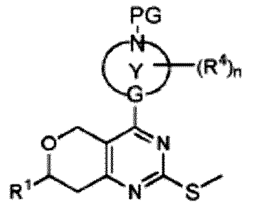
A7

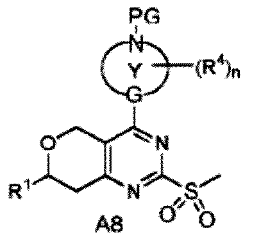
A8

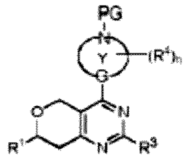
A9

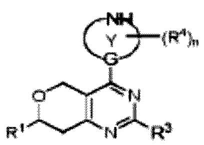
A10

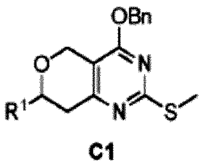
C1

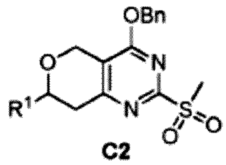
C2

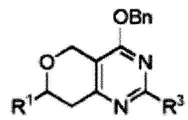
C3

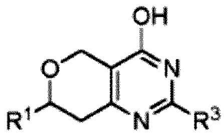
C4
.
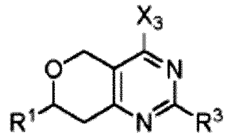
C5
:

dans lequel $R^1$, $R^3$, $R^4$, G, Y et n sont tels que définis dans l'une quelconque des revendications 1 à 15 ;
$X^1$ et $X^3$ sont indépendamment des groupes partants PG est un groupe amino protecteur ;
le groupe partant est OTf ou Cl ;
le groupe amino protecteur est Boc ou Cbz ;
par exemple, le composé représenté par la formule A5, A6, A7, A8, A9, A10, C1, C2, C3, C4 ou C5 est l'un quelconque des composés suivants :

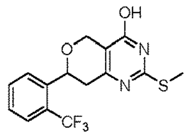
2-f

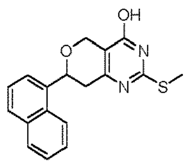
3-f

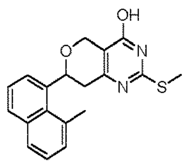
4-f

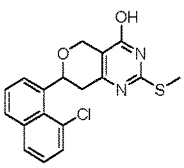
12-f

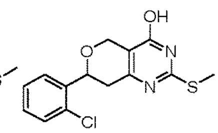
30-f

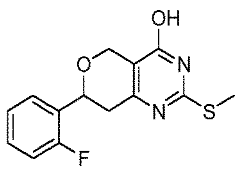
35-f

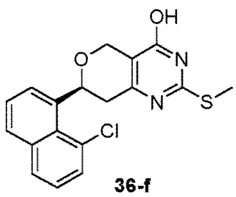
36-f

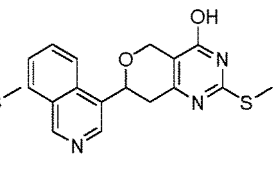
40-f

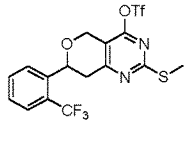
2-e

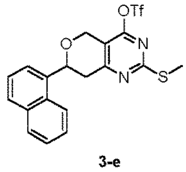
3-e

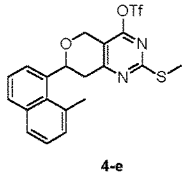
4-e

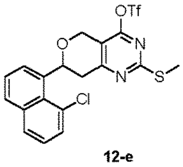
12-e

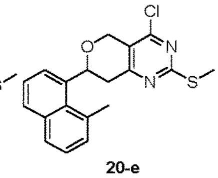
20-e

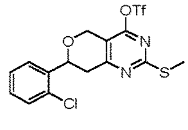
30-e

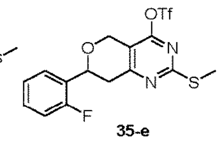
35-e

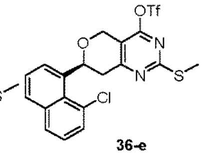
36-e

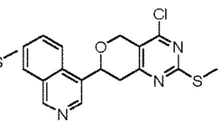
40-e

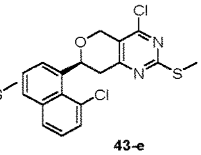
43-e

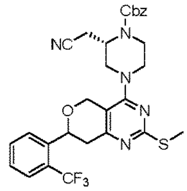
**2-d**

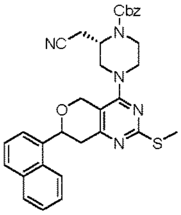
**3-d**

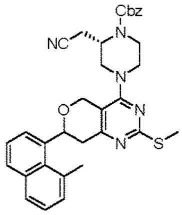
**4-d**

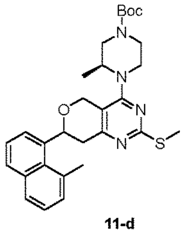
**11-d**

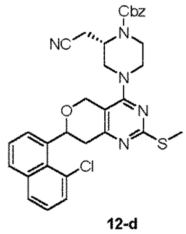
**12-d**

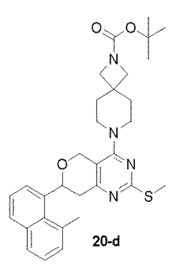
**20-d**

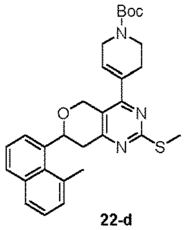
**22-d**

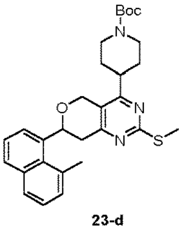
**23-d**

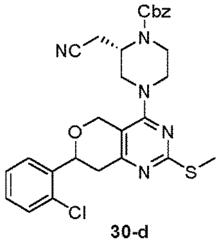
**30-d**

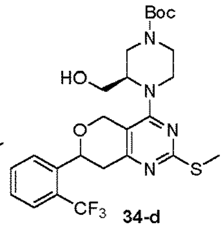
**34-d**

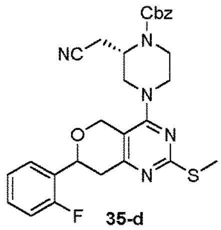
**35-d**

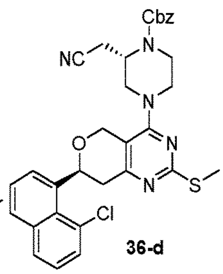
**36-d**

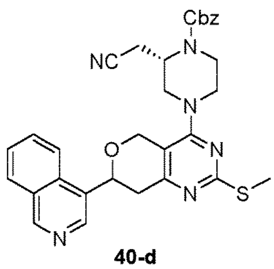
**40-d**

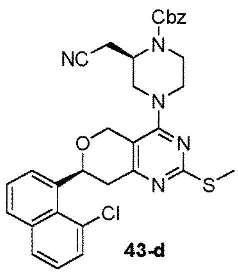
**43-d**

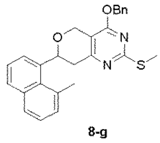
**8-g**

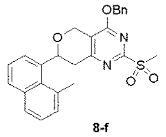
**8-f**

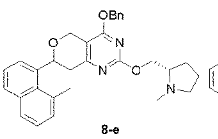
**8-e**

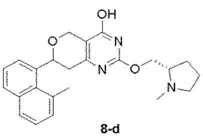
**8-d**

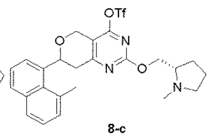
**8-c**

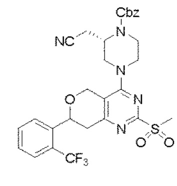
**2-c**

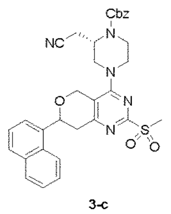
**3-c**

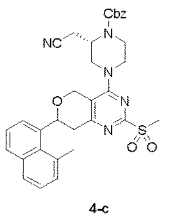
**4-c**

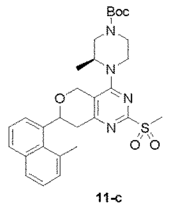
**11-c**

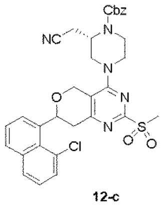
**12-c**

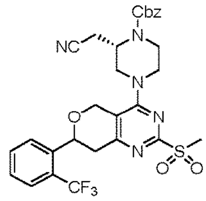
**19-c**

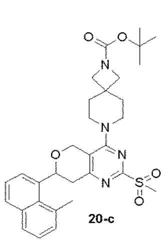
**20-c**

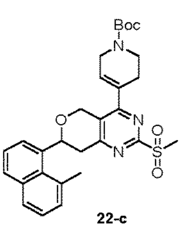
**22-c**

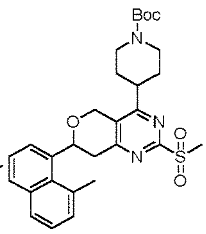
**23-c**

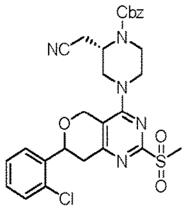
**30-c**

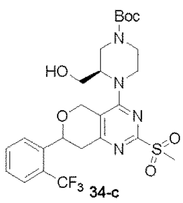
34-c

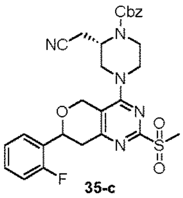
35-c

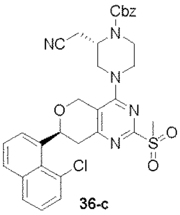
36-c

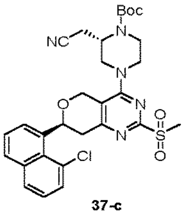
37-c

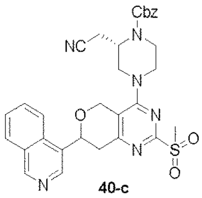
40-c

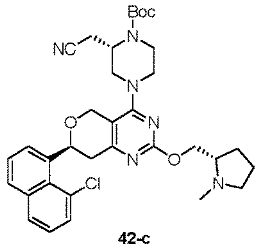
42-c

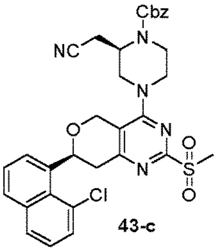
43-c

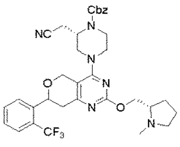
2-b

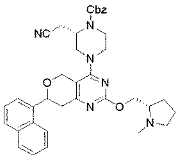
3-b

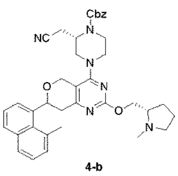
4-b

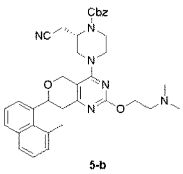
5-b

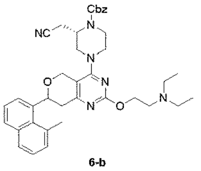
6-b

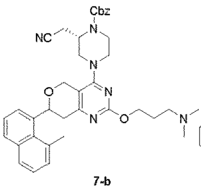
7-b

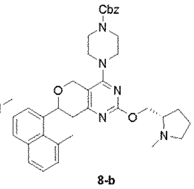
8-b

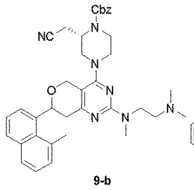
9-b

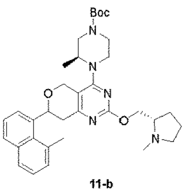
11-b

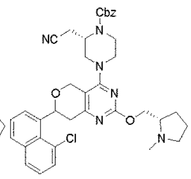
12-b

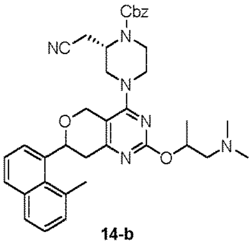
14-b

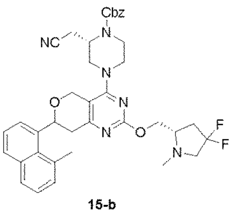
15-b

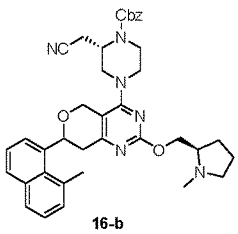
16-b

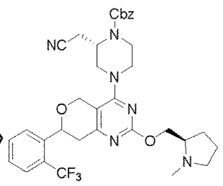
19-b

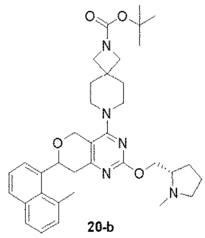
20-b

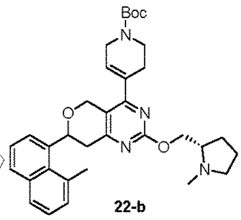
22-b

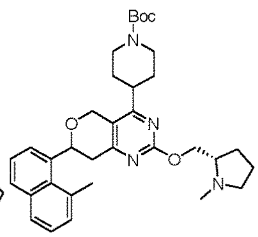
23-b

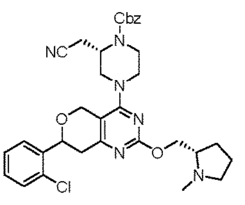
30-b

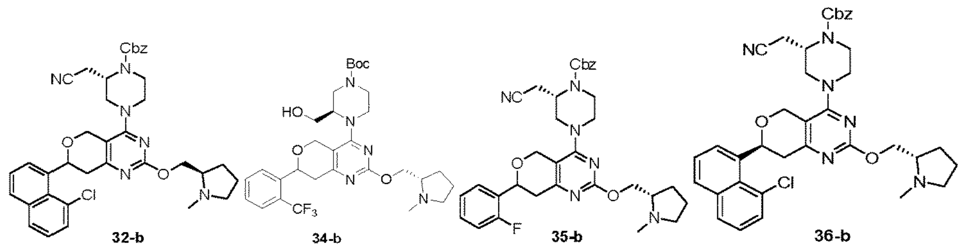

32-b 34-b 35-b 36-b

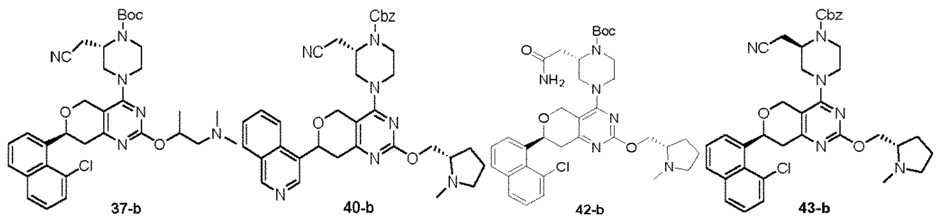

37-b 40-b 42-b 43-b

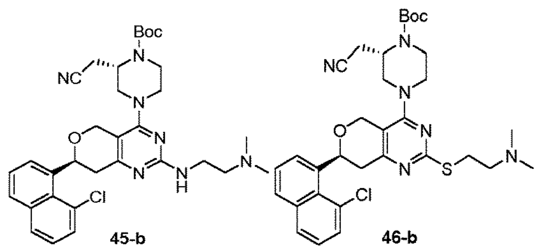

45-b 46-b

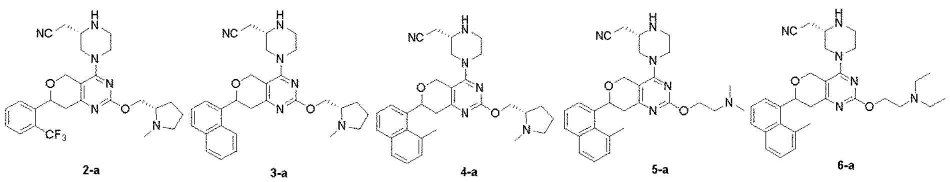

2-a 3-a 4-a 5-a 6-a

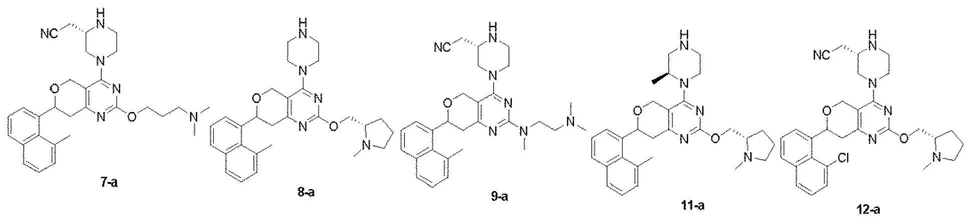

7-a 8-a 9-a 11-a 12-a

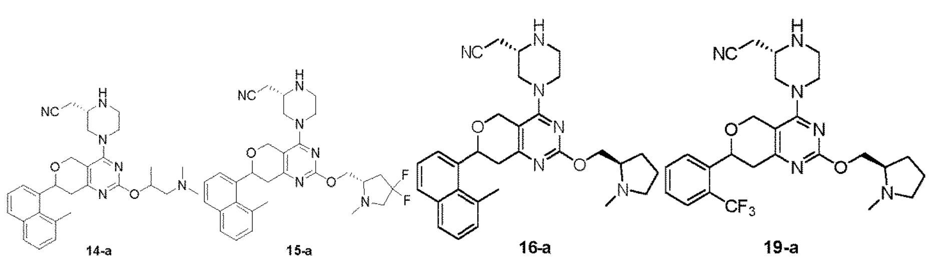

14-a 15-a 16-a 19-a

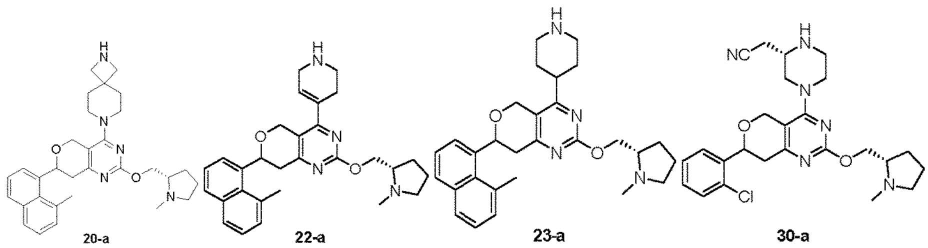

20-a 22-a 23-a 30-a

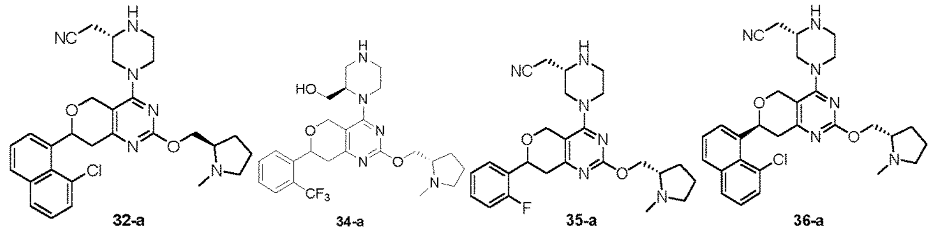

32-a 34-a 35-a 36-a

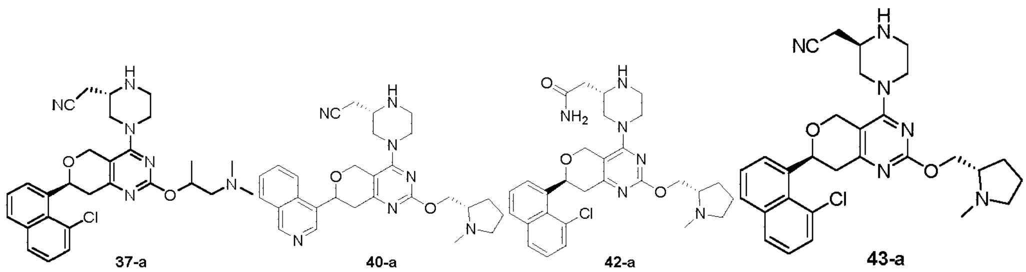

37-a 40-a 42-a 43-a

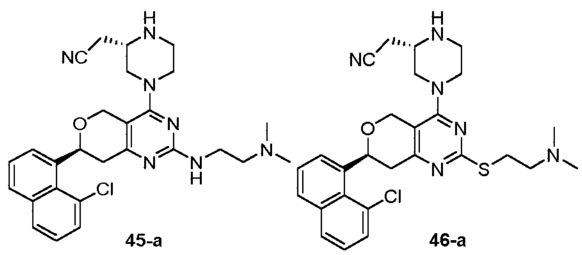

45-a 46-a

**18.** Composition pharmaceutique, comprenant une substance A et un adjuvant pharmaceutique, dans laquelle la substance A est le composé hétérocyclique contenant de l'oxygène représenté par la formule I telle que définie dans l'une quelconque des revendications 1 à 15, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci.

**19.** Substance A destinée à être utilisée comme médicament, dans laquelle la substance A est le composé hétérocyclique contenant de l'oxygène représenté par la formule I telle que définie dans l'une quelconque des revendications 1 à 15, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci.

**20.** Substance A destinée à être utilisée comme médicament, dans laquelle la substance A est le composé hétérocyclique contenant de l'oxygène représenté par la formule I telle que définie dans l'une quelconque des revendications 1 à 15, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci ;
dans le traitement ou la prévention du cancer, dans laquelle le cancer est l'un ou plusieurs parmi le cancer du côlon, le cancer du pancréas, le cancer du sein, le cancer de la prostate, le cancer du poumon, le cancer du cerveau, le cancer de l'ovaire, le cancer du col de l'utérus, le cancer des testicules, le carcinome rénal, le cancer de la tête ou du cou, le cancer des os, le cancer de la peau, le cancer du rectum, le cancer du foie, le cancer du côlon, le cancer de

l'œsophage, le cancer gastrique, le cancer du pancréas, le cancer de la thyroïde, le cancer de la vessie, le lymphome, la leucémie et le mélanome.

**21.** Substance A destinée à être utilisée comme inhibiteur de RAS in *ex vivo,* dans laquelle la substance A est le composé hétérocyclique contenant de l'oxygène représenté par la formule I telle que définie dans l'une quelconque des revendications 1 à 15, un sel pharmaceutiquement acceptable de celui-ci, un solvate de celui-ci, un solvate du sel pharmaceutiquement acceptable de celui-ci, une forme cristalline de celui-ci, un stéréoisomère de celui-ci, un tautomère de celui-ci ou un composé isotopique de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019099524 A1
- CN 109843856 A
- US 20190248767 A1
- WO 2017201161 A1
- WO 2019155399 A1


### Non-patent literature cited in the description


- *Nature*, 2013, vol. 503, 548-551
- *Science*, 2016, vol. 351, 604-608
- **R. LAROCK**. Comprehensive Organic Transformations. VCH Publishers, 1989
- **T. W. GREENE** ; **P. G. M. WUTS**. Protective Groups in Organic Synthesis,. John Wiley and Sons, 1999
- **L. FIESER** ; **M. FIESER**. Fieser and Fieser's Reagents for Organic Synthesis,. John Wiley and Sons, 1994
- Encyclopedia of Reagents for Organic Synthesis. John Wiley and Sons, 1995
- **T. W. GREENE** ; **P. G. M. WUTS**. Protective Groups in Organic Synthesis. John Wiley and Sons, 1999
- **L. FIESER** ; **M. FIESER**. Fieser and Fieser's Reagents for Organic Synthesis. John Wiley and Sons, 1994
- **BERGE et al.** Pharmaceutical Salts. *Journal of Pharmaceutical Science*, 1977, vol. 66, 1-19
- Handbook of Pharmaceutical Salts: Properties, Selection, and Use. Wiley-VCH, 2002
- Pharmacopoeia of the People's Republic of China. 2015
- **RAYMOND C ROWE**. Handbook of Pharmaceutical Excipients. 2009